# EUROPEAN PATENT SPECIFICATION

(11) **EP 3 705 488 B1**
(45) Date of publication and mention of the grant of the patent: **03.08.2022**
(21) Application number: 20171670.1
(22) Date of filing: 29.05.2015
(51) Int. Cl.: C07J 43/00, A61P 25/14, A61K 31/57, A61K 31/58

(54) **NEUROACTIVE STEROIDS, COMPOSITIONS AND USES THEREOF**
NEUROAKTIVE STEROIDE, IHRE ZUSAMMENSETZUNGEN UND VERWENDUNGEN DAVON
STÉROÏDES NEUROACTIFS, LEURS COMPOSITIONS ET UTILISATIONS

(30) Priority: 29.05.2014 WO PCT/CN2014/078820
(43) Date of publication of application: 09.09.2020
(62) Divisional of application: 15799762.8
(73) Proprietor: Sage Therapeutics, Inc., Cambridge, MA 02142 (US)
(72) Inventor: BOTELLA, Gabriel Martinez, Wayland, MA 01778 (US); HARRISON, Boyd L., Princeton Junction, NJ 08550 (US); ROBICHAUD, Albert Jean, Cambridge, MA 02141 (US); SALITURO, Francesco G., Marlborough, MA 01752 (US); BERESIS, Richard Thomas, Shanghai 200040 (CN)
(74) Representative: Coles, Andrea Birgit

(56) References cited:
- WO-A1-2013/056181
- WO-A1-2016/061537
- GABRIEL MARTINEZ BOTELLA ET AL: "Neuroactive Steroids. 1. Positive Allosteric Modulators of the ([gamma]-Aminobutyric Acid) A Receptor: Structure-Activity Relationships of Heterocyclic Substitution at C-21", JOURNAL OF MEDICINAL CHEMISTRY, vol. 58, no. 8, 23 April 2015 (2015-04-23) , pages 3500-3511, XP055239491, US ISSN: 0022-2623, DOI: 10.1021/acs.jmedchem.5b00032

## Description

### Background of the Invention

Brain excitability is defined as the level of arousal of an animal, a continuum that ranges from coma to convulsions, and is regulated by various neurotransmitters. In general, neurotransmitters are responsible for regulating the conductance of ions across neuronal membranes. At rest, the neuronal membrane possesses a potential (or membrane voltage) of approximately -70 mV, the cell interior being negative with respect to the cell exterior. The potential (voltage) is the result of ion (K⁺, Na⁺, Cl⁻, organic anions) balance across the neuronal semipermeable membrane. Neurotransmitters are stored in presynaptic vesicles and are released under the influence of neuronal action potentials. When released into the synaptic cleft, an excitatory chemical transmitter such as acetylcholine will cause membrane depolarization (change of potential from - 70 mV to -50 mV). This effect is mediated by postsynaptic nicotinic receptors which are stimulated by acetylcholine to increase membrane permeability to Na⁺ ions. The reduced membrane potential stimulates neuronal excitability in the form of a postsynaptic action potential.

In the case of the GABA receptor complex (GRC), the effect on brain excitability is mediated by GABA, a neurotransmitter. GABA has a profound influence on overall brain excitability because up to 40% of the neurons in the brain utilize GABA as a neurotransmitter. GABA regulates the excitability of individual neurons by regulating the conductance of chloride ions across the neuronal membrane. GABA interacts with its recognition site on the GRC to facilitate the flow of chloride ions down an electrochemical gradient of the GRC into the cell. An intracellular increase in the levels of this anion causes hyperpolarization of the transmembrane potential, rendering the neuron less susceptible to excitatory inputs (*i.e.,* reduced neuron excitability). In other words, the higher the chloride ion concentration in the neuron, the lower the brain excitability (the level of arousal).

It is well-documented that the GRC is responsible for the mediation of anxiety, seizure activity, and sedation. Thus, GABA and drugs that act like GABA or facilitate the effects of GABA *(e.g.,* the therapeutically useful barbiturates and benzodiazepines (BZs), such as Valium^{®}) produce their therapeutically useful effects by interacting with specific regulatory sites on the GRC.

Accumulated evidence has now indicated that in addition to the benzodiazepine and barbiturate binding site, the GRC contains a distinct site for neuroactive steroids (Lan, N. C. et al., Neurochem. Res. 16:347-356 (1991)).

Neuroactive steroids can occur endogenously. The most potent endogenous neuroactive steroids are 3α-hydroxy-5-reduced pregnan-20-one and 3α-21-dihydroxy-5-reduced pregnan-20-one, metabolites of hormonal steroids progesterone and deoxycorticosterone, respectively. The ability of these steroid metabolites to alter brain excitability was recognized in 1986 (Majewska, M. D. et al., Science 232:1004-1007 (1986); Harrison, N. L. et al., J Pharmacol. Exp. Ther. 241:346-353 (1987)).

The ovarian hormone progesterone and its metabolites have been demonstrated to have profound effects on brain excitability (Backstrom, T. et al., Acta Obstet. Gynecol. Scand. Suppl. 130:19-24 (1985); Pfaff, D.W and McEwen, B. S., Science 219:808-814 (1983); Gyermek et al., J Med Chem. 11: 117 (1968); Lambert, J. et al., Trends Pharmacol. Sci. 8:224-227 (1987)). The levels of progesterone and its metabolites vary with the phases of the menstrual cycle. It has been well documented that the levels of progesterone and its metabolites decrease prior to the onset of menses. The monthly recurrence of certain physical symptoms prior to the onset of menses has also been well documented. These symptoms, which have become associated with premenstrual syndrome (PMS), include stress, anxiety, and migraine headaches (Dalton, K., Premenstrual Syndrome and Progesterone Therapy, 2nd edition, Chicago Yearbook, Chicago (1984)). Subjects with PMS have a monthly recurrence of symptoms that are present in premenses and absent in postmenses.

In a similar fashion, a reduction in progesterone has also been temporally correlated with an increase in seizure frequency in female epileptics, *i.e.,* catamenial epilepsy (Laidlaw, J., Lancet, 1235-1237 (1956)). A more direct correlation has been observed with a reduction in progesterone metabolites (Rosciszewska et al., J. Neurol. Neurosurg. Psych. 49:47-51 (1986)). In addition, for subjects with primary generalized petit mal epilepsy, the temporal incidence of seizures has been correlated with the incidence of the symptoms of premenstrual syndrome (Backstrom, T. et al., J. Psychosom. Obstet. Gynaecol. 2:8-20 (1983)). The steroid deoxycorticosterone has been found to be effective in treating subjects with epileptic spells correlated with their menstrual cycles (Aird, R.B. and Gordan, G., J. Amer. Med. Soc. 145:715-719 (1951)).

A syndrome also related to low progesterone levels is postnatal depression (PND). Immediately after birth, progesterone levels decrease dramatically leading to the onset of PND. The symptoms of PND range from mild depression to psychosis requiring hospitalization. PND is also associated with severe anxiety and irritability. PND-associated depression is not amenable to treatment by classic antidepressants, and women experiencing PND show an increased incidence of PMS (Dalton, K., Premenstrual Syndrome and Progesterone Therapy, 2nd edition, Chicago Yearbook, Chicago (1984)).

Collectively, these observations imply a crucial role for progesterone and deoxycorticosterone and more specifically their metabolites in the homeostatic regulation of brain excitability, which is manifested as an increase in seizure activity or symptoms associated with catamenial epilepsy, PMS, and PND. The correlation between reduced levels of progesterone and the symptoms associated with PMS, PND, and catamenial epilepsy (Backstrom, T. et al., J Psychosom.Obstet. Gynaecol. 2:8-20 (1983)); Dalton, K., Premenstrual Syndrome and Progesterone Therapy, 2nd edition, Chicago Yearbook, Chicago (1984)) has prompted the use of progesterone in their treatment (Mattson et al., "Medroxyprogesterone therapy of catamenial epilepsy," in Advances in Epileptology: XVth Epilepsy International Symposium, Raven Press, New York (1984), pp. 279-282, and Dalton, K., Premenstrual Syndrome and Progesterone Therapy, 2nd edition, Chicago Yearbook, Chicago (1984)). However, progesterone is not consistently effective in the treatment of the aforementioned syndromes. For example, no dose-response relationship exists for progesterone in the treatment of PMS (Maddocks et al., Obstet. Gynecol. 154:573-581 (1986); Dennerstein et al., Brit. Med J 290:16-17 (1986)).

New and improved neuroactive steroids are needed that act as modulating agents for brain excitability, as well as agents for the prevention and treatment of CNS-related diseases. The compounds, compositions, and methods described herein are directed toward this end.

### Summary of the Invention

The claimed invention relates to a compound of Formula (SI-4), 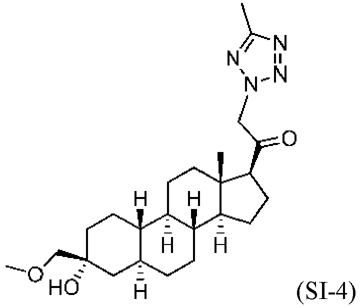 or a pharmaceutically acceptable salt thereof, or a pharmaceutical composition comprising the compound of Formula (SI-4) and a pharmaceutically acceptable excipient, for use in a method for treating essential tremor in a subject.

The compound, the pharmaceutically acceptable salt, or the pharmaceutical composition may be administered orally, subcutaneously, intravenously, or intramuscularly. The compound the pharmaceutically acceptable salt, or the pharmaceutical composition may be administered chronically.

The present invention is based, in part, on the desire to provide novel 19-nor (i.e., C19 desmethyl) compounds, e.g., related to progesterone, deoxycorticosterone, and their metabolites, with good potency, pharmacokinetic (PK) properties, oral bioavailability, formulatability, stability, safety, clearance and/or metabolism. One key feature of the compounds as described herein is disubstitution at the C3 position (e.g., with one substituent being a 3a hydroxy moiety. The inventors envision disubstitution at C-3 will eliminate the potential for oxidation of the hydroxy moiety to the ketone, prevent further metabolism, and reduce the potential for secondary elimination pathways, such as glucuronidation. The inventors further envision the overall effect of C3 disubstitution should be of improving the overall PK parameters and reducing potential toxicities and side effects, which may allow, in certain embodiments, administration orally and/or chronically. Another key feature of the compounds as described herein is the presence of a hydrogen at the C19 position ("19-nor") rather than a methyl group. The inventors envision 19-nor compounds, as compared to their C19-methyl counterparts, will have improved physical properties, such as improved solubility. The inventors envision further enhancement of solubility, for example, when the AB ring system is in the *cis* configuration.

In another aspect, provided is a pharmaceutical composition comprising the compound of the present invention and a pharmaceutically acceptable excipient. In certain embodiments, the compound of the present invention is provided in an effective amount in the pharmaceutical composition. In certain embodiments, the compound of the present invention is provided in a therapeutically effective amount. In certain embodiments, the compound of the present invention is provided in a prophylactically effective amount.

Compounds of the present claimed invention and as described herein, act, in certain embodiments, as GABA modulators, e.g., effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, provided is a compound of the present invention for use in methods of treating a CNS-related disorder in a subject, as set out in the claims. In certain embodiments, the compound is administered orally, subcutaneously, intravenously, or intramuscularly. In certain embodiments, the compound is administered chronically.

Other objects and advantages will become apparent to those skilled in the art from a consideration of the ensuing Detailed Description, Examples, and Claims.

### Definitions

### Chemical Definitions

Definitions of specific functional groups and chemical terms are described in more detail below. The chemical elements are identified in accordance with the Periodic Table of the Elements, CAS version, *Handbook of Chemistry and Physics,* 75^{th} Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

When a range of values is listed, it is intended to encompass each value and sub-range within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The following terms are intended to have the meanings presented therewith below and are useful in understanding the description and intended scope of the present invention. When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein. The articles "a" and "an" may be used herein to refer to one or to more than one (*i.e.* at least one) of the grammatical objects of the article. By way of example "an analogue" means one analogue or more than one analogue.

"Alkyl" refers to a radical of a straight-chain or branched saturated hydrocarbon group having from 1 to 20 carbon atoms ("C₁₋₂₀ alkyl"). In some embodiments, an alkyl group has 1 to 12 carbon atoms ("C₁₋₁₂ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl", also referred to herein as "lower alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently optionally substituted, *i.e.,* unsubstituted (an "unsubstituted alkyl") or substituted (a "substituted alkyl") with one or more substituents; e.g., for instance from 1 to 5 substituents, 1 to 3 substituents, or 1 substituent. In certain embodiments, the alkyl group is unsubstituted C₁₋₁₀ alkyl *(e.g.,* -CH₃). In certain embodiments, the alkyl group is substituted C₁₋₁₀ alkyl. Common alkyl abbreviations include Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

As used herein, "alkylene," "alkenylene," and "alkynylene," refer to a divalent radical of an alkyl, alkenyl, and alkynyl group, respectively. When a range or number of carbons is provided for a particular "alkylene," "alkenylene," and "alkynylene" group, it is understood that the range or number refers to the range or number of carbons in the linear carbon divalent chain. "Alkylene," "alkenylene," and "alkynylene" groups may be substituted or unsubstituted with one or more substituents as described herein.

"Alkylene" refers to an alkyl group wherein two hydrogens are removed to provide a divalent radical, and which may be substituted or unsubstituted. Unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Exemplary substituted alkylene groups, e.g., substituted with one or more alkyl (methyl) groups, include but are not limited to, substituted methylene (-CH(CH₃)-, (-C(CH₃)₂-), substituted ethylene (-CH(CH₃)CH₂-,-CH₂CH(CH₃)-, -C(CH₃)₂CH₂-,-CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-,-CH₂C(CH₃)₂CH₂-, -CH₂CH₂C(CH₃)₂-), and the like.

"Alkenyl" refers to a radical of a straight-chain or branched hydrocarbon group having from 2 to 20 carbon atoms, one or more carbon-carbon double bonds *(e.g.,* 1, 2, 3, or 4 carbon-carbon double bonds), and optionally one or more carbon-carbon triple bonds *(e.g.,* 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents e.g., for instance from 1 to 5 substituents, 1 to 3 substituents, or 1 substituent. In certain embodiments, the alkenyl group is unsubstituted C₂₋₁₀ alkenyl. In certain embodiments, the alkenyl group is substituted C₂₋₁₀ alkenyl.

"Alkenylene" refers to an alkenyl group wherein two hydrogens are removed to provide a divalent radical, and which may be substituted or unsubstituted. Exemplary unsubstituted divalent alkenylene groups include, but are not limited to, ethenylene (-CH=CH-) and propenylene (e.g., - CH=CHCH₂-, -CH₂-CH=CH-). Exemplary substituted alkenylene groups, e.g., substituted with one or more alkyl (methyl) groups, include but are not limited to, substituted ethylene (-C(CH₃)=CH-, -CH=C(CH₃)-), substituted propylene (e.g., -C(CH₃)=CHCH₂-, -CH=C(CH₃)CH₂-,-CH=CHCH(CH₃)-, -CH=CHC(CH₃)₂-, -CH(CH₃)-CH=CH-,-C(CH₃)₂-CH=CH-, -CH₂-C(CH₃)=CH-, -CH₂-CH=C(CH₃)-), and the like.

"Alkynyl" refers to a radical of a straight-chain or branched hydrocarbon group having from 2 to 20 carbon atoms, one or more carbon-carbon triple bonds *(e.g.,* 1, 2, 3, or 4 carbon-carbon triple bonds), and optionally one or more carbon-carbon double bonds *(e.g.,* 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently optionally substituted, *i.e.*, unsubstituted (an "unsubstituted alkynyl") or substituted (a "substituted alkynyl") with one or more substituents; e.g., for instance from 1 to 5 substituents, 1 to 3 substituents, or 1 substituent. In certain embodiments, the alkynyl group is unsubstituted C₂₋₁₀ alkynyl. In certain embodiments, the alkynyl group is substituted C₂₋₁₀ alkynyl.

"Alkynylene" refers to a linear alkynyl group wherein two hydrogens are removed to provide a divalent radical, and which may be substituted or unsubstituted. Exemplary divalent alkynylene groups include, but are not limited to, substituted or unsubstituted ethynylene, substituted or unsubstituted propynylene, and the like.

The term "heteroalkyl," as used herein, refers to an alkyl group, as defined herein, which further comprises 1 or more *(e.g.,* 1, 2, 3, or 4) heteroatoms *(e.g.,* oxygen, sulfur, nitrogen, boron, silicon, phosphorus) within the parent chain, wherein the one or more heteroatoms is inserted between adjacent carbon atoms within the parent carbon chain and/or one or more heteroatoms is inserted between a carbon atom and the parent molecule, *i.e.,* between the point of attachment. In certain embodiments, a heteroalkyl group refers to a saturated group having from 1 to 10 carbon atoms and 1, 2, 3, or 4 heteroatoms ("heteroC₁₋₁₀ alkyl"). In some embodiments, a heteroalkyl group is a saturated group having 1 to 9 carbon atoms and 1, 2, 3, or 4 heteroatoms ("heteroC₁₋₉ alkyl"). In some embodiments, a heteroalkyl group is a saturated group having 1 to 8 carbon atoms and 1, 2, 3, or 4 heteroatoms ("heteroC₁₋₈ alkyl"). In some embodiments, a heteroalkyl group is a saturated group having 1 to 7 carbon atoms and 1, 2, 3, or 4 heteroatoms ("heteroC₁₋₇ alkyl"). In some embodiments, a heteroalkyl group is a group having 1 to 6 carbon atoms and 1, 2, or 3 heteroatoms ("heteroC₁₋₆ alkyl"). In some embodiments, a heteroalkyl group is a saturated group having 1 to 5 carbon atoms and 1 or 2 heteroatoms ("heteroC₁₋₅ alkyl"). In some embodiments, a heteroalkyl group is a saturated group having 1 to 4 carbon atoms and 1 or 2 heteroatoms ("heteroC₁₋₄ alkyl"). In some embodiments, a heteroalkyl group is a saturated group having 1 to 3 carbon atoms and 1 heteroatom ("heteroC₁₋₃ alkyl"). In some embodiments, a heteroalkyl group is a saturated group having 1 to 2 carbon atoms and 1 heteroatom ("heteroC₁₋₂ alkyl"). In some embodiments, a heteroalkyl group is a saturated group having 1 carbon atom and 1 heteroatom ("heteroC₁ alkyl"). In some embodiments, a heteroalkyl group is a saturated group having 2 to 6 carbon atoms and 1 or 2 heteroatoms ("heteroC₂₋₆ alkyl"). Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₁₀ alkyl. In certain embodiments, the heteroalkyl group is a substituted heteroC₁₋₁₀ alkyl.

The term "heteroalkenyl," as used herein, refers to an alkenyl group, as defined herein, which further comprises one or more *(e.g.,* 1, 2, 3, or 4) heteroatoms *(e.g.,* oxygen, sulfur, nitrogen, boron, silicon, phosphorus) wherein the one or more heteroatoms is inserted between adjacent carbon atoms within the parent carbon chain and/or one or more heteroatoms is inserted between a carbon atom and the parent molecule, *i.e.,* between the point of attachment. In certain embodiments, a heteroalkenyl group refers to a group having from 2 to 10 carbon atoms, at least one double bond, and 1, 2, 3, or 4 heteroatoms ("heteroC₂₋₁₀ alkenyl"). In some embodiments, a heteroalkenyl group has 2 to 9 carbon atoms at least one double bond, and 1, 2, 3, or 4 heteroatoms ("heteroC₂₋₉ alkenyl"). In some embodiments, a heteroalkenyl group has 2 to 8 carbon atoms, at least one double bond, and 1, 2, 3, or 4 heteroatoms ("heteroC₂₋₈ alkenyl"). In some embodiments, a heteroalkenyl group has 2 to 7 carbon atoms, at least one double bond, and 1, 2, 3, or 4 heteroatoms ("heteroC₂₋₇ alkenyl"). In some embodiments, a heteroalkenyl group has 2 to 6 carbon atoms, at least one double bond, and 1, 2, or 3 heteroatoms ("heteroC₂₋₆ alkenyl"). In some embodiments, a heteroalkenyl group has 2 to 5 carbon atoms, at least one double bond, and 1 or 2 heteroatoms ("heteroC₂₋₅ alkenyl"). In some embodiments, a heteroalkenyl group has 2 to 4 carbon atoms, at least one double bond, and 1 or 2 heteroatoms ("heteroC₂₋₄ alkenyl"). In some embodiments, a heteroalkenyl group has 2 to 3 carbon atoms, at least one double bond, and 1 heteroatom ("heteroC₂₋₃ alkenyl"). In some embodiments, a heteroalkenyl group has 2 to 6 carbon atoms, at least one double bond, and 1 or 2 heteroatoms ("heteroC₂₋₆ alkenyl"). Unless otherwise specified, each instance of a heteroalkenyl group is independently unsubstituted (an "unsubstituted heteroalkenyl") or substituted (a "substituted heteroalkenyl") with one or more substituents. In certain embodiments, the heteroalkenyl group is an unsubstituted heteroC₂₋₁₀ alkenyl. In certain embodiments, the heteroalkenyl group is a substituted heteroC₂₋₁₀ alkenyl.

The term "heteroalkynyl," as used herein, refers to an alkynyl group, as defined herein, which further comprises one or more *(e.g.,* 1, 2, 3, or 4) heteroatoms *(e.g.,* oxygen, sulfur, nitrogen, boron, silicon, phosphorus) wherein the one or more heteroatoms is inserted between adjacent carbon atoms within the parent carbon chain and/or one or more heteroatoms is inserted between a carbon atom and the parent molecule, *i.e.,* between the point of attachment. In certain embodiments, a heteroalkynyl group refers to a group having from 2 to 10 carbon atoms, at least one triple bond, and 1, 2, 3, or 4 heteroatoms ("heteroC₂₋₁₀ alkynyl"). In some embodiments, a heteroalkynyl group has 2 to 9 carbon atoms, at least one triple bond, and 1, 2, 3, or 4 heteroatoms ("heteroC₂₋₉ alkynyl"). In some embodiments, a heteroalkynyl group has 2 to 8 carbon atoms, at least one triple bond, and 1, 2, 3, or 4 heteroatoms ("heteroC₂₋₈ alkynyl"). In some embodiments, a heteroalkynyl group has 2 to 7 carbon atoms, at least one triple bond, and 1, 2, 3, or 4 heteroatoms ("heteroC₂₋₇ alkynyl"). In some embodiments, a heteroalkynyl group has 2 to 6 carbon atoms, at least one triple bond, and 1, 2, or 3 heteroatoms ("heteroC₂₋₆ alkynyl"). In some embodiments, a heteroalkynyl group has 2 to 5 carbon atoms, at least one triple bond, and 1 or 2 heteroatoms ("heteroC₂₋₅ alkynyl"). In some embodiments, a heteroalkynyl group has 2 to 4 carbon atoms, at least one triple bond, and 1 or 2 heteroatoms ("heteroC₂₋₄ alkynyl"). In some embodiments, a heteroalkynyl group has 2 to 3 carbon atoms, at least one triple bond, and 1 heteroatom ("heteroC₂₋₃ alkynyl"). In some embodiments, a heteroalkynyl group has 2 to 6 carbon atoms, at least one triple bond, and 1 or 2 heteroatoms ("heteroC₂₋₆ alkynyl"). Unless otherwise specified, each instance of a heteroalkynyl group is independently unsubstituted (an "unsubstituted heteroalkynyl") or substituted (a "substituted heteroalkynyl") with one or more substituents. In certain embodiments, the heteroalkynyl group is an unsubstituted heteroC₂₋₁₀ alkynyl. In certain embodiments, the heteroalkynyl group is a substituted heteroC₂₋₁₀ alkynyl.

As used herein, "alkylene," "alkenylene," "alkynylene," "heteroalkylene," "heteroalkenylene," and "heteroalkynylene," refer to a divalent radical of an alkyl, alkenyl, alkynyl group, heteroalkyl, heteroalkenyl, and heteroalkynyl group respectively. When a range or number of carbons is provided for a particular "alkylene," "alkenylene," "alkynylene," "heteroalkylene," "heteroalkenylene," or "heteroalkynylene," group, it is understood that the range or number refers to the range or number of carbons in the linear carbon divalent chain. "Alkylene," "alkenylene," "alkynylene," "heteroalkylene," "heteroalkenylene," and "heteroalkynylene" groups may be substituted or unsubstituted with one or more substituents as described herein.

"Aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

In certain embodiments, an aryl group substituted with one or more of groups selected from halo, C₁-C₈ alkyl, C₁-C₈ haloalkyl, cyano, hydroxy, C₁-C₈ alkoxy, and amino.

Examples of representative substituted aryls include the following 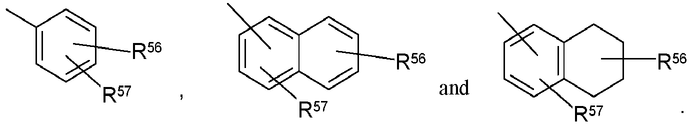 wherein one of R⁵⁶ and R⁵⁷ may be hydrogen and at least one of R⁵⁶ and R⁵⁷ is each independently selected from C₁-C₈ alkyl, C₁-C₈ haloalkyl, 4-10 membered heterocyclyl, alkanoyl, C₁-C₈ alkoxy, heteroaryloxy, alkylamino, arylamino, heteroarylamino, NR⁵⁸COR⁵⁹, NR⁵⁸SOR⁵⁹ NR⁵⁸SO₂R⁵⁹, COOalkyl, COOaryl, CONR⁵⁸R⁵⁹, CONR⁵⁸OR⁵⁹, NR⁵⁸R⁵⁹, SO₂NR⁵⁸R⁵⁹, S-alkyl, SOalkyl, SO₂alkyl, Saryl, SOaryl, SO₂aryl; or R⁵⁶ and R⁵⁷ may be joined to form a cyclic ring (saturated or unsaturated) from 5 to 8 atoms, optionally containing one or more heteroatoms selected from the group N, O, or S. R⁶⁰ and R⁶¹ are independently hydrogen, C₁-C₈ alkyl, C₁-C₄ haloalkyl, C₃-C₁₀ cycloalkyl, 4-10 membered heterocyclyl, C₆-C₁₀ aryl, substituted C₆-C₁₀ aryl, 5-10 membered heteroaryl, or substituted 5-10 membered heteroaryl .

Other representative aryl groups having a fused heterocyclyl group include the following: 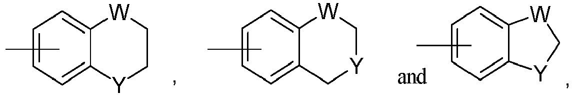 wherein each W is selected from C(R⁶⁶)₂, NR⁶⁶, O, and S; and each Y is selected from carbonyl, NR⁶⁶, O and S; and R⁶⁶ is independently hydrogen, C₁-C₈ alkyl, C₃-C₁₀ cycloalkyl, 4-10 membered heterocyclyl, C₆-C₁₀ aryl, and 5-10 membered heteroaryl.

"Fused aryl" refers to an aryl having two of its ring carbon in common with a second aryl or heteroaryl ring or with a carbocyclyl or heterocyclyl ring.

"Aralkyl" is a subset of alkyl and aryl, as defined herein, and refers to an optionally substituted alkyl group substituted by an optionally substituted aryl group.

"Heteroaryl" refers to a radical of a 5-10 membered monocyclic or bicyclic 4n+2 aromatic ring system (e.g., having 6 or 10 π electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, *i.e.,* either the ring bearing a heteroatom *(e.g.,* 2-indolyl) or the ring that does not contain a heteroatom (*e.g.*, 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 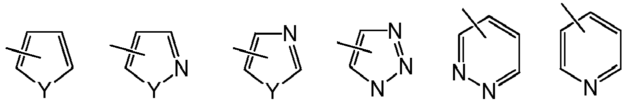 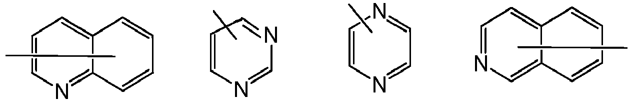 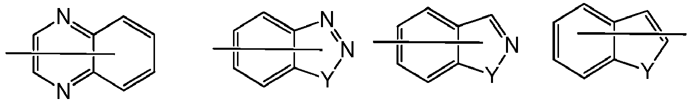 wherein each Y is selected from carbonyl, N, NR⁶⁵, O, and S; and R⁶⁵ is independently hydrogen, C₁-C₈ alkyl, C₃-C₁₀ cycloalkyl, 4-10 membered heterocyclyl, C₆-C₁₀ aryl, and 5-10 membered heteroaryl.

"Heteroaralkyl" is a subset of alkyl and heteroaryl, as defined herein, and refers to an optionally substituted alkyl group substituted by an optionally substituted heteroaryl group.

"Carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently optionally substituted, *i.e.*, unsubstituted (an "unsubstituted carbocyclyl") or substituted (a "substituted carbocyclyl") with one or more substituents. In certain embodiments, the carbocyclyl group is unsubstituted C₃₋₁₀ carbocyclyl. In certain embodiments, the carbocyclyl group is a substituted C₃₋₁₀ carbocyclyl.

In some embodiments, "carbocyclyl" is a monocyclic, saturated carbocyclyl group having from 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 5 to 6 ring carbon atoms ("C₅₋₆ cycloalkyl"). In some embodiments, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (C₅). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted C₃₋₁₀ cycloalkyl. In certain embodiments, the cycloalkyl group is substituted C₃₋₁₀ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

Particular examples of heterocyclyl groups are shown in the following illustrative examples: 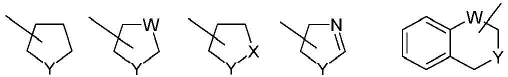 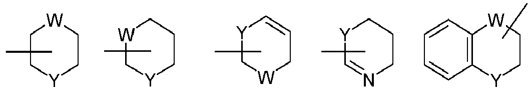 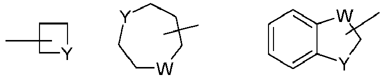 wherein each W is selected from CR⁶⁷, C(R⁶⁷)₂, NR⁶⁷, O, and S; and each Y is selected from NR⁶⁷, O, and S; and R⁶⁷ is independently hydrogen, C₁-C₈ alkyl, C₃-C₁₀ cycloalkyl, 4-10 membered heterocyclyl, C₆-C₁₀ aryl, 5-10 membered heteroaryl. These heterocyclyl rings may be optionally substituted with one or more groups selected from the group consisting of acyl, acylamino, acyloxy, alkoxy, alkoxycarbonyl, alkoxycarbonylamino, amino, substituted amino, aminocarbonyl (carbamoyl or amido), aminocarbonylamino, aminosulfonyl, sulfonylamino, aryl, aryloxy, azido, carboxyl, cyano, cycloalkyl, halogen, hydroxy, keto, nitro, thiol, -S-alkyl, -S-aryl, -S(O)-alkyl,-S(O)-aryl, -S(O)₂-alkyl, and -S(O)₂-aryl. Substituting groups include carbonyl or thiocarbonyl which provide, for example, lactam and urea derivatives.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.,* heteroalkyl, cycloalkyl, *e.g.,* heterocyclyl, aryl, *e.g,.* heteroaryl, cycloalkenyl, *e.g*,. cycloheteroalkenyl, and the like having from 1 to 5, and particularly from 1 to 3 heteroatoms.

"Acyl" refers to a radical -C(O)R²⁰, where R²⁰ is hydrogen, substituted or unsubstitued alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstitued heteroaryl, as defined herein. "Alkanoyl" is an acyl group wherein R²⁰ is a group other than hydrogen. Representative acyl groups include, but are not limited to, formyl (-CHO), acetyl (-C(=O)CH₃), cyclohexylcarbonyl, cyclohexylmethylcarbonyl, benzoyl (-C(=O)Ph), benzylcarbonyl (-C(=O)CH₂Ph), -C(O)-C₁-C₈ alkyl, -C(O)-(CH₂)ₜ(C₆-C₁₀ aryl), - C(O)-(CH₂)ₜ(5-10 membered heteroaryl), -C(O)-(CH₂)ₜ(C₃-C₁₀ cycloalkyl), and -C(O)-(CH₂)ₜ(4-10 membered heterocyclyl), wherein t is an integer from 0 to 4. In certain embodiments, R²¹ is C₁-C₈ alkyl, substituted with halo or hydroxy; or C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy.

"Acylamino" refers to a radical -NR²C(O)R²³, where each instance of R²² and R²³ is independently hydrogen, substituted or unsubstitued alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstitued heteroaryl,, as defined herein, or R²² is an amino protecting group. Exemplary "acylamino" groups include, but are not limited to, formylamino, acetylamino, cyclohexylcarbonylamino, cyclohexylmethyl-carbonylamino, benzoylamino and benzylcarbonylamino. Particular exemplary "acylamino" groups are -NR²⁴C(O)-C₁-C₈ alkyl, -NR²⁴C(O)-(CH₂)ₜ(C₆-C₁₀ aryl), -NR²⁴C(O)-(CH₂)ₜ(5-10 membered heteroaryl), -NR²⁴C(O)-(CH₂)ₜ(C₃-C₁₀ cycloalkyl), and -NR²⁴C(O)-(CH₂)ₜ(4-10 membered heterocyclyl), wherein t is an integer from 0 to 4, and each R²⁴ independently represents H or C₁-C₈ alkyl.In certain embodiments, R²⁵ is H, C₁-C₈ alkyl, substituted with halo or hydroxy; C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy; and R²⁶ is H, C₁-C₈ alkyl, substituted with halo or hydroxy; C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxyl; provided at least one of R²⁵ and R²⁶ is other than H.

"Acyloxy" refers to a radical -OC(O)R²⁷, where R²⁷ is hydrogen, substituted or unsubstitued alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstitued heteroaryl, as defined herein. Representative examples include, but are not limited to, formyl, acetyl, cyclohexylcarbonyl, cyclohexylmethylcarbonyl, benzoyl and benzylcarbonyl. In certain embodiments, R²⁸ is C₁-C₈ alkyl, substituted with halo or hydroxy; C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy.

"Alkoxy" refers to the group -OR²⁹ where R²⁹ is substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstitued heteroaryl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

In certain embodiments, R²⁹ is a group that has 1 or more substituents, for instance from 1 to 5 substituents, and particularly from 1 to 3 substituents, in particular 1 substituent, selected from the group consisting of amino, substituted amino, C₆-C₁₀ aryl, aryloxy, carboxyl, cyano, C₃-C₁₀ cycloalkyl, 4-10 membered heterocyclyl, halogen, 5-10 membered heteroaryl, hydroxyl, nitro, thioalkoxy, thioaryloxy, thiol, alkyl-S(O)-, aryl-S(O)-, alkyl-S(O)₂- and aryl-S(O)₂-. Exemplary 'substituted alkoxy' groups include, but are not limited to, -O-(CH₂)ₜ(C₆-C₁₀ aryl), -O-(CH₂)ₜ(5-10 membered heteroaryl), -O-(CH₂)ₜ(C₃-C₁₀ cycloalkyl), and -O-(CH₂)ₜ(4-10 membered heterocyclyl), wherein t is an integer from 0 to 4 and any aryl, heteroaryl, cycloalkyl or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy. Particular exemplary 'substituted alkoxy' groups are - OCF₃, -OCH₂CF₃, -OCH₂Ph, -OCH₂-cyclopropyl, -OCH₂CH₂OH, and -OCH₂CH₂NMe₂.

"Amino" refers to the radical -NH₂.

"Substituted amino" refers to an amino group of the formula -N(R³⁸)₂ wherein R³⁸ is hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of R³⁸ is not a hydrogen. In certain embodiments, each R³⁸ is independently selected from hydrogen, C₁-C₈ alkyl, C₃-C₈ alkenyl, C₃-C₈ alkynyl, C₆-C₁₀ aryl, 5-10 membered heteroaryl, 4-10 membered heterocyclyl, or C₃-C₁₀ cycloalkyl; or C₁-C₈ alkyl, substituted with halo or hydroxy; C₃-C₈ alkenyl, substituted with halo or hydroxy; C₃-C₈ alkynyl, substituted with halo or hydroxy, or -(CH₂)ₜ(C₆-C₁₀ aryl), -(CH₂)ₜ(5-10 membered heteroaryl), - (CH₂)ₜ(C₃-C₁₀ cycloalkyl), or -(CH₂)ₜ(4-10 membered heterocyclyl), wherein t is an integer between 0 and 8, each of which is substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy; or both R³⁸ groups are joined to form an alkylene group.

Exemplary "substituted amino" groups include, but are not limited to, -NR³⁹-C₁-C₈ alkyl, -NR³⁹-(CH₂)ₜ(C₆-C₁₀ aryl), -NR³⁹-(CH₂)ₜ(5-10 membered heteroaryl), -NR³⁹-(CH₂)ₜ(C₃-C₁₀ cycloalkyl), and -NR³⁹-(CH₂)ₜ(4-10 membered heterocyclyl), wherein t is an integer from 0 to 4, for instance 1 or 2, each R³⁹ independently represents H or C₁-C₈ alkyl; and any alkyl groups present, may themselves be substituted by halo, substituted or unsubstituted amino, or hydroxy; and any aryl, heteroaryl, cycloalkyl, or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy. For the avoidance of doubt the term 'substituted amino' includes the groups alkylamino, substituted alkylamino, alkylarylamino, substituted alkylarylamino, arylamino, substituted arylamino, dialkylamino, and substituted dialkylamino as defined below. Substituted amino encompasses both monosubstituted amino and disubstituted amino groups.

"Azido" refers to the radical -N₃.

"Carbamoyl" or "amido" refers to the radical -C(O)NH₂.

"Substituted carbamoyl" or "substituted amido" refers to the radical -C(O)N(R⁶²)₂ wherein each R⁶² is independently hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of R⁶² is not a hydrogen. In certain embodiments, R⁶² is selected from H, C₁-C₈ alkyl, C₃-C₁₀ cycloalkyl, 4-10 membered heterocyclyl, C₆-C₁₀ aryl, aralkyl, 5-10 membered heteroaryl, and heteroaralkyl; or C₁-C₈ alkyl substituted with halo or hydroxy; or C₃-C₁₀ cycloalkyl, 4-10 membered heterocyclyl, C₆-C₁₀ aryl, aralkyl, 5-10 membered heteroaryl, or heteroaralkyl, each of which is substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy; provided that at least one R⁶² is other than H.

Exemplary "substituted carbamoyl" groups include, but are not limited to, -C(O) NR⁶⁴-C₁-C₈ alkyl, -C(O)NR⁶⁴-(CH₂)ₜ(C₆-C₁₀ aryl), -C(O)N⁶⁴-(CH₂)ₜ(5-10 membered heteroaryl), -C(O)NR⁶⁴-(CH₂)ₜ(C₃-C₁₀ cycloalkyl), and -C(O)NR⁶⁴-(CH₂)ₜ(4-10 membered heterocyclyl), wherein t is an integer from 0 to 4, each R⁶⁴ independently represents H or C₁-C₈ alkyl and any aryl, heteroaryl, cycloalkyl or heterocyclyl groups present, may themselves be substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy.

"Carboxy" refers to the radical -C(O)OH.

"Cyano" refers to the radical -CN.

"Halo" or "halogen" refers to fluoro (F), chloro (Cl), bromo (Br), and iodo (I). In certain embodiments, the halo group is either fluoro or chloro.

"Hydroxy" refers to the radical -OH.

"Nitro" refers to the radical -NO₂.

"Cycloalkylalkyl" refers to an alkyl radical in which the alkyl group is substituted with a cycloalkyl group. Typical cycloalkylalkyl groups include, but are not limited to, cyclopropylmethyl, cyclobutylmethyl, cyclopentylmethyl, cyclohexylmethyl, cycloheptylmethyl, cyclooctylmethyl, cyclopropylethyl, cyclobutylethyl, cyclopentylethyl, cyclohexylethyl, cycloheptylethyl, and cyclooctylethyl, and the like.

"Heterocyclylalkyl" refers to an alkyl radical in which the alkyl group is substituted with a heterocyclyl group. Typical heterocyclylalkyl groups include, but are not limited to, pyrrolidinylmethyl, piperidinylmethyl, piperazinylmethyl, morpholinylmethyl, pyrrolidinylethyl, piperidinylethyl, piperazinylethyl, morpholinylethyl, and the like.

"Cycloalkenyl" refers to substituted or unsubstituted carbocyclyl group having from 3 to 10 carbon atoms and having a single cyclic ring or multiple condensed rings, including fused and bridged ring systems and having at least one and particularly from 1 to 2 sites of olefinic unsaturation. Such cycloalkenyl groups include, by way of example, single ring structures such as cyclohexenyl, cyclopentenyl, cyclopropenyl, and the like.

"Fused cycloalkenyl" refers to a cycloalkenyl having two of its ring carbon atoms in common with a second aliphatic or aromatic ring and having its olefinic unsaturation located to impart aromaticity to the cycloalkenyl ring.

"Ethylene" refers to substituted or unsubstituted -(C-C)-.

"Ethenyl" refers to substituted or unsubstituted -(C=C)-.

"Ethynyl" refers to - (C≡C)-.

"Nitrogen-containing heterocyclyl" group means a 4- to 7- membered non-aromatic cyclic group containing at least one nitrogen atom, for example, but without limitation, morpholine, piperidine *(e.g.* 2-piperidinyl, 3-piperidinyl and 4-piperidinyl), pyrrolidine *(e.g.* 2-pyrrolidinyl and 3-pyrrolidinyl), azetidine, pyrrolidone, imidazoline, imidazolidinone, 2-pyrazoline, pyrazolidine, piperazine, and N-alkyl piperazines such as N-methyl piperazine. Particular examples include azetidine, piperidone and piperazone.

"Thioketo" refers to the group =S.

Alkyl, alkenyl, alkynyl, carbocyclyl, heterocyclyl, aryl, and heteroaryl groups, as defined herein, are optionally substituted (e.g., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group *(e.g.,* a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, -NO₂, -N₃,-SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa},-SSR^{cc}, -C(=O)R^{aa}, -CO₂H, -CHO, -C(OR^{cc})₂, -CO₂R^{aa}, -OC(=O)R^{aa}, -OCO₂R^{aa},-C(=O)N(R^{bb})₂, -OC(=O)N(R^{bb})_{2,} -NR^{bb}C(=O)R^{aa}, -NR^{bb}CO₂R^{aa}, -NR^{bb}C(=O)N(R^{bb})₂,-C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂,-OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂,-SO₂R^{aa}, -SO₂OR^{aa}, -OSO₂R^{aa}, -S(O)R^{aa}, *e*.*g*.,-S(=O)R^{aa}, -OS(=O)R^{aa}, -Si(R^{aa})₃, -OSi(R^{aa} )₃-C(=S)N(R^{bb})₂, -C(=O)SR^{aa}, -C(=S)SR^{aa}, -SC(=S)SR^{aa}, -SC(=O)SR^{aa}, -OC(=O)SR^{aa},-SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂,-OP(=O)(OR^{cc})₂, -P(=O)₂N(R^{bb})₂, -OP(=O)₂N(R^{bb})₂, -P(=O)(NR^{bb})₂, -OP(=O)(NRb^{b})₂,-NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂,-B(OR^{cc})₂, -BR^{aa}(OR^{cc}), C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, -N(R^{cc})₂,-CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂,-SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc},-P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, -SO₂H,-SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee},-C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂,-NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee},-OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(O)R^{ee}, *e*.*g*.,-S(=O)R^{ee},-Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, C₁₋₆ alkyl, C₁₋₆ perhaloalkyl, C₂₋₆ alkenyl, C₂₋₆ alkynyl, C₃₋₁₀ carbocyclyl, 3-10 membered heterocyclyl, C₆₋₁₀ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S;
each instance of R^{ee} is, independently, selected from C₁₋₆ alkyl, C₁₋₆ perhaloalkyl, C₂₋₆ alkenyl, C₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ perhaloalkyl, C₂₋₆ alkenyl, C₂₋₆ alkynyl, C₃₋₁₀ carbocyclyl, 3-10 membered heterocyclyl, C₆₋₁₀ aryl and 5-10 membered heteroaryl, or two R^{ff} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH,-OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl),-NH(OH), -SH, -SC₁₋₆ alkyl, -SS(C₁₋₆, alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂,-OC(=O)NH(C₁₋₆ alkyl), -NHC(=O)(C₁₋₆ alkyl), -N(C₁₋₆ alkyl)C(=O)( C₁₋₆ alkyl),-NHCO₂(C₁₋₆ alkyl), -NHC(=O)N(C₁₋₆ alkyl)₂, -NHC(=O)NH(C₁₋₆ alkyl),-NHC(=O)NH₂, -C(=NH)O(C₁₋₆ alkyl),-OC(=NH)(C₁₋₆ alkyl), -OC(=NH)OC₁₋₆ alkyl,-C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂,-NHC(=NH)NH₂, -NHSO₂(C₁₋₆ alkyl), -SO₂N(C₁₋₆ alkyl)₂, -SO₂NH(C₁₋₆ alkyl),-SO₂NH₂,-SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂,-C(=O)S(C₁₋₆ alkyl), -C(=S)SC₁₋₆ alkyl, -SC(=S)SC₁₋₆ alkyl, -P(=O)₂(C₁₋₆ alkyl),-P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

A "counterion" or "anionic counterion" is a negatively charged group associated with a cationic quaternary amino group in order to maintain electronic neutrality. Exemplary counterions include halide ions (*e.g.*, F⁻, Cl⁻, Br⁻, I⁻), NO₃⁻, ClO₄⁻, OH⁻, H₂PO₄⁻, HSO₄⁻, SO₄⁻²sulfonate ions *(e.g.,* methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions (e.g., acetate, ethanoate, propanoate, benzoate, glycerate, lactate, tartrate, glycolate, and the like).

Nitrogen atoms can be substituted or unsubstituted as valency permits, and include primary, secondary, tertiary, and quarternary nitrogen atoms. Exemplary nitrogen atom substituents include, but are not limited to, hydrogen, -OH, -OR^{aa}, -N(R^{cc})₂, -CN, -C(=O)R^{aa},-C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂,-SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups attached to a nitrogen atom are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined above.

These and other exemplary substituents are described in more detail in the Detailed Description, Examples, and claims.

### Other definitions

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describes pharmaceutically acceptable salts in detail in J. Pharmaceutical Sciences (1977) 66:1-19. Pharmaceutically acceptable salts of the compounds of the present invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (*i.e.*, a male or female of any age group, *e.g.,* a pediatric subject *(e.g,* infant, child, adolescent) or adult subject *(e.g.,* young adult, middle-aged adult or senior adult)) and/or a non-human animal, *e.g.,* a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

In general, the "effective amount" of a compound refers to an amount sufficient to elicit the desired biological response. As will be appreciated by those of ordinary skill in this art, the effective amount of a compound of the invention may vary depending on such factors as the desired biological endpoint, the pharmacokinetics of the compound, the disease being treated, the mode of administration, and the age, health, and condition of the subject. An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

### Brief Description of the Drawings

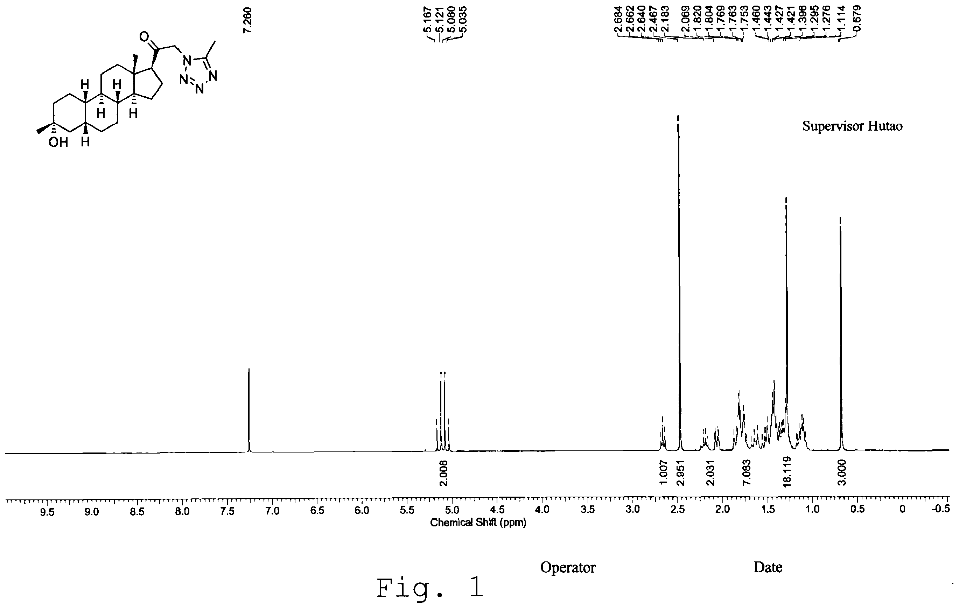

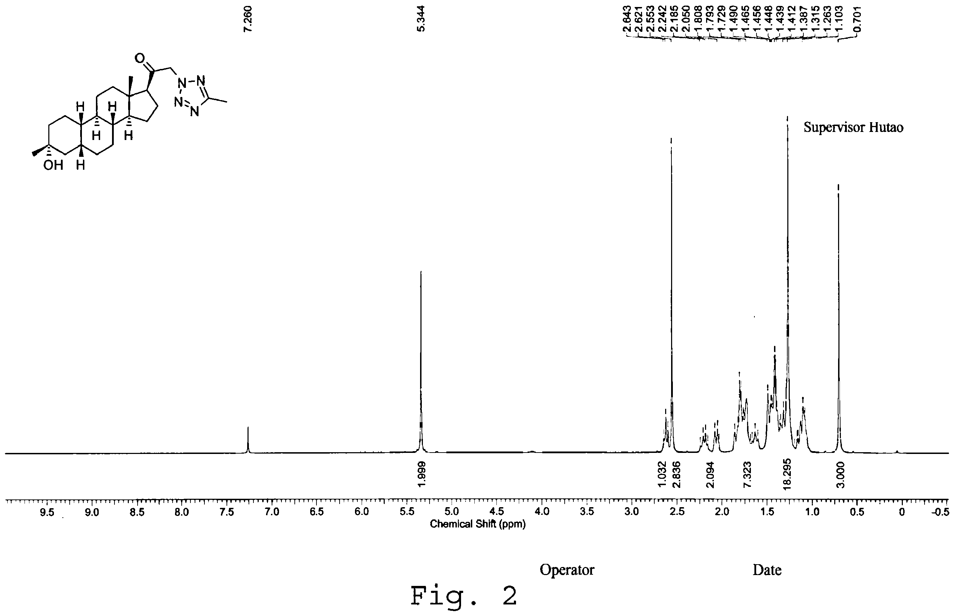

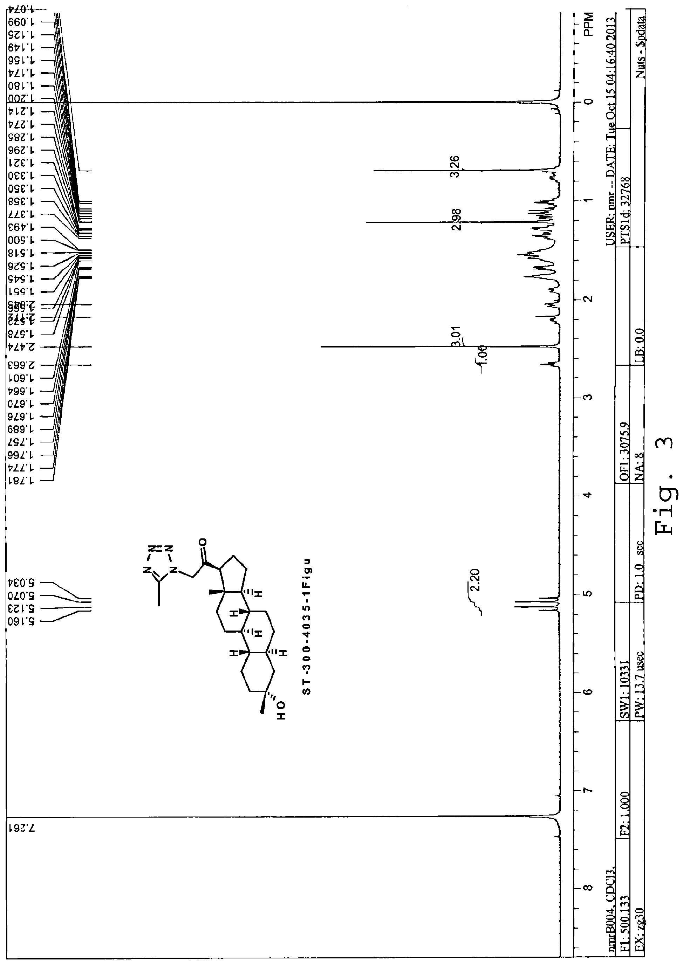

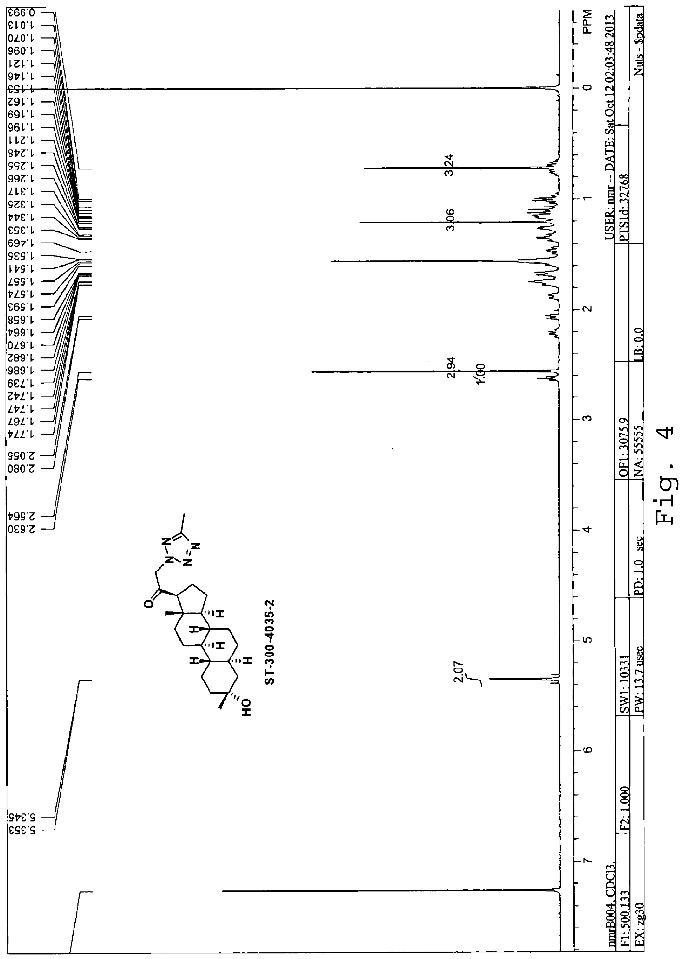

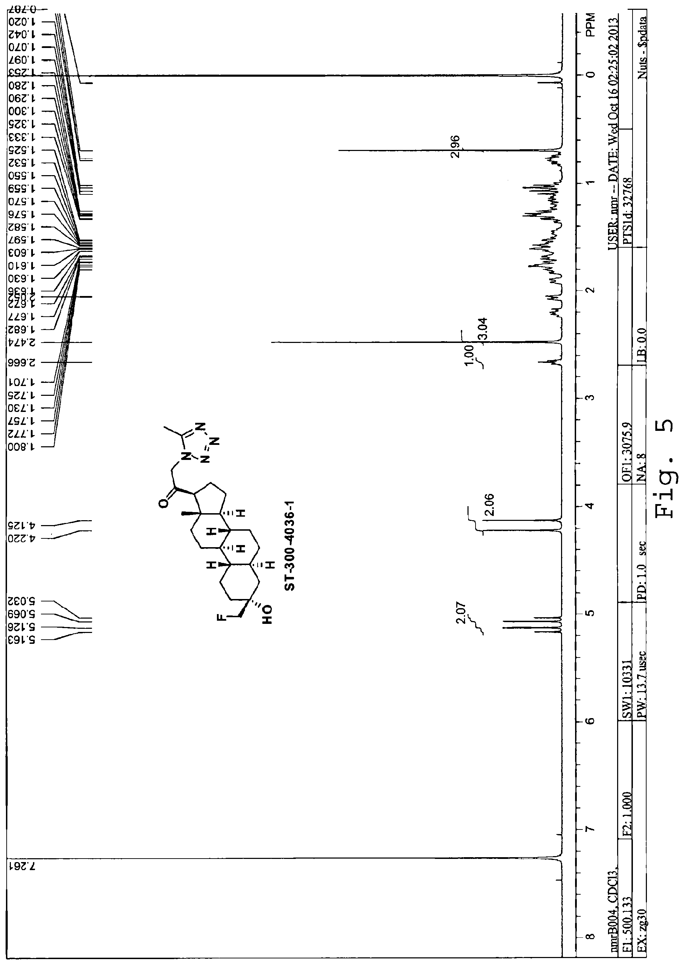

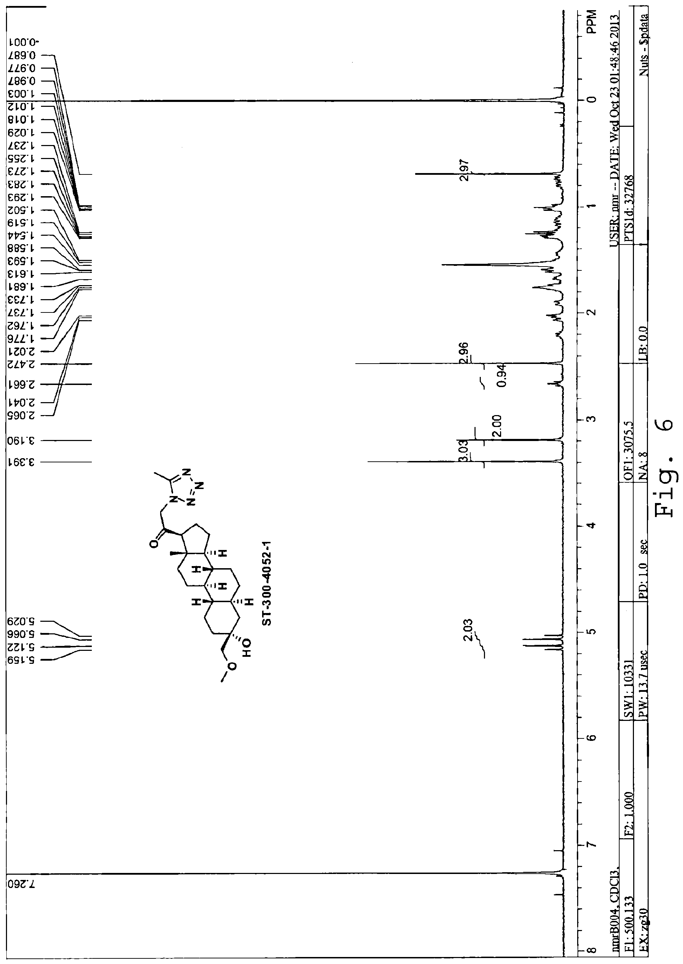

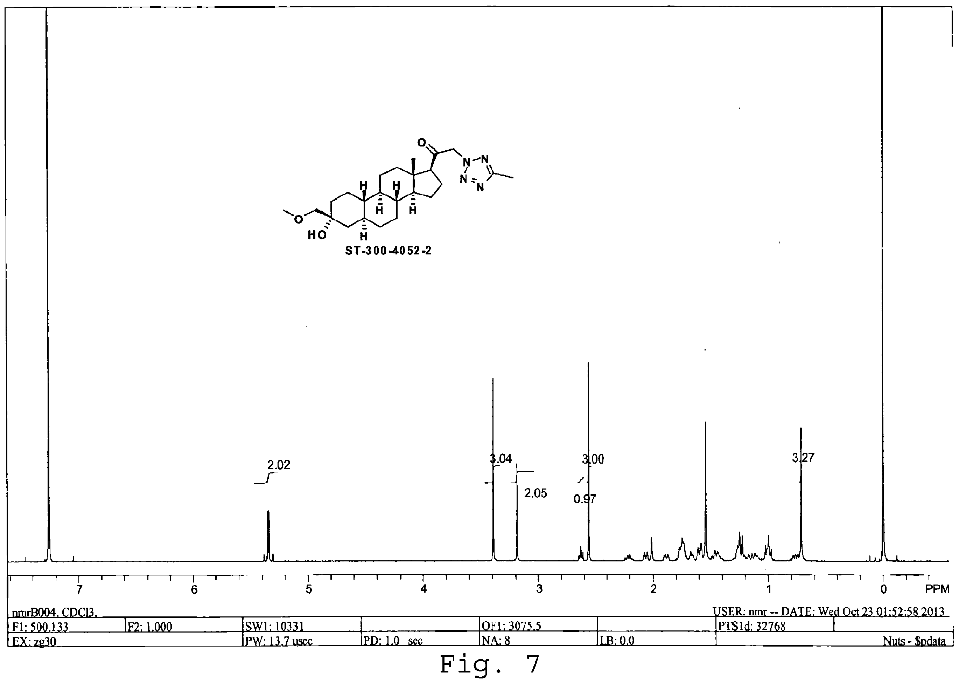

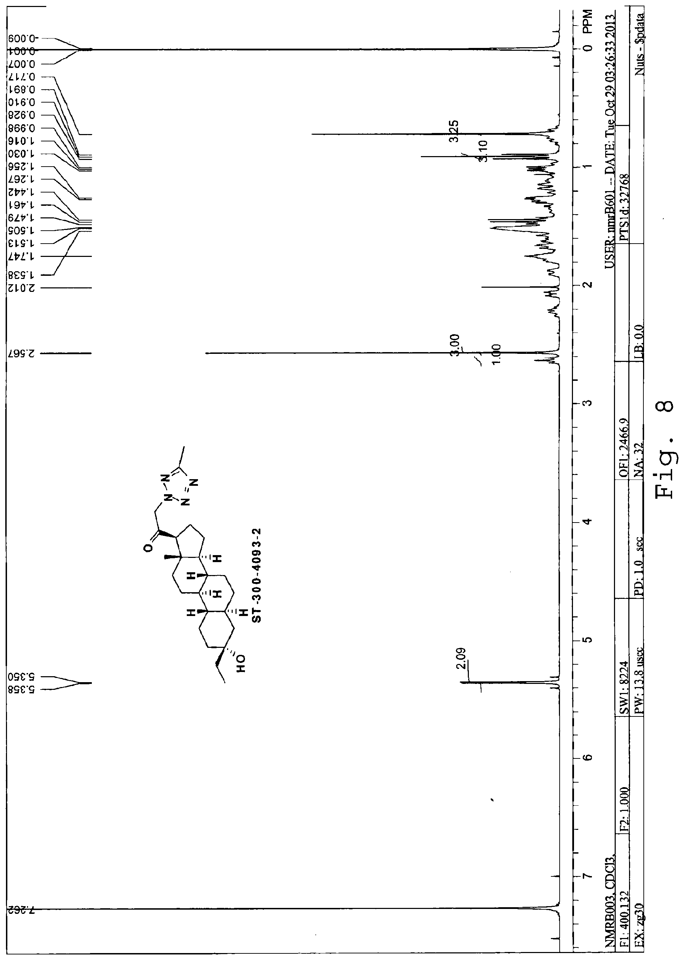

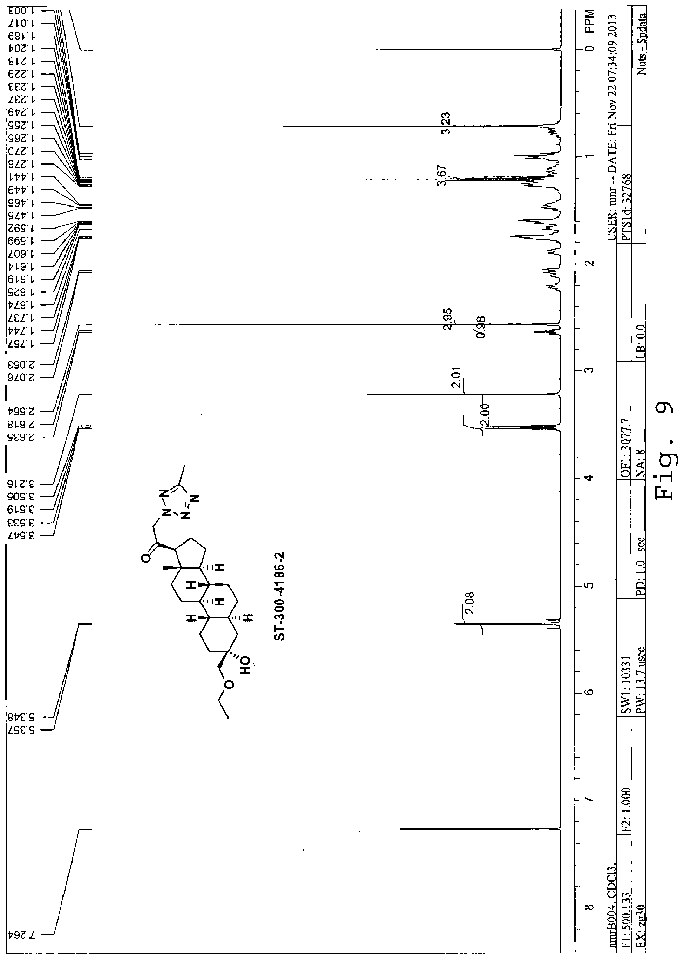

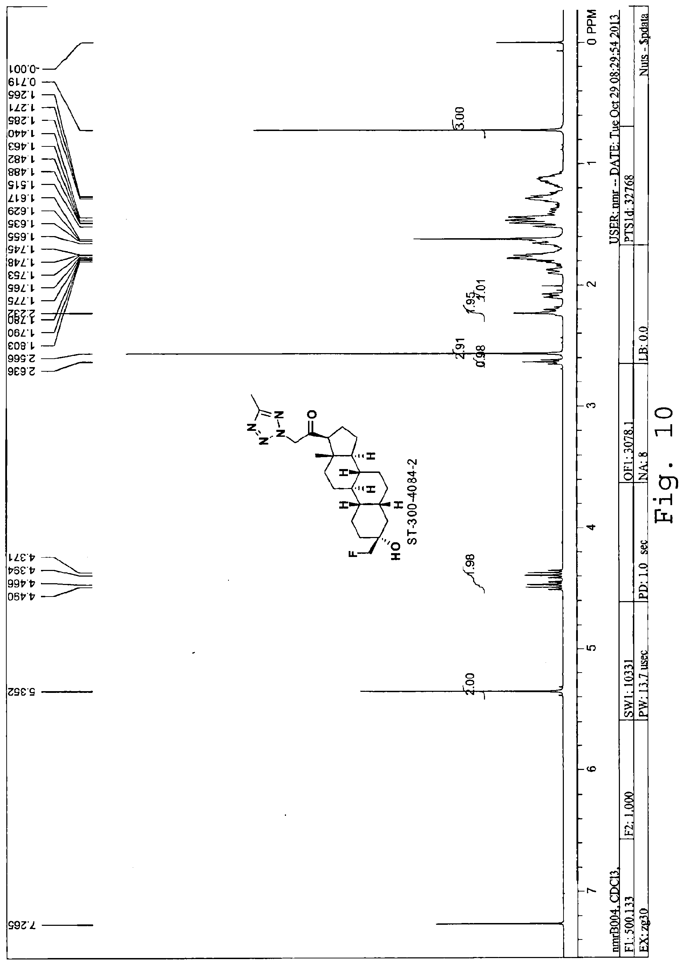

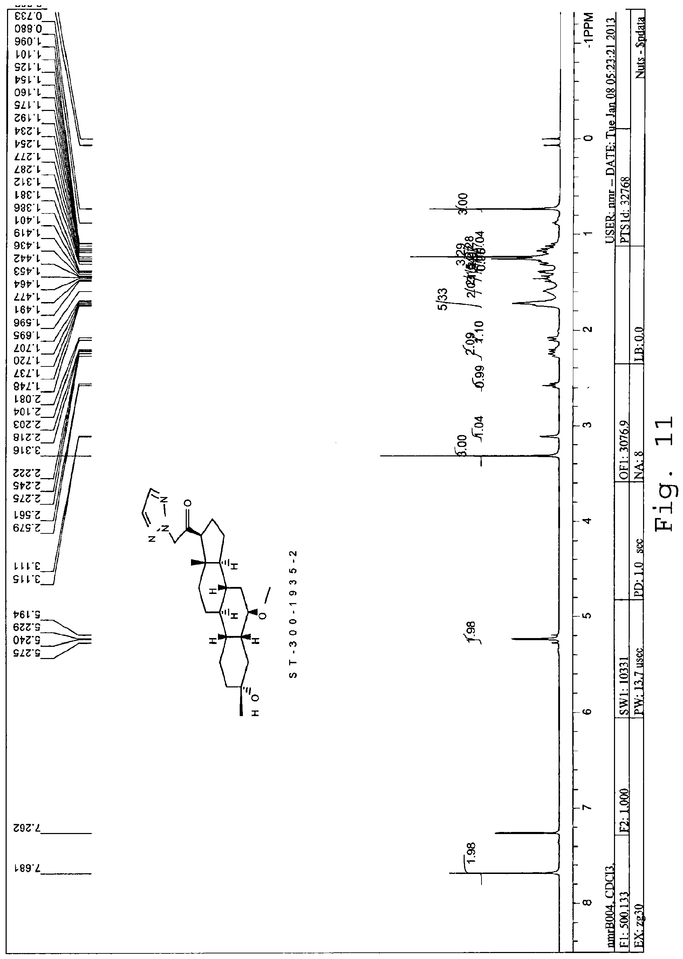

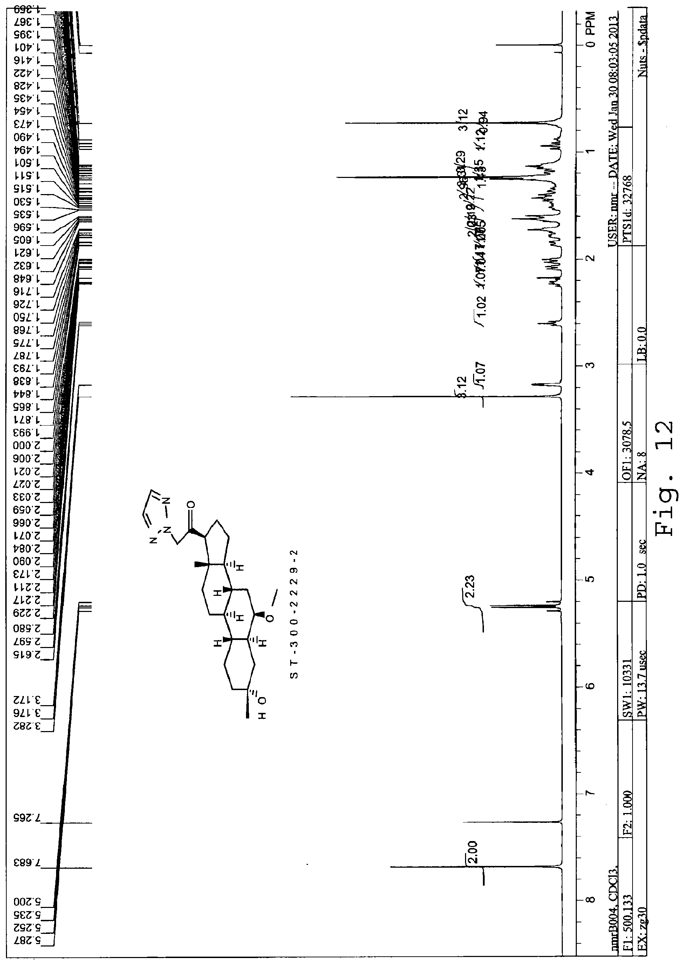

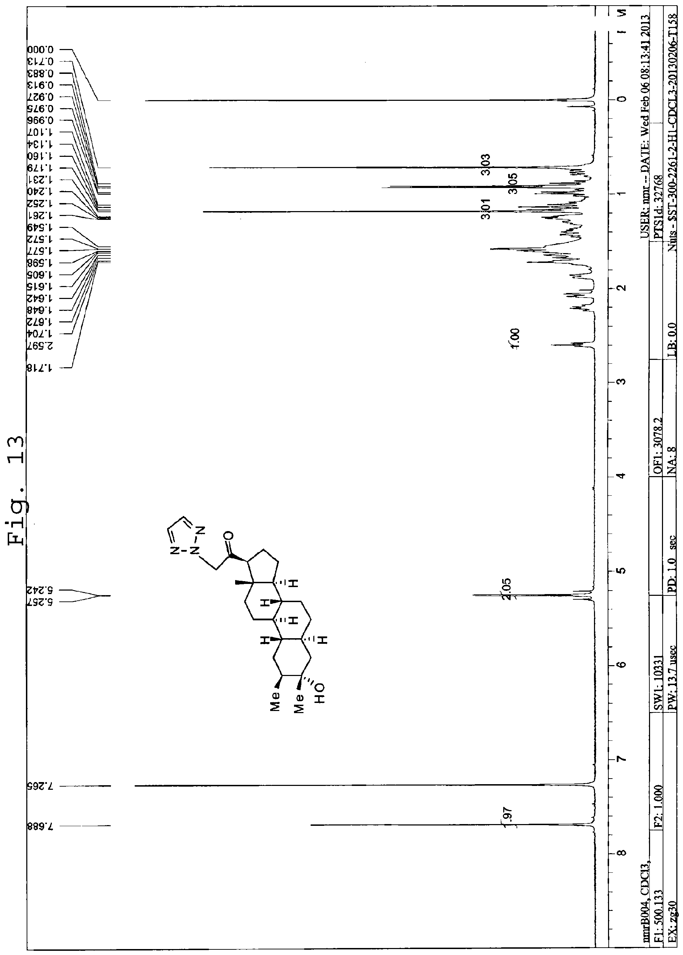

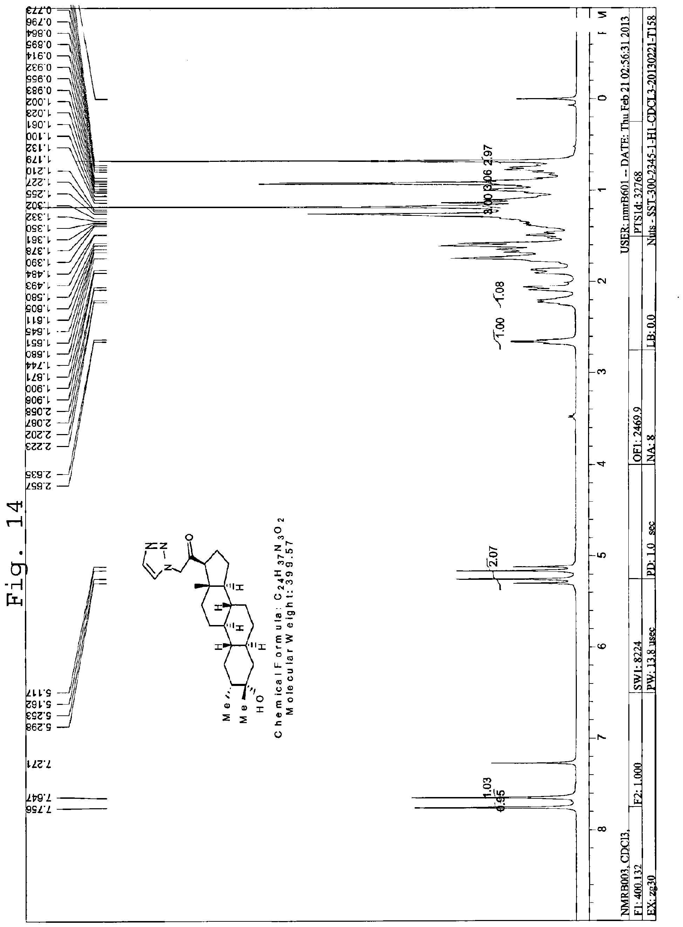

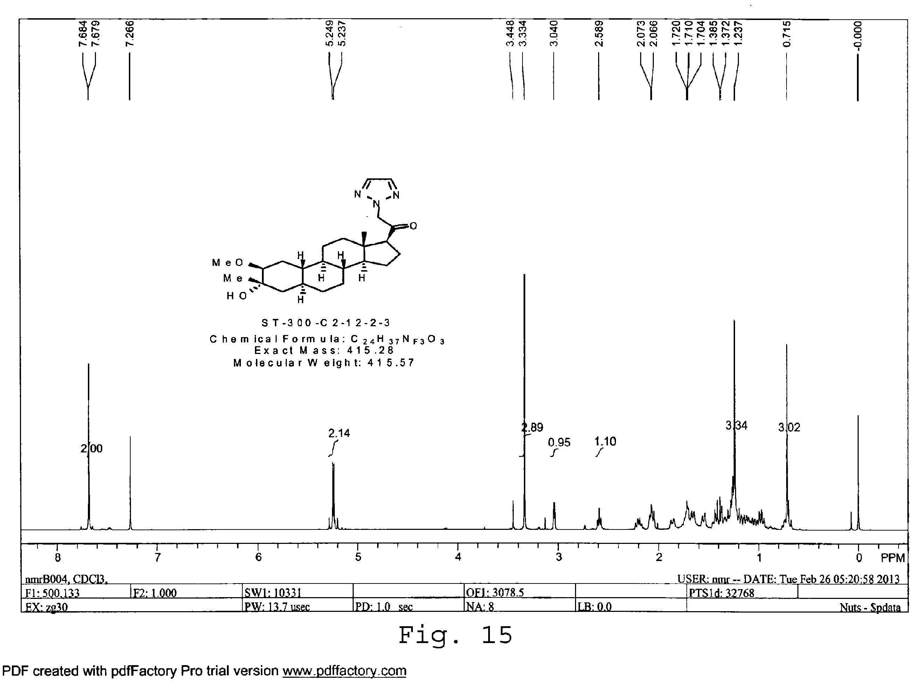

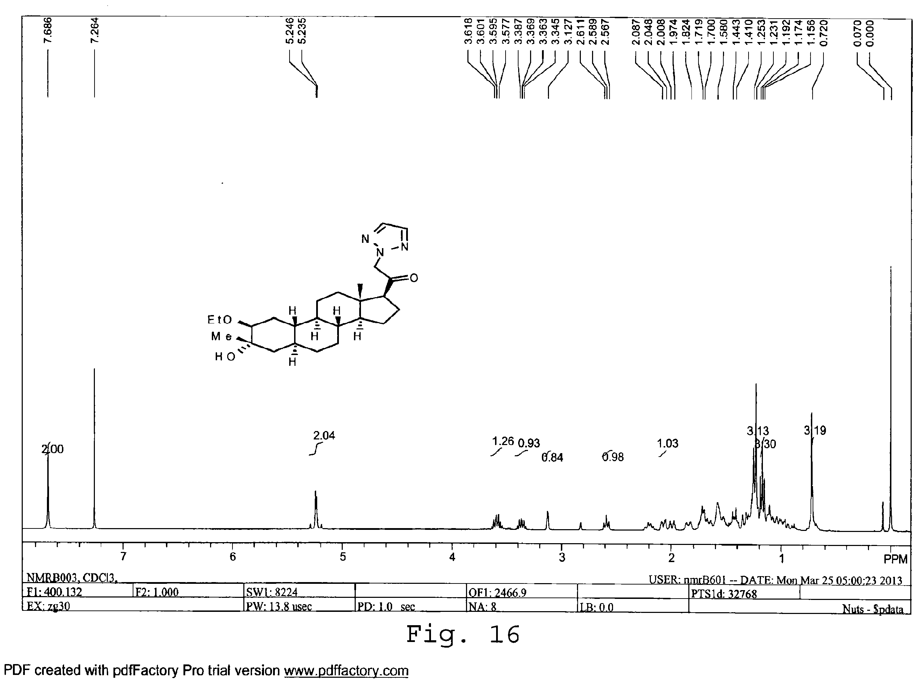

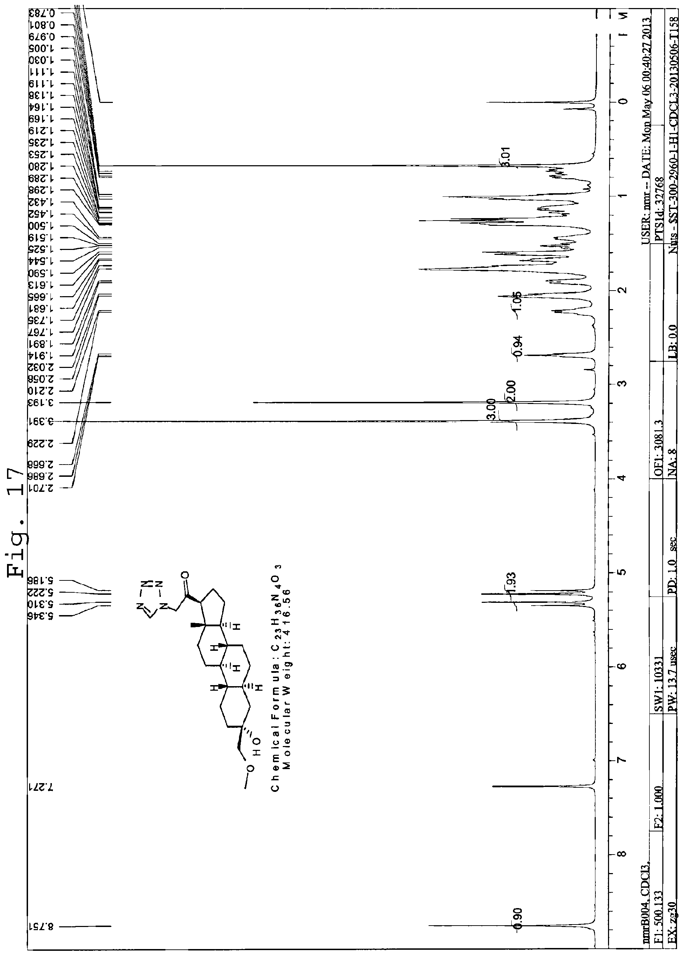

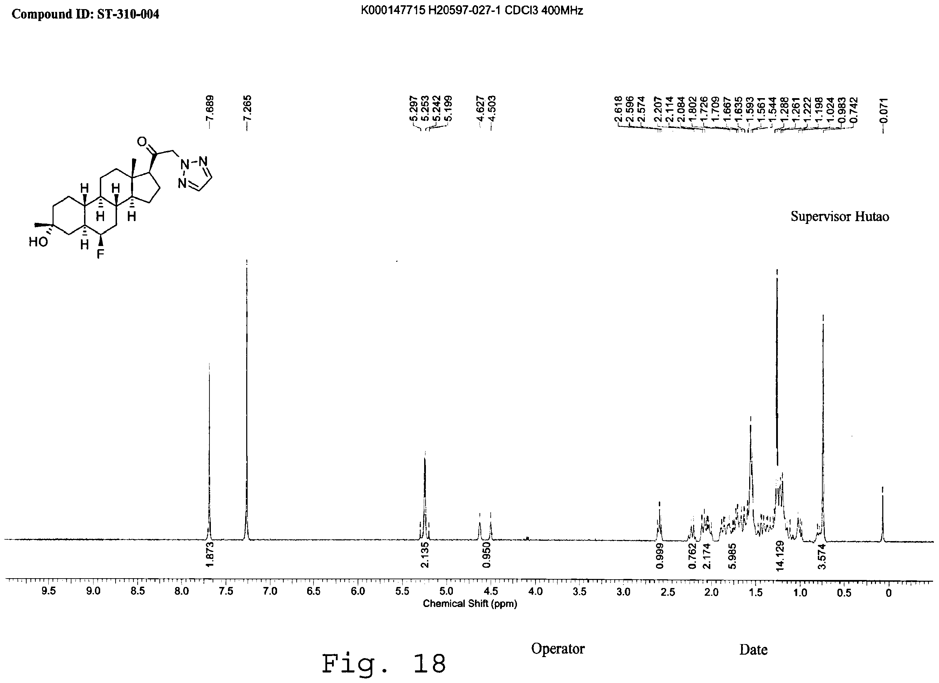

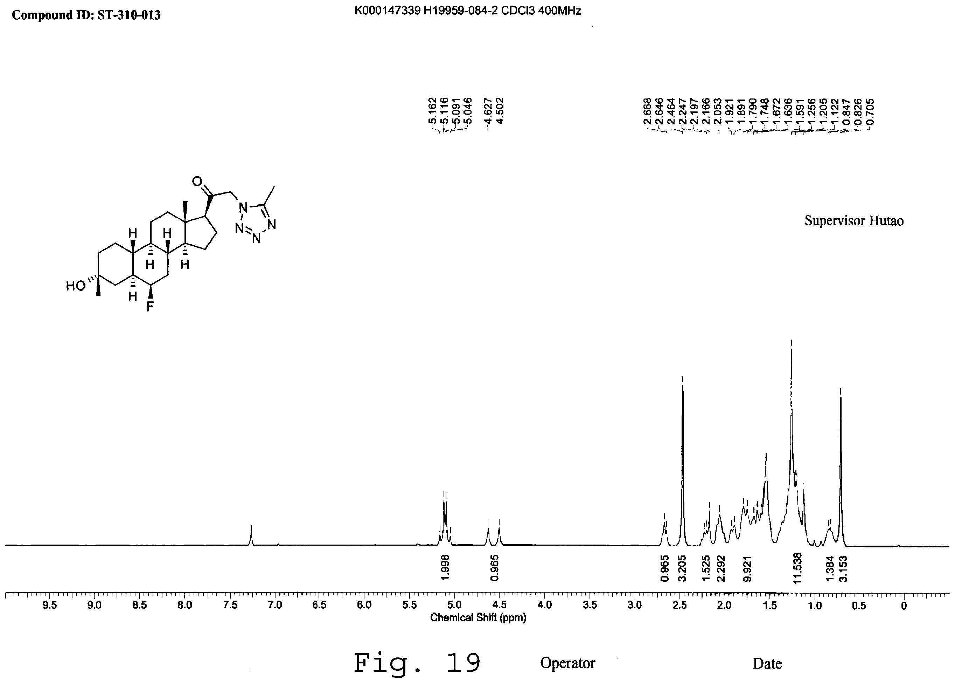

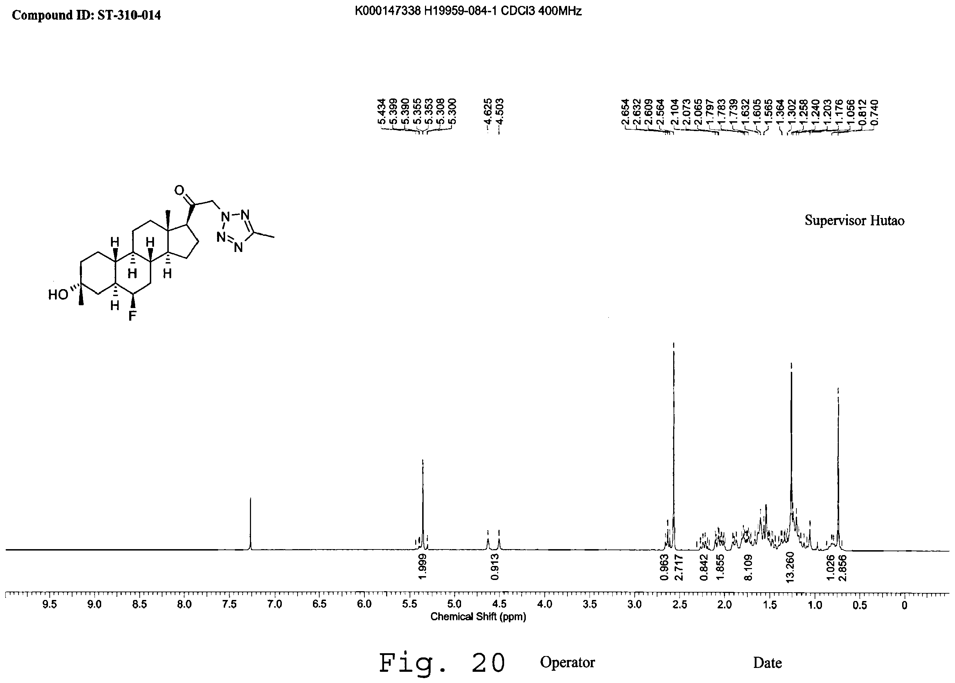

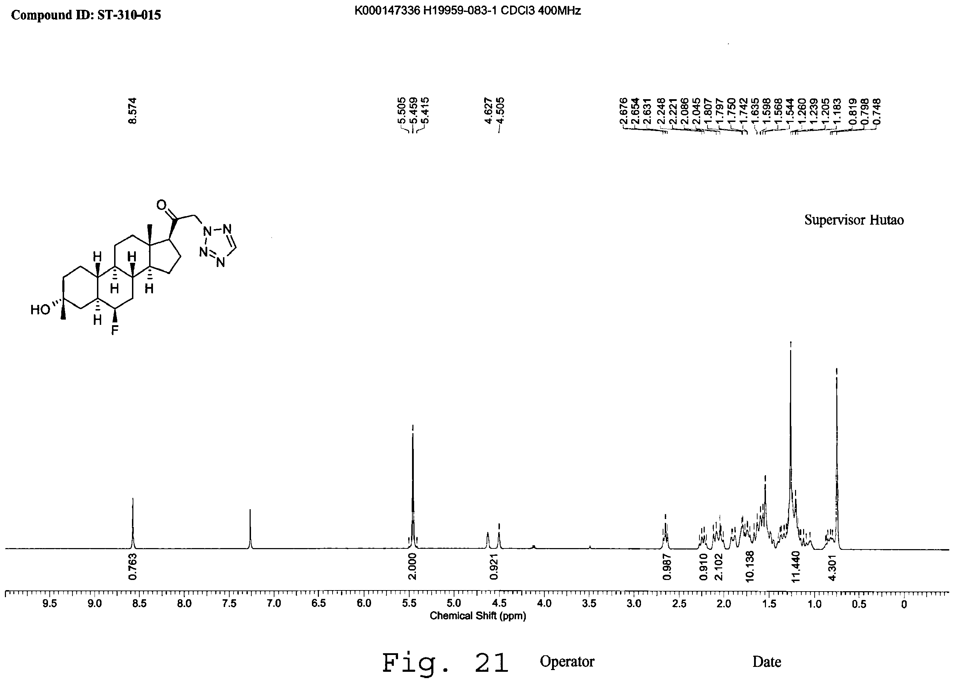

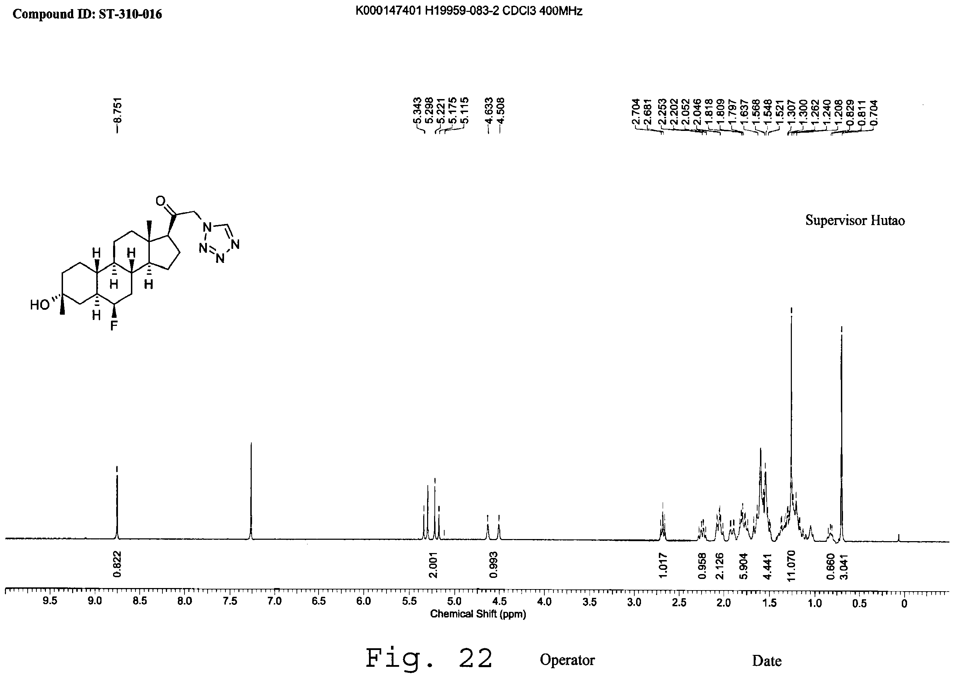

FIGS. 1-22 depict representative ¹H NMR spectra of exemplary compounds described herein.

### Detailed Description of Certain Embodiments of the Invention

Described herein, are 19-nor C3,3-disubstituted C21-triazole and C21-tetrazole neuroactive steroids of Formula (I), which is defined more narrowly in the claims: 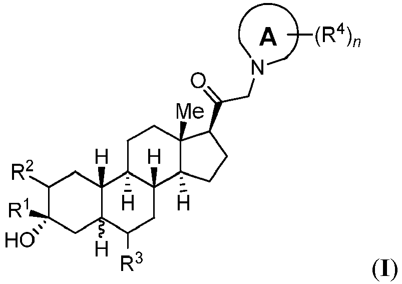 or a pharmaceutically acceptable salt thereof; wherein: A is selected from the group: 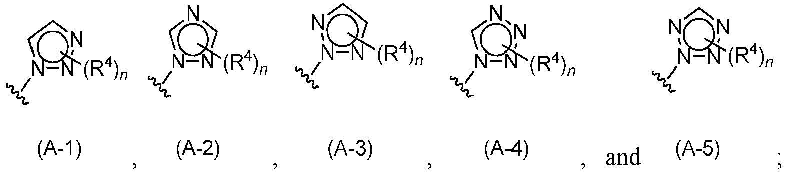 R¹ is C₁-C₆ haloalkyl (CHF₂, CH₂F) or C₁-C₆ alkyl (e.g., CH₃, CH₂CH₃, CH₂OCH₃, CH₂OCH₂CH₃); R² and R³ is independently selected from H, halo (e.g., F), C₁-C₆ alkyl (e.g., CH₃) or alkoxy (OCH3, OCH₂CH₃); R⁴ is halo (e.g., Cl, F), cyano, nitro, -S(O)ₓR^{a}, -NR^{b}R^{c}, C₁-C₆ alkyl (e.g., CH₃, CF₃), C₁-C₆ alkoxy, -C(O)R^{a}, -C(O)OR^{a}, or -C(O)NR^{b}R^{c}; R^{a} is H or C₁-C₆ alkyl; each R^{b} and R^{c} is independently H, -S(O)ₓR^{a}, -C(O)R^{a}, C₁-C₆ alkyl, or C₁-C₆ alkoxy, or R^{b} and R^{c} taken together with the atom to which they are attached form a ring (e.g., R^{b} and R^{c} taken together with the atom to which they are attached form a 4-8-membered ring, e.g., a heterocyclic ring, e.g., a morpholine ring, a pyrrolidine ring, a piperidine ring); *n* is an integer from 0 to 2; and x is an integer from 0 to 2.

The following definitions for compounds of Formula (**I**) are provided for the general understanding of the present invention, with the claimed compound set out below.

In some compounds of disclosed Formula **(I),** when A is (A-1) or (A-2), then R¹ is selected from: -CHF₂, CH₂F, -CCl₃, -CHCl₂, CH₂Cl, - CBr₃, CHBr₂, CH₂Br, or C₁-C₆ alkyl; or when A is (A-3) or (A-5), R¹ is -CH₃, -CH₂F, -CH₂OCH₃, or -CHF₂, and *n* is 0, then at least one of R² and R³ is not H.

In some compounds of disclosed Formula **(I),** when A is (A-1), (A-3), or (A-5), and *n* is 0, then at least one of R² and R³ is not H.

In some compounds of disclosed Formula **(I),** when A is (A-1), (A-3), or (A-5), then at least one of R² and R³ is not H.

In some compounds of disclosed Formula **(I),** when A is (A-1) or (A-2), and *n* is 0, then R¹ is selected from: -CHF₂, CH₂F, -CCl₃, -CHCl₂, CH₂Cl, - CBr₃, CHBr₂, CH₂Br, or C₁-C₆ alkyl.

In some compounds of disclosed Formula **(I),** *n* is 0 or 1. In some compounds of disclosed Formula **(I),** *n* is 0. In some compounds of disclosed Formula **(I),** *n* is 1.

Some compounds of disclosed Formula **(I),** described but not claimed herein, are selected from a compound of Formula (**Ia**):

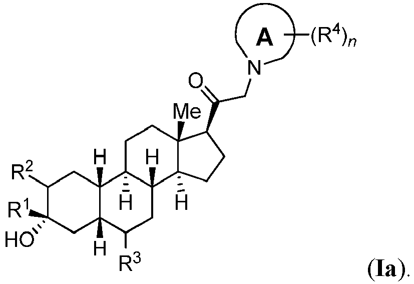

The compound of the Formula (I) can be selected from a compound of Formula (**Ib**), defined more narrowly in the claims:

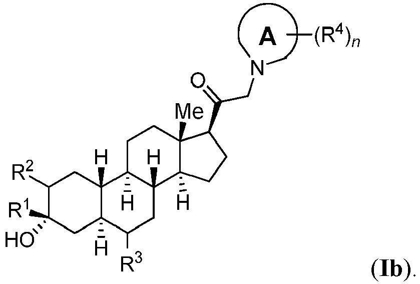

Some compounds of Formula (**I**), described but not claimed herein, are selected from a compound of Formula (**II**):

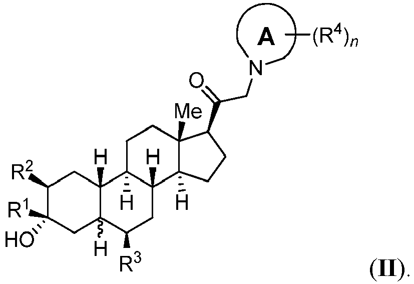

The following definitions are provided for the general understanding of the present invention, with the claimed compound set out below.

In some compounds, *n* is 1 and R⁴ is halo, cyano, -S(O)ₓR^{a}, or C₁-C₆ alkyl. In some compounds, R⁴ is -CH₃. In some compounds, R⁴ is cyano. In some compounds, R⁴ is -S(O)₂CH₃. In some compounds, A is selected from the group:

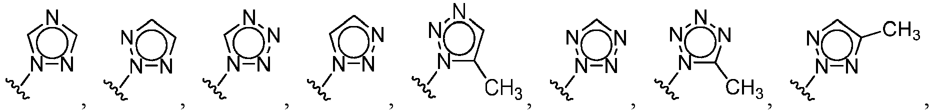

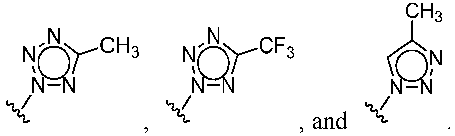

In some compounds, R¹ is C₁-C₆ alkyl. In some compounds, R¹ is -CH₃.

In some compounds, R² and R³ are H.

In some compounds, *n* is 1 and R⁴ is halo, cyano, -S(O)ₓR^{a}, or C₁-C₆ alkyl.

In some compounds, R⁴ is -CH₃.

In some compounds, R⁴ is -C(O)OR^{a}. In some compounds, R^{a} is H. In some compounds, R^{a} is C₁-C₆ alkyl. In some compounds, R^{a} is -CH₂CH₃.

In some compounds, R⁴ is -C(O)NR^{b}R^{c}. In some compounds, R^{b} and R^{c} are H.

In some compounds, R⁴ is cyano. In some compounds, R⁴ is -S(O)₂CH₃.

In some compounds, A is selected from the group:

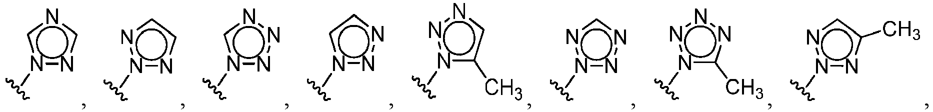

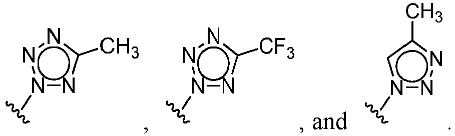

The compound of the present invention claimed herein is:

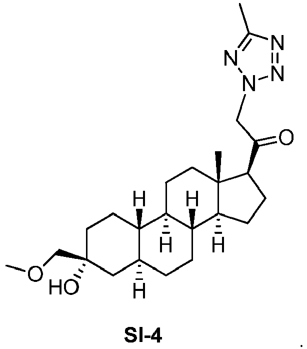

Other compounds disclosed herein but not forming part of the claimed invention are: 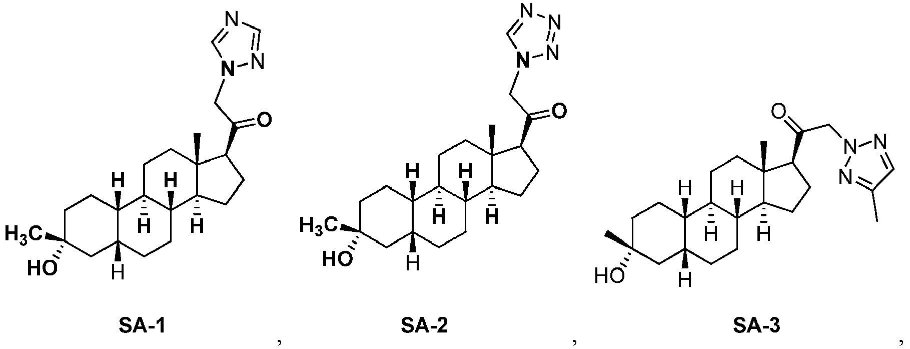 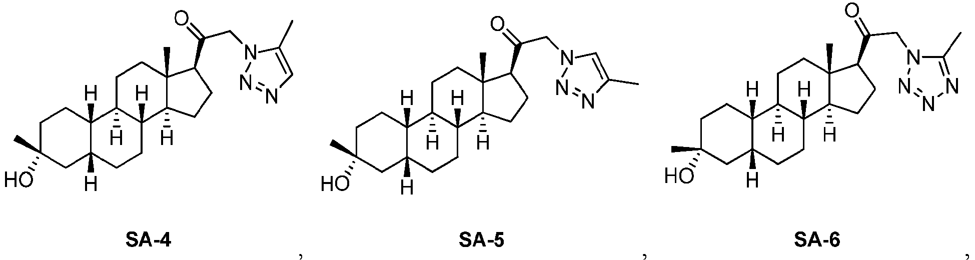 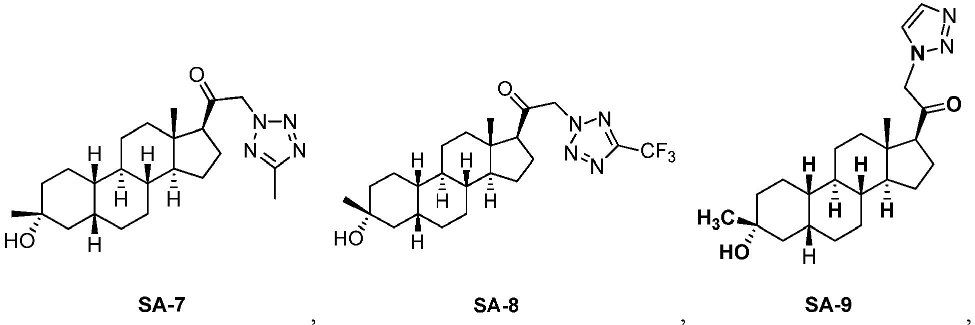 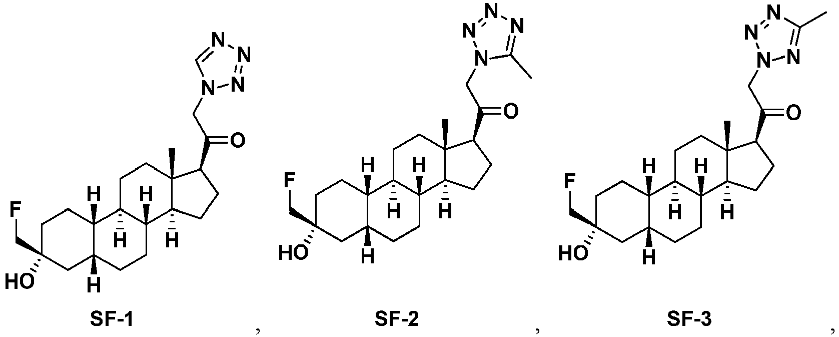 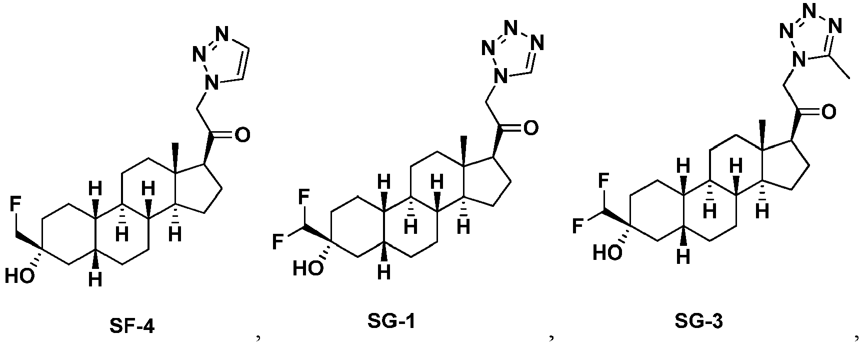 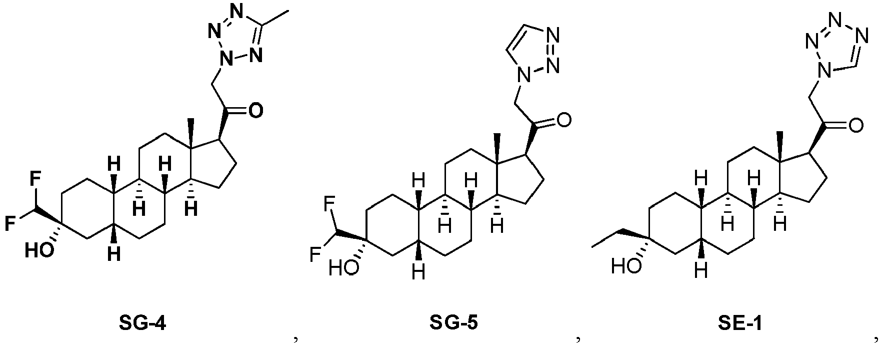 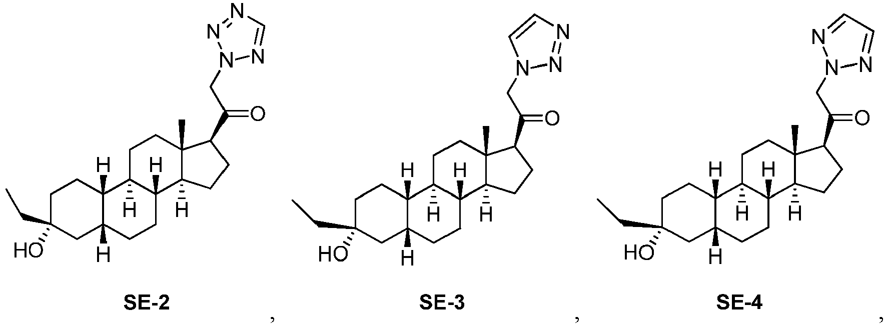 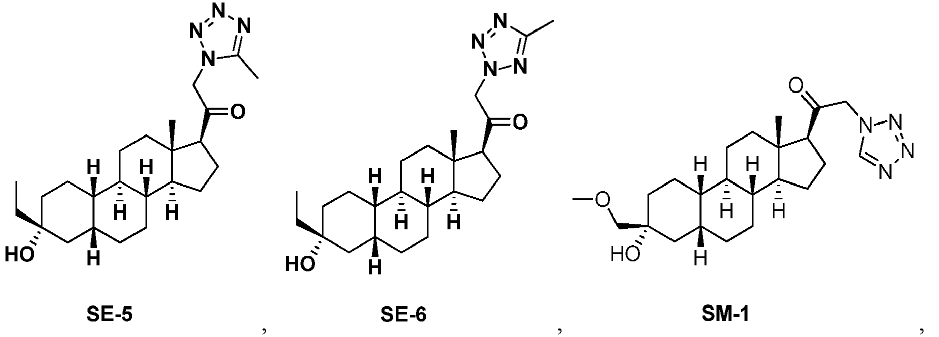 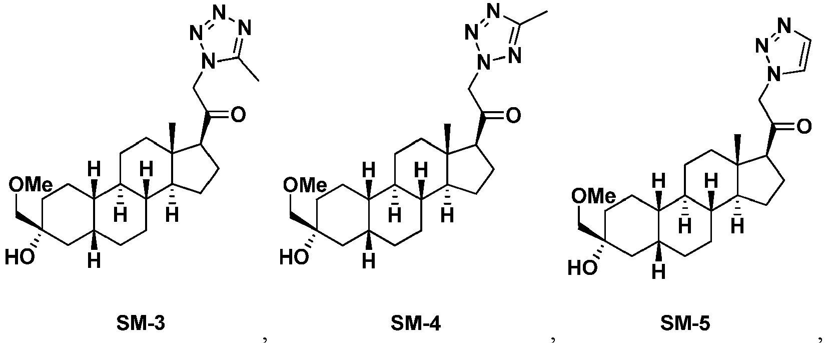 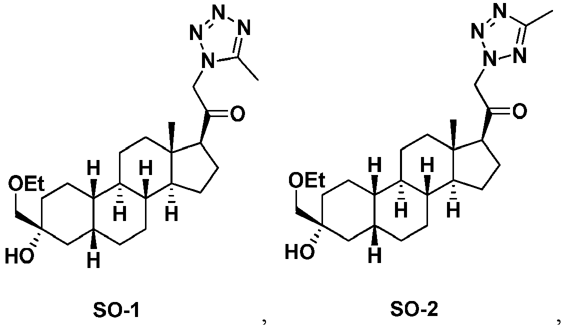 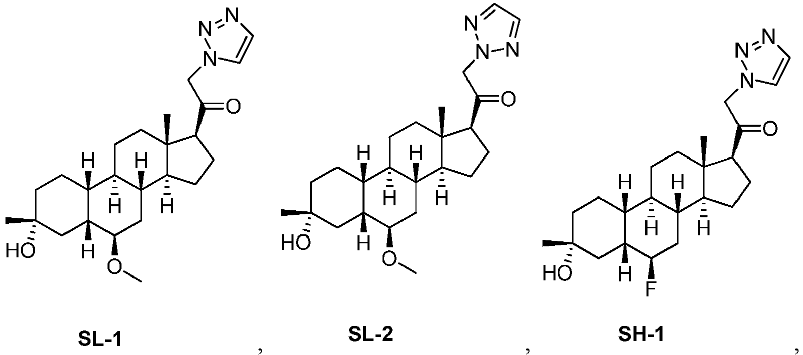 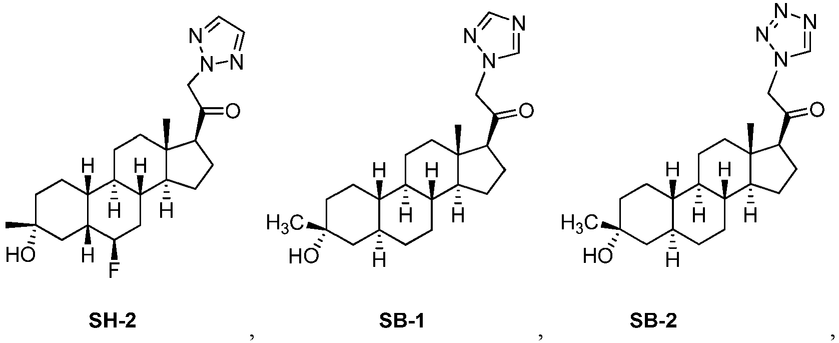 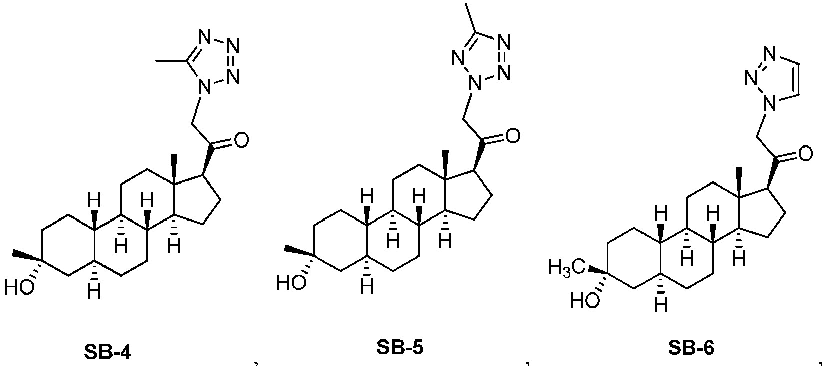 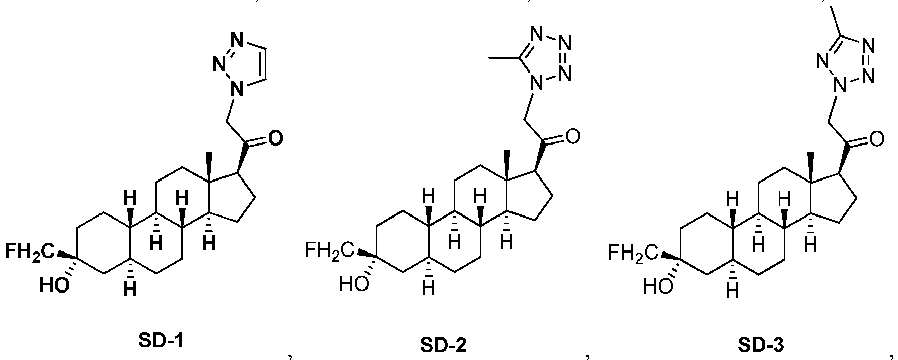 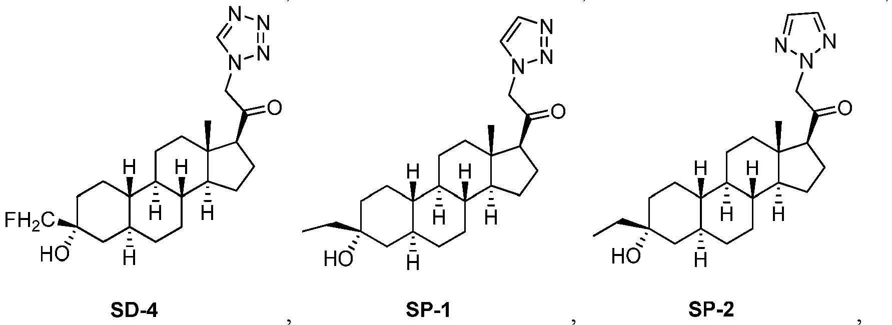 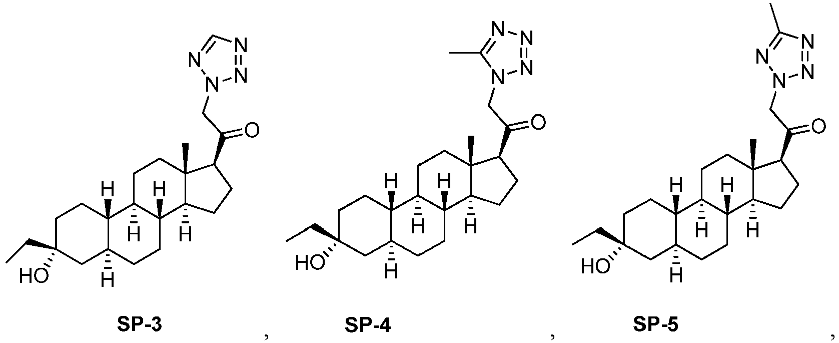 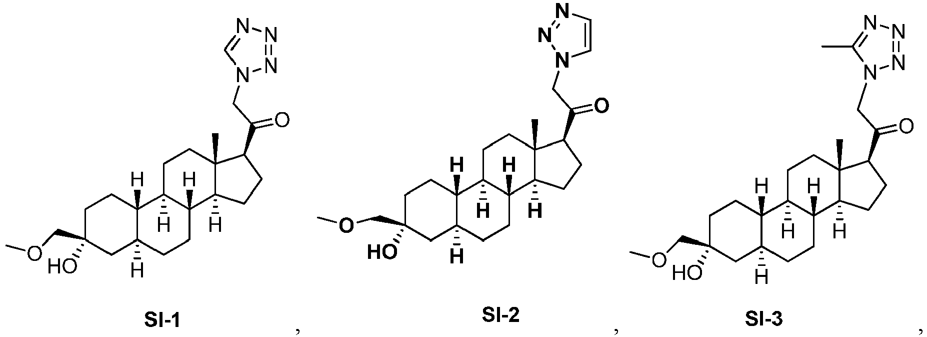 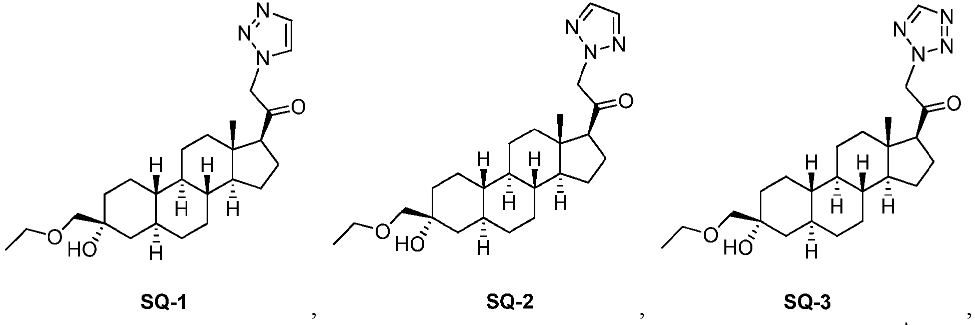 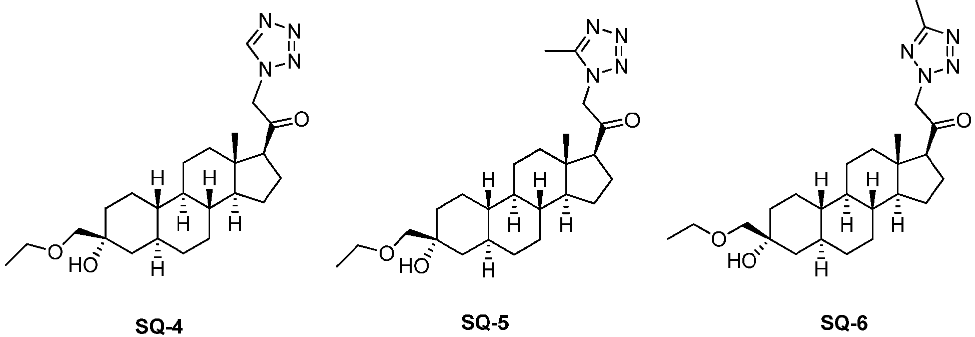 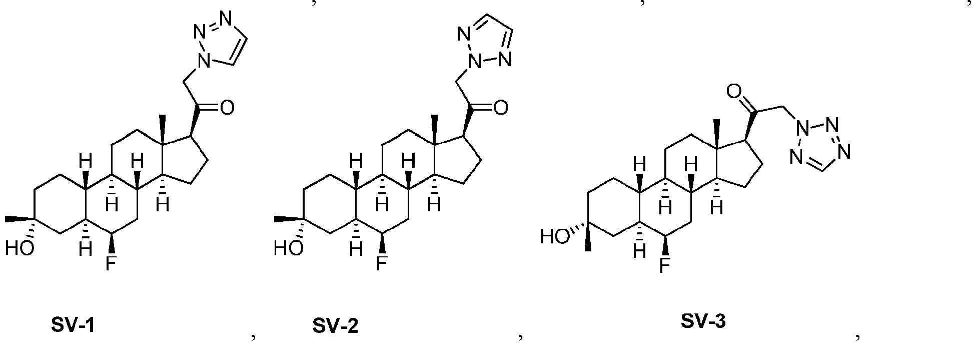 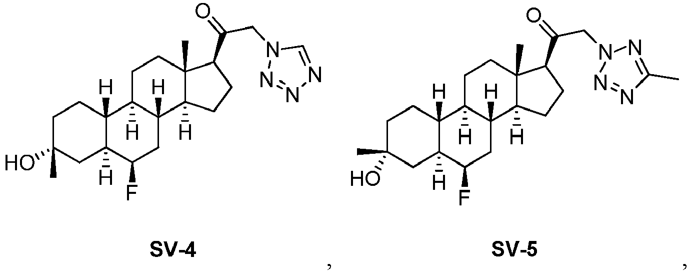 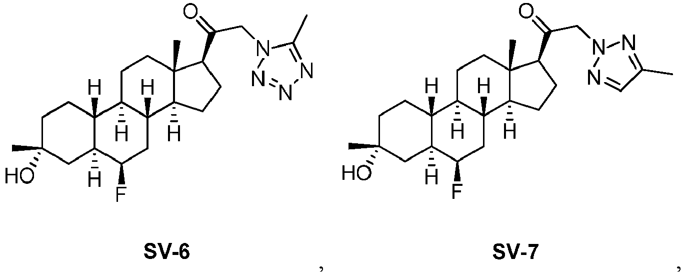 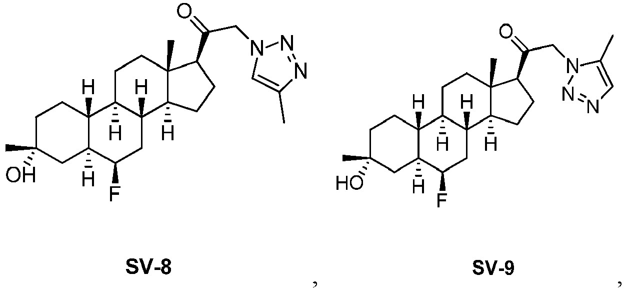 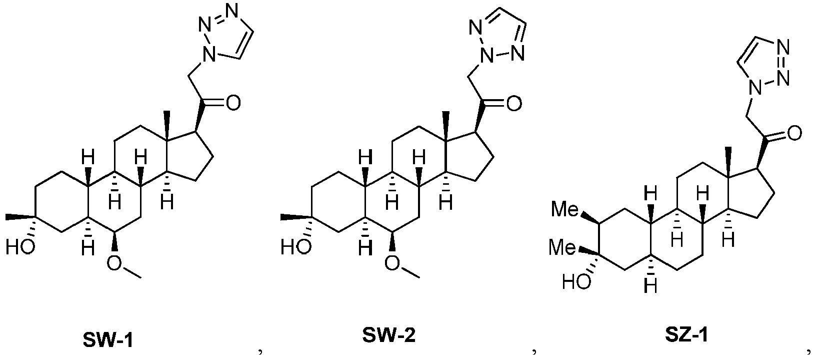 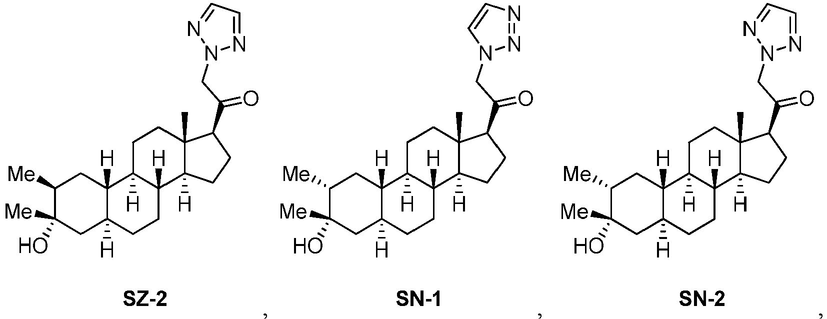 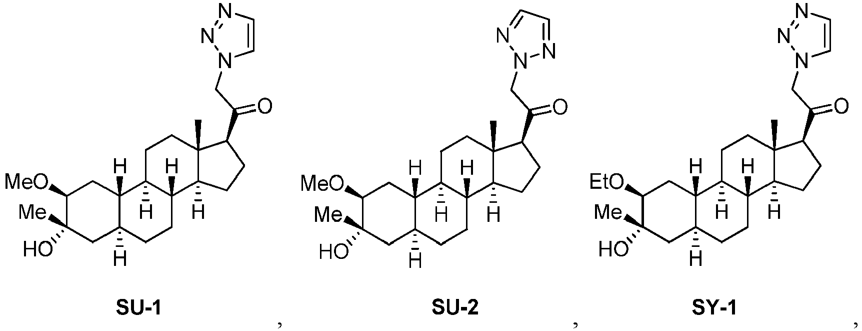 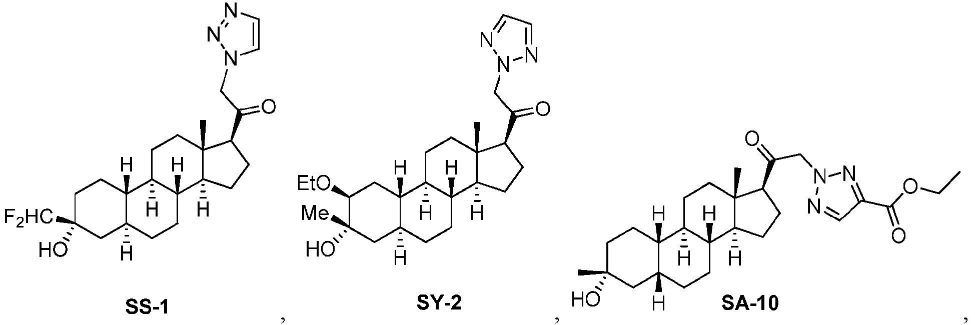 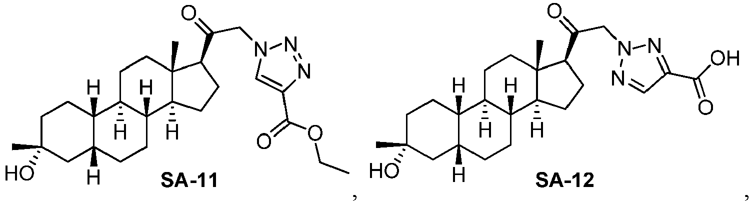 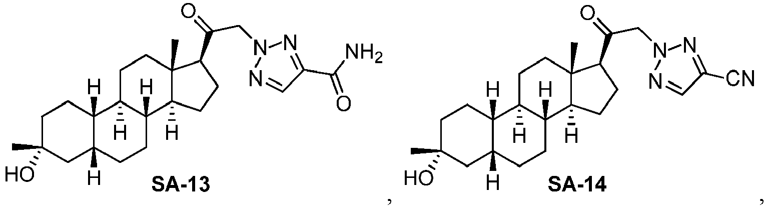 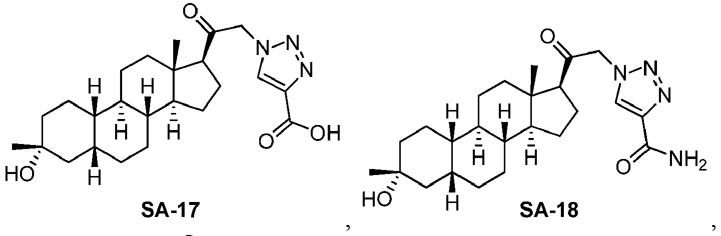 and ,

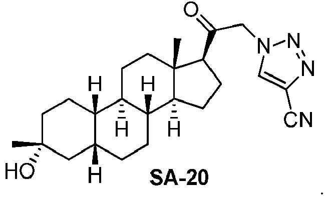

In an aspect, provided herein is a pharmaceutical composition comprising a compound as claimed herein, or pharmaceutically acceptable salt thereof, and a pharmaceutically acceptable excipient.

Described herein are compounds (e.g., a compound of Formula (**I**)), defined more narrowly in the claims, or a pharmaceutically acceptable salt thereof, for use in a method for treating a CNS-related disorder in a subject in need thereof. The CNS-related disorder can be a sleep disorder, an eating disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, or tinnitus; depression (e.g., post-partum depression); or an eating disorder (e.g., anorexia nervosa, bulimia nervosa, binge-eating disorder, cachexia). In the invention claimed herein, the CNS-related disorder is tremor (e.g., essential tremor).

In some embodiments, the compound is administered orally, subcutaneously, intravenously, or intramuscularly. In some embodiments, the compound is administered chronically.

### Pharmaceutical Compositions

In another aspect, the invention provides a pharmaceutical composition comprising a compound of the present invention (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the onset of a CNS-disorder, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present invention may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, preferably from about 0.1 to about 20% by weight, preferably from about 0.1 to about 10% by weight, and more preferably from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds of the present invention can also be administered in sustained release forms or from sustained release drug delivery systems. A description of representative sustained release materials can be found in Remington's Pharmaceutical Sciences.

The present invention also relates to the pharmaceutically acceptable formulations of a compound of the present invention. In one embodiment, the formulation comprises water. In another embodiment, the formulation comprises a cyclodextrin derivative. The most common cyclodextrins are α-, β- and γ- cyclodextrins consisting of 6, 7 and 8 α-1 ,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as Captisol^{®}. See, *e.g.,* U.S. 5,376,645. In certain embodiments, the formulation comprises hexapropyl-β-cyclodextrin *(e.g.,* 10-50% in water).

The present invention also relates to the pharmaceutically acceptable acid addition salt of a compound of the present invention. The acid which may be used to prepare the pharmaceutically acceptable salt is that which forms a non-toxic acid addition salt, *i.e.,* a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

The following formulation examples illustrate representative pharmaceutical compositions that may be prepared in accordance with this invention. The present invention, however, is not limited to the following pharmaceutical compositions.

*Exemplary Formulation 1* - *Tablets:* A compound of the present invention may be admixed as a dry powder with a dry gelatin binder in an approximate 1:2 weight ratio. A minor amount of magnesium stearate is added as a lubricant. The mixture is formed into 240-270 mg tablets (80-90 mg of active compound per tablet) in a tablet press.

Exemplary Formulation 2 - Capsules: A compound of the present invention may be admixed as a dry powder with a starch diluent in an approximate 1:1 weight ratio. The mixture is filled into 250 mg capsules (125 mg of active compound per capsule).

*Exemplary Formulation 3* - *Liquid*: A compound of the present invention (125 mg) may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color are diluted with water and added with stirring. Sufficient water may then be added to produce a total volume of 5 mL.

*Exemplary Formulation 4* - *Tablets:* A compound of the present invention may be admixed as a dry powder with a dry gelatin binder in an approximate 1:2 weight ratio. A minor amount of magnesium stearate is added as a lubricant. The mixture is formed into 450-900 mg tablets (150-300 mg of active compound) in a tablet press.

*Exemplary Formulation 5* - *Injection*: A compound of the present invention may be dissolved or suspended in a buffered sterile saline injectable aqueous medium to a concentration of approximately 5 mg/mL.

*Exemplary Formulation 6* - *Tablets:* A compound of the present invention may be admixed as a dry powder with a dry gelatin binder in an approximate 1:2 weight ratio. A minor amount of magnesium stearate is added as a lubricant. The mixture is formed into 90-150 mg tablets (30-50 mg of active compound per tablet) in a tablet press.

*Exemplary Formulation 7* - *Tablets:* A compound of the present invention may be admixed as a dry powder with a dry gelatin binder in an approximate 1:2 weight ratio. A minor amount of magnesium stearate is added as a lubricant. The mixture is formed into 30-90 mg tablets (10-30 mg of active compound per tablet) in a tablet press.

*Exemplary Formulation 8* - *Tablets:* A compound of the present invention may be admixed as a dry powder with a dry gelatin binder in an approximate 1:2 weight ratio. A minor amount of magnesium stearate is added as a lubricant. The mixture is formed into 0.3-30 mg tablets (0.1-10 mg of active compound per tablet) in a tablet press.

*Exemplary Formulation 9* - *Tablets:* A compound of the present invention may be admixed as a dry powder with a dry gelatin binder in an approximate 1:2 weight ratio. A minor amount of magnesium stearate is added as a lubricant. The mixture is formed into 150-240 mg tablets (50-80 mg of active compound per tablet) in a tablet press.

*Exemplary Formulation 10* - *Tablets:* A compound of the present invention may be admixed as a dry powder with a dry gelatin binder in an approximate 1:2 weight ratio. A minor amount of magnesium stearate is added as a lubricant. The mixture is formed into 270-450 mg tablets (90-150 mg of active compound per tablet) in a tablet press.

### Use in Methods of Treatment

As generally described herein, the present invention is directed to neuroactive steroids that may act, for example, as GABA modulators. In certain embodiments, a compound of the present invention or a composition thereof is useful as a therapeutic agent for treating a disorder described herein, e.g., tremor (e.g., essential tremor) in a subject. In certain embodiments, the compound is administered by intravenous administration.

Earlier studies (see, *e.g.,* Gee et al., European Journal of Pharmacology, 136:419-423 (1987)) demonstrated that certain 3α-hydroxylated steroids are orders of magnitude more potent as modulators of the GABA receptor complex (GRC) than others had reported (see, *e.g.,* Majewska et al., Science 232:1004-1007 (1986); Harrison et al., J Pharmacol. Exp. Ther. 241:346-353 (1987)). Majewska *et al.* and Harrison *et al.* taught that 3α-hydroxylated-5-reduced steroids are only capable of much lower levels of effectiveness. *In vitro* and *in vivo* experimental data have now demonstrated that the high potency of these steroids allows them to be therapeutically useful in the modulation of brain excitability via the GRC (see, *e.g.,* Gee et al., European Journal of Pharmacology, 136:419-423 (1987); Wieland et al., Psychopharmacology 118(1):65-71 (1995)).

Various synthetic steroids have also been prepared as neuroactive steroids. See, for example, U.S. Patent 5,232,917, which discloses neuroactive steroid compounds useful in treating stress, anxiety, insomnia, seizure disorders, and mood disorders, that are amenable to GRC-active agents, such as depression, in a therapeutically beneficial manner. Furthermore, it has been previously demonstrated that these steroids interact at a unique site on the GRC which is distinct from other known sites of interaction (e.g., barbiturates, benzodiazepines, and GABA) where therapeutically beneficial effects on stress, anxiety, sleep, mood disorders and seizure disorders have been previously elicited (see, *e.g.,* Gee, K.W. and Yamamura, H.I., "Benzodiazepines and Barbiturates: Drugs for the Treatment of Anxiety, Insomnia and Seizure Disorders," in Central Nervous System Disorders, Horvell, ed., Marcel-Dekker, New York (1985), pp. 123-147; Lloyd, K.G. and Morselli, P.L., "Psychopharmacology of GABAergic Drugs," in Psychopharmacology: The Third Generation of Progress, H.Y. Meltzer, ed., Raven Press, N.Y. (1987), pp. 183-195; and Gee et al., European Journal of Pharmacology, 136:419-423 (1987). These compounds are desirable for their duration, potency, and oral activity (along with other forms of administration).

Compounds of the claimed invention and present disclosure, as described herein, can modulate GABA function, and therefore can act as neuroactive steroids for the treatment and prevention of CNS-related conditions in a subject. Modulation, as used herein, refers to the inhibition or potentiation of GABA receptor function. Accordingly, the compounds and pharmaceutical compositions provided herein find use as therapeutics for preventing and/or treating CNS conditions in mammals including humans and non-human mammals. Thus, and as stated earlier, described herein are compounds for use in recited methods of treatment, and the use of such compounds for the preparation of medicaments useful for such methods.

Exemplary CNS conditions related to GABA-modulation include, but are not limited to, sleep disorders [*e.g.*, insomnia], mood disorders [*e.g.*, depression, dysthymic disorder *(e.g.,* mild depression), bipolar disorder *(e.g.,* I and/or II), anxiety disorders (*e.g.*, generalized anxiety disorder (GAD), social anxiety disorder), stress, post-traumatic stress disorder (PTSD), compulsive disorders (*e*.*g*.*,* obsessive compulsive disorder (OCD))], schizophrenia spectrum disorders [*e*.*g*.*,* schizophrenia, schizoaffective disorder], convulsive disorders [*e.g.,* epilepsy (*e.g.,* status epilepticus (SE)), seizures], disorders of memory and/or cognition [*e.g.,* attention disorders (*e.g.,* attention deficit hyperactivity disorder (ADHD)), dementia (*e*.*g*.*,* Alzheimer's type dementia, Lewis body type dementia, vascular type dementia], movement disorders [*e.g.,* Huntington's disease, Parkinson's disease], personality disorders [*e.g.,* antisocial personality disorder, obsessive compulsive personality disorder], autism spectrum disorders (ASD) [*e.g.,* autism, monogenetic causes of autism such as synaptophathy's, *e.g.,* Rett syndrome, Fragile X syndrome, Angelman syndrome], pain [*e*.*g*.*,* neuropathic pain, injury related pain syndromes, acute pain, chronic pain], traumatic brain injury (TBI), vascular diseases [*e*.*g*.*,* stroke, ischemia, vascular malformations], substance abuse disorders and/or withdrawal syndromes [*e*.*g*.*,* addition to opiates, cocaine, and/or alcohol], and tinnitus.

In yet another aspect, provided is a combination of the compound of the present invention and another pharmacologically active agent. The compound provided herein can be administered as the sole active agent or can be administered in combination with other agents. Administration in combination can proceed by any technique apparent to those of skill in the art including, for example, separate, sequential, concurrent and alternating administration.

Provided but not claimed herein is a compound of the present invention for use in a method of treating or preventing brain excitability in a subject susceptible to or afflicted with a condition associated with brain excitability.

In another aspect, provided is the compound of the present invention for use in a method of treating or preventing tremor in a subject. In certain embodiments the tremor is essential tremor.

Provided but not claimed herein is a compound of the present invention for use in a method of treating or preventing mood disorders in a subject. The mood disorder may be depression. The mood disorder may be postpartum depression.

Provided but not claimed herein is a compound of the present invention for use in a method of alleviating or preventing PMS or PND in a subject.

Provided but not claimed herein is a compound of the present invention, or a composition thereof for use in a method of treating or preventing stress or anxiety in a subject.

Provided but not claimed herein is a compound of the present invention, or a composition thereof for use in a method of alleviating or preventing insomnia in a subject.

Provided but not claimed herein is a compound of the present invention for use in a method of inducing sleep and maintaining substantially the level of REM sleep that is found in normal sleep, wherein substantial rebound insomnia is not induced.

Provided but not claimed herein is a compound of the present invention for use in a method of cognition enhancement or treating memory disorder. The disorder may be Alzheimer's disease. The disorder may be Rett syndrome.

Provided but not claimed herein is a compound of the present invention for use in a method of treating attention disorders. The attention disorder may be ADHD.

In certain embodiments, the compound is administered to the subject chronically. In certain embodiments, the compound is administered to the subject orally, subcutaneously, intramuscularly, or intravenously.

### Anxiety Disorders

**Anxiety disorder** is a blanket term covering several different forms of abnormal and pathological fear and anxiety. Current psychiatric diagnostic criteria recognize a wide variety of anxiety disorders.

**Generalized anxiety disorder** is a common chronic disorder characterized by long-lasting anxiety that is not focused on any one object or situation. Those suffering from generalized anxiety experience non-specific persistent fear and worry and become overly concerned with everyday matters. Generalized anxiety disorder is the most common anxiety disorder to affect older adults.

In **panic disorder**, a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attacks' potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

**Obsessive compulsive disorder** is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of **phobia**, which includes all cases in which fear and anxiety is triggered by a specific stimulus or situation. Sufferers typically anticipate terrifying consequences from encountering the object of their fear, which can be anything from an animal to a location to a bodily fluid.

**Post-traumatic stress disorder** or **PTSD** is an anxiety disorder which results from a traumatic experience. Post-traumatic stress can result from an extreme situation, such as combat, rape, hostage situations, or even serious accident. It can also result from long term (chronic) exposure to a severe stressor, for example soldiers who endure individual battles but cannot cope with continuous combat. Common symptoms include flashbacks, avoidant behaviors, and depression.

### Eating Disorders

Eating disorders feature disturbances in eating behavior and weight regulation, and are associated with a wide range of adverse psychological, physical, and social consequences. An individual with an eating disorder may start out just eating smaller or larger amounts of food, but at some point, their urge to eat less or more spirals out of control. Eating disorders may be characterized by severe distress or concern about body weight or shape, or extreme efforts to manage weight or food intake. Eating disorders include anorexia nervosa, bulimia nervosa, binge-eating disorder, cachexia, and their variants.

Individuals with **anorexia nervosa** typically see themselves as overweight, even when they are underweight. Individuals with anorexia nervosa can become obsessed with eating, food, and weight control. Individuals with anorexia nervosa typically weigh themselves repeatedly, portion food carefully, and eat very small quantities of only certain foods. Individuals with anorexia nervosa may engage in binge eating, followed by extreme dieting, excessive exercise, self-induced vomiting, or misuse of laxatives, diuretics, or enemas. Symptoms include extremely low body weight, severe food restriction, relentless pursuit of thinness and unwillingness to maintain a normal or healthy weight, intense fear of gaining weight, distorted body image and self-esteem that is heavily influenced by perceptions of body weight and shape, or a denial of the seriousness of low body weight, lack of menstruation among girls and women. Other symptoms include the thinning of the bones, brittle hair and nails, dry and yellowish skin, growth of fine hair all over the body, mild anemia, muscle wasting, and weakness, severe constipation, low blood pressure or slowed breathing and pulse, damage to the structure and function of the heart, brain damage, multi-organ failure, drop in internal body temperature, lethargy, sluggishness, and infertility.

Individuals with **bulimia nervosa** have recurrent and frequent episodes of eating unusually large amounts of food and feel a lack of control over these episodes. This binge eating is followed by behavior that compensates for the overeating such as forced vomiting, excessive use of laxatives or diuretics, fasting, excessive exercise, or a combination of these behaviors.

Unlike anorexia nervosa, people with bulimia nervosa usually maintain what is considered a healthy or normal weight, while some are slightly overweight. But like people with anorexia nervosa, they typically fear gaining weight, want desperately to lose weight, and are unhappy with their body size and shape. Usually, bulimic behavior is done secretly because it is often accompanied by feelings of disgust or shame. The binge eating and purging cycle can happen anywhere from several times a week to many times a day. Other symptoms include chronically inflamed and sore throat, swollen salivary glands in the neck and jaw area, worn tooth enamel, and increasingly sensitive and decaying teeth as a result of exposure to stomach acid, acid reflux disorder and other gastrointestinal problems, intestinal distress and irritation from laxative abuse, severe dehydration from purging of fluids, electrolyte imbalance (that can lead to a heart attack or stroke).

Individuals with **binge-eating disorder** lose control over their eating. Unlike bulimia nervosa, periods of binge eating are not followed by compensatory behaviors like purging, excessive exercise, or fasting. Individuals with binge-eating disorder often are overweight or obese. Obese individuals with binge-eating disorder are at higher risk for developing cardiovascular disease and high blood pressure. They also experience guilt, shame, and distress about their binge eating, which can lead to more binge eating.

**Cachexia** is also known as "wasting disorder," and is an eating-related issue experienced by many cancer patients. Individuals with cachexia may continue to eat normally, but their body may refuse to utilize the vitamins and nutrients that it is ingesting, or they will lose their appetite and stop eating. When an individual experiences loss of appetite and stops eating, they can be considered to have developed anorexia nervosa.

### Neurodegenerative Diseases and Disorders

The term "neurodegenerative disease" includes diseases and disorders that are associated with the progressive loss of structure or function of neurons, or death of neurons. Neurodegenerative diseases and disorders include, but are not limited to, Alzheimer's disease (including the associated symptoms of mild, moderate, or severe cognitive impairment); amyotrophic lateral sclerosis (ALS); anoxic and ischemic injuries; ataxia and convulsion (including for the treatment and prevention and prevention of seizures that are caused by schizoaffective disorder or by drugs used to treat schizophrenia); benign forgetfulness; brain edema; cerebellar ataxia including McLeod neuroacanthocytosis syndrome (MLS); closed head injury; coma; contusive injuries (*e*.*g*., spinal cord injury and head injury); dementias including multi-infarct dementia and senile dementia; disturbances of consciousness; Down syndrome; drug-induced or medication-induced Parkinsonism (such as neuroleptic-induced acute akathisia, acute dystonia, Parkinsonism, or tardive dyskinesia, neuroleptic malignant syndrome, or medication-induced postural tremor); epilepsy; fragile X syndrome; Gilles de la Tourette's syndrome; head trauma; hearing impairment and loss; Huntington's disease; Lennox syndrome; levodopa-induced dyskinesia; mental retardation; movement disorders including akinesias and akinetic (rigid) syndromes (including basal ganglia calcification, corticobasal degeneration, multiple system atrophy, Parkinsonism-ALS dementia complex, Parkinson's disease, postencephalitic parkinsonism, and progressively supranuclear palsy); muscular spasms and disorders associated with muscular spasticity or weakness including chorea (such as benign hereditary chorea, drug-induced chorea, hemiballism, Huntington's disease, neuroacanthocytosis, Sydenham's chorea, and symptomatic chorea), dyskinesia (including tics such as complex tics, simple tics, and symptomatic tics), myoclonus (including generalized myoclonus and focal cyloclonus), tremor (such as rest tremor, postural tremor, and intention tremor) and dystonia (including axial dystonia, dystonic writer's cramp, hemiplegic dystonia, paroxysmal dystonia, and focal dystonia such as blepharospasm, oromandibular dystonia, and spasmodic dysphonia and torticollis); neuronal damage including ocular damage, retinopathy or macular degeneration of the eye; neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest; Parkinson's disease; seizure; status epilecticus; stroke; tinnitus; tubular sclerosis, and viral infection induced neurodegeneration (e.g., caused by acquired immunodeficiency syndrome (AIDS) and encephalopathies). Neurodegenerative diseases also include, but are not limited to, neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest. Methods of treating or preventing a neurodegenerative disease also include treating or preventing loss of neuronal function characteristic of neurodegenerative disorder.

### Epilepsy

Epilepsy is a brain disorder characterized by repeated seizures over time. Types of epilepsy can include, but are not limited to generalized epilepsy, e.g., childhood absence epilepsy, juvenile nyoclonic epilepsy, epilepsy with grand-mal seizures on awakening, West syndrome, Lennox-Gastaut syndrome, partial epilepsy, e.g., temporal lobe epilepsy, frontal lobe epilepsy, benign focal epilepsy of childhood.

### Status epilepticus (SE)

Status epilepticus (SE) can include, *e.g.,* convulsive status epilepticus, *e.g.,* early status epilepticus, established status epilepticus, refractory status epilepticus, super-refractory status epilepticus; non-convulsive status epilepticus, e.g., generalized status epilepticus, complex partial status epilepticus; generalized periodic epileptiform discharges; and periodic lateralized epileptiform discharges. Convulsive status epilepticus is characterized by the presence of convulsive status epileptic seizures, and can include early status epilepticus, established status epilepticus, refractory status epilepticus, super-refractory status epilepticus. Early status epilepticus is treated with a first line therapy. Established status epilepticus is characterized by status epileptic seizures which persist despite treatment with a first line therapy, and a second line therapy is administered. Refractory status epilepticus is characterized by status epileptic seizures which persist despite treatment with a first line and a second line therapy, and a general anesthetic is generally administered. Super refractory status epilepticus is characterized by status epileptic seizures which persist despite treatment with a first line therapy, a second line therapy, and a general anesthetic for 24 hours or more.

Non-convulsive status epilepticus can include, *e.g.,* focal non-convulsive status epilepticus, *e*.*g*., complex partial non-convulsive status epilepticus, simple partial non-convulsive status epilepticus, subtle non-convulsive status epilepticus; generalized non-convulsive status epilepticus, *e.g.,* late onset absence non-convulsive status epilepticus, atypical absence non-convulsive status epilepticus, or typical absence non-convulsive status epilepticus.

Compositions described herein can also be administered as a prophylactic to a subject having a CNS disorder *e.g.,* a traumatic brain injury, status epilepticus, *e.g.,* convulsive status epilepticus, *e*.*g*., early status epilepticus, established status epilepticus, refractory status epilepticus, super-refractory status epilepticus; non-convulsive status epilepticus, e.g., generalized status epilepticus, complex partial status epilepticus; generalized periodic epileptiform discharges; and periodic lateralized epileptiform discharges; prior to the onset of a seizure.

### Seizure

A seizure is the physical findings or changes in behavior that occur after an episode of abnormal electrical activity in the brain. The term "seizure" is often used interchangeably with "convulsion." Convulsions are when a person's body shakes rapidly and uncontrollably. During convulsions, the person's muscles contract and relax repeatedly.

Based on the type of behavior and brain activity, seizures are divided into two broad categories: generalized and partial (also called local or focal). Classifying the type of seizure helps doctors diagnose whether or not a patient has epilepsy.

Generalized seizures are produced by electrical impulses from throughout the entire brain, whereas partial seizures are produced (at least initially) by electrical impulses in a relatively small part of the brain. The part of the brain generating the seizures is sometimes called the focus.

There are six types of generalized seizures. The most common and dramatic, and therefore the most well known, is the generalized convulsion, also called the grand-mal seizure. In this type of seizure, the patient loses consciousness and usually collapses. The loss of consciousness is followed by generalized body stiffening (called the "tonic" phase of the seizure) for 30 to 60 seconds, then by violent jerking (the "clonic" phase) for 30 to 60 seconds, after which the patient goes into a deep sleep (the "postictal" or after-seizure phase). During grand-mal seizures, injuries and accidents may occur, such as tongue biting and urinary incontinence.

Absence seizures cause a short loss of consciousness (just a few seconds) with few or no symptoms. The patient, most often a child, typically interrupts an activity and stares blankly. These seizures begin and end abruptly and may occur several times a day. Patients are usually not aware that they are having a seizure, except that they may be aware of "losing time."

Myoclonic seizures consist of sporadic jerks, usually on both sides of the body. Patients sometimes describe the jerks as brief electrical shocks. When violent, these seizures may result in dropping or involuntarily throwing objects.

Clonic seizures are repetitive, rhythmic jerks that involve both sides of the body at the same time.

Tonic seizures are characterized by stiffening of the muscles.

Atonic seizures consist of a sudden and general loss of muscle tone, particularly in the arms and legs, which often results in a fall.

Seizures described herein can include epileptic seizures; acute repetitive seizures; cluster seizures; continuous seizures; unremitting seizures; prolonged seizures; recurrent seizures; status epilepticus seizures, e.g., refractory convulsive status epilepticus, non-convulsive status epilepticus seizures; refractory seizures; myoclonic seizures; tonic seizures; tonic-clonic seizures; simple partial seizures; complex partial seizures; secondarily generalized seizures; atypical absence seizures; absence seizures; atonic seizures; benign Rolandic seizures; febrile seizures; emotional seizures; focal seizures; gelastic seizures; generalized onset seizures; infantile spasms; Jacksonian seizures; massive bilateral myoclonus seizures; multifocal seizures; neonatal onset seizures; nocturnal seizures; occipital lobe seizures; post traumatic seizures; subtle seizures; Sylvan seizures; visual reflex seizures; or withdrawal seizures.

### Tremor

**Tremor** is an involuntary, at times rhythmic, muscle contraction and relaxation that can involve oscillations or twitching of one or more body parts (e.g., hands, arms, eyes, face, head, vocal folds, trunk, legs).

**Cerebellar tremor** or **intention tremor** is a slow, broad tremor of the extremities that occurs after a purposeful movement. Cerebellar tremor is caused by lesions in or damage to the cerebellum resulting from, *e.g.,* tumor, stroke, disease *(e.g.,* multiple sclerosis, an inherited degenerative disorder).

**Dystonic tremor** occurs in individuals affected by dystonia, a movement disorder in which sustained involuntary muscle contractions cause twisting and repetitive motions and/or painful and abnormal postures or positions. Dystonic tremor may affect any muscle in the body. Dystonic tremors occurs irregularly and often can be relieved by complete rest.

**Essential tremor** or benign essential tremor is the most common type of tremor. Essential tremor may be mild and nonprogressive in some, and may be slowly progressive, starting on one side of the body but affect both sides within 3 years. The hands are most often affected, but the head, voice, tongue, legs, and trunk may also be involved. Tremor frequency may decrease as the person ages, but severity may increase. Heightened emotion, stress, fever, physical exhaustion, or low blood sugar may trigger tremors and/or increase their severity.

**Orthostatic tremor** is characterized by fast *(e.g.,* greater than 12 Hz) rhythmic muscle contractions that occurs in the legs and trunk immediately after standing. Cramps are felt in the thighs and legs and the patient may shake uncontrollably when asked to stand in one spot. Orthostatic tremor may occurs in patients with essential tremor.

**Parkinsonian tremor** is caused by damage to structures within the brain that control movement. Parkinsonian tremor is often a precursor to Parkinson's disease and is typically seen as a "pill-rolling" action of the hands that may also affect the chin, lips, legs, and trunk. Onset of parkinsonian tremor typically begins after age 60. Movement starts in one limb or on one side of the body and can progress to include the other side.

**Physiological tremor** can occur in normal individuals and have no clinical significance. It can be seen in all voluntary muscle groups. Physiological tremor can be caused by certain drugs, alcohol withdrawl, or medical conditions including an overactive thyroid and hypoglycemia. The tremor classically has a frequency of about 10 Hz.

**Psychogenic tremor** or hysterical tremor can occur at rest or during postural or kinetic movement. Patient with psychogenic tremor may have a conversion disorder or another psychiatric disease.

**Rubral tremor** is characterized by coarse slow tremor which can be present at rest, at posture, and with intention. The tremor is associated with conditions that affect the red nucleus in the midbrain, classical unusual strokes.

### Mood disorders

**Clinical depression** is also known as major depression, major depressive disorder (MDD), severe depression, unipolar depression, unipolar disorder, and recurrent depression, and refers to a mental disorder characterized by pervasive and persistent low mood that is accompanied by low self-esteem and loss of interest or pleasure in normally enjoyable activities. Some people with clinical depression have trouble sleeping, lose weight, and generally feel agitated and irritable. Clinical depression affects how an individual feels, thinks, and behaves and may lead to a variety of emotional and physical problems. Individuals with clinical depression may have trouble doing day-to-day activities and make an individual feel as if life is not worth living.

**Postnatal depression (PND)** is also referred to as **postpartum depression (PPD)**, and refers to a type of clinical depression that affects women after childbirth. Symptoms can include sadness, fatigue, changes in sleeping and eating habits, reduced sexual desire, crying episodes, anxiety, and irritability. In some embodiments, the PND is a treatment-resistant depression (e.g., a treatment-resistant depression as described herein). In some embodiments, the PND is refractory depression (e.g., a refractory depression as described herein).

**Atypical depression (AD)** is characterized by mood reactivity (e.g., paradoxical anhedonia) and positivity, significant weight gain or increased appetite. Patients suffering from AD also may have excessive sleep or somnolence (hypersomnia), a sensation of limb heaviness, and significant social impairment as a consequence of hypersensitivity to perceived interpersonal rejection.

**Melancholic depression** is characterized by loss of pleasure (anhedonia) in most or all activities, failures to react to pleasurable stimuli, depressed mood more pronounced than that of grief or loss, excessive weight loss, or excessive guilt.

**Psychotic major depression (PMD)** or psychotic depression refers to a major depressive episode, in particular of melancholic nature, where the individual experiences psychotic symptoms such as delusions and hallucinations.

**Catatonic depression** refers to major depression involving disturbances of motor behavior and other symptoms. An individual may become mute and stuporose, and either is immobile or exhibits purposeless or bizarre movements.

**Seasonal affective disorder (SAD)** refers to a type of seasonal depression wherein an individual has seasonal patterns of depressive episodes coming on in the fall or winter.

**Dysthymia** refers to a condition related to unipolar depression, where the same physical and cognitive problems are evident. They are not as severe and tend to last longer *(e.g.,* at least 2 years).

**Double depression** refers to fairly depressed mood (dysthymia) that lasts for at least 2 years and is punctuated by periods of major depression.

**Depressive Personality Disorder (DPD)** refers to a personality disorder with depressive features.

**Recurrent Brief Depression (RBD)** refers to a condition in which individuals have depressive episodes about once per month, each episode lasting 2 weeks or less and typically less than 2-3 days.

**Minor depressive disorder** or minor depression refers to a depression in which at least 2 symptoms are present for 2 weeks.

**Bipolar disorder or manic depressive disorder** causes extreme mood swings that include emotional highs (mania or hypomania) and lows (depression). During periods of mania the individual may feel or act abnormally happy, energetic, or irritable. They often make poorly thought out decisions with little regard to the consequnces. The need for sleep is usually reduced. During periods of depression there may be crying, poor eye contact with others, and a negative outlook on life. The risk of suicide among those with the disorder is high at greater than 6% over 20 years, while self harm occurs in 30-40%. Other mental health issues such as anxiety disorder and substance use disorder are commonly associated with bipolar disorder.

**Depression caused by chronic medical conditions** refers to depression caused by chronic medical conditions such as cancer or chronic pain, chemotherapy, chronic stress.

**Treatment-resistant depression** refers to a condition where the individuals have been treated for depression, but the symptoms do not improve. For example, antidepressants or physchological counseling (psychotherapy) do not ease depression symptoms for individuals with treatment-resistant depression. In some cases, individuals with treatment-resistant depression improve symptoms, but come back. **Refractory depression** occurs in patients suffering from depression who are resistant to standard pharmacological treatments, including tricyclic antidepressants, MAOIs, SSRIs, and double and triple uptake inhibitors and/or anxiolytic drugs, as well as non-pharmacological treatments (e.g., psychotherapy, electroconvulsive therapy, vagus nerve stimulation and/or transcranial magnetic stimulation).

**Suicidality, suicidal ideation, suicidal behavior** refers to the tendency of an individual to commit suicide. Suicidal ideation concerns thoughts about or an unusual preoccupation with suicide. The range of suicidal ideation varies greatly, from e.g., fleeting thoughts to extensive thoughts, detailed planning, role playing, incomplete attempts. Symptoms include talking about suicide, getting the means to commit suicide, withdrawing from social contact, being preoccupied with death, feeling trapped or hopeless about a situation, increasing use of alcohol or drugs, doing risky or self-destructive things, saying goodbye to people as if they won't be seen again.

**Symptoms** of depression include persistent anxious or sad feelings, feelings of helplessness, hopelessness, pessimism, worthlessness, low energy, restlessness, irritability, fatigue, loss of interest in pleasurable activities or hobbies, absence of positive thoughts or plans, excessive sleeping, overeating, appetite loss, insomnia,self-harm, thoughts of suicide, and suicide attempts. The presence, severity, frequency, and duration of symptoms may vary on a case to case basis. Symptoms of depression, and relief of the same, may be ascertained by a physician or psychologist (e.g., by a mental state examination).

### Anesthesia / Sedation

Anesthesia is a pharmacologically induced and reversible state of amnesia, analgesia, loss of responsiveness, loss of skeletal muscle reflexes, decreased stress response, or all of these simultaneously. These effects can be obtained from a single drug which alone provides the correct combination of effects, or occasionally with a combination of drugs (e.g., hypnotics, sedatives, paralytics, analgesics) to achieve very specific combinations of results. Anesthesia allows patients to undergo surgery and other procedures without the distress and pain they would otherwise experience.

Sedation is the reduction of irritability or agitation by administration of a pharmacological agent, generally to facilitate a medical procedure or diagnostic procedure.

Sedation and analgesia include a continuum of states of consciousness ranging from minimal sedation (anxiolysis) to general anesthesia.

**Minimal sedation is a**lso known as anxiolysis. Minimal sedation is a drug-induced state during which the patient responds normally to verbal commands. Cognitive function and coordination may be impaired. Ventilatory and cardiovascular functions are typically unaffected.

**Moderate sedation/analgesia (conscious sedation) is a** drug-induced depression of consciousness during which the patient responds purposefully to verbal command, either alone or accompanied by light tactile stimulation. No interventions are usually necessary to maintain a patent airway. Spontaneous ventilation is typically adequate. Cardiovascular function is usually maintained.

**Deep sedation/analgesia is a** drug-induced depression of consciousness during which the patient cannot be easily aroused, but responds purposefully (not a reflex withdrawal from a painful stimulus) following repeated or painful stimulation. Independent ventilatory function may be impaired and the patient may require assistance to maintain a patent airway. Spontaneous ventilation may be inadequate. Cardiovascular function is usually maintained.

**General anesthesia is a** drug-induced loss of consciousness during which the patient is not arousable, even to painful stimuli. The ability to maintain independent ventilatory function is often impaired and assistance is often required to maintain a patent airway. Positive pressure ventilation may be required due to depressed spontaneous ventilation or drug-induced depression of neuromuscular function. Cardiovascular function may be impaired.

Sedation in the intensive care unit (ICU) allows the depression of patients' awareness of the environment and reduction of their response to external stimulation. It can play a role in the care of the critically ill patient, and encompasses a wide spectrum of symptom control that will vary between patients, and among individuals throughout the course of their illnesses. Heavy sedation in critical care has been used to facilitate endotracheal tube tolerance and ventilator synchronization, often with neuromuscular blocking agents.

In some embodiments, sedation (*e*.*g*., long-term sedation, continuous sedation) is induced and maintained in the ICU for a prolonged period of time (*e.g.,* 1 day, 2 days, 3 days, 5 days, 1 week, 2 week, 3 weeks, 1 month, 2 months). Long-term sedation agents may have long duration of action. Sedation agents in the ICU may have short elimination half-life.

Procedural sedation and analgesia, also referred to as conscious sedation, is a technique of administering sedatives or dissociative agents with or without analgesics to induce a state that allows a subject to tolerate unpleasant procedures while maintaining cardiorespiratory function.

### Examples

In order that the invention described herein may be more fully understood, the following examples are set forth. The synthetic and biological examples described in this application are offered to illustrate the compounds, pharmaceutical compositions and methods provided herein and are not to be construed in any way as limiting.

### Materials and Methods

The compounds provided herein can be prepared from readily available starting materials using the following general methods and procedures. It will be appreciated that where typical or preferred process conditions (*i.e.*, reaction temperatures, times, mole ratios of reactants, solvents, pressures, *etc*.) are given, other process conditions can also be used unless otherwise stated. Optimum reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include (but are not limited to) recrystallization, column chromatography, HPLC, or supercritical fluid chromatography (SFC). The following schemes are presented with details as to the preparation of representative triazole and tetrazoles that have been listed herein. The compounds provided herein may be prepared from known or commercially available starting materials and reagents by one skilled in the art of organic synthesis. Exemplary chiral columns available for use in the separation/purification of the enantiomers/diastereomers provided herein include, but are not limited to, CHIRALPAK^{®} AD-10, CHIRALCEL^{®} OB, CHIRALCEL^{®} OB-H, CHIRALCEL^{®} OD, CHIRALCEL^{®} OD-H, CHIRALCEL^{®} OF, CHIRALCEL^{®} OG, CHIRALCEL^{®} OJ and CHIRALCEL^{®} OK.

**¹H-NMR** reported herein (e.g., for intermediates) may be a partial representation of the full NMR spectrum of a compound, e.g., a compound described herein. For example, the reported **¹H NMR** may exclude the region between δ (ppm) of about 1 to about 2.5 ppm. Copies of full ¹H-NMR spectrum for representative examples are provided in the **Figures**.

Exemplary general method for preparative HPLC: Column: Waters RBridge prep 10 µm C18, 19^{∗}250 mm. Mobile phase: aectonitrile, water (NH₄HCO₃) (30 L water, 24 g NH₄HCO₃, 30 mL NH₃.H₂O). Flow rate: 25 mL/min

Exemplary general method for analytical HPLC: Mobile phase: A: water (10 mM NH₄HCO₃), B: acetonitrileGradient: 5%-95% B in 1.6 or 2 min Flow rate: 1.8 or 2 mL/min; Column: XBridge C18, 4.6^{∗}50mm, 3.5 µm at 45 C.

### Synthetic Procedures

The compounds of the invention and as described herein can be prepared in accordance with methods described in the art (Upasani et al., J. Med. Chem. 1997, 40:73-84; and Hogenkamp et al., J. Med. Chem. 1997, 40:61-72) and using the appropriate reagents, starting materials, and purification methods known to those skilled in the art. In some embodiments, compounds described herein can be prepared using methods shown in general Schemes 1-3, comprising a nucleophilic substitution of 19-nor pregnane bromide with a neucleophile. In certain embodiments, the nucleophile reacts with the 19-nor pregnane bromide in the presence of K₂CO₃ in THF.

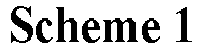

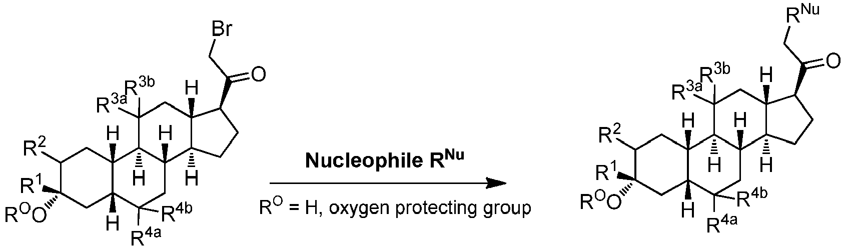

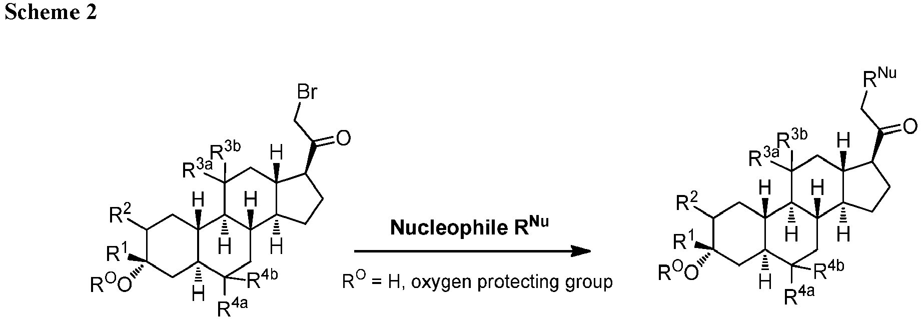

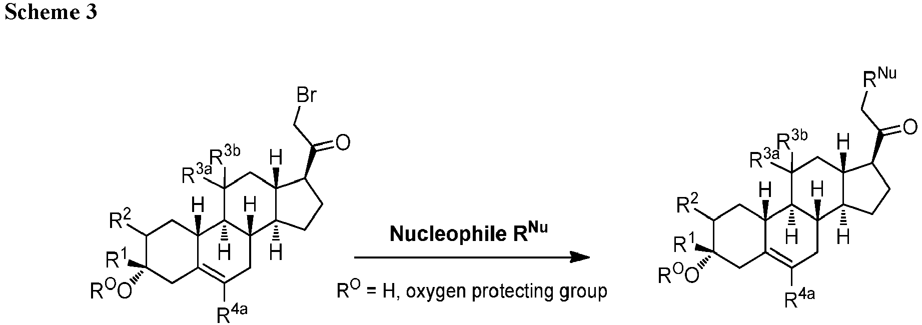

### Reference Example 1. Synthesis of SA and SA intermediates

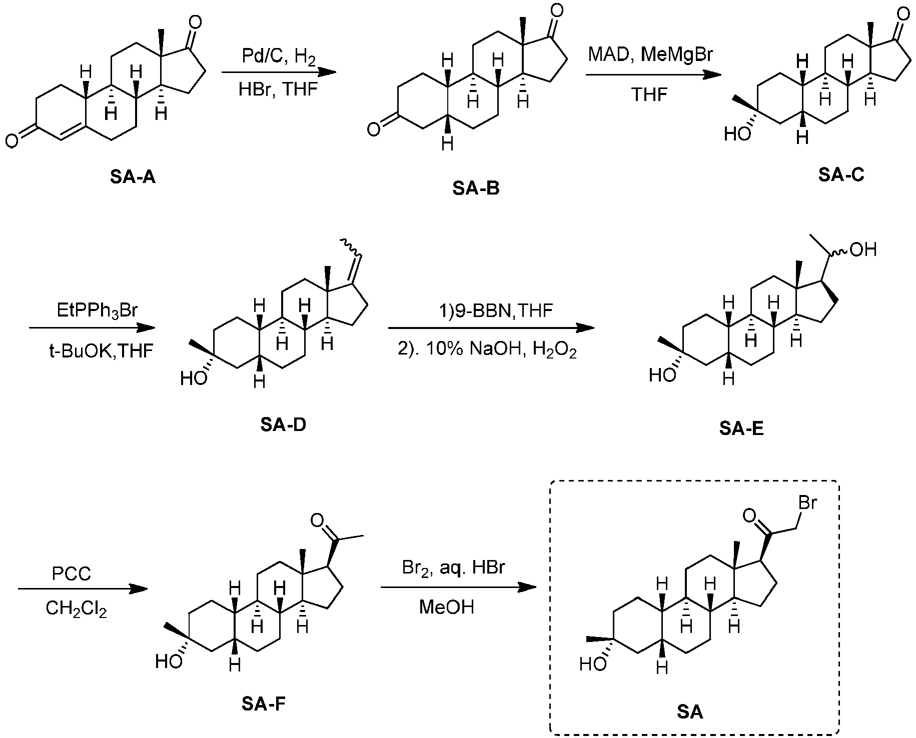

**Synthesis of compound SA-B**. Compound SA-A (50 g, 184 mmol) and palladium black (2.5 g) in tetrahydrofuran (300 mL) and concentrated hydrobromic acid (1.0 mL) was hydrogenated with 10 atm hydrogen. After stirring at room temperature for 24h, the mixture was filtered through a pad of celite and the filtrate was concentrated in vacuo to afford the crude compound. Recrystallization from acetone gave compound **SA-B** (42.0 g, yield: 83.4%) as white powder. **¹H NMR:** (400 MHz, CDCl3) δ 2.45-2.41 (m, 1H), 2.11-3.44 (m, 2H), 3.24 (s, 3H), 2.18-2.15 (m, 1H), 2.01-1.95 (m, 1H), 1.81-1.57 (m, 7H), 1.53-1.37 (m, 7H), 1.29-1.13 (m, 3H), 1.13-0.90 (m, 2H), 0.89 (s, 3H).

**Synthesis of compound SA-C.** A solution of **SA-B** (42.0 g, 153.06 mmol) in 600 mL anhydrous toluene was added dropwise to the methyl aluminum bis(2,6-di-tert-butyl-4-methylphenoxide (MAD) (459.19 mmol, 3.0 eq, freshly prepared) solution under N₂ at -78°C. After the addition was completed, the reaction mixture was stirred for 1 hr at -78°C. Then 3.0 M MeMgBr (153.06 mL, 459.19 mmol) was slowly added dropwise to the above mixture under N₂ at -78°C. Then the reaction mixture was stirred for 3 hr at this temperature. TLC (Petroleum ether/ethyl acetate = 3:1) showed the reaction was completed. Then saturated aqueous NH₄Cl was slowly added dropwise to the above mixture at -78°C. After the addition was completed, the mixture was filtered, the filter cake was washed with EtOAc, the organic layer was washed with water and brine, dried over anhydrous Na₂SO₄, filtered and concentrated, purified by flash Chromatography on silica gel (Petroleum ether/ ethyl acetate20:1 to 3:1) to afford compound **SA-C** (40.2 g, yield: 90.4%) as white powder. **¹H NMR:** (400 MHz, CDCl3) δ 2.47-2.41 (m, 1H), 2.13-2.03 (m, 1H), 1.96-1.74 (m, 6H), 1.70-1.62 (m, 1H), 1.54-1.47 (m, 3H), 1.45-1.37 (m, 4H), 1.35-1.23 (m, 8H), 1.22-1.10 (m, 2H), 1.10-1.01 (m, 1H), 0.87 (s, 3H).

**Synthesis of compound SA-D.** To a solution of PPh₃EtBr (204.52 g, 550.89 mmol) in THF (500 mL) was added a solution of t-BuOK (61.82 g, 550.89 mmol) in THF (300 mL) at 0 °C. After the addition was completed, the reaction mixture was stirred for 1 h 60°C, then **SA-C** (40.0 g, 137.72 mmol) dissolved in THF (300 mL) was added dropwise at 60°C. The reaction mixture was heated to 60°C for 18 h. The reaction mixture was cooled to room temperature and quenched with Sat. NH₄Cl, extracted with EtOAc (3^{∗}500 mL). The combined organic layers were washed with brine, dried and concentrated to give the crude product, which was purified by a flash column chromatography (Petroleum ether/ ethyl acetate50:1 to 10:1) to afford compound **SA-D** (38.4 g, yield: 92%) as a white powder. **¹H NMR:** (400 MHz, CDCl3) δ 5.17-5.06 (m, 1H), 2.42-2.30 (m, 1H), 2.27-2.13 (m, 2H), 1.89-1.80 (m, 3H), 1.76-1.61 (m, 6H), 1.55-1.43 (m, 4H), 1.42-1.34 (m, 3H), 1.33-1.26 (m, 6H), 1.22-1.05 (m, 5H), 0.87 (s, 3H).

**Synthesis of compound SA-E**. To a solution of **SA-D** (38.0 g, 125.62 mmol) in dry THF (800 mL) was added dropwise a solution of BH₃.Me₂S (126 mL, 1.26 mol) under ice-bath. After the addition was completed, the reaction mixture was stirred for 3 h at room temperature (14-20 °C). TLC (Petroleum ether/ ethyl acetate3:1) showed the reaction was completed. The mixture was cooled to 0 °C and 3.0 M aqueous NaOH solution (400 mL) followed by 30% aqueous H₂O₂ (30%, 300 mL) was added. The mixture was stirred for 2 h at room temperature (14-20 °C), and then filtered, extracted with EtOAc (3^{∗}500 mL). The combined organic layers were washed with saturated aqueous Na₂S₂O₃, brine, dried over Na₂SO₄ and concentrated in vacuum to give the crude product (43 g , crude) as colorless oil. The crude product was used in the next step without further purification.

**Synthesis of compound SA-F.** To a solution of **SA-E** (43.0 g, 134.16 mmol) in dichloromethane (800 mL) at 0 °C and PCC (53.8 g, 268.32 mmol) was added portion wise. Then the reaction mixture was stirred at room temperature (16-22 °C) for 3 h. TLC (Petroleum ether/ ethyl acetate3:1) showed the reaction was completed, then the reaction mixture was filtered, washed with DCM. The organic phase was washed with saturated aqueous Na₂S₂O₃, brine, dried over Na₂SO₄ and concentrated in vacuum to give the crude product. The crude product was purified by a flash column chromatography (Petroleum ether/ ethyl acetate50:1 to 8:1) to afford compound **SA-F** (25.0 g, yield:62.5%, over two steps) as a white powder. **¹H NMR (SA-F):** (400 MHz, CDCl3) δ 2.57-2.50 (m, 1H), 2.19-2.11 (m, 4H), 2.03-1.97 (m, 1H), 1.89-1.80 (m, 3H), 1.76-1.58 (m, 5H), 1.47-1.42 (m, 3H), 1.35-1.19 (m, 10H), 1.13-1.04 (m, 3H), 0.88-0.84 (m, 1H), 0.61 (s, 3H).

**Synthesis of compound SA.** To a solution of **SA-F** (10 g, 31.4 mmol) and aq. HBr (5 drops, 48% in water) in 200 mL of MeOH was added dropwise bromine (5.52 g, 34.54 mmol). The reaction mixture was stirred at 17 °C for 1.5 h. The resulting solution was quenched with saturated aqueous NaHCO₃ at 0°C and extracted with EtOAc (150 mL×2). The combined organic layers were dried and concentrated. The residue was purified by column chromatography on silica gel eluted with (PE: EA=15:1 to 6:1) to afford compound **SA** (9.5 g, yield: 76.14%) as an off white solid. LC/MS: rt 5.4 min ; m/z 379.0, 381.1, 396.1.

### Reference Example 2. Synthesis of compound SA-1.

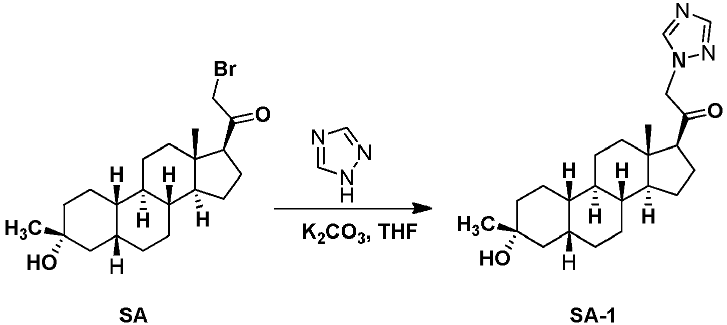

To a suspension of K₂CO₃ (25 mg, 0.18mmol) in THF (5 mL) was added 3H-1,2,4-triazole ( 32mg, 0.46 mmol) and **SA** ( 36 mg, 0.09 mmol). The mixture was stirred at rt for 24h. The reaction mixture was poured in to 5 mL H₂O and extracted with EtOAc (2 × 10 mL). The combined organic layers were washed with brine, dried over sodium sulfate, filtered and concentrated. The residure was purified with by reverse-phase prep-HPLC to afford the title compound as an off white solid (11mg,31.3% )

**¹HNMR** (400 MHz, CDCl₃), δ (ppm), 7.67 (s, 1H), 7.64 (s, 1H), 5.27(AB,1H), 4.18(AB,1H) 2.65(1H, t), 1.27(s, CH₃), 0.67 (s, 3H).

### Reference Example 3. Synthesis of compound SA-2.

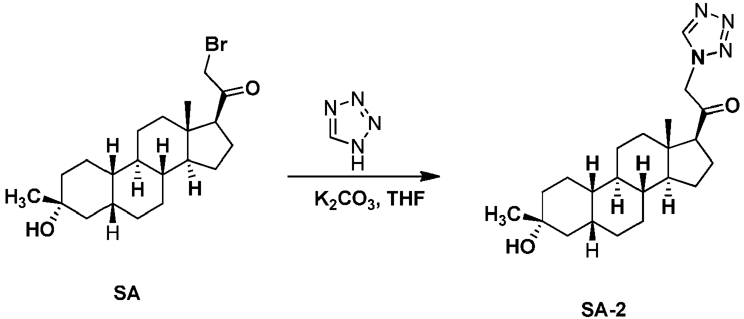

To a suspension of K₂CO₃ (25 mg, 0.18mmol) in THF (5 mL) was added 1H-tetrazole (16 mg, 0.23 mmol) and **SA** (70 mg, 0.09 mmol). The mixture was stirred at rt for 15h. The reaction mixture was poured in to 5 mL H₂O and extracted with EtOAc (2 × 10 mL). The combined organic layers were washed with brine, dried over sodium sulfate, filtered and concentrated. The residue was purified with by reverse-phase prep-HPLC to afford the title compound as an off white solid, **SA-2** (8mg, 11.7%), and a byproduct (10mg, 14.0%).

**SA-2: ¹HNMR** (500 MHz, CDCl₃), δ (ppm), 8.74 (s, 1H), 5.31(AB,1H),5.17(AB,1H), 2.65(1H, t), 1.28(s, CH₃), 0.67 (s, 3H).

### Reference Example 4. Synthesis of compound SA-3.

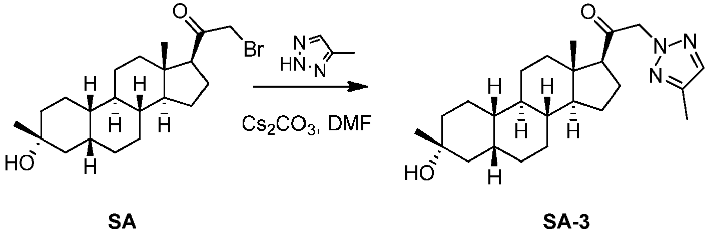

To a suspension of **SA** (1 g, 2.52 mmol) in DMF (20 mL) was added K₂CO₃ (1.04 g, 7.55 mmol) and 4-methyl-2H-1,2,3-triazole (313.64 mg, 3.77 mmol). The mixture was stirred at room temperature for 3h. Then the reaction mixture was poured into 5 mL H₂O and extracted with EtOAc (30 mL). The combined organic layers were washed with brine (10 mL^{∗}3), dried over sodium sulfate, filtered and concentrated. The residue was purified with by prep-HPLC to afford the title compound **SA-3** ( 269.2 mg, Yield=26.59%) as an off white solid. **¹HNMR** (**SA-3**) (400 MHz, CDCl₃) δ 7.42 (s, 1H), 5.14-5.13 (m, 2H), 2.57-2.56 (m, 1H), 2.33 (s, 3H), 2.01-2.00 (m, 2H), 1.81-1.70 (m, 6H), 1.45-1.39 (m, 7H), 1.27-1.24 (m, 9H), 1.01-1.00 (m, 3H), 0.70 (s , 3H).

### Reference Example 5. Synthesis of compounds SA-4 and SA-5.

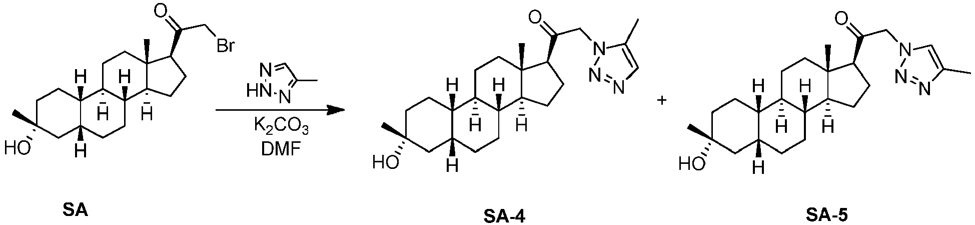

To a solution of 4-methyl-2H-1,2,3-triazole (836.4 mg, 10.07 mmol) and K₂CO₃ (1.39 g, 10.07 mmol) in DMF (20 mL) was added compound **SA** (2.0 g, 5.03 mmol) at room temperature (13-17 °C) under N₂. The reaction mixture was stirred at room temperature (13-17 °C) for 4 h. TLC showed the reaction was completed. Then the reaction mixture was poured into water and extracted with EtOAc (50 mLx3). The combined organic layers were washed with brine, dried over anhydrous Na₂SO₄, filtered and concentrated in vacuum. The residue was purified by silica gel to afford 730 mg mixture of **SA-4/SA-5** and a byproduct (500 mg, yield :25%). The mixture was split by SFC purification to give **SA-4** (249.8 mg, yield: 12.5%) and **SA-5** (426.2 mg, yield:21.3%) as off white solid. **¹H NMR (SA-4):** (400 MHz, CDCl3) δ 7.49 (s, 1H), 5.14-5.02 (m, 2H), 2.67-2.63 (m, 1H), 2.21-2.16 (m, 4H), 2.11-2.08 (m, 1H), 1.88-1.75 (m, 6H), 1.65-1.55 (m, 1H), 1.51-1.37 (m, 7H), 1.33-1.22 (m, 8H), 1.14-1.08 (m, 3H), 0.69 (s, 3H). **¹H NMR (SA-5):** (400 MHz, CDCl3) δ 7.35 (s, 1H), 5.20-5.04 (m, 2H), 2.65-2.61 (m, 1H), 2.38 (s, 3H), 2.25-2.17 (m, 1H), 2.09-2.05 (m, 1H), 1.88-1.63 (m, 7H), 1.50-1.28 (m, 15H), 1.15-1.06 (m, 3H), 0.67 (s, 3H).

### Reference Example 6. Synthesis of compounds SA-6 and SA-7.

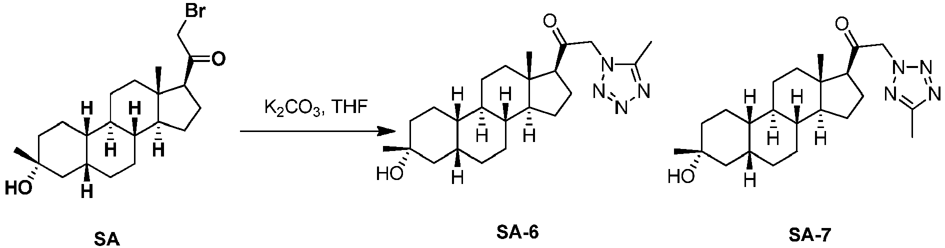

To a solution of compound **SA** (120 mg, 0.29 mmol) in THF (3 mL) was added K₂CO₃ (210 mg, 1.5 mmol) and 5-methyl-2H-tetrazole (126 mg, 1.5 mmol). The resulting solution was stirred at room temperature overnight when LCMS analysis showed the reaction to be complete. The reaction was then diluted with EtOAc (20 mL) and the resulting solution was washed with brine (10 mL), dried over Na₂SO₄ and concentrated *in vacuo.* The residue was purified by prep-HPLC to give **SA-6** (10 mg, 0.025 mmol, Yield=8%), **SA-7** (8 mg, 0.020 mmol, Yield=7%) as an off white solid. **SA-6: ¹H NMR** (400 MHz, CDCl3) δ 5.16-5.03 (m, 2H), 2.66 (t, 1H), 2.46 (s, 3 H), 2.25-2.10 (m, 1H), 2.08-2.02 (m, 1H), 1.90-1.70 (m, 7H), 1.68-1.02 (m, 18H),0.67 (s, 3 H). **LC-MS:** rt=2.20 min; m/z=401.3 (M+H)⁺. **SA-7: ¹H NMR:** (400 MHz, CDCl3) δ 5.40-5.30 (m, 2H), 2.62 (t, 1H), 2.55 (s, 3 H), 2.30-2.00 (m, 2H), 1.90-1.56 (m, 7H), 1.50-1.02 (m, 18H), 0.70 (s, 3 H). **LC-MS:** rt=2.30 min; m/z=401.2 (M+H)⁺

### Reference Example 7. Synthesis of compound SA-8.

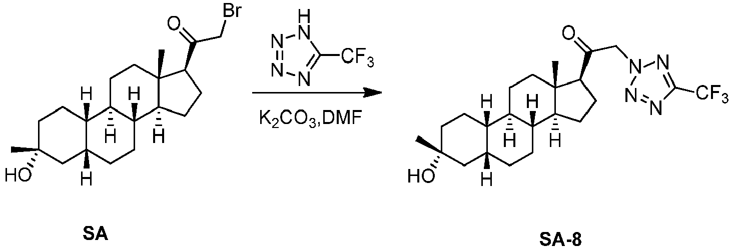

To a solution of compound **SA** (150 mg, 0.377 mmol) and K₂CO₃ (104.3 mg, 0.755 mmol) in dry DMF (10 mL) was added 5-(trifluoromethyl)-1H-tetrazole (104.2 mg, 0.755 mmol) under N₂ at room temperature (14-20°C). The reaction mixture was stirred for 18h at the same temperature. The reaction mixture was poured to water, extracted with EtOAc (50 mLx3). The organic layers were washed with brine, dried over anhydrous Na₂SO₄, filtered and concentrated in vacuum. The residue was purified by silica gel column (PE:EtOAc=10: 1 to 1:1) to afford **SA-8** (89.1 mg, yield: 51.9%) as a white powder. **¹H NMR (SA-8):** (400 MHz, CDCl3) δ 5.51 (s, 2H), 2.69-2.65 (m, 1H), 2.26-2.18 (m, 1H), 2.09-2.05 (m, 1H), 1.87-1.77 (m, 6H), 1.69-1.62 (m, 1H), 1.55-1.43 (m, 7H), 1.37-1.26 (m, 8H), 1.19-1.09 (m, 3H), 0.72 (s, 3H).

### Reference Example 8. Synthesis of compound SA-9.

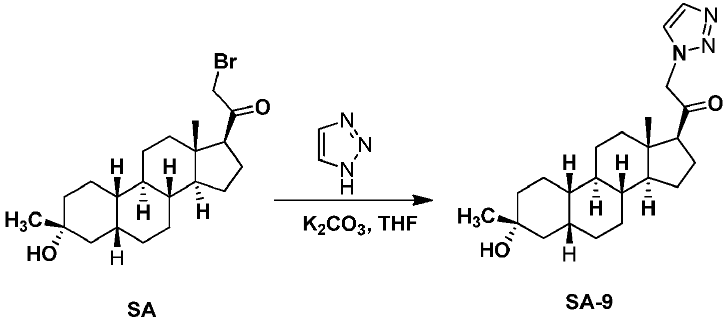

To a suspension of K₂CO₃ (25 mg, 0.18mmol) in THF (5 mL) was added 3H-1,2,4-triazole (16 mg, 0.23 mmol) and **SA** (70 mg, 0.09 mmol). The mixture was stirred at rt for 15h. The reaction mixture was poured into 5 mL H₂O and extracted with EtOAc (2 × 10 mL). The combined organic layers were washed with brine, dried over sodium sulfate, filtered and concentrated. The residue was purified with by reverse-phase prep-HPLC to afford the title compound as an off white solid, **SA-9** (15mg,22% ). **SA-9: ¹HNMR** (400 MHz, CDCl₃), δ (ppm), 7.76 (s, 1H), 7.64 (s, 1H), 5.27(AB,1H),5.14(AB,1H), 2.65(1H, t), 1.27(s, 3H), 0.67 (s, 3H).

### Reference Example 9. Synthesis of SC-SS and SC-SS intermediates

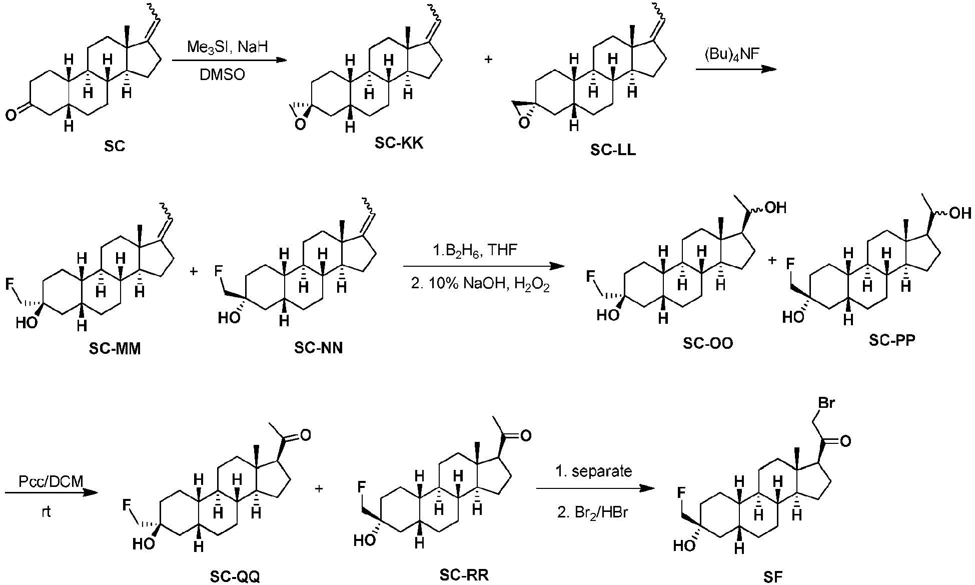

**Synthesis of compound SC-KK** and **SC-LL**. To a stirred solution of trimethylsufoxonium iodide (43 g , 210 mmol) in 200 mL of DMSO was added NaH (60%, 8.4 g, 210 mmol). After stirring at room temperature for 1h, a suspension of Compound **SC** (30 g, 105 mmol) in 20 mL of DMSO was added dropwise. After 2.5 h, the reaction mixture was poured into ice-cold water and extracted with ethyl acetate (100 mL×3).The combined ethyl acetate layers were then washed with brine (100 mL×3), dried with MgSO4, filtered, and concentrated. The residue was purified by column chromatography (petroleum ether/ethyl acetate = 20:1 to 15:1) to afford compound **SC-LL** (14.7 g, 49 mmol, 47%).

**Synthesis of compound SC-MM** and **SC-NN.** A mixture of reactant mixture **SA-KK** and **SA-LL** (3.0g, 10.0mmol, 1:1) was added dry (Bu)4NF, then the mixture was heated 100 °C overnight. The residual mixture was poured in to 50 mL H₂O and extracted with EtOAc (2 × 50 mL). The combined organic layers were washed with brine solution, dried over sodium sulfate, filtered and concentrated. The residue was purified by flash chromatography ( petroleum ether/ ethyl acetate=20:1) to afford product mixture **SC-MM** and **SC-NN** (2.1g, 6.5 mmol, 65%) as off white solid.

**Synthesis of compound SC-OO** and **SC-PP.** To a solution of reactant mixture **SC-MM** and **SC-NN** (2.1g, 6.5 mmol) in anhydrous THF (30 mL) was added BH₃.THF (1.0 M, 13.0 mL, 13.0 mmol), the solution was stirred at 25 °C overnight. Then the reaction was quenched by addition of water (5 mL). 2 M NaOH solution (20 mL) was added followed by 30 % H₂O₂ (20 mL). The mixture was stirred at room temperature for 1 hour. The mixture was diluted with ethyl acetate (200 mL) and resulting solution was washed with brine (2×100 mL), dried over magnesium sulfate and concentrated *in vacuo.* The crude product mixture was used directly in the next step without further purification.

**Synthesis of compound SC-QQ and SC-RR.** To a solution of crude reactant mixture **SC-OO** and **SC-PP** (2.2g, 6.5 mmol, theoretical amount) in dichloromethane (40 mL) was added Pyridinium chlorochromate (Pcc) in portions (2.8g, 13.0 mmol). The solution was stirred at 25 °C overnight. Then the mixture was filtered through a short pad of silica gel and the silica gel was washed with dichloromethane (3×50 mL). All filtrate was combined and concentrated *in vacuo.* The residue was purified by flash chromatography ( petroleum ether/ ethyl acetate=15:1) to afford product **SC-QQ** (910 mg, 2.7 mmol, Yield=41% (2 steps)) as off white solid and product **SC-RR** (850 mg, 2.5 mmol, Yield=39% (2 steps)) as off white solid. **Compound SC-QQ: ¹HNMR** (500 MHz, CDCl3) δ(ppm): 4.17 (d, 2H), 2.53 (t, 1H), 2.17-2.13 (m, 2H), 2.11 (s, 3H), 2.03-2.00 (m, 1H), 0.62 (s, 3H). **Compound SC-RR: ¹HNMR** (500 MHz, CDCl3) δ(ppm): 4.45 (AB×d, 1H), 4.39 (AB×d, 1H), 2.54 (t, 1H), 0.62 (s, 3H).

**Synthesis of compound SF.** To a solution of reactant **SC-RR** (100 mg, 0.301 mmol) in methanol (10 mL) was added 48% hydrobromic acid (152 mg, 0.903 mmol) followed by bromine (241 mg, 0.077 mL, 1.505 mmol). The solution was heated at 25 °C for 1.5 hours. Then the mixture was poured into cooled water (50 mL). The resulting solid was extracted with ethyl acetate (2×50 mL). The combined organic extracts were washed with brine (50 mL), dried over magnesium sulfate and concentrated *in vacuo.* The crude product **SF** was used directly without further purification in the next step.

### Reference Example 10. Synthesis of compound SF-1.

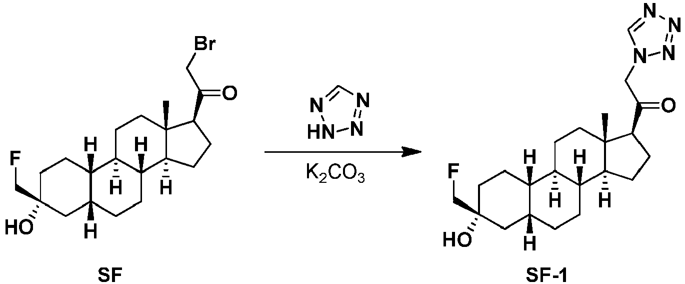

To a suspension of K₂CO₃ (55 mg, 0.4 mmol) in THF (5 mL) was added 2H-tetrazole (28mg, 0.4mmol) and Compound **SF** (83 mg, 0.2 mmol). The mixture was stirred at RT for 15h then the residue mixture was poured into 5 mL H₂O and extracted with EtOAc (2 × 10 mL). The combined organic layers were washed with brine, dried over sodium sulfate, filtered and concentrated. The residue mixture was purified by reverse-phase prep-HPLC to afford **SF-1** as an off white solid (6 mg,8%). **SF-1**: ¹HNMR (500 MHz, CDCl3) δ (ppm): 8.75 (s, 1H), 5.32 (AB, 1H), 5.19 (AB, 1H), 4.48 (AB×d, 1H), 4.38 (AB×d, 1H), 2.68 (t, 1H), 0.68 (s, 3H). **LC-MS**: rt = 2.10 min, m/z = 405.4 [M+H]⁺

### Reference Example 11. Synthesis of compounds SF-2 and SF-3.

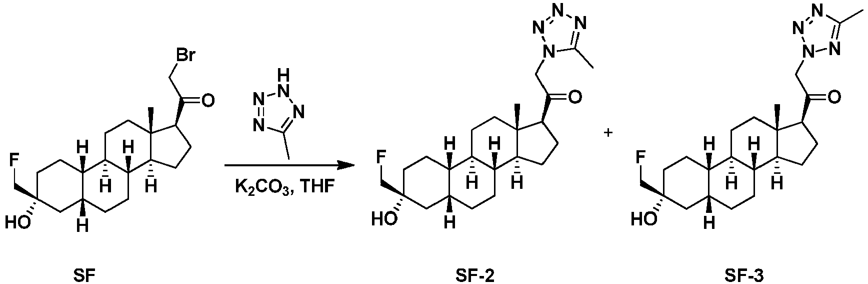

To a suspension of K₂CO₃ (55mg, 0.4mmol) in THF (5mL) was added 5-methyl-2H-tetrazole (33.6mg, 0.4mmol) and **SF** (85 mg, 0.2mmol) and the mixture was stirred at RT for 15h. The residue mixture was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SF-2** as an off white solid (22mg,26% ) and **SF-3** as an off white solid (38mg, 45%). **SF-2:** ¹HNMR (500 MHz, CDCl3) δ (ppm): 5.15 (AB, 1H), 5.06 (AB, 1H), 4.48 (AB×d, 1H), 4.39 (AB×d, 1H), 2.68 (t, 1H), 2.47 (s, 3H), 0.69 (s, 3H). **LC-MS**: rt = 2.09 min, m/z = 419.3 [M+H]⁺. **SF-3**: ¹HNMR (500 MHz, CDCl3) δ (ppm): 5.35 (t, 2H), 4.48 (AB×d, 1H), 4.38 (AB×d, 1H), 2.63 (t, 1H), 2.56 (s, 3H), 2.25-2.18 (m, 2H), 2.10-2.04 (m, 1H), 0.72 (s, 3H). **LC-MS**: rt = 2.20 min, m/z = 419.1 [M+H]⁺

### Reference Example 12. Synthesis of compounds SF-4.

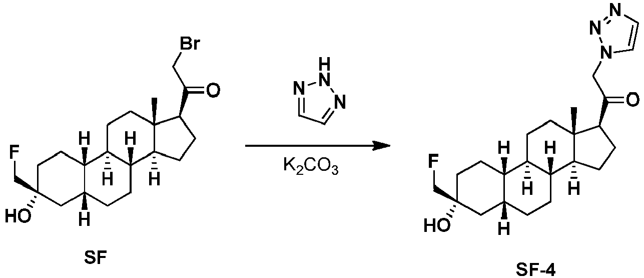

To a suspension of K₂CO₃ (55 mg, 0.4 mmol) in THF (5 mL) was added 2H-1,2,3-triazole (28 mg, 0.4 mmol) and Compound **SF** (85 mg, 0.2 mmol). The mixture was stirred at RT for 15h then the residue mixture was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SF-4** as an off white solid (12 mg, 15%). Compound **SF-4: ¹HNMR** (500 MHz, CDCl3) δ (ppm): 7.76 (d, 1H), 7.64 (d, 1H), 5.28 (AB, 1H), 5.14 (AB, 1H), 4.48 (AB × d, 1H), 4.38 (AB × d, 1H), 2.66 (t, 1H), 2.25 (s, 1H), 2.23-2.20 (m, 1H), 2.11-2.08 (m, 1H), 0.68 (s, 3H). **LC-MS**: rt = 2.05 min, m/z = 404.3 [M+H]⁺

### Reference Example 13. Synthesis of SG and SG intermediates.

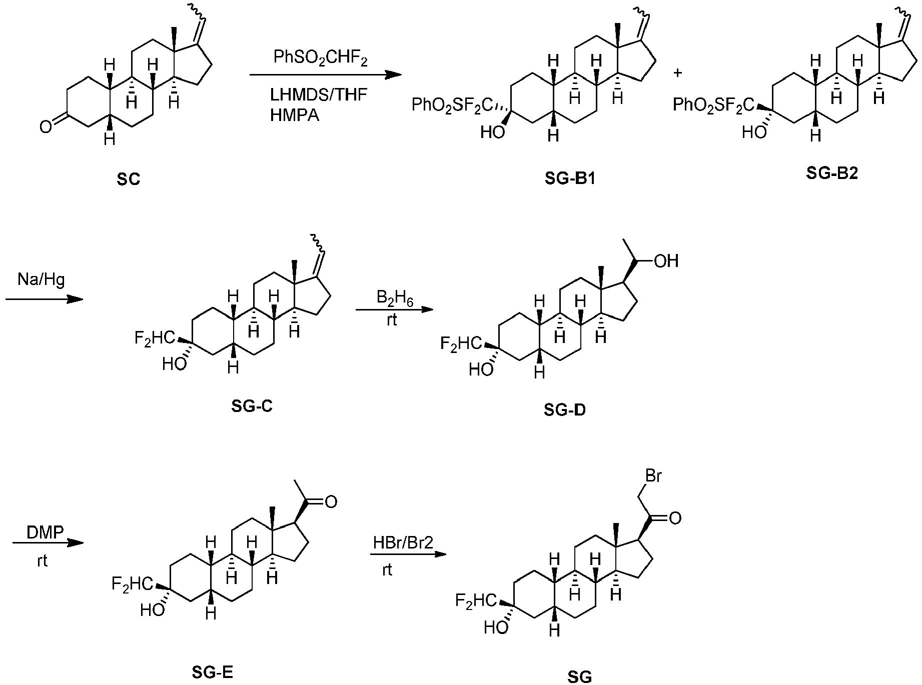

**Synthesis of compounds SG-B1 and SG-B2.** To a solution of compound **SC** (800 mg, 2.79 mmol) and PhSO₂CF₂H (540 mg, 2.79 mmol) in THF (25 mL) and HMPA (0.5 mL) at -78 °C under N₂ was added LHMDS (4 mL, 1M in THF) dropwise. After stirring at -78 °C for 2 h, the reaction mixture was quenched with saturated aqueous NH₄Cl solution (10 mL) and allowed to warm to room temperature then extracted with Et₂O (20 mL × 3). The combined organic layers were washed with brine, dried over sodium sulfate, filtered and concentrate. The residue was purified by silica gel column chromatography (pertroleum ether/ ethyl acetate = 10/ 1) to give the mixture of compound **SG-B1** and **SG-B2** (700 mg). The mixture was further purified by chiral-HPLC to afford compound **SG-B1** (200 mg, t= 4.31 min). **¹H NMR** (400 MHz, CDCl₃), δ (ppm), 7.99-7.97 (d, 2H), 7.77-7.75 (m, 1H), 7.64-7.60 (m, 2H), 5.14-5.08 (m, 1H), 0.88 (s, 3H); compound **SG-B2** (260 mg, t= 5.66 min). **¹H NMR** (400 MHz, CDCl₃), δ (ppm), 8.00--7.98 (d, 2H), 7.77-7.75 (m, 1H), 7.64-7.60 (m, 2H), 5.14-5.09 (m, 1H), 0.88 (s, 3H).

**Synthesis of compound SG-C**. To a solution of compound **SG-B2** (100 mg, 0.209 mmol) and anhydrous Na₂HPO₄ (100 mg) in anhydrous methanol (5 mL) at -20 °C under N₂ was added Na/Hg amalgam (500 mg). After stirring at -20 °C to 0 °C for 1 h, the methanol solution was decanted out and the solid residue was washed with Et₂O (5 × 3 mL). The combined organic layers were washed with brine (20 mL), dried over MgSO₄, filtered and concentrated. The residue was purified by silica gel chromatography (pertroleum ether/ ethyl acetate = 10/ 1) to give compound **SG-C** (36 mg, 0.106 mmol, 51%).

**¹H NMR** (400 MHz, CDCl₃), δ (ppm), 6.02-5.88 (t, 1H), 5.17-5.15 (m, 1H), 0.88 (s, 3H).

**Synthesis of compound SG-D**. To a solution of compound **SG-C** (150 mg, 0.443 mmol) in dry THF (5 mL) was added borane-tetrahydrofuran complex (1.34 mL of 1.0 M solution in THF). After stirring at room temperature for 1 hour, the reaction mixture was cooled in an ice bath then quenched slowly with 10% aqueous NaOH (1 mL) followed 30% aqueous solution of H₂O₂ (1.2 mL). The mixture was allowed to stir at room temperature for 1 hour then extracted with EtOAc (3 × 10 mL). The combined organic layers were washed with 10% aqueous Na₂S₂O₃ (10 mL), brine (10 mL), dried over MgSO₄, filtered and concentrated to afford crude compound **SG-D** (210 mg). The crude product was used in the next step without further purification.

**Synthesis of compound SG-E.** To a solution of crude compound **SG-D** (210 mg) was dissolved in 10 mL of H₂O saturated dichloromethane (dichloromethane had been shaken with several milliliters of H₂O then separated from the water layer) was added Dess-Martin periodinate (380 mg, 0.896 mmol). After stirring at room temperature for 24 h, the reaction mixture was extracted with dichloromethane (3 × 10 mL). The combined organic layers were washed with 10 % aqueous Na₂S₂O₃ (10 mL), brine (10 mL), dried over MgSO₄, filtered and concentrated. The residue was purified by chromatography on silica gel (pertroleum ether/ ethyl acetate = 5: 1) to afford compound **SG-E** (90 mg, 0.254 mmol, 57%) as an off white solid. **¹H NMR** (400 MHz, CDCl₃), δ (ppm), 6.01-5.73 (t, 1H), 2.55-2.54 (m), 2.12 (s), 0.62 (s, 3H).

**Synthesis of compound SG**. To a solution of compound **SG-E** (80 mg, 0.226 mmol) in MeOH (5 mL) was added 2 drops of HBr (48%) followed by bromine (100 mg, 0.63 mmol). After stirring at room temperature for 1h, the reaction mixture was poured into ice-water then extracted with ethyl acetate (15 mL × 3), The combined organic layers were washed with brine (20 mL), dried over MgSO4, filtered and concentrated to give crude compound SG (95 mg). The crude product was used in the next step without further purification.

### Reference Example 14. Synthesis of compounds SG-1 and SG-2.

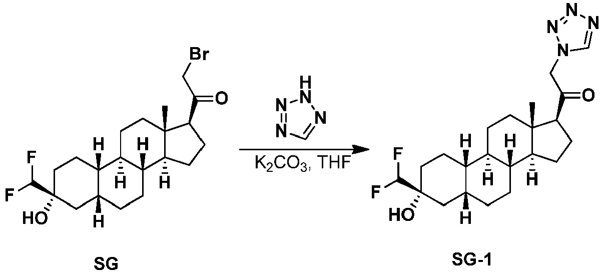

To a suspension of K₂CO₃ (55mg, 0.4mmol) in THF (5mL) was added 2H-tetrazole (28mg, 0.4mmol) and 10 (86 mg, 0.2mmol). The mixture was stirred at RT for 15h. The residue mixture was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SG-1** as an off white solid (12mg,14.2% ) and an off white solid byproduct (15mg,17.7% ).

**SG-1:** ¹HNMR (500 MHz, CDCl3) δ (ppm): 8.74 (s, 1H), 5.87 (t, 1H), 5.32 (AB, 1H, J=18.0Hz), 5.19 (AB, 1H), 2.68 (t, 1H, J=8.5Hz), 2.26-2.20 (m), 2.09-2.05 (m), 0.68 (s, 3H). **LC-MS**: rt = 2.11 min, m/z = 423.3 [M+H]⁺

### Reference Example 15. Synthesis of compounds SG-3 and SG-4.

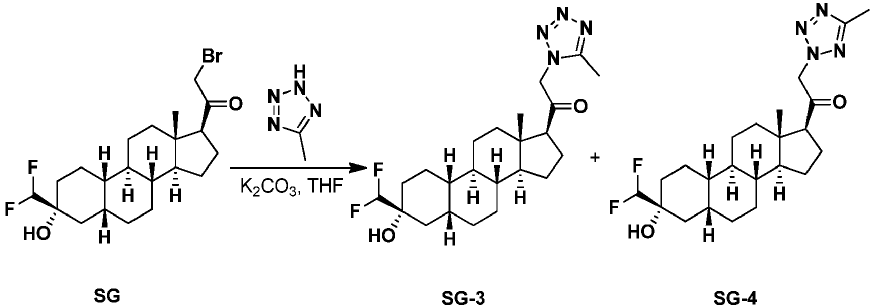

To a suspension of K₂CO₃ (55mg, 0.4mmol) in THF (5mL) was added 5-methyl-2H-tetrazole (28mg, 0.4mmol) and **SG** (86 mg, 0.2mmol). The mixture was stirred at RT for 15h. The residue mixture was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SG-3** as an off white solid (15mg, 17%) and **SG-4** as an off white solid (30mg, 34%). **SG-3**: ¹HNMR (500 MHz, CDCl3) δ (ppm): 5.87 (t, 1H), 5.15 (AB, 1H), 5.05 (AB, 1H), 2.67 (t, 1H), 2.47 (s, 3H), 2.22-2.20 (m, 1H) 2.09-2.07 (m, 1H), 0.69 (s, 3H). **LC-MS**: rt = 2.14 min, m/z = 437.1 [M+H]⁺. **SG-4**: ¹HNMR (500 MHz, CDCl3) δ (ppm): 5.87 (t, 1H), 5.35 (s, 2H), 2.63 (t, 1H), 2.56 (s, 3H), 0.72 (s, 3H). **LC-MS**: rt = 2.24 min, m/z = 437.0 [M+H]⁺

### Reference Example 16. Synthesis of compounds SG-5.

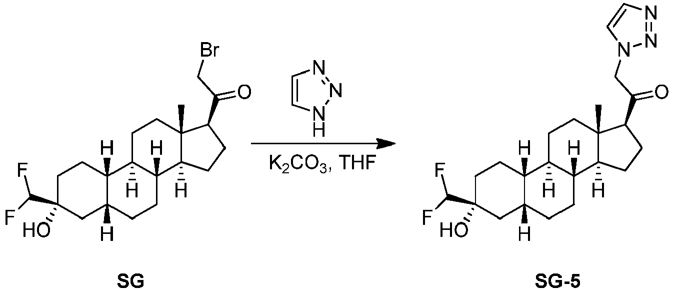

To a suspension of K₂CO₃ (25 mg, 0.18 mmol) in THF (5 mL) was added 1H-1,2,3-triazole (50 mg, 0.72 mmol) and the reactant (100 mg, 0.23 mmol). The mixture was stirred at room temperature for 15h, then the reaction mixture was poured into 10 mL H₂O and extracted with EtOAc (2 × 20 mL). The combined organic layers were washed with brine (10 mL), dried over sodium sulfate, filtered and concentrated *in vacuo.* The residual mixture was purified by reverse-phase prep-HPLC to afford the title compound **SG-5** (15.4 mg, 0.0365 mmol, 22%). **SG-5: ¹HNMR** (400 MHz, CDCl₃) δ (ppm): 7.75(s,1H), 7.64(s,1H), 5.87(t, 1H), 5.27 (AB, 1H), 5.14 (AB, 1H), 2.66 (t,1H), 0.69 (s, 3H).

### Reference Example 17. Synthesis of SE and SE intermediates.

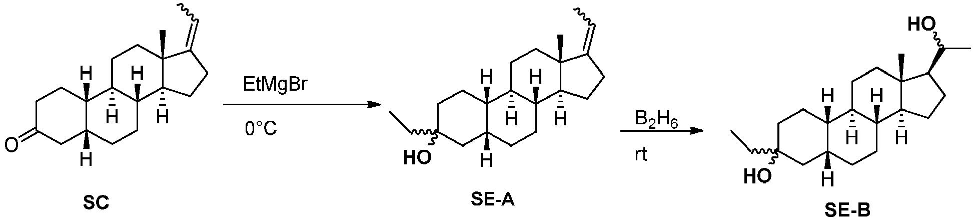

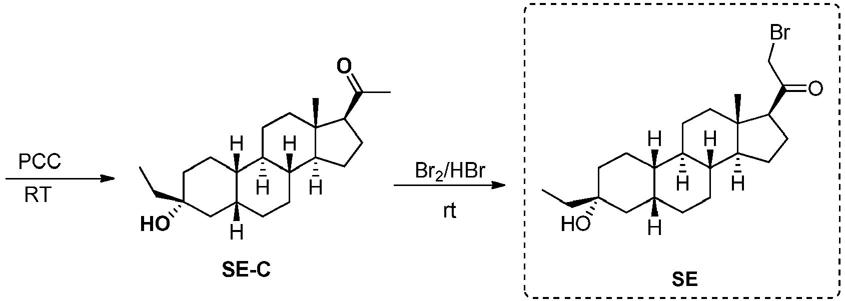

**Synthesis of compound SE-A**. To a solution of EtMgBr (5 mmol, 1M in THF) in THF (20 mL) at 0°C was added a solution of compound **SC** (858mg, 3 mmol) in dry THF (5 mL) via syringe pump over 30 min. After stirring at 0°C for 5h, the reaction mixture was allowed to warm up and stirred at room temperature overnight. The reaction mixture was quenched with iced-cold water and extracted with EtOAc (15 mL × 3). The combined organic layers were washed with brine, dried over sodium sulfate, filtered and concentrated. The white residue was purified by flash column chromatography (petroleum ether/ethyl acetate= 20:1 to 10:1) to give compound **SE-A** (900mg).

**Synthesis of compound SE-B**. To a solution of compound **SE-A** (200 mg, 0.66 mmol) in dry THF (5 mL) was added borane-tetrahydrofuran complex (2 mL of 1.0 M solution in THF). After stirring at room temperature for 1 hour, the reaction mixture was cooled in an ice bath then quenched slowly with 10% aqueous NaOH (1 mL) followed by 30% aqueous solution of H₂O₂ (1.2 mL). The mixture was allowed to stir at room temperature for 1 hour then extracted with EtOAc (3 × 10 mL). The combined organic layers were washed with 10% aqueous Na₂S₂O₃ (10 mL), brine (10 mL), dried over MgSO₄, filtered and concentrated to afford compound **SE-B** (260 mg, crude). The crude product was used in the next step without further purification.

**Synthesis of compound SE-C**. To a solution of compound **SE-B** (260mg, crude) was dissolved in 10 mL dichloromethane was added PCC (449 mg,). After stirring at room temperature for 24 h, the reaction mixture was extracted with dichloromethane (3 × 10 mL). The combined organic layers were washed with 10 % aqueous NaCl (10 mL), brine (10 mL), dried over MgSO₄, filtered and concentrated. The residue was purified by chromatography on silica gel (petroleum ether/ethyl acetate = 4:1 to 2:1) to afford title **SE-C** (15 mg,) as an off white solid. **¹H NMR** (500 MHz, CDCl₃), δ (ppm), 2.49 (1H, t), 0.84(,t 3H), 0.59 (s, 3H).

**Synthesis of compound SE**. To a solution of compound **SE-C** (30 mg, 0.09mmol) in MeOH (5 mL) was added 2 drops of HBr (48%) followed by bromine (100 mg, 0.62 mmol). After stirring at room temperature for 1h, the reaction mixture was poured into ice-water then extracted with ethyl acetate (15 mL × 3), The combined organic layers were washed with brine (20 mL), dried over MgSO₄, filtered and concentrated to give compound **SE** (36mg crude ). The crude product was used in the next step without further purification.

### Reference Example 18. Synthesis of compounds SE-1 and SE-2.

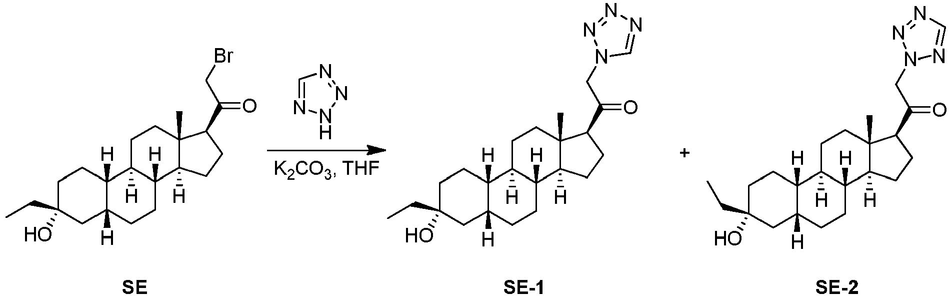

To a suspension of K₂CO₃ (50 mg, 0.36 mmol) in THF (5 mL) was added 1H-tetrazole (40 mg, 0.46 mmol) and SM (100 mg, 0.243 mmol). The mixture was stirred at rt for 15h. The reaction mixture was poured into 5 mL H₂O and extracted with EtOAc (2 × 10 mL). The combined organic layers were washed with brine, dried over sodium sulfate, filtered and concentrated. The residue was purified with by reverse-phase prep-HPLC to afford the title compound as an off white solid **SE-1** (9 mg, 9.2%), **SE-2** (15 mg,15.6%). **SE-1: ¹HNMR** (400 MHz, CDCl₃) δ (ppm): 8.75 (s, 1H), 5.32 (AB, 1H), 5.20 (AB, 1H), 2.67 (t, 1H), 1.59 (q, 2H), 0.88 (t, 3H), 0.68 (s, 3H). **LC-MS**: rt=2.27min,m/z = 383.4 (M⁺ -H₂O+ 1). **SE-2: ¹HNMR** (400 MHz, CDCl₃), δ (ppm): 8.57 (s, 1H), 5.46 (s, 2H), 2.67 (t, 1H), 1.59 (q, 2H), 0.88 (t, 3H), ,0.71(s, 3H). LC-**MS**: rt=2.36min,m/z = 383.4 (M⁺-H₂O + 1).

### Reference Example 19. Synthesis of compounds SE-3 and SE-4.

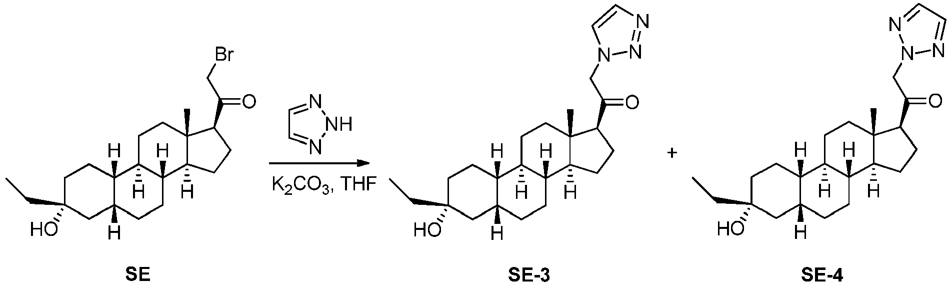

To a suspension of K₂CO₃ (50 mg, 0.36 mmol) in THF (5 mL) was added 2H-1,2,3-triazole (36 mg, 0.52 mmol) and **SE** ( 100 mg, 0.25 mmol). The mixture was stirred at rt for 24h. Then the reaction mixture was poured into 5 mL H₂O and extracted with EtOAc (2 × 10 mL). The combined organic layers were washed with brine, dried over sodium sulfate, filtered and concentrated. The residure was purified with by reverse-phase prep-HPLC to afford the title compound as an off white solid, **SE-3** (9 mg, 9.3%), **SE-4** (10 mg,10.3% ),

**SE-3:** ¹HNMR (500 MHz, CDCl₃) δ (ppm): 7.75 (d, 1H), 7.64 (d, 1H), 5.27 (AB,1H),5.13 (AB, 1H), 2.67 (1H, t), 1.59(2H, q), 0.90(3H, t), 1.28 (s, 3H), 0.67 (s, 3H). **LC-MS**: rt=2.31min, m/z = 400.4 (M⁺ + 1). **SE-4:** ¹HNMR (500 MHz, CDCl₃) δ (ppm): 7.68 (s, 2H), 5.25 (AB, 1H), 5.21(AB, 1H), 2.58 (t, 1H), 1.59 (2H, q), 0.90 (3H, t), 0.71 (s, 3H). **LC-MS**: rt=2.42min, m/z = 400.4 (M⁺ + 1).

### Reference Example 20. Synthesis of compounds SE-5 and SE-6.

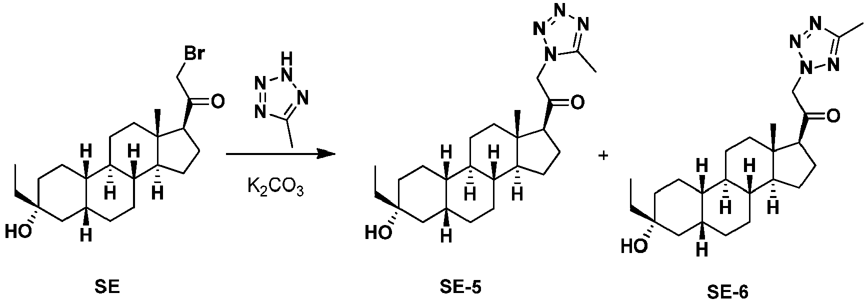

To a suspension of K₂CO₃ (55 mg, 0.4 mmol) in THF (5 mL) was added 5-methyl-2H-tetrazole (33.6 mg, 0.4 mmol) and compound **SE** (82 mg, 0.2 mmol). The mixture was stirred at RT for 15h then the residue mixture was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SE-5** as an off white solid (11.1 mg,13.5%) and **SE-6** as an off white solid (30.6 mg, 37.2%). **SE-5**: ¹HNMR (500 MHz, CDC13) δ (ppm): 5.13 (AB, 1H), 5.07 (AB, 1H), 2.66 (t, 1H), 2.47 (s, 3H), 2.24-2.17 (m, 1H), 2.11-2.05 (m, 1H), 1.47 (q, 2H), 0.93 (t, 3H), 0.69 (s, 3H). **LC-MS**: rt = 2.13 min, m/z = 415.1 [M+H]⁺. **SE-6**: ¹HNMR (500 MHz, CDC13) δ (ppm): 5.37 (AB, 1H), 5.33 (AB, 1H), 2.62 (t, 1H), 2.56 (s, 3H), 2.25-2.18 (m, 1H), 2.09-2.06 (m, 1H), 1.47 (q, 2H), 0.93 (t, 3H), 0.72 (s, 3H). **LC-MS**: rt = 2.26 min, m/z = 415.3 [M+H]⁺

### Reference Example 21. Synthesis of SM and SM intermediates.

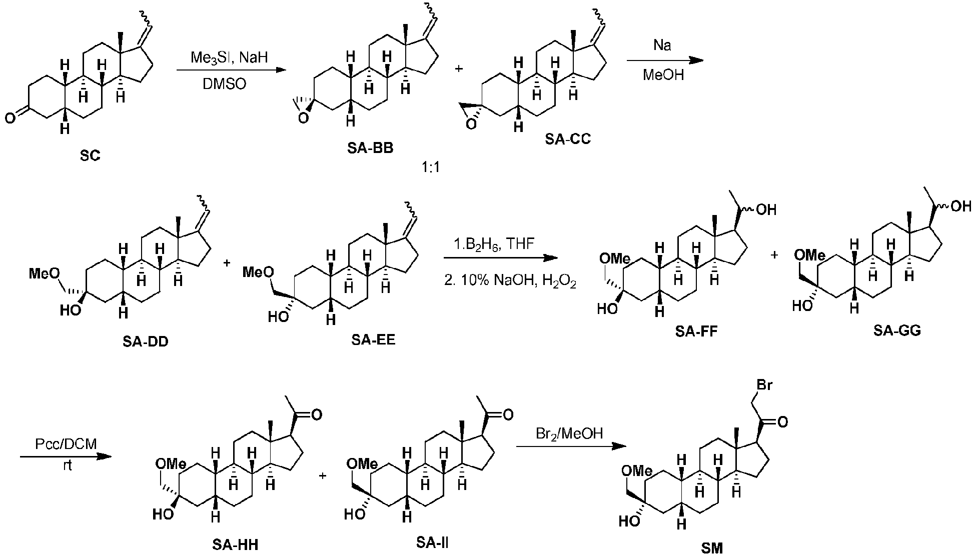

**Synthesis of compound SA-DD and SA-EE.** Compound mixture **SA-BB** and **SA-CC** (5.0 g, 16.7 mmol) was dissolved in dry methanol (250 mL), and Na metal (1.2g, 50.0 mmol) was added and the solution was refluxed for 16 h. Methanol was then evaporated off and the residue was dissolved in dichloromethane and washed with H₂O (3 × 50 mL) and brine (100 mL), dried over MgSO₄, filtered, and concentrated. The crude target compound was purified by via silica gel chromatography (petroleum ether/ethyl acetate = 10:1 to 5:1), and concentrated to give the product mixture **SA-DD** and **SA-EE** (4.6g, 83%) as an off white solid.

**Synthesis of compound SA-FF** and **SA-GG.** To a solution of reactant mixture **SA-DD** and **SA-EE** (4.6g, 13.9 mmol) in anhydrous THF (30 mL) was added BH₃.THF (1.0 M, 27.7 mL, 27.7 mmol), the solution was stirred at 25 °C overnight, then the reaction was quenched by addition of water (5 mL). 2 M NaOH solution (30 mL) was added followed by 30 % H₂O₂ (30 mL). The mixture was stirred at room temperature for 1 hour. The mixture was diluted with ethyl acetate (200 mL) and resulting solution was washed with brine (2× 100 mL), dried over magnesium sulfate and concentrated *in vacuo.* The crude product mixture was used directly in the next step without further purification.

**Synthesis of compound SA-HH and SA-II.** To a solution of crude reactant mixture **SA-FF** and **SA-GG** (4.9g, 13.9 mmol, theoretical amount) in dichloromethane (40 mL) was added Pyridinium chlorochromate (PCC) in portions (6.0g, 27.8 mmol). The solution was stirred at 25 °C overnight then the mixture was filtered through a short pad of silica gel and the silica gel was washed with dichloromethane (3×50 mL). All filtrates were combined and concentrated *in vacuo.* The residue was purified by flash chromatography ( petroleum ether/ ethyl acetate=15:1) to afford product **SA-HH** (2.1g, 6.03 mmol, Yield=43% (2 steps)) as off white solid and product **SA-II** (2.2g, 6.32 mmol, Yield=45% (2 steps)) as off white solid. **Compound SA-HH**: ¹HNMR (500 MHz, CDC13) δ (ppm): 3.40 (s, 3H), 3.20 (s, 2H), 2.62-2.51 (m, 2H), 2.11 (s, 3H), 2.02-1.99 (m, 2H), 0.62 (s, 3H). **Compound SA-II**: ¹HNMR (500 MHz, CDC13) δ (ppm): 3.42 (AB, 1H), 3.38 (AB, 1H), 3.40 (s, 3H), 2.65 (s, 1H), 2.54 (t, 1H), 2.16-2.14 (m, 1H), 2.11 (s, 3H), 2.02-1.98 (m, 1H), 0.61 (s, 3H).

**Synthesis of compound SM**. To a solution of reactant **SA-II** (100 mg, 0.301 mmol) in methanol (10 mL) was added 48% hydrobromic acid (152 mg, 0.903 mmol) followed by bromine (241 mg, 0.077 mL, 1.51 mmol). The solution was heated at 25 °C for 1.5 hours then the mixture was poured into cold water (50 mL) and the resulting solid was extracted with ethyl acetate (2×50 mL). The combined organic extracts were washed with brine (50 mL), dried over magnesium sulfate and concentrated *in vacuo.* The crude product **SM** was used directly without further purification in the next step.

### Reference Example 22. Synthesis of compounds SM-1.

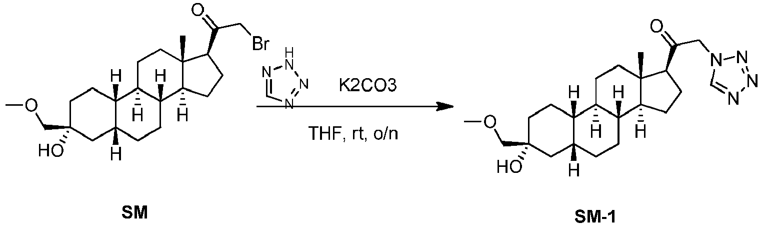

To a solution of compound **SM** (120 mg, 0.28 mmol) in THF (3 mL) was added K₂CO₃ (190 mg, 1.4 mmol) and 1H-tetrazole (100 mg, 1.4 mmol). The resulting solution was stirred at room temperature overnight then the reaction was diluted with EtOAc (20 mL). The resulting solution was washed with brine (10 mL), dried over Na₂SO₄ and concentrated *in vacuo.* The residue was purified by prep-HPLC to give **SM-1** (12 mg, 10%), and an off white solid byproduct (14 mg, 12%). **SM-1: 1H NMR:** (500 MHz, CDCl₃), δ (ppm), 8.74 (s, 1H), 5.32 (AB, 1H), 5.19 (AB, 1H), 3.42 (AB, 1H), 3.40 (S, 3H), 3.39 (AB, 1H), 2.68 (t, 1H), 2.66 (s, 1H), 0.67 (s, 3H). **LC-MS:** rt=2.19 min; m/z=399.2 (M-18)⁺

### Reference Example 23. Synthesis of compounds SM-3 and SM-4.

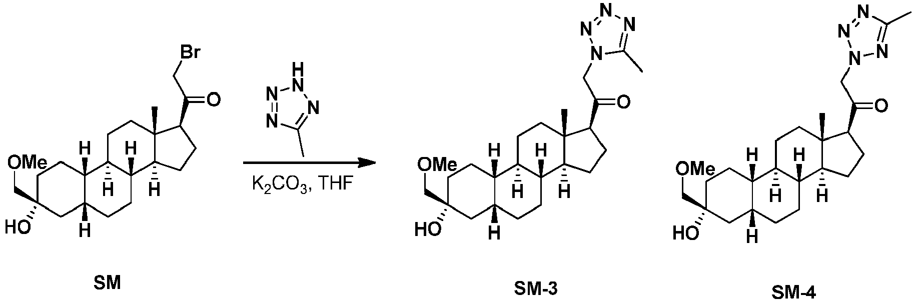

To a suspension of K₂CO₃ (55mg, 0.4mmol) in THF (5mL) was added 5-methyl-2H-tetrazole (33.6mg, 0.4mmol) and 10 (85 mg, 0.2mmol). The mixture was stirred at RT for 15h then was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SM-3** as an off white solid (8.6mg, 10%) and an off white solid (12mg, 13.9%). **SM-3**: ¹HNMR (500 MHz, CDC13) δ (ppm): 5.15 (AB, 1H), 5.05 (AB, 1H), 3.42 (AB, 1H), 3.39 (AB, 1H), 3.40 (s, 3H), 2.67 (t, 1H), 2.64 (s, 1H), 2.47 (s, 3H), 2.21-2.17 (m, 1H), 2.08-2.05 (m, 1H), 0.68 (s, 3H). **LC-MS**: rt = 2.14 min, m/z = 431.2 [M+H]⁺.**SM-4:** ¹HNMR (500 MHz, CDC13) δ (ppm): 5.37 (AB, 1H), 5.33 (AB, 1H), 3.42 (AB, 1H), 3.38 (AB, 1H), 3.40 (s, 3H), 2.63 (t, 1H), 2.56 (s, 3H), 0.71 (s, 3H). **LC-MS:** rt = 2.25 min, m/z = 431.2 [M+H]+

### Reference Example 24. Synthesis of compounds SM-5.

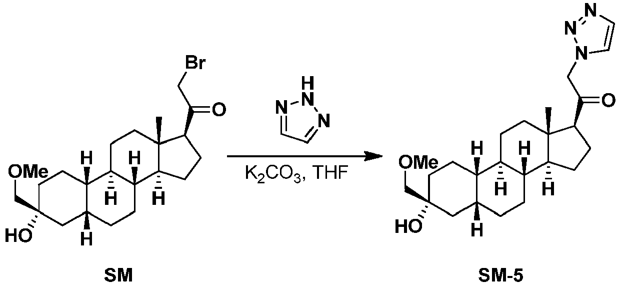

To a suspension of K₂CO₃ (55mg, 0.4mmol) in THF (5mL) was added 2H-1,2,3-triazole (28mg, 0.4mmol) and Compound **SM** (85 mg, 0.2mmol). The mixture was stirred at RT for 15h then the residue mixture was poured into 5 mL H₂O and extracted with EtOAc (2 × 10 mL). The combined organic layers were washed with brine, dried over sodium sulfate, filtered and concentrated. The residue mixture was purified by reverse-phase prep-HPLC to afford **SM-5** as an off white solid (25mg,30% ). Compound **SM-5: ¹HNMR** (500 MHz, CDC13) δ (ppm): 7.76 (s, 1H), 7.65 (s, 1H), 5.28 (AB, 1H), 5.14 (AB, 1H), 3.42 (AB, 1H), 3.39 (AB, 1H), 3.40 (s, 3H), 2.66 (t, 1H), 2.23-2.20 (m, 1H), 2.10-2.08 (m, 1H), 0.67 (s, 3H). **LC-MS**: rt = 2.14 min, m/z = 415.8 [M+H]⁺

### Reference Example 25. Synthesis of SO and SO intermediates.

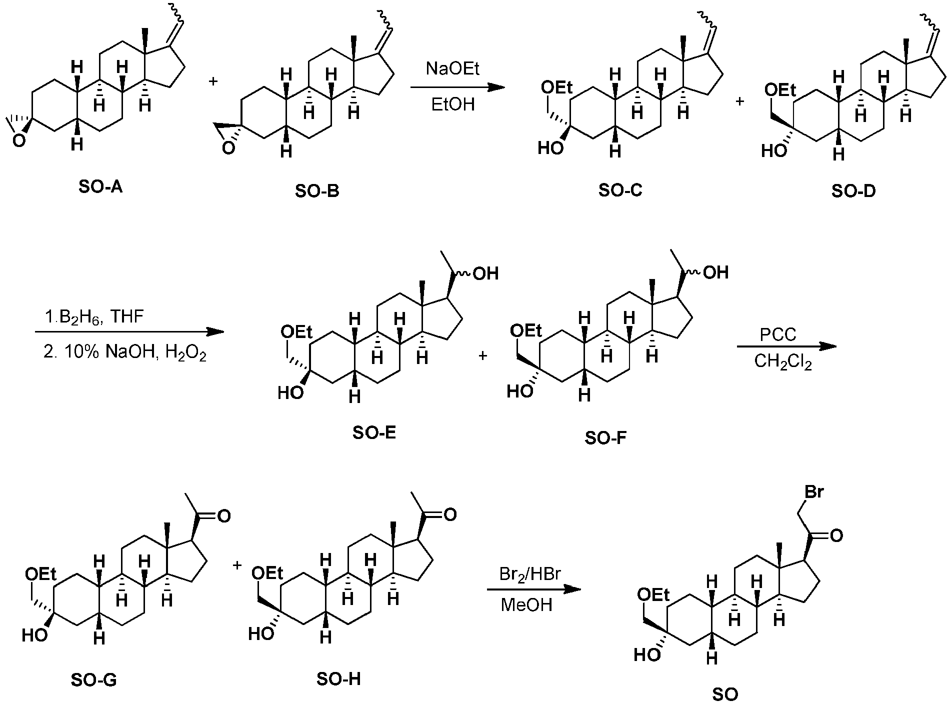

**Synthesis of compound SO-C and SO-D.** Compound mixture **SO-A** and **SO-B** (5.0 g, 16.7 mmol) was dissolved in dry ethanol (250 mL), and Na (1.2g, 50.0 mmol) was added. The solution was refluxed for 16 h. Ethanol was evaporated off and the residue was dissolved in dichloromethane and washed with H₂O (3 × 50 mL) and brine (100 mL), dried over MgS0₄, filtered, and concentrated. The crude target compound was purified by silica gel chromatography (petroleum ether/ethyl acetate = 10:1 to 5:1), and concentrated to give the product mixture **SO-C** and **SO-D** (4.5g, 78%) as an off white solid.

**Synthesis of compounds SO-E and SO-F.** To a solution of reactant mixture **SO-C** and **SO-D** (4.5g, 13.0 mmol) in anhydrous THF (30 mL) was added BH₃.THF (1.0 M, 27.7 mL, 27.7 mmol), the solution was stirred at 25 °C overnight. Then the reaction was quenched by addition of water (5 mL). 2 M NaOH solution (30 mL) was added followed by 30 % H₂O₂ (30 mL). The mixture was stirred at room temperature for 1 hour. The mixture was diluted with ethyl acetate (200 mL) and resulting solution was washed with brine (2×100 mL), dried over magnesium sulfate and concentrated in vacuo. The crude product mixture was used directly in the next step without further purification.

**Synthesis of compound SO-G and SO-H**. To a solution of crude reactant mixture **SO-E** and **SO-F** (4.5g, 13.0 mmol, theoretical amount) in dichloromethane (40 mL) was added Pyridinium chlorochromate (PCC) in portions (5.7g, 26.0 mmol). The solution was stirred at 25 °C overnight. Then the mixture was filtered through a short pad of silica gel and the silica gel was washed with dichloromethane (3×50 mL). All filtrate was combined and concentrated in vacuo. The residue was purified by flash chromatography (eluant: petroleum ether/ ethyl acetate=15:1) to afford product **SO-G** (2.0g, 5.5 mmol, Yield=42% (2 steps)) as off white solid and product **SO-H** (1.8g, 4.97 mmol, Yield=38% (2 steps)) as off white solid. **SO-H**: ¹HNMR (500 MHz, CDC13) δ (ppm): 3.53 (q, 2H), 3.45 (AB, 1H), 3.41 (AB, 1H), 2.54 (t, 1H), 2.16-2.12 (m), 2.11 (s), 2.02-1.98 (m), 1.2 (t, 3H), 0.61 (s, 3H).

**Synthesis of compound SO.** To a solution of reactant **SO-H** (100 mg, 0.301 mmol) in methanol (10 mL) was added 48% hydrobromic acid (152 mg, 0.903 mmol) followed by bromine (241 mg, 0.077 mL, 1.505 mmol). The solution was heated at 25 °C for 1.5 hours. Then the mixture was poured into cooled water (50 mL). The resulting solid was extracted with ethyl acetate (2×50 mL). The combined organic extracts were washed with brine (50 mL), dried over magnesium sulfate and concentrated in vacuo. The crude product **SO** was used directly without further purification in the next step.

### Reference Example 26. Synthesis of compounds SO-1 and SO-2.

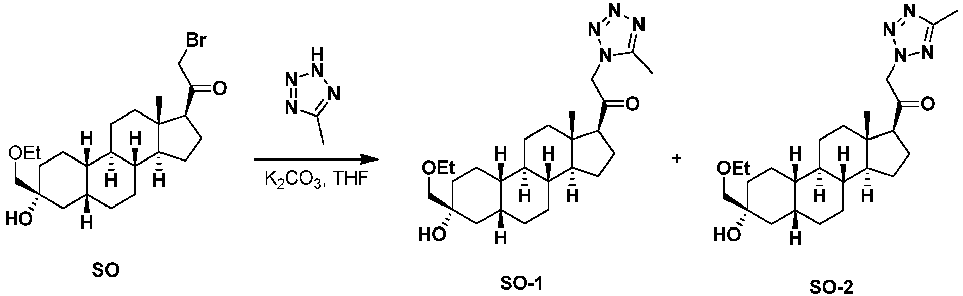

To a suspension of K₂CO₃ (55mg, 0.4mmol) in THF (5mL) was added 5-methyl-2H-tetrazole (33.6mg, 0.4mmol) and 10 (85 mg, 0.2mmol). The mixture was stirred at RT for 15h. The residue mixture was poured into 5mL H₂O and extracted with EtOAc (2 × 10 mL). The combined organic layers were washed with brine, dried over sodium sulfate, filtered and concentrated. The residue mixture was purified by reverse-phase prep-HPLC to afford **SO-1** as an off white solid (9.6mg,10.8%) and **SO-2** as an off white solid (17.5mg, 19.7%). **SO-1**: ¹HNMR (500 MHz, CDC13) δ (ppm): 5.15 (AB, 1H), 5.05 (AB, 1H), 3.54 (q, 2H), 3.45 (AB, 1H), 3.41 (AB, 1H), 2.75 (s, 1H), 2.66 (t, 1H), 2.47 (s, 3H), 2.24-2.17 (m, 1H), 2.08-2.05 (m, 1H), 1.21 (t, 3H), 0.68 (s, 3H). **LC-MS**: rt = 2.24 min, m/z = 445.3 [M+H]⁺. **SO-2**: ¹HNMR (500 MHz, CDC13) δ (ppm): 5.36 (AB, 1H), 5.35 (AB, 1H), 3.54 (q, 2H), 3.45 (AB, 1H), 3.41 (AB, 1H), 2.75 (s, 1H), 2.63 (t, 1H), 2.56 (s, 3H), 2.24-2.17 (m, 1H), 2.09-2.05 (m, 1H), 1.21 (t, 3H), 0.71 (s, 3H). **LC-MS**: rt = 2.35 min, m/z = 427.3 [M-H2O+H]⁺

### Reference Example 27. Synthesis of SL and SL intermediates.

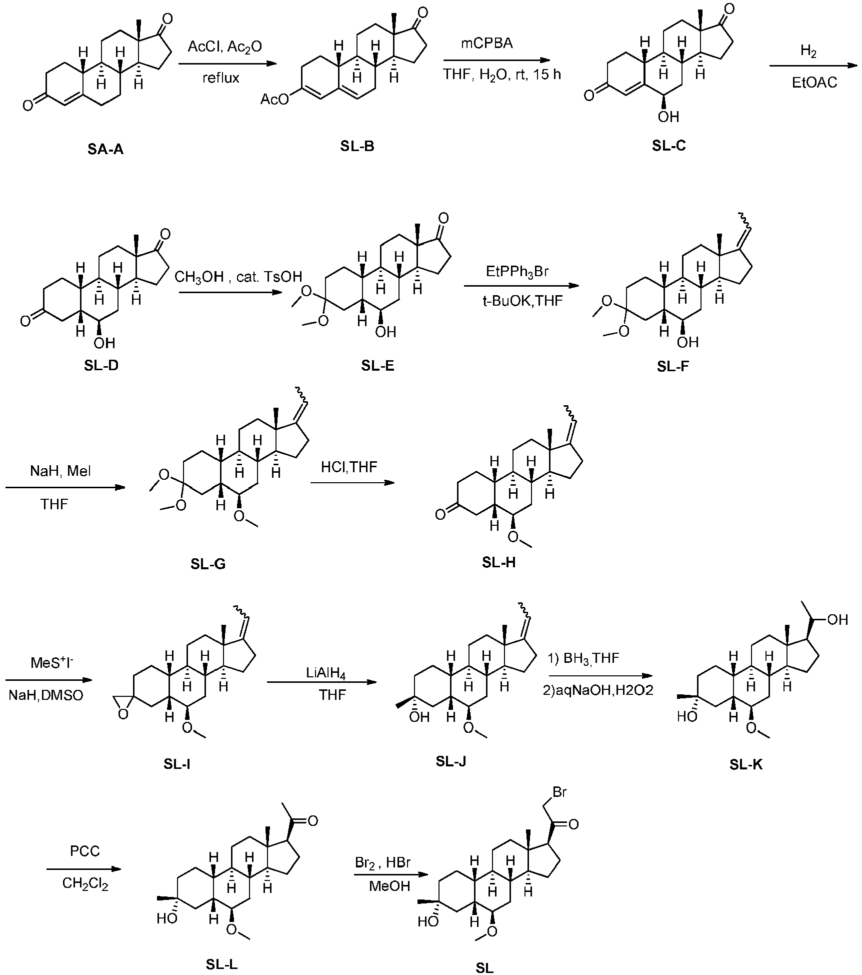

**Synthesis of compound SL-B. SA-A** (10 g, 36.7 mmol) was added to 50 mL acetyl chloride and 50 ml L acetic anhydride. The reaction mixture was heated to 120°C for 5 h, evaporated *in vacuo* to afford crude **SL-B** as the off white solid (10 g, 87% yield). **¹H NMR** (400 MHz, CDCl₃), δ (ppm), 5.78 (s, 1H), 5.55 (s, 1H),2.4(2H,dd), 2.13 (s, 3H), 0.90 (s, 3H).

**Synthesis of compound SL-C.** To a solution **SL-B** (10 g, 31.8 mmol) in 200 mL THF and 20 mL H₂O, was added mCPBA (11 g, 63.6 mmol) at 0°C, stirred at rt for 15 h, the reaction mixture was extracted 500 mL EtOAc, washed with 100 mL saturated Na₂SO₃, 100 mL saturated NaHCO₃ and 100 mL brine and evaporated *in vacuo* then purified by chromatography (PE:EtOAc = 5:1) to afford **SL-C** as the off white solid ( 2.2 g, 24% yield). **¹H NMR** (400 MHz, CDCl₃), δ (ppm), 5.92 (s, 1H), 4.44 (s, 1H), 0.95 (s, 3H).

**Synthesis of compound SL-D.** To a solution of **SL-C** (2 g, 6.94 mmol) in 50 mL EtOAc, was added Pd/C 200 mg. The reaction mixture was hydrogenated in 1 atm H₂ for 15 h. The reaction mixture was evaporated *in vacuo* then purified by chromatography (PE:EtOAc = 1:2) to afford **SL-D** as the off white solid (1 g, 50% yield). **¹H NMR** (400 MHz, CDCl₃), δ (ppm), 3.83 (s, 1H), 0.93 (s, 3H).

**Synthesis of compound SL-E.** To a solution of **SL-D** (1 g, 3.4 mmol) in 100 mL MeOH, was added TsOH 50 mg, heated to 60°C for 2 h. The reaction mixture was extracted 500 mL EtOAc, washed with 100 mL saturated NaHCO₃, 100 mL brine and evaporated *in vacuo* to afford **SL-E** as the off white solid (1 g, 91% yield). **¹H NMR** (400 MHz, MeOD), δ (ppm), 3.80 (s, 1H), 3.20 (s, 3H), 3.15 (s, 3H), 0.89 (s, 3H).

**Synthesis of compound SL-F.** To a solution of ethyltriphenylphosphonium bromide (10.67 g, 28.84 mmol) in 30 mL THF, was added KOt-Bu (3.23 g, 28.80 mmol). The reaction was heated to 60°C for 1 h then **SL-E** (3.23 g, 9.6 mmol) was added to the mixture, stirred at 60°C for 15 h. The reaction mixture was extracted 500 mL EtOAc, washed with brine and evaporated *in vacuo* then purified by chromatography (PE:EtOAc = 3:1) to afford **SL-F** as the off white solid (2.18 g, 65% yield). **¹H NMR** (400 MHz, d₆-acetone), δ (ppm), 5.09-5.07 (m, 1H), 3.65 (s, 1H), 3.11 (s, 3H), 3.08 (s, 3H), 0.88 (s, 3H).

**Synthesis of compound SL-G.** To a solution of **SL-F** (1 g, 2.9 mmol) in 50 mL THF, was added NaH (2 g, 5.8 mmol) , stirred at rt for 1 h. Then 1 mL Mel was added to themixture, stirred at rt overnight. The reaction mixture was quenched with 5 mL H₂O and extracted with 100 mL EtOAc, washed with brine and evaporated *in vacuo* then purified by chromatography (PE:EtOAc = 10:1) to afford **SL-G** as the off white solid (577 mg, 55% yield). **¹H NMR** (400 MHz, d₆-acetone), δ (ppm), 4.96-4.93 (m, 1H), 3.12 (s, 3H), 3.00 (s, 1H), 2.98 (s, 3H), 2.96 (s, 3H), 0.75 (s, 3H).

**Synthesis of compound SL-H.** To a solution of **SL-G** (1 g, 2.8 mmol) in 20 mL THF, was added 2 M aqueous HCl 2 mL, stirred at rt for 1 h. The reaction mixture was quenched with 5 mL H₂O and extracted with 100 mL EtOAc, washed with brine and evaporated *in vacuo* then purified by chromatography (PE:EtOAc = 10:1) to afford **SL-H** as the off white solid (750 mg, 83% yield). **¹H NMR** (400 MHz, CDCl₃), δ (ppm), 5.15-5.11 (m, 1H), 3.32 (s, 3H), 3.14 (s, 1H), 0.92 (s, 3H).

**Synthesis of compound SL-I.** To a stirred solution of trimethylsulfonium iodide (6.4 g, 31.5 mmol) in 10 mL of DMSO was added NaH (60%, 800 mg, 31.5 mmol).After stirring at room temperature for 1h, a suspension of **SL-H** (1 g, 3.2 mmol) in 5 mL of DMSO was added dropwise. After 15 h, the reaction mixture was poured into ice-cold water and extracted with 300 mL EtOAc, washed with 100 mL brine, dried and evaporated *in vacuo* then purified by chromatography (PE:EtOAc = 10:1) to afford **SL-I** and its isomer as the off white solid (793 mg, 76% yield).

**Synthesis of compound SL-J.** To a solution of **SL-I** and its isomer (150 mg, 0.45 mmol) in 10 mL THF, was added LiAH₄ (50 mg, 1.35 mmol) , stirred at rt for 1 h. The reaction mixture was quenched with 5 mL H₂O and extracted with 100 mL EtOAc, washed with brine and evaporated *in vacuo* then purified by chromatography (PE:EA = 3:1) to afford SL-J as the off white solid (72 mg, 48% yield). **¹H NMR** (400 MHz, CDCl₃), δ (ppm), 5.11-5.10 (m, 1H), 3.33 (s, 3H), 3.12 (s, 1H), 1.22 (s, 3H), 0.89 (s, 3H).

**Synthesis of compound SL-K.** To a solution of **SL-J** (100 mg, 0.3 mmol) in dry THF (5 mL) was added borane-tetrahydrofuran complex (1 mL; 1.0 M solution in THF). After stirring at room temperature for 1 hour, the reaction mixture was cooled in an ice bath then quenched slowly with 10% aqueous NaOH (1 mL) followed by 30% aqueous solution of H₂O₂ (1 mL). After stirring at room temperature for one hour, the mixture was extracted with EtOAc (3 × 100 mL). The combined organic layers were washed with 10% aqueous Na₂S₂O₃ (100 mL), brine (100 mL), dried over MgSO₄, filtered and concentrated to afford **SL-K** as the off white solid (100 mg, 91%). The crude product was used in the next step without further purification.

**Synthesis of compound SL-L.** To a solution of **SL-K** (100 mg, 0.29 mmol) in 20 mL DCM, was added PCC (190 mg, 0.87 mmol), stirred at rt for 2 h. The reaction mixture was quenched with 5 mL H₂O and extracted with 100 ml EtOAc, washed with brine and evaporated *in vacuo* then purified by chromatography (PE:EtOAc = 3:1) to afford **SL-L** as the off white solid (55 mg, 55% yield). **¹H NMR** (400 MHz, CDCl₃), δ (ppm), 3.30 (s, 3H), 3.10 (s, 1H),2.5(1H,t,J=10Hz),2.1(s, 3H), 1.16 (s, 3H), 0.56 (s, 3H).

**Synthesis of compound SL.** To a solution of **SL-L** (40 mg, 0.11 mmol) in MeOH (5 mL) was added 2 drops of HBr (48%) followed by bromine (150 mg, 0.33 mmol). After stirring at room temperature for 1h, the reaction mixture was poured into ice-water then extracted with EtOAc (10 mL × 3). The combined organic layers were washed with brine (20 mL), dried over MgSO₄, filtered and concentrated to give crude compound **SL** as the off white solid (40 mg, 80% yield). The crude product was used in the next step without further purification.

### Reference Example 28. Synthesis of compounds SL-1 and SL-2.

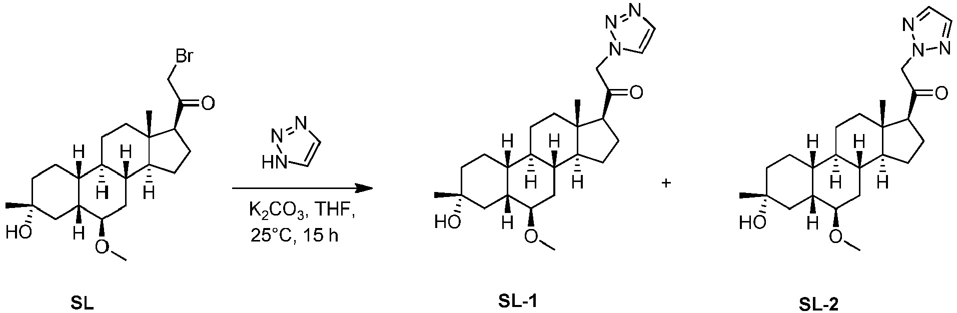

To a suspension of **SL** ( 40 mg, 0.09 mmol) in THF (5 mL) was added 1H-1,2,3-triazole (30 mg, 0.45 mmol) and K₂CO₃ ( 60 mg, 0.45mmol). The mixture was stirred at 25°C for 15h. The reaction mixture was purified by reverse-phase prep-HPLC to afford **SL-1** as an off white solid (5 mg, 13% yield) and **SL-2** as an off white solid (5 mg, 13% yield). **SL-1: ¹H NMR** (400 MHz, CDCl₃), δ (ppm), 7.75 (s, 1H), 7.64 (s, 1H), 5.25-5.13 (m, 2H), 3.31 (s, 3H), 3.11 (s, 1H), 1.24 (s, 3H), 0.71 (s, 3H). **SL-2: ¹H NMR** (400 MHz, CDCl₃), δ (ppm), 7.68 (s, 2H), 5.27-5.19 (m, 2H), 3.31 (s, 3H), 3.11 (s, 1H), 1.21 (s, 3H), 0.75 (s, 3H).

### Reference Example 29. Synthesis of SH and SH intermediates.

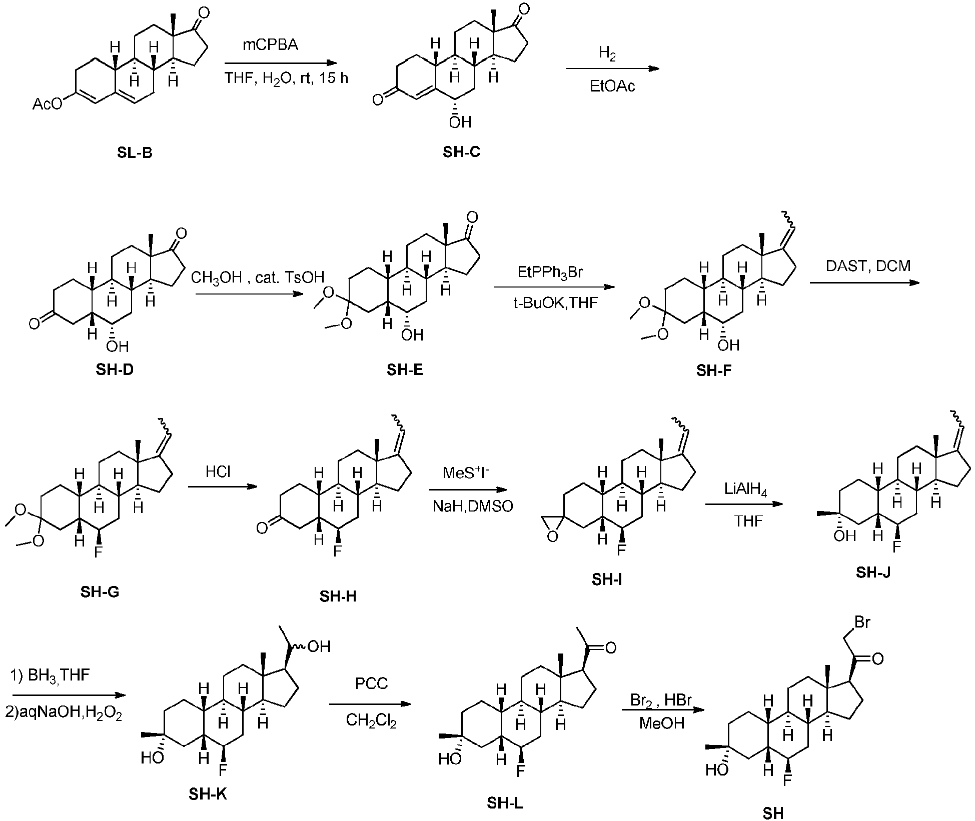

**Synthesis of compound SH-C.** To a solution of Compound **SL-B** (10 g, 31.8 mmol) in 200 mL THF and 20 mL H₂O was added m-CPBA (11 g, 63.6 mmol) at 0°C. After stirring at rt for 15 h, the reaction mixture was diluted with 500 mL EtOAc. The resulting solution was washed with 300 mL sat. Na₂SO₃, 300 mL sat. NaHCO₃ and 300 mL brine and evaporated *in vacuo.* The residue was purified by chromatography (PE:EA = 5:1) to afford **SH-C** as the off white solid (1.1 g, 3.8 mmol,12% yield). **¹H NMR** (500 MHz, CDCl₃), δ (ppm), 6.25 (s, 1H), 4.27 (dd, 1H), 0.93 (s, 3H).

**Synthesis of compound SH-D.** To a solution of Compound **SH-C** (2 g, 6.94 mmol) in 50 mL EtOAc was added Pd\C 200 mg. The reaction mixture was hydrogenated in 1 atm H₂ for 15 h. The reaction mixture was evaporated *in vacuo* then purified by chromatography (PE:EA = 1:2) to afford **SH-D** as the off white solid (1.5 g, 5.2 mmol,75% yield). **¹H NMR** (500 MHz, CDCl₃), δ (ppm), 3.97 (td, 1H), 0.88 (s, 3H).

**Synthesis of compound SH-E.** To a solution of Compound **SH-D** (1 g, 3.4 mmol) in 100 mL MeOH, was added TsOH 50 mg. The solution was heated to 60 °C for 2 h. Then the reaction mixture was diluted with 500 mL EtOAc, washed with 100 mL sat. NaHCO₃, 100 mL brine and evaporated *in vacuo* to afford **SH-E** as the off white solid (1 g, 91% yield).

**Synthesis of compound SH-F.** To a solution of ethyltriphenylphosphonium bromide (10.67 g, 28.84 mmol) in 30 mL THF was added KOt-Bu (3.23 g, 28.80 mmol). The reaction was heated to 60 °C for 1 h, then Compound **SH-E** (3.23 g, 9.6 mmol) was added to the mixture. The solution was heated at 60 °C for 15 h. Then the reaction mixture was diluted with 500 mL EtOAc. The resulting solution was washed with 100 mL brine, evaporated *in vacuo,* and then purified by chromatography (PE:EA = 3:1) to afford **SH-F** as the off white solid (2 g, 5.74 mmol ,62% yield). **¹H NMR** (500 MHz, MeOD), δ (ppm), 5.15-5.12 (m, 1H), 3.80-3.78 (m, 1H), 3.21 (s, 3H), 3.15 (s, 3H), 1.67 (d, 3H), 0.95 (s, 3H).

**Synthesis of compound SH-G.** To a solution of Compound **SH-F** (0.5 g, 1.43 mmol) in 10 mL DCM was added DAST (0.5 ml, 10 mmol) at -78°C. The reaction mixture was stirred at -78°C for 30 min, then was quenched with 5 Ll sat. NaHCO₃, extracted with 50 ml DCM, washed with 100 ml brine, dried over Na₂SO₄, concentrated *in vacuo,* and purified by chromatography (PE:EA = 30:1) to afford **SH-G** as the off white solid (175 mg, 0.5 mmol, 35% yield).

**Synthesis of compound SH-H.** To a solution of Compound **SH-G** (350 mg, 1 mmol) in 20 mL THF was added 2 M HCl (2 mL). The solution was stirred at rt for 1 h, then the reaction mixture was extracted with 100 mL EtOAc, washed with 100 mL brine and evaporated *in vacuo.* The resulting residue was then purified by chromatography (PE:EA = 10:1) to afford **SH-H** as the off white solid (210 mg, 0.7 mmol, 60% yield). **¹H NMR** (500 MHz, CDCl₃), δ (ppm), 5.17-5.14 (m, 1H), 4.80-4.66 (m, 1H), 2.61-2.57 (m, 1H), 1.79 (d, 3H), 0.93 (s, 3H).

**Synthesis of compound SH-I.** To a stirred suspension of trimethylsulfonium iodide (3.2 g, 16 mmol) in 10 mL DMSO was added NaH (60%, 400 mg, 16 mmol). After stirring at room temperature for 1h, a suspension of Compound **SH-H** (486 mg, 1.6 mmol) in 5 mL DMSO was added dropwise. After 15 h, the reaction mixture was poured into ice-cold water and extracted with 300 mL EtOAc. The resulting solution was washed with 100 mL brine, dried (NaSO₄) and evaporated *in vacuo.* The resulting residue was then purified by chromatography (PE:EA = 10:1) to afford a mixture of **SH-I** and its C-3 isomer as the off white solid (290 mg, 0.91 mmol,58% yield).

**Synthesis of compound SH-J.** To a solution of **SH-I** and its C-3 isomer (300 mg, 0.94 mmol) in 10 ml THF, was added LiAH₄ (100 mg, 2.7 mmol) .The suspension was stirred at rt for 1 h. Then the reaction mixture was quenched with 5 mL H₂O and extracted with 100 mL EtOAc. The resulting solution was washed with brine and evaporated *in vacuo.* The resulting residue was then purified by chromatography (PE:EA = 3:1) to afford **SH-J** as the off white solid (140 mg, 48% yield). **¹H NMR** (500 MHz, CDCl₃), δ (ppm), 5.15-5.12 (m, 1H), 4.72-4.60 (m, 1H), 1.70 (d, 3H), 1.27 (s, 3H), 0.92 (s, 3H).

**Synthesis of compound SH-K.** To a solution of Compound **SH-J** (100 mg, 0.3 mmol) in dry THF (5 mL) was added borane-tetrahydrofuran complex (1 mL; 1.0 M solution in THF). After stirring at room temperature for 1 hour, the reaction mixture was cooled in an ice bath then quenched slowly with 10% aqueous NaOH (1 mL) followed by 30% aqueous solution of H₂O₂ (1 mL). After stirring at room temperature for one hour, the mixture was extracted with EtOAc (3 × 100 mL). Then the combined organic extracts were washed with 10% aqueous Na₂S₂O₃ (100 mL), brine (100 mL), dried over MgSO₄, filtered and concentrated to afford crude **SH-K** as the off white solid (100 mg, 91%). The crude product was used in the next step without further purification.

**Synthesis of compound SH-L**. To a solution of Compound **SH-K** (100 mg, 0.29 mmol) in 20 m DCLM was added PCC (190 mg, 0.87 mmol) and the resulting solution was stirred at rt for 2 h. Then, reaction mixture was filtered through a pad of cerite and the filtrate was evaporated *in vacuo.* The residue was then purified by chromatography (PE:EA = 3:1) to afford **SH-L** as the off white solid (53 mg, 53% yield). **¹H NMR** (400 MHz, CDCl₃), δ (ppm), 4.71-4.57 (m, 1H), 2.54(1H, t),2.15(s, 3H), 1.28 (s, 3H), 0.58 (s, 3H).

**Synthesis of compound SH.** To a solution of Compound **SH-L** (40 mg, 0.11 mmol) in MeOH (5 mL) was added 2 drops of HBr (48%) followed by bromine (150 mg, 0.33 mmol). After stirring at room temperature for 1h, the reaction mixture was poured into ice-water then extracted with ethyl acetate (10 mL × 3). The combined organic layers were washed with brine (20 mL), dried over MgSO₄, filtered and concentrated to give crude compound **SH** as the yellow solid (40 mg, 80% yield). The crude product was used in the next step without further purification.

### Reference Example 30. Synthesis of compounds SH-1 and SH-2.

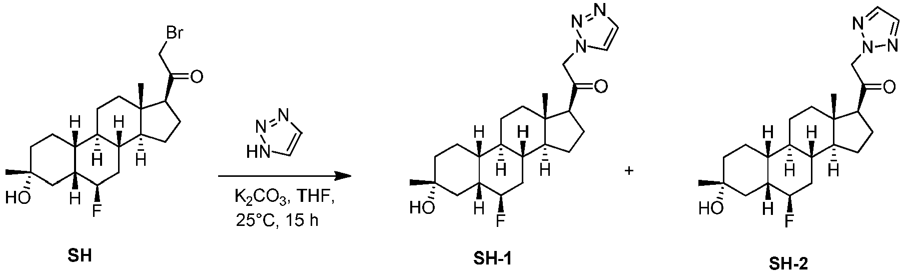

To a suspension of Compound **SH** (50 mg, 0.12 mmol) in THF (5 mL) was added 2H-1,2,3-triazole (120 mg, 1.8 mmol) and K₂CO₃ ( 200 mg, 1.2 mmol). The mixture was stirred at 25°C for 15h. The reaction mixture was extracted with ethyl acetate (20 mLx3). The combined organic layers were washed with brine (20 mL), dried over MgSO₄, filtered and concentrated to give crude product. This crude product was purified with by reverse-phase prep-HPLC to afford **SH-1** as an off white solid ( 12 mg, 0.03mmol, 25% yield) and **SH-2** as an off white solid ( 5.7 mg, 0.014mmol, 8.33% yield). **SH-1: ¹H NMR** (500 MHz, CDCl₃), δ (ppm), 7.76 (s, 1H), 7.65 (s, 1H), 5.29(1H,AB), 5.14(1H,AB), 4.73-4.59 (m, 1H),2.68(1H,t),1.30 (s, 3H), 0.67 (s, 3H). **SH-2: ¹H NMR** (500 MHz, CDCl₃), δ (ppm), 7.69 (s, 2H), 5.27(1H,AB), 5.23(1H,AB), 4.73-4.59 (m, 1H), 4.64-4.59 (m, 1H),2.60(1H,t),1.29 (s, 3H), 0.70 (s, 3H).

### Reference Example 31. Synthesis of SB and SB intermediates

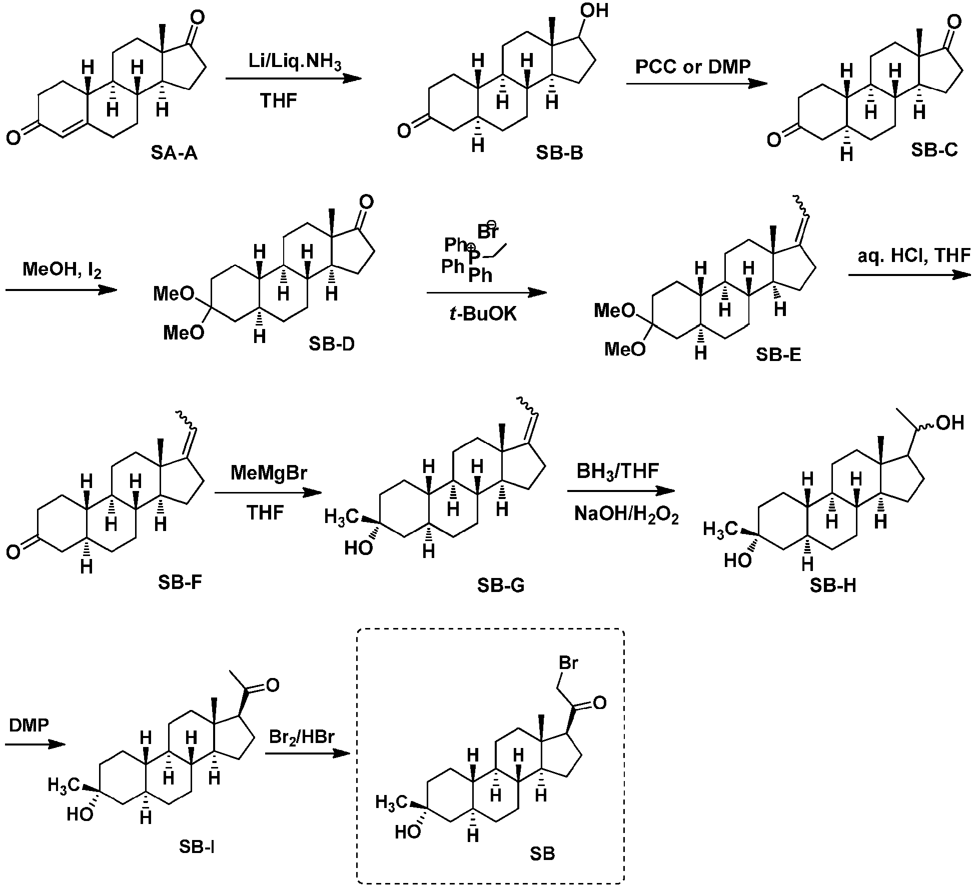

**Synthesis of compounds SB-B and SB-C.** Small pieces of lithium (7.63 g, 1.1 mol) were added to 2.7 L of condensed ammonia in a three neck flask at -70 °C. As soon as all lithium was dissolved, the blue solution was warmed to -50 °C. A solution of 19-norandrost-4-ene-3,17-dione **SB-A** (1, 30 g, 110 mmol) and *tert*-BuOH (8.14 g, 110 mmol) in 800 ml of anhydrous tetrahydrofuran was added dropwise and stirred for 90 min until the reaction mixture turned light yellow. Ammonium chloride (70 g) was added and excess ammonia was left to evaporate. The residue was extracted with 0.5N HCl (500 mL) and dichloromethane (500 mL × 2). The combined organic layers were washed with saturated NaHCO₃ solution, dried over Na₂SO₄, filtered and concentrated to give a mixture of **SB-B** and SB-C (21 g, 70%) which was directly used in the next step without further purification. A solution of **SB-B** and **SB-C** (21 g, 76 mmol) in 50 mL of anhydrous dichloromethane was added to a suspension of pyridinium chlorochromate (PCC) (32.8 g, 152 mmol) in 450 mL of dichloromethane. After stirring at room temperature for 2h, 2N NaOH solution (500 mL) was added to the dark brown reaction mixture and stirred for another 10 min. The resulting solution was extracted with dichloromethane, the combined organic layers were washed with 2N HCl, brine, dried over Na₂SO₄, filtered and concentrated. The residue was purified by chromatography on silica gel (pertroleum ether/ethyl acetate = 20:1 to 10:1) to afford title compound **SB-C** (16.8 g, 80%) as an off white solid. **¹H NMR** of **SB-B** (400 MHz, CDCl₃), δ (ppm), 3.65 (t, 1H, 1H), 0.77 (s, 3H). **¹H NMR** of **SB-C** (400 MHz, CDCl₃), δ (ppm), 0.88 (s, 3H) .

**Synthesis of compound SB-D**. To a solution of compound **SB-C** (16.8 g. 61.3 mmol) in methanol (250 mL) was added iodine (1.54 g, 6.1 mmol). After stirring at 60°C for 12h, the solvent was removed in vacuo. The crude product was dissolved in dichloromethane (200 mL) and washed with saturated NaHCO₃ (150 mL), brine, dried over Na₂SO₄, filtered and concentrated. The residue was purified by chromatography on basic alumina (pertroleum ether/ ethyl acetate = 100:1) to give compound **SB-D** (14 g, 43.8 mmol, 71%). **¹H NMR** (400 MHz, CDCl₃), δ (ppm), 3.18 (s, 3H), 3.12 (s, 3H), 0.85 (s, 3H).

**Synthesis of compound SB-E.** To a suspension oft-BuOK (7.36 g, 65.7 mmol) in THF (100 mL) at 0 °C was added ethyltriphenylphosphonium bromide (26 g, 70 mmol) slowly. After stirring at 60°C for 3h, compound **SB-D** (7g, 21.9 mmol) was added and the mixture was stirred at 60°C for another 2h. After cooling to room temperature, the reaction mixture was poured into saturated ammonium chloride and extracted with EtOAc (2 × 500 mL). The combined organic layers were washed with brine, dried over sodium sulfate, filtered and concentrate to afford the crude compound **SB-E** (7.36 g, 100%). The crude product was used in the next step without further purification.

**Synthesis of compound SB-F.** A solution of crude compound **SB-E** (7.36g, 21.9 mmol) in THF ( 50 mL) was acidified to pH = 3 by 1N aqueous HCl. After stirring at room temperature for 12 h, the reaction mixture was extracted with ethyl acetate (250 mL × 3). The combined organic layers were washed with brine, dried over sodium sulfate, filtered and concentrated. The residue was purified by column chromatography (pertroleum ether/ethyl acetate = 30:1 to 20:1) to afford compound **SB-F** (4.8 g, 16.7 mmol, 76% for two steps). **¹H NMR** (400 MHz, CDCl₃), δ (ppm), 5.12-5.10 (m, 1H), 0.77 (s, 3H).

**Synthesis of compound SB-G.** To a solution of MeMgBr (28 mmol, 1M in THF) in THF (50 mL) at 0 °C was added a solution of compound **SB-F** (4.8 g, 16.8 mmol) in dry THF (10 mL) via syringe pump over 30 min. After stirring at 0 °C for 5 h, the reaction mixture was allowed to warm up and stirred at room temperature overnight. The reaction mixture was quenched with iced-cold water and extracted with ethyl acetate (150 mL × 3). The combined organic layers were washed with brine, dried over sodium sulfate, filtered and concentrated. The white residue was purified by flash column chromatography (pertroleum ether/ ethyl acetate = 20:1 to 10:1) to give compound **SB-G** (2.5 g, 8.28 mmol, 49%; Rf = 0.35, petroleum ether/ethyl acetate = 10:1). **¹H NMR** (400 MHz, CDCl₃), δ (ppm), 5.05-5.03 (m, 1H), 1.21 (s, 3H), 0.90 (s, 3H).

**Synthesis of compound SB-H.** To a solution of compound **SB-G** (2 g, 6.62 mmol) in dry THF (50 mL) was added borane-tetrahydrofuran complex (20 mL; 1.0 M solution in THF). After stirring at room temperature for 1 hour, the reaction mixture was cooled in an ice bath then quenched slowly with 10% aqueous NaOH (10 mL) followed by 30% aqueous solution of H₂O₂ (12 mL). After stirring at room temperature for one hour, the mixture was extracted with EtOAc (3 × 100 mL). The combined organic layers were washed with 10% aqueous Na₂S₂O₃ (100 mL), brine (100 mL), dried over MgSO₄, filtered and concentrated to afford crude compound **SB-H** (2g, 100%). The crude product was used in the next step without further purification.

**Synthesis of compound SB-I.** To a solution of crude compound **SB-H** (2 g, 6.62 mmol) in 60 mL of wet dichloromethane (dichloromethane had been shaken with several milliliters of H₂O then separated from the water layer) was added Dess-Martin periodinate (5.5 g, 13 mmol). After stirring at room temperature for 24 h, the reaction mixture was extracted with dichloromethane (3 × 100 mL). The combined organic layers were washed with 10 % aqueous Na₂S₂O₃ (100 mL), brine (100 mL), dried over MgSO₄, filtered and concentrated. The residue was purified by chromatography on silica gel (pertroleum ether/ ethyl acetate = 10:1 to 5:1) to afford compound **SB-I** (1g, 3.14 mmol, 47% for two steps) as an off white solid. **¹H NMR** (400 MHz, CDCl₃), δ (ppm), 2.56 (t, 1H), 2.11 (s and m, 4H), 2.0 (dt, 1H), 1.8 (dm, 2H), 1.54 (m, 6 H) 1.43 (m, 1H), 1.34 (m, 2H),1.20 (m, 12H), 0.7 (m, 2H), 0.62(s, 3H).

**Synthesis of compound SB.** To a solution of compound **SB-I** (600 mg, 1.89 mmol) in MeOH (20 mL) was added 5 drops of HBr (48%) followed by bromine (302 mg, 1.89 mmol). After stirring at room temperature for 1h, the reaction mixture was poured into ice-water then extracted with ethyl acetate (100 mL × 3). The combined organic layers were washed with brine (200 mL), dried over MgSO₄, filtered and concentrated to give crude compound **SB** (600 mg).

### Reference Example 32. Synthesis of compound SB-1.

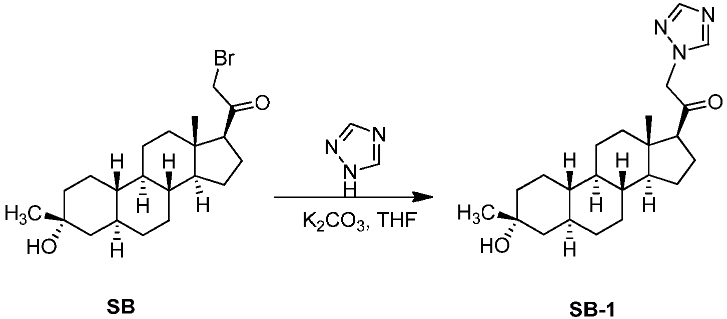

To a suspension of K₂CO₃ (25 mg, 0.18 mmol) in THF (5 mL) was added 1,2,4-triazole (13 mg, 0.18 mmol) and compound **SB** (36 mg, 0.09 mmol). After stirring at room temperature for 15h, the reaction mixture was poured in to 5 mL H₂O and extracted with EtOAc (2 × 10 mL). The combined organic layers were washed with brine, dried over sodium sulfate, filtered and concentrate. The reaction mixture was purified with by reverse-phase prep-HPLC to afford the title compound as an off white solid (15 mg, 42%). **SB-1: ¹HNMR** (500 MHz, CDCl₃), δ (ppm), 8.14 (s, 1H), 7.96 (s, 1H), 5.02 (AB, 1H), 4.93 (AB, J = 18.0 Hz, 1H), 2.63 (t, 1H), 1.21 (s, CH₃), 0.69 (s, 3H).

### Reference Example 33. Synthesis of compounds SB-2.

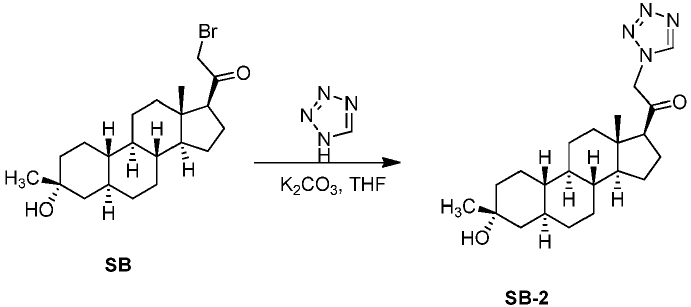

To a suspension of K₂CO₃ (25 mg, 0.18 mmol) in THF (5 mL) was added tetrazole (13 mg, 0.18 mmol) and compound **SB** (36 mg, 0.09 mmol). After stirring at room temperature for 15h, the reaction mixture was poured in to 5 mL H₂O and extracted with EtOAc (2 × 10 mL). The combined organic layers were washed with brine, dried over sodium sulfate, filtered and concentrate. The reaction mixture was purified with by reverse-phase prep-HPLC to afford **SB-2** as an off white solid (7 mg, 19%) and an off white solid byproduct (4 mg, 11%). **SB-2: ¹HNMR** (500 MHz, CDCl₃), δ (ppm), 8.58 (s, 1H), 5.49 (AB, 1H), 5.44 (AB, 1H), 2.63 (t, 1H), 1.21 (s, CH₃), 0.72 (s, 3H).

### Reference Example 34. Synthesis of compounds SB-4 and SB-5.

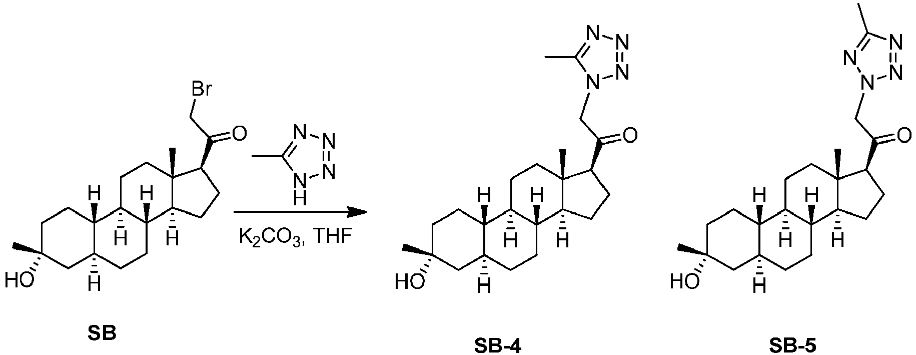

To a suspension of K₂CO₃ (67 mg, 0.50 mmol) in THF (5 mL) was added 5-methyl-1H-tetrazole (42.0 mg, 0.50 mmol) and compound **SB** (100 mg, 0.25 mmol). After stirring at room temperature for 15h, the reaction mixture was poured into 5 mL H₂O and extracted with EtOAc (2 × 10 mL). The combined organic layers were washed with brine (2 × 10 mL), dried over sodium sulfate, filtered and concentrated *in vacuo.* The residue was purified by reverse-phase prep-HPLC to afford **SB-4** as an off white solid ( 10.1 mg, 0.025 mmol, 10.1%) and **SB-5** as an off white solid ( 21.3 mg, 0.053 mmol, 21.2%). **SB-4: ¹HNMR** (500 MHz, CDCl₃) δ (ppm): 5.12 (AB, 1H), 5.06 (AB, 1H), 2.66 (t, 1H), 2.47 (s, 3H), 1.21 (s, CH₃), 0.69 (s, 3H). **LCMS:** Rt = 2.19 min. m/z = 401.3 [M+H]⁺. **SB-5: ¹HNMR** (500 MHz, CDCl₃) δ (ppm): 5.35 (AB, 1H), 5.34 (AB, 1H), 2.63 (t, 1H), 2.56 (s, 3H), 1.21 (s, CH₃), 0.72 (s, 3H). **LCMS:** Rt = 2.30 min. m/z = 401.3 [M+H]⁺.

### Reference Example 35. Synthesis of compounds SB-6.

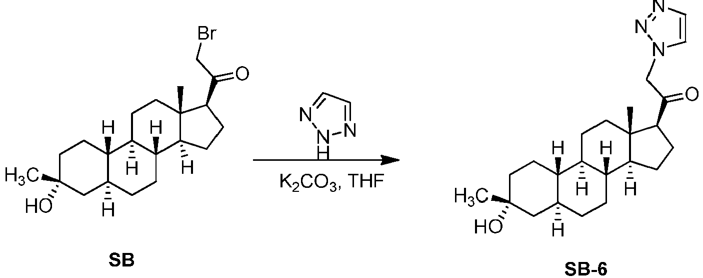

To a suspension of K₂CO₃ (25 mg, 0.18 mmol) in THF (5 mL) was added 1,2,3-1H-Triazole (13 mg, 0.18 mmol) and compound **SB** (36 mg, 0.09 mmol). After stirring at room temperature for 15h, the reaction mixture was poured in to 5 mL H₂O and extracted with EtOAc (2 × 10 mL). The combined organic layers were washed with brine, dried over sodium sulfate, filtered and concentrate. The reaction mixture was purified with by reverse-phase prep-HPLC to afford **SB-6** as an off white solid (12 mg, 33%). **SB-6: ¹HNMR** (500 MHz, CDCl₃), δ (ppm), 7.76 (s, 1H), 7.64 (d, 1H), 5.26 (AB, 1H), 5.14 (AB, 1H), 2.59 (t, 1H), 1.21 (s, 3H), 0.68 (s, 3H).

### Reference Example 36. Synthesis of SD and SD intermediates.

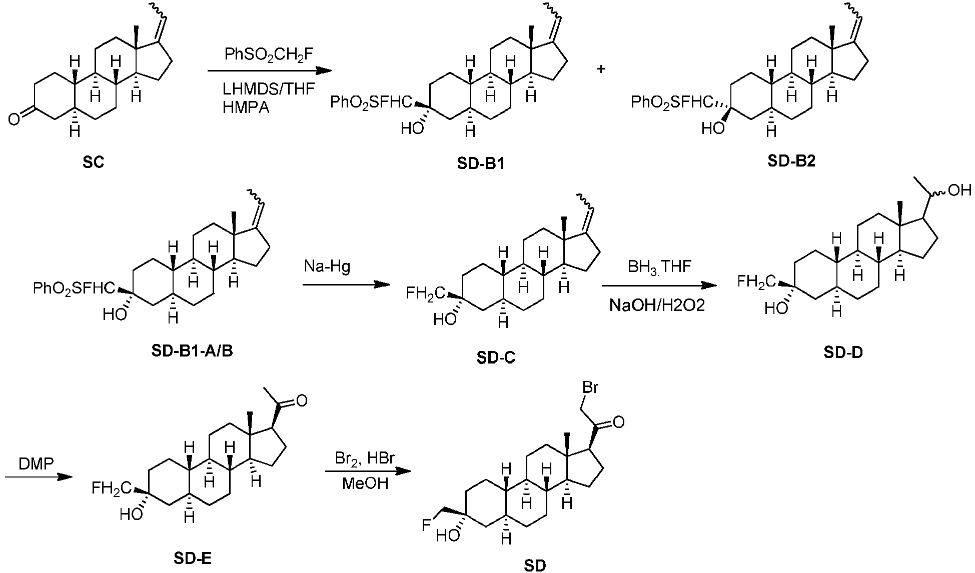

**Synthesis of compound SD-B1 and SD-B2.** To a solution of compound **SC** (1.3g, 4.5 mmol) and PhSO₂CH₂F (790 mg, 4.5 mmol) in THF (25 mL) and HMPA (0.5 mL) at -78 °C under N₂ was added LHMDS (5.5 mL, 1M in THF) dropwise. After stirring at -78 °C for 2 h, the reaction mixture was quenched with saturated aqueous NH₄Cl solution (10 mL) and allowed to warm to room temperature then extracted with Et₂O (20 mL × 3). The combined organic layers were washed with brine, dried over sodium sulfate, filtered and concentrate. The residue was purified by silica gel column chromatography (pertroleum ether/ ethyl acetate =10/1) to give the mixture of compound **SD-B1** and **SD-B2** (1.53 g). The mixture was further purified by chiral-HPLC to afford compound **SD-B1-A** (220 mg, t= 3.41min). **¹H NMR** (500 MHz, CDCl₃), δ (ppm), 7.99-7.97 (m, 2H), 7.75-7.74 (m, 1H), 7.62-7.55 (m, 2H), 5.13-5.09 (m, 1H), 4.86-4.78 (d, 1H), 0.88 (s, 3H); **SD-B1-B** (200 mg, t= 3.66 min); **¹H NMR** (500 MHz, CDCl3), δ (ppm), 7.96-7.95 (m, 1H), 7.71-7.69 (m, 1H), 7.62-7.58 (m, 2H), 5.13-5.09 (m, 1H), 4.87-4.77 (d, 1H), 0.88 (s, 3H); **SD-B2-A** (235 mg, t= 4.9min). **¹H NMR** (500 MHz, CDC13), δ (ppm), 7.99-7.97 (m, 1H), 7.72-7.70 (m, 1H), 7.62-7.59 (m, 2H), 5.29-5.20 (d, 1H), 4.88-4.78 (m,1H), 0.88 (s, 3H); **SD-B2-B** (220 mg, t= 5.2 min). **¹H NMR** (500 MHz, CDC13), δ (ppm), 7.99-7.97 (m, 2H), 7.72 (m, 1H), 7.62-7.59 (m, 2H), 5.30-5.20 (d, 1H), 5.09-5.08 (m,1H), 0.88 (s, 3H).

**Synthesis of compound SD-C**. To a solution of compound **SD-B1-A** (200 mg, 0.434 mmol) and anhydrous Na₂HPO₄ (100 mg) in anhydrous methanol (15 mL) at -20 °C under N₂ was added Na/Hg amalgam (400 mg). After stirring at -20 °C to 0 °C for 1 h, the methanol solution was decanted out and the solid residue was washed with Et₂O (5 × 3 mL). The solvent of combined organic phase was removed under vacuum, and 20 ml brine was added, followed by extracting with Et2O. The combined ether phase was dried with MgSO4, and the ether was removed to give the crude product, which was further purified by silica gel chromatography (PE/EA=10/1) to give product 99 mg, 69%. **¹H NMR** (500 MHz, CDC13), δ (ppm), 5.12-5.10 (m, 1H,), 4.21-24.11 (d, 2H), 0.88 (s, 3H).

**Synthesis of compound SD-D.** To a solution of compound **SD-C** (95 mg, 0.296 mmol) in dry THF (5 mL) was added borane-tetrahydrofuran complex (1 mL of 1.0 M solution in THF). After stirring at room temperature for 1 hour, the reaction mixture was cooled in an ice bath then quenched slowly with 10% aqueous NaOH (1 mL) followed by 30% aqueous solution of H₂O₂ (1.2 mL). The mixture was allowed to stir at room temperature for 1 hour then extracted with EtOAc (3 × 10 mL). The combined organic layers were washed with 10% aqueous Na₂S₂O₃ (10 mL), brine (10 mL), dried over MgSO₄, filtered and concentrated to afford compound **SD-D** (120mg crude). The crude product was used in the next step without further purification.

**Synthesis of compound SD-E.** To a solution of compound **SD-D** (120 mg crude) was dissolved in 10 mL of wet dichloromethane (dichloromethane had been shaken with several milliliters of H₂O then separated from the water layer) was added Dess-Martin periodinate (300 mg, 707 mmol). After stirring at room temperature for 24 h, the reaction mixture was extracted with dichloromethane (3 × 10 mL). The combined organic layers were washed with 10 % aqueous Na₂S₂O₃ (10 mL), brine (10 mL), dried over MgSO₄, filtered and concentrated. The residue was purified by chromatography on silica gel (pertroleum ether/ ethyl acetate = 1: 5) to afford compound **SD-E** (70 mg, 70% for two steps) as an off white solid. **¹H NMR** (500 MHz, CDC13), δ (ppm), 4.21-4.11 (d, 2H), 2.19 (s, 3H), 0.62 (s, 3H).

**Synthesis of compound SD.** To a solution of reactant (200 mg, 0.594 mmol) in methanol (5 mL) was added 48% hydrobromic acid (300 mg, 1.782 mmol) followed by bromine (475 mg, 0.152 mL, 2.97 mmol). The solution was heated at 25 °C for 2 hours. Then the mixture was poured into cooled water (50 mL). The resulting solid was extracted with ethyl acetate (2×100 mL). The combined organic extracts were washed with brine (100 mL), dried over magnesium sulfate and concentrated in vacuo. The crude product was used directly without further purification in the next step.

### Reference Example 37. Synthesis of compounds SD-1.

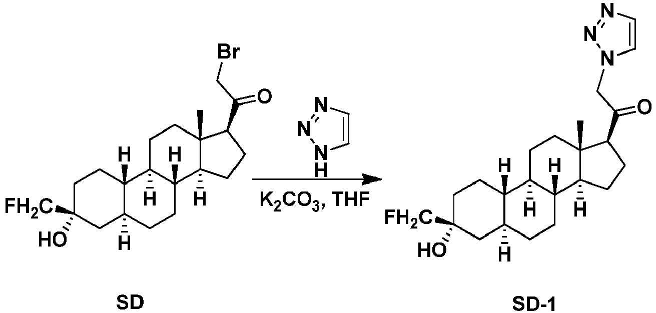

To a suspension of K₂CO₃ (63 mg, 0.47 mmol) in THF (10 mL) was added 1,2,3-1H-Triazole (11.4 mg, 0.47 mmol) and compound **SD** (100 mg, 0.23 mmol). After stirring at room temperature for 15h, the reaction mixture was poured into 5 mL H₂O and extracted with EtOAc (2 × 10 mL). The combined organic layers were washed with brine (2 × 10 mL), dried over sodium sulfate, filtered and concentrated under vacuum. The residue was purified by reverse-phase prep-HPLC to afford **SD-1** as an off white solid ( 28.7 mg, 29.5%) and SGE-00921-01-A as an off white solid ( 22.8 mg, 23.4%). **SD-1: ¹HNMR** (500 MHz, CDCl₃), δ (ppm), 7.76 (d, 1H), 7.65 (d, 1H), 5.28 (AB, 1H), 5.14 (AB, 1H), 4.17 (d, 2H), 2.66 (t, 1H), 0.68 (s, 3H). **LCMS:** Rt = 2.18 min. m/z = 404.2 [M+H]⁺.

### Reference Example 38. Synthesis of compounds SD-2 and SD-3.

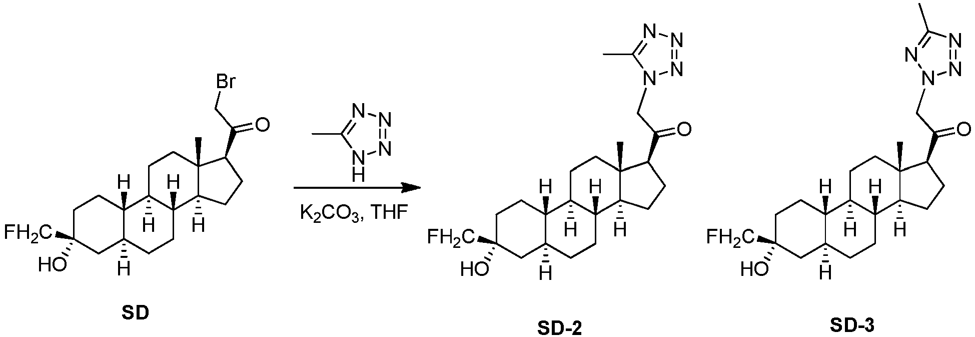

To a suspension of K₂CO₃ (63 mg, 0.47mmol) in THF (10mL) was added 5-methyl-1H-tetrazole (39.5 mg, 0.47mmol) and compound **SD** (100 mg, 0.24mmol). After stirring at room temperature for 15h, the reaction mixture was poured into 5mL H₂O and extracted with EtOAc (2 × 10mL). The combined organic layers were washed with brine(2 × 10mL), dried over sodium sulfate, filtered and concentrated in vacuum. The residue was purified by reverse-phase prep-HPLC to afford **SD-2** as an off white solid ( 6.5 mg, 0.016 mmol, 6.7%) and **SD-3** as an off white solid ( 25.8 mg, 0.062 mmol, 25.8%). **SD-2: ¹HNMR** (500 MHz, CDCl₃) δ (ppm): 5.12 (AB, 1H), 5.06 (AB, 1H), 4.17 (d, J = 47.8 Hz, 2H), 2.67 (t, 1H), 2.47 (s, 3H), 0.69 (s, 3H). **LCMS:** Rt = 2.11 min. m/z = 419.3 [M+H]⁺. **SD-3: ¹HNMR** (500 MHz, CDCl₃) δ (ppm): 5.35 (AB, 1H), 5.34 (AB, 1H), 4.17 (d, 2H), 2.63 (t, 1H), 2.56 (s, 3H), 0.72 (s, 3H). **LCMS:** Rt = 2.21 min. m/z = 419.3 [M+H]⁺.

### Reference Example 39. Synthesis of compounds SD-4 and SD-5.

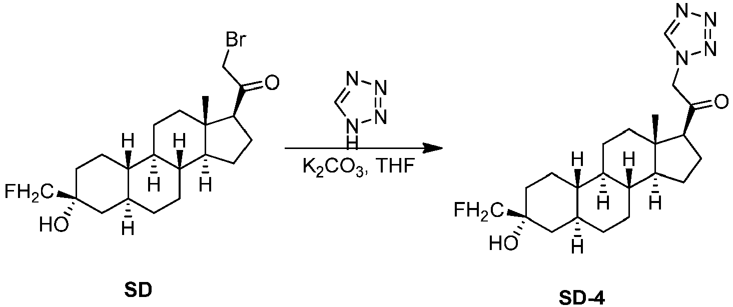

To a solution of crude reactant 11(100 mg, 0.241 mmol) in anhydrous THF (5 mL) was added (140 mg, 1.2 mmol) followed by potassium carbonate (85 mg, 1.2 mmol). The solution was heated at 60 °C for 2h then the solution was cooled to room temperature and diluted with ethyl acetate (100 mL). The resulting solution was washed with brine (2×50 mL), dried over magnesium sulfate and concentrated *in vacuo.* The crude product was purified by reverse phase prep-HPLC to afford product **SD-4** (15mg, 0.04mmol, Yield=17%) and an off white solid byproduct (26 mg, 0.06mmol, Yield=25%). **SD-4:** ¹HNMR (500 MHz, CDC13) δ(ppm): 8.75 (1H, s),5.32 (1H, AB, J=18.5Hz), 5.18 (1H, AB), 4.17 (2H, d), 2.68 (1H, t), 0.68 (3H, s). **LCMS:** rt=2.14min, *m*/*z*=405 [M+H]⁺

### Reference Example 40. Synthesis of SP and SP intermediates.

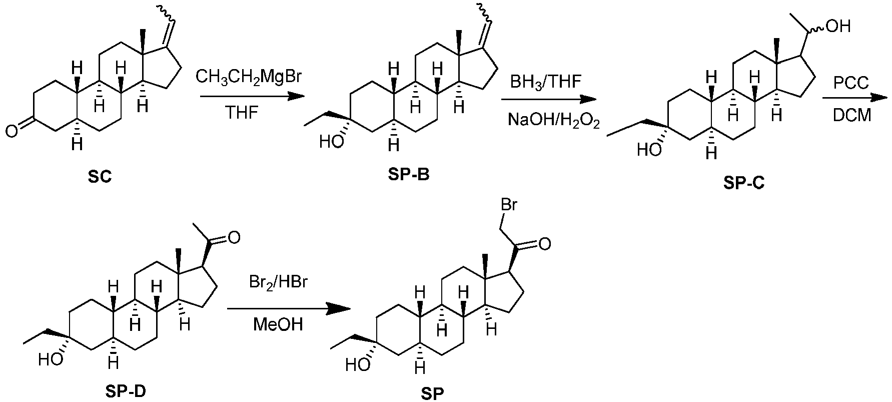

**Synthesis of compound SP-B.** To a solution of reactant SC (4.4 g, 15.38 mmol) in dry THF (50 mL) was added ethylmagnesium bromide (3M in THF, 51.28 mL) dropwise at 0°C. The solution was then slowly warmed and stirred at ambient temperature for 15h. Sat. NH₄Cl solution (20mL) was added to quench the reaction and the resulting solution was extracted with ethyl acetate (3×100mL). The extracts were washed with brine, dried over Na₂SO₄ and concentrated *in vacuo.* The residue was purified by flash chromatography (eluant: petroleum ether: ethyl acetate=10:1) to afford product **SP-B** (3.15g, 10.00 mmol, 64.8%) as an off white solid.

**Synthesis of compound SP-C.** To a solution of reactant **SP-B** (500 mg, 1.58 mmol) in anhydrous THF (10 mL) was added BH₃.THF (1.0 M, 7.23 mL, 7.23 mmol) at room temperature, and the solution was stirred at 25 °C overnight. Then the reaction was quenched by addition of water (5 mL), 2 M NaOH solution (10 mL) was added followed by 30 % H₂O₂ (10 mL). The resulting mixture was stirred at room temperature for 1 hour. Then the mixture was diluted with ethyl acetate (200 mL) and resulting solution was washed with brine (2×100 mL), dried over magnesium sulfate and concentrated *in vacuo.* The crude product SP-C was used directly in the next step without further purification.

**Synthesis of compound SP-D.** To a solution of reactant **SP-C** (6.53 g, 19.67 mmol) in anhydrous DCM (100mL) cooled in an ice-water cooling bath was added pyridinium chlorochromate (8.48g, 39.34mol) in portions. The mixture was stirred at ambient temperature overnight. The solution was then diluted with DCM (50mL) and filtered. The combined organic solutions were washed with brine (100mL), dried over Na₂SO₄ and concentrated *in vacuo.* The residue was purified by flash chromatography (eluant: petroleum ether: elthyl acetate=10:1) to afford product **SP-D** (2.5g, 7.53mmol, yield39%) as an off white solid. **SP-D:** ¹HNMR (500 MHz, CDC13) δ(ppm): 2.54 (1H, t), 2.11 (3H,s), 1.42-1.45 (2H, q), 0.91 (3H, t), 0.62 (3H, s).

**Synthesis of compound SP.** To a solution of reactant **SP-D** (80 mg, 0.24 mmol) in methanol (5 mL) was added 48% hydrobromic acid (148 mg, 0.884mmol) followed by bromine (241 mg, 0.077 mL, 1.505 mmol). The solution was heated at 25 °C for 1.5 hours, then the mixture was poured into cooled water (50 mL). The resulting solid was extracted with ethyl acetate (2×50 mL). The combined organic extracts were washed with brine (20 mL), dried over magnesium sulfate and concentrated *in vacuo.* The crude product **SP** was used directly without further purification in the next step.

### Reference Example 41. Synthesis of compounds SP-1 and SP-2.

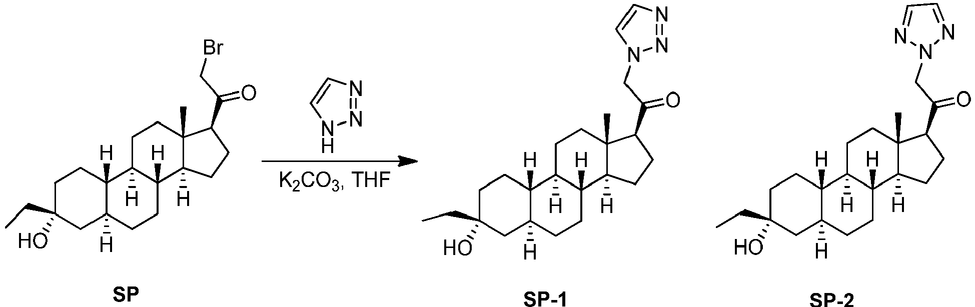

To a solution of crude reactant **SP** (500 mg, 1.2 mmol) in anhydrous THF (10 mL) was added 1,2,4-1H-Triazole (500 mg, 6.0 mmol) followed by potassium carbonate (1.02g, 6 mmol). The solution was heated at 60 °C for 2h, then the solution was cooled to room temperature and diluted with ethyl acetate (100 mL). The resulting solution was washed with brine (2×50 mL), dried over magnesium sulfate and concentrated *in vacuo.* The crude product was purified by reverse phase prep-HPLC to afford product **SP-1** (105 mg, 0.26 mmol, Yield=22%) and **SP-2** (62 mg, 0.15 mmol, Yield=13%) as off white solid. **SP-1:** ¹HNMR (500 MHz, CDC13) δ(ppm): 7.75(1H,s), 7.64(1H,s), 5.26(1H, AB), 5.14(1H, AB),2.66 (1H,t), 0.91 (3H, t), 0.68 (3H, s). **LCMS:** rt=2.35min, *m*/*z*=400 [M+H]⁺. **SP-2:** ¹HNMR (500 MHz, CDC13) δ(ppm): 7.68(2H, s), 5.25(1H, AB), 5.23(1H, AB, 2.59 (1H,t), 0.91 (3H, t), 0.70 (3H, s). **LCMS:** rt=2.49min, *m*/*z*=400 [M+H]⁺

### Reference Example 42. Synthesis of compound SP-3.

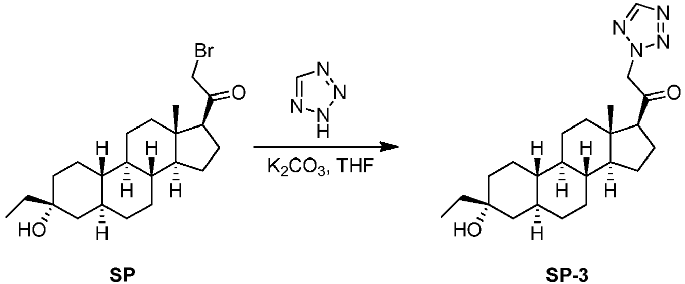

To a solution of crude reactant **SP** (247.5 mg, 0.603 mmol, theoretical amount) in THF (5 mL) was added tetrazole (84 mg, 1.202 mmol) followed by potassium carbonate (166 mg, 1.202 mmol) and the mixture was heated at 50 °C for 2 hours. Then the reaction mixture was diluted with ethyl acetate (100 mL). The resulting solution was washed with brine (2×50 mL), dried over magnesium sulfate and concentrated *in vacuo.* The residue was purified by reverse phase prep-HPLC to afford desired product **SP-3** (14.4 mg, 0.0359 mmol, Yield=6.0% (2 steps)) as off white solid. Another desired product was not obtained in prep-HPLC purification due to its very weak absorption (214 nm, 254 nm). **SP-3:** ¹HNMR (400 MHz, CDC13) δ(ppm): 8.57 (1H, s), 5.46 (1H, AB), 5.45 (1H, AB), 2.65 (1H, t), 1.45 (2H, q), 0.91 (3H, t), 0.73 (3H, s). **LCMS:** rt=2.48 min, *m*/*z*=401.1 [M+H]⁺

### Reference Example 43. Synthesis of compounds SP-4 and SP-5.

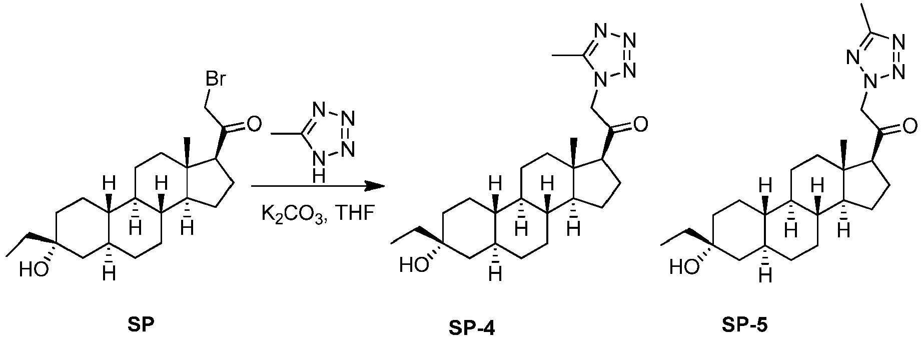

To a suspension of K₂CO₃ (67 mg, 0.50 mmol) in THF (5 mL) was added 5-methyl-1H-tetrazole (42.0 mg, 0.50 mmol) and compound **SP** (100 mg, 0.24 mmol). After stirring at room temperature for 15h, the reaction mixture was poured into 5 mL H₂O and extracted with EtOAc (2 ×10 mL). The combined organic layers were washed with brine (2 ×10 mL), dried over sodium sulfate, filtered and concentrated in vacuum. The residue was purified by reverse-phase prep-HPLC to afford **SP-4** as an off white solid ( 15.2 mg, 0.037 mmol, 15.2%) and **SP-5** as an off white solid ( 13.3 mg, 0.032 mmol, 13.3%). **SP-4: ¹HNMR** (500 MHz, CDCl₃) δ (ppm): 5.13 (AB, 1H), 5.05 (AB, 1H), 2.66 (t, 1H), 2.48 (s, 3H), 0.91(t, 1H), 0.69 (s, 3H). **LCMS:** Rt = 2.30 min. m/z = 415.3 [M+H]⁺. **SP-5: ¹HNMR** (400 MHz, CDCl₃) δ (ppm): 5.36 (AB, 1H), 5.35 (AB, 1H), 2.63 (t, 1H), 2.58 (s, 3H), 0.91(t, 1H), 0.72 (s, 3H). **LCMS:** Rt = 2.38 min. m/z = 415.3 [M+H]⁺.

### Example 44. Synthesis of SI and SI intermediates.

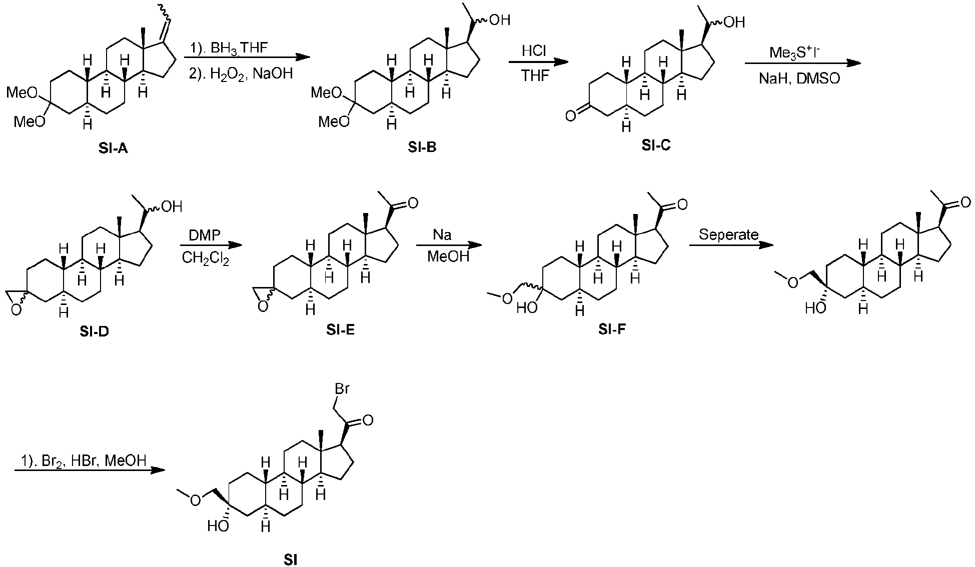

**Synthesis of compound SI-B.** To a solution of compound **SI-A** (5 g, 15 mmol) in dry THF (20 mL) was added borane-tetrahydrofuran complex (30 mL of 1.0 M solution in THF) and the reaction mixture was stirred at ambient temperature for 1 hour then 10 % aqueous NaOH (56 mL) was slowly added. The mixture was cooled in ice and 30 % aqueous solution of H₂O₂ (67mL) was slowly added. The mixture was stirred at ambient temperature for 1 hour and then extracted with EtOAc (3 x 100 mL). The combined EtOAc extracts were washed with 10 % aqueous Na₂S₂O₃ (100 mL), brine (100 mL), dried over MgSO₄. Filtration and removal of the solvent gave the crude product 3.2 g for next step reaction.

**Synthesis of compound SI-C.** To a solution of compound **SI-B** (3.2 g, 9 mmol) in THF (40 mL) was added 2M HCl (3 mL). The reaction solution was stirred at RT for 12h then the solvent was removed under reduced pressure. The crude target compound was purified by silica gel chromatography (eluant: petroleum ether/ethyl acetate = 10:1 to 5:1) to give 2.2 g of the product as an off white solid, yield:81.40%.

**Synthesis of compound SI-D.** To a stirred solution of trimethylsufonium iodide (6.43 g, 31.5 mmol) in 100 mL of DMSO was added 60wt% NaH (1.26 g, 31.5 mmol). After stirring at room temperature (15°C) for 1h, a solution of compound **SI-C** (2.2 g, 7.2 mmol) in 20 mL of DMSO was added dropwise. After 2.5 h, the reaction mixture was poured into ice-cold water and extracted with ether (100 mLx3). The combined ether layers were then washed with brine (100 mLx3), dried (MgSO₄), filtered, and concentrated to give the crude product 1.6 g for next step reaction.

**Synthesis of compound SI-E.** Compound **SI-D** (1.6 g, 5 mmol) was dissolved in 60 mL of H₂O saturated CH₂Cl₂. (Using a separatory funnel, the CH₂Cl₂ had been shaken with several milliliters of H₂O and then separated from the water layer). DMP was added (4.2 g, 10 mmol), and the resultant reaction mixture was vigorously stirred for 24 h. The reaction solution was diluted with DCM (100 mL), washed with 10 % aqueous Na₂S₂O₃ (100 mL), brine (100 mL), dried over MgSO₄, filtered, and concentrated. The residue was purified by chromatography on silica gel (eluant: petroleum ether/ethyl acetate = 20:1 to 10:1) to afford title compound (1.2 g, 3.79 mmol, 75%) as an off white solid. **¹H NMR** (400 MHz, CDC13) δ (ppm): 2.63 (s, 1H), 2.59 (s, 1H), 2.12 (s, 3H), 0.63 (s, 3H) .

### Synthesis of compounds SI-F1 and SI-F2.

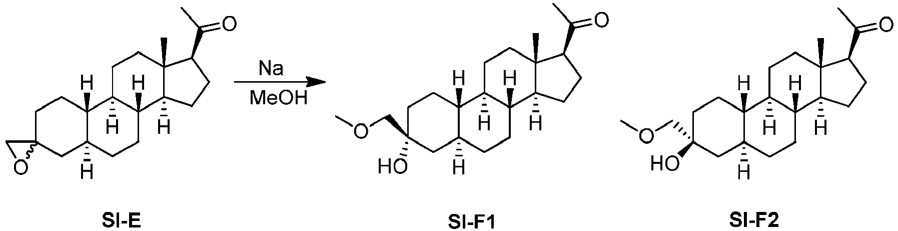

**SI-E** (1.2 g, 3.8 mmol) was dissolved in dry methanol (250 mL), and Na (262 mg, 11.4 mmol) was added. The solution was refluxed for 16 h. Methanol was evaporated off and the residue was dissolved in dichloromethane and washed with H₂O (3 × 50 mL) and brine (100 mL), dried over MgSO₄, filtered, and concentrated. The crude target compound was purified by silica gel chromatography (eluant: petroleum ether/ethyl acetate = 10:1 to 5:1) to give **SI-F1** (300 mg, 25%) , **SI-F2** (300mg, 25%) as an off white solid. **SI-F1: ¹H NMR** (400 MHz, CDC13) δ (ppm): 3.39 (s, 3H), 3.19 (s, 2H), 2.54 (t, 1H), 2.11(s, 3H), 0.61 (s, 3H). **SI-F2: ¹H NMR** (400 MHz, CDC13) δ (ppm): 3.39 (s, 5H), 3.37 (s, 2H), 2.52 (t, 1H), 2.11 (s, 3H), 0.62 (s, 3H).

**Synthesis of compound SI.** A solution of **SI-F1** (50 mg, 0.14mmol) in MeOH and was treated with 2 drops of HBr(48%) followed by bromine (6 drops),. The mixture was stirred at rt for 1h and was poured into ice-water. The mixture was extracted with EA (50 mL) and dried over sodium sulfate. Filtration

### Reference Example 45. Synthesis of SI-1.

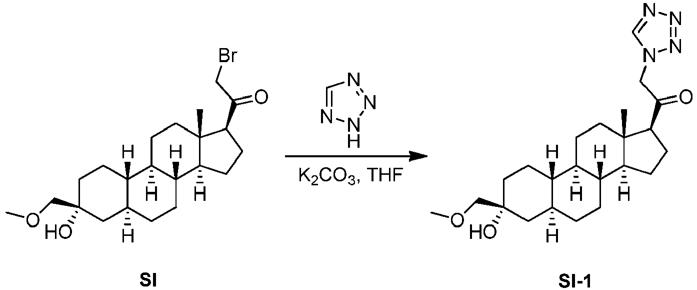

To a solution of crude reactant (245.3 mg, 0.574 mmol, theoretical amount) in THF (5 mL) was added tetrazole (201 mg, 2.87 mmol) followed by potassium carbonate (397 mg, 2.87 mmol). The mixture was heated at 60 °C overnight. Then the solution was diluted with ethyl acetate (100 mL). The resulting solution was washed with brine (2×50 mL), dried over magnesium sulfate and concentrated *in vacuo.* The residue was purified by reverse phase prep-HPLC to afford fraction 1 and fraction 2. Fraction 2 was pure product SI-1 (27.5 mg, 0.066 mmol, two steps overall yield=11.5%) as off white solid. Fraction 1 was additionally purified by silica gel chromatography (eluant: petroleum ether / ethyl acetate = 1: 4) to afford an off white solid byproduct (8.2 mg, 0.0197 mmol, two steps overall yield=3.49%). **SI-1:** ¹HNMR (500 MHz, CDCl₃) δ(ppm): 8.75 (1H, s), 5.32 (1H, AB), 5.21 (1H, AB), 3.39 (3H, s), 3.19 (2H, s), 2.67 (1H, t), 0.68 (3H, s). **LC-MS:** rt = 2.19 min, m/z =417.3 [M+H]⁺

### Reference Example 46. Synthesis of compounds SI-2.

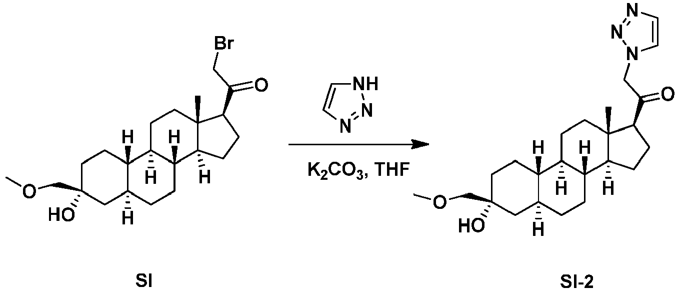

To a suspension of K₂CO₃ (248 mg, 1.8 mmol) in THF (50 mL) was added 1,2,3-1H-triazole (130 mg, 1.8 mmol) and compound **SI** (400 mg, 0.94 mmol). After stirring at room temperature for 15h, the reaction mixture was poured in to 50 mL H₂O and extracted with EtOAc (2 × 100 mL). The combined organic layers were washed with brine, dried over sodium sulfate, filtered and concentrate. The reaction mixture was purified with by reverse-phase prep-HPLC to afford **SI-2** as an off white solid (80 mg, 20%). **SI-2: ¹HNMR** (500 MHz, CDCl₃), δ (ppm), 7.76 (d, 1H), 7.64 (d, 1H), 5.27 (AB, 1H), 5.13 (AB, 1H), 3.39 (s, 3H), 3.19 (s, 2H), 2.66 (t, 1H), 0.68 (s, 3H).

### Example 47. Synthesis of compounds SI-3 and SI-4.

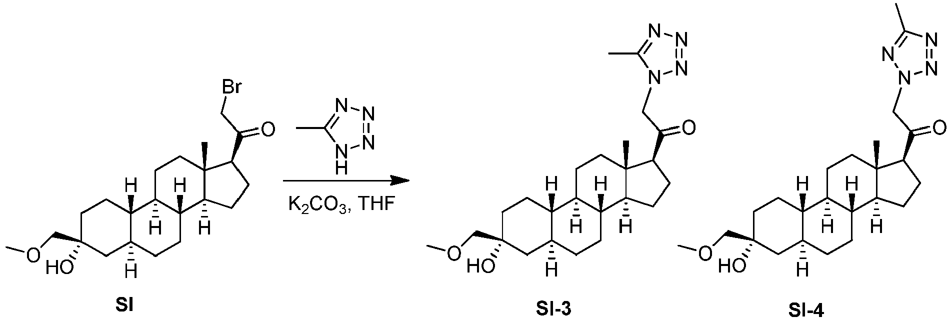

To a suspension of K₂CO₃ (67 mg, 0.50 mmol) in THF (5 mL) was added 5-methyl-1H-tetrazole (42.0 mg, 0.50 mmol) and compound **SI** (100 mg, 0.23 mmol). After stirring at room temperature for 15h, the reaction mixture was poured into 5 mL H₂O and extracted with EtOAc (2 × 10 mL). The combined organic layers were washed with brine (2 × 10 mL), dried over sodium sulfate, filtered and concentrated *in vacuo.* The residue was purified by reverse-phase prep-HPLC to afford **SI-3** as an off white solid ( 12.6 mg, 0.029 mmol, 12.7%) and **SI-4** as an off white solid ( 22.3 mg, 0.052 mmol, 22.5%). **SI-3: ¹HNMR** (500 MHz, CDCl₃) δ (ppm): 5.13 (AB, 1H), 5.05 (AB, 1H), 3.39 (s, 3H), 3.19 (s, 2H), 2.66 (t, 1H), 2.47 (s, 3H), 0.69 (s, 3H). **LC-MS:** Rt = 2.14 min. m/z = 431.3 [M+H]⁺. S**I-4: ¹HNMR** (500 MHz, CDCl₃) δ (ppm): 5.35 (AB, 1H), 5.34 (AB, 1H), 3.39 (s, 3H), 3.19 (s, 2H), 2.63 (t, 1H), 2.56 (s, 3H), 0.72 (s, 3H). LC-MS: Rt = 2.25 min. m/z = 401.3 [M+H]⁺.

### Reference Example 48. Synthesis of SQ and SQ intermediates.

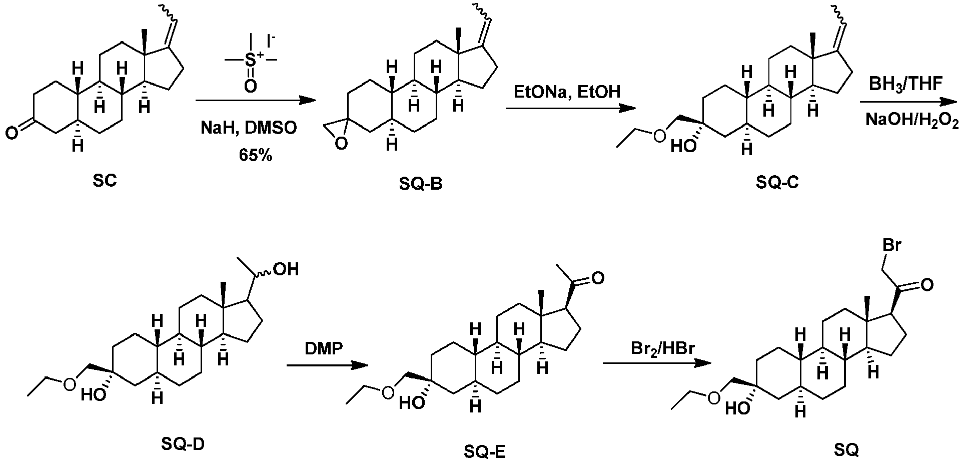

**Synthesis of compound SQ-B.** To a stirred solution of trimethylsulfonium iodide (8.1 g, 36.9 mmol) in 100mL of DMSO was added NaH (60%; 1.26 g, 31.5 mmol). After stirring at room temperature for 1h, a suspension of compound **SC** (2.2 g , 7.2 mmol) in DMSO (20 mL) was added dropwise. The mixture was stirred for another 2.5 h, then poured into ice-cold water and extracted with ether (100 mL x 3). The combined ether layers were then washed with brine (100 mLx 3), dried over MgSO₄, filtered, and concentrated to give the crude product **SQ-B** (2.2 g). The crude product was used in the next step without further purification.

**Synthesis of compound SQ-C.** Compound **SQ-B** (2.2 g, 7.3 mmol) was dissolved in dry methanol (250 mL), and Na (672 mg, 29.2 mmol) was added. The solution was stirred reflux for 6 h. Methanol was evaporated off and the residue was dissolved in dichloromethane and washed with H₂O (3 × 50 mL) and brine (100 mL), dried over MgSO₄, filtered, and concentrated. The crude target compound was purified by via silica gel chromatography (pertroleum ether/ethyl acetate = 10:1 to 5:1),and concentrated to give **SQ-C** (1.8 g, 82%) as an off white solid.

**¹H NMR** (500 MHz, CDCl₃), δ (ppm), 5.03-5.01 (m, 1H), 3.43 (q, 2H), 3.13 (s, 2H), 0.80 (s, 3H) . **Synthesis of compound SQ-D.** To a solution of compound **SQ-C** ( 1.8 g, 5.2 mmol) in dry THF ( 50 mL) was added borane-tetrahydrofuran complex ( 20 mL of 1.0 M solution in THF). After stirring at room temperature for 1 hour, the reaction mixture was cooled in an ice bath then quenched slowly with 10% aqueous NaOH (10 mL) followed 30% aqueous solution of H₂O₂ (12mL). The mixture was allowed to stir at room temperature for 1 hour then extracted with EtOAc (3 × 100 mL). The combined organic layers were washed with 10% aqueous Na₂S₂O₃ (100 mL), brine (100 mL), dried over MgSO₄, filtered and concentrated to afford crude compound **SQ-D** ( 1.8g, 100%). The crude product was used in the next step without further purification.

**Synthesis of compound SQ-E.** To a solution of crude compound **SQ-D** ( 1.8g, 5.2mmol ) was dissolved in 60 mL of H₂O saturated dichloromethane (dichloromethane had been shaken with several milliliters of H₂O then separated from the water layer) was added Dess-Martin periodinate ( 4.4g, 10.4 mmol). After stirring at room temperature for 24 h, the reaction mixture was extracted with dichloromethane (3 × 100 mL). The combined organic layers were washed with 10 % aqueous Na₂S₂O₃ (100 mL), brine (100 mL), dried over MgSO₄, filtered and concentrated. The residue was purified by chromatography on silica gel (pertroleum ether/ ethyl acetate = 10:1 to 5:1) to afford **SQ-E** ( 1g, 2.8 mmol, 56% for two steps) as an off white solid.

**¹H NMR** (400 MHz, CDCl₃), δ (ppm), 3.52 (q, 2H), 3.21 (s, 2H), 2.54 (t, 2H), 2.11 (s, 3H), 1.20 (t, 3H), 0.61 (s, 3H). **LCMS:** Rt = 7.25 min. m/z = 345.1 [M-17]⁺.

**Synthesis of compound SQ.** To a solution of compound **SQ-E** (600 mg, 1.65 mmol) in MeOH (20 mL) was added 5 drops of HBr (48%) followed by bromine (264 mg, 1.65 mmol). After stirring at room temperature for 1h, the reaction mixture was poured into ice-water then extracted with ethyl acetate (100 mL x 3). The combined organic layers were washed with brine (200 mL), dried over MgSO₄, filtered and concentrated to give crude compound **SQ** (600 mg, 100%). The crude product was used in the next step without further purification. **LCMS:** Rt = 7.25 min. m/z = 463.1 [M+Na]⁺.

### Reference Example 49. Synthesis of compound SQ-1 and SQ-2.

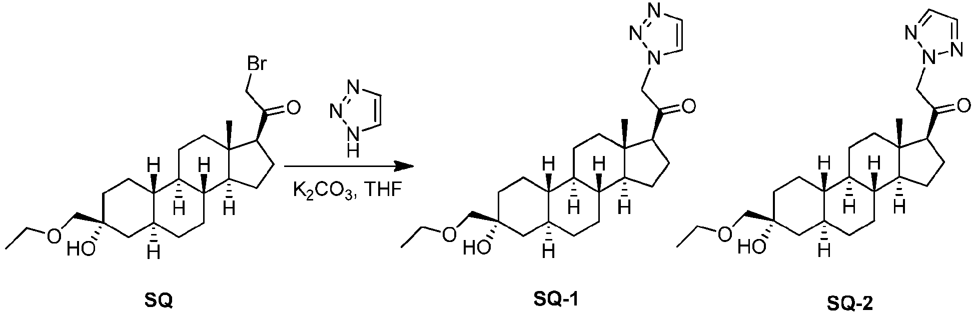

To a suspension of K₂CO₃ (188 mg, 1.36 mmol) in THF (10 mL) was added 1,2,3-1H-Triazole (94 mg, 1.36 mmol) and compound **SQ** (300 mg, 0.68 mmol). After stirring at room temperature for 15h, the reaction mixture was poured into 5 mL H₂O and extracted with EtOAc (2 × 10 mL). The combined organic layers were washed with brine(2 × 10 mL), dried over sodium sulfate, filtered and concentrated under vacuum. The residue was purified by reverse-phase prep-HPLC to afford **SQ-1**as an off white solid ( 81 mg, 0.19 mmol, 27.9%) and **SQ-2** as an off white solid ( 41 mg, 0.10 mmol, 14.7%) . **SQ-1: ¹HNMR** (400 MHz, CDCl₃) δ (ppm): 7.76 (s, 1H), 7.64 (s, 1H), 5.28 (AB, 1H), 5.14 (AB, 1H), 3.53 (q, 2H), 3.22 (s, 2H), 2.66 (t, 1H), 1.20 (t, 3H), 0.68 (s, 3H). **LCMS:** Rt = 2.21 min. m/z = 430.3 [M+H]⁺. **SQ-2: ¹HNMR** (400 MHz, CDCl₃) δ (ppm): 7.69 (s, 2H), 5.27 (AB, 1H), 5.22 (AB, 1H), 3.53 (q, 2H), 3.22 (s, 2H), 2.60 (t, 1H), 1.20 (t, 3H), 0.71 (s, 3H). **LCMS:** Rt = 2.34 min. m/z = 430.3 [M+H]⁺.

### Reference Example 50. Synthesis of compounds SQ-3 and SQ-4.

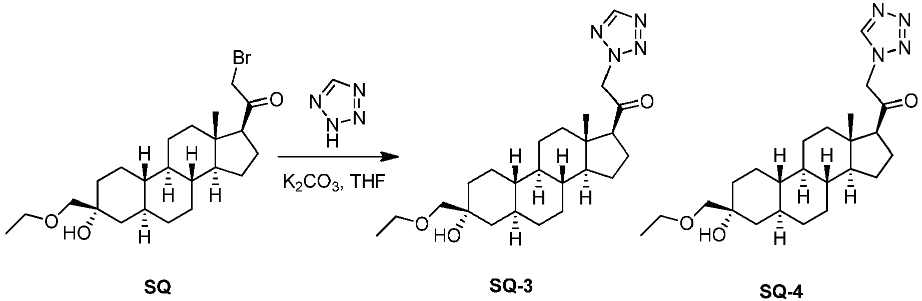

To a suspension of K₂CO₃ (94 mg, 0.68 mmol) in THF (10 mL) was added 1,2,3-1H-Triazole (48 mg, 0.68 mmol) and compound **SQ** (150 mg, 0.34 mmol). After stirring at room temperature for 15h, the reaction mixture was poured into 5 mL H₂O and extracted with EtOAc (2 × 10 mL). The combined organic layers were washed with brine (2 × 10 mL), dried over sodium sulfate, filtered and concentrated under vacuum. The residue was purified by reverse-phase prep-HPLC to afford **SQ-3** as an off white solid (20.9 mg, 0.049 mmol, 14.4%) and **SQ-4** as an off white solid (15.2 mg, 0.035 mmol, 10.3%) . **SQ-3: ¹HNMR** (400 MHz, CDCl₃) δ (ppm): 8.57 (s, 1H), 5.46 (AB, 1H), 5.45 (AB, 1H), 3.53 (q, 2H), 3.22 (s, 2H), 2.66 (t, 1H), 1.21 (t, 3H), 0.72 (s, 3H). **LCMS:** Rt = 2.35 min. m/z = 431.4 [M+H]⁺. **SQ-4: ¹HNMR** (400 MHz, CDCl₃) δ (ppm): 8.74 (s, 1H), 5.32 (AB, J = 18.0 Hz, 1H), 5.18 (AB, J = 18.1 Hz, 1H), 3.52 (q, 2H), 3.22 (s, 2H), 2.68 (t, 1H), 1.20 (t, 3H), 0.68 (s, 3H). **LCMS:** Rt = 2.22 min. m/z = 431.4 [M+H]⁺.

### Reference Example 51. Synthesis of compounds SQ-5 and SQ-6.

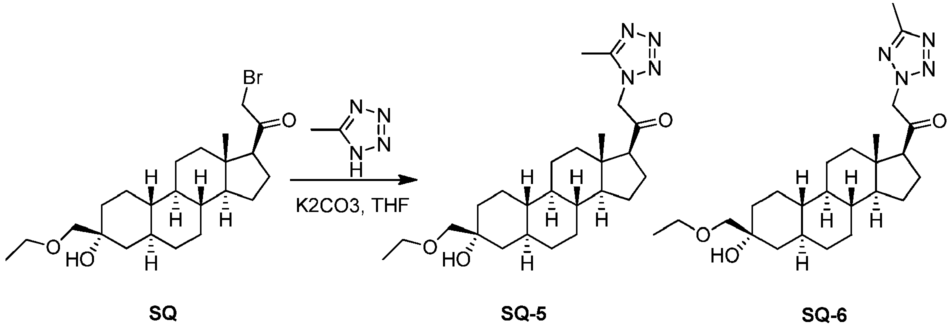

To a suspension of K₂CO₃ (67 mg, 0.50 mmol) in THF (5 mL) was added 5-methyl-1H-tetrazole (42.0 mg, 0.50 mmol) and compound **SQ** (100 mg, 0.25 mmol). After stirring at room temperature for 15h, the reaction mixture was poured into 5 mL H₂O and extracted with EtOAc (2 × 10 mL). The combined organic layers were washed with brine (2 × 10 mL), dried over sodium sulfate, filtered and concentrated in vacuum. The residue was purified by reverse-phase prep-HPLC to afford **SQ-5** as an off white solid ( 8.5 mg, 0.019mmol, 8.1%) and SQ-6 as an off white solid ( 14.8 mg, 0.034mmol, 13.2%). **SQ-5: ¹HNMR** (500 MHz, CDCl₃) δ (ppm): 5.13 (AB, 1H), 5.06 (AB, 1H), 3.53 (q, 2H), 3.22 (s, 2H), 2.67 (t, 1H), 1.21 (t, 3H), 0.69 (s, 3H). **LCMS:** Rt = 2.26 min. m/z = 445.4 [M+H]⁺. S**Q-6: ¹HNMR** (500 MHz, CDCl₃) δ (ppm): 5.36 (AB, 1H), 5.35 (AB, 1H), 3.53 (q, 2H), 3.22 (s, 2H), 2.64 (t, 1H), 2.56 (s, 2H), 1.20 (t, 3H), 0.72 (s, 3H). **LCMS:** Rt = 2.35 min. m/z = 445.3 [M+H]⁺.

### Reference Example 52. Synthesis of SV and SV intermediates.

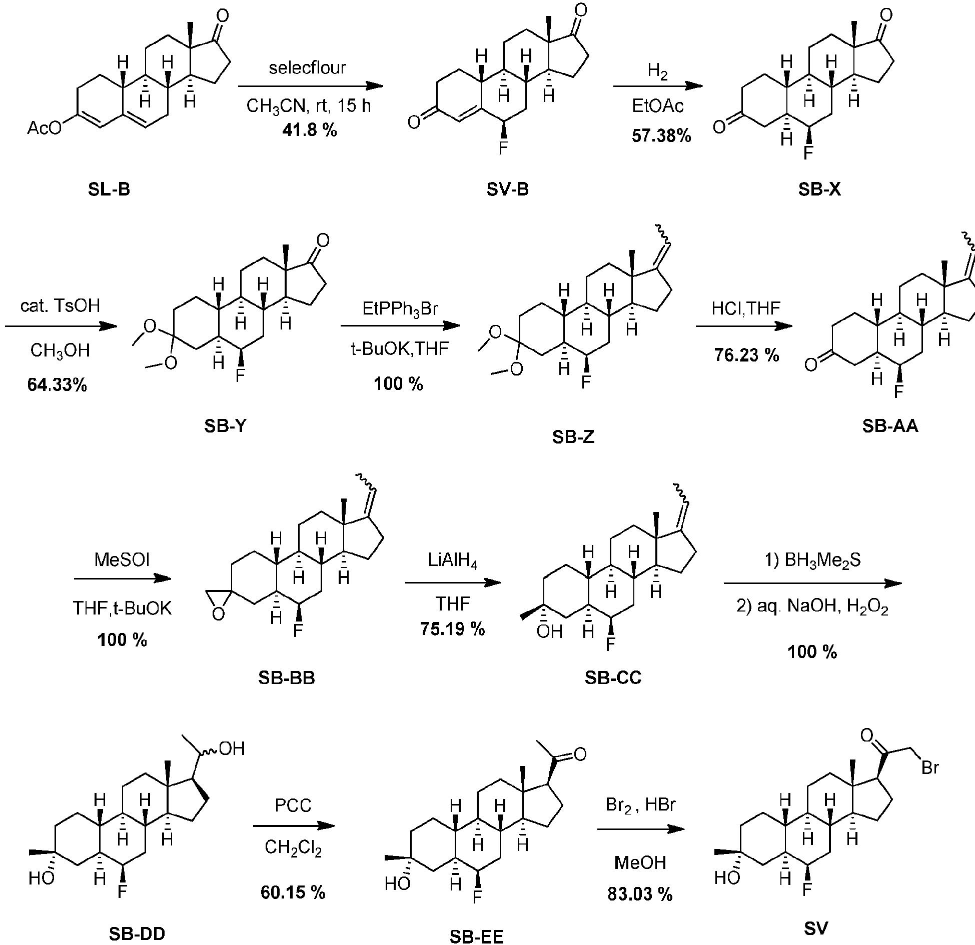

**Synthesis of compound SV-B.** To a solution of **SL-B** (68 g, 216.27 mmol) in 600 mL CH₃CN, was added selectflour (90.22 g, 324.4 mmol) in portions at -4 °C. The resulting reaction mixture wasstirred at -4 °C for 3 h. After the TLC showed the reaction was completed, then the mixture was filtered and concentrated. The product was purified by column chromatograph on silica gel eluted with (Petroleum ether/ ethyl acetate20:1-15:1-10:1-8:1-6:1-5:1) to afford **SV-B** (26.3 g, 41.8 % yield) as off white solid. **¹H NMR (SV-B)** (400 MHz, CDCl₃), δ (ppm), 6.02-5.94 (m, 1H,), 5.20-5.01 (m, 1H), 2.55-2.26 (m, 6H), 2.16-2.05 (m, 1H), 2.01-1.83 (m, 4H), 1.48-1.22 (m, 5H), 0.98-0.78 (m, 6H).

**Synthesis of compound SB-X.** To a solution **of SV-B** (27 g, 92.98 mmol) in EtOAc (350 mL) at 20 °C, then Pd/C(2.7 g, 5 % ) was added in the mixture. The solution was stirred at 20 °C, 1 atm for 10 h under hydrogen..After the LCMS showed the reaction was completed, and then the mixture was filtered and concentrated. The product was purified by column chromatograph on silica gel eluted with (Petroleum ether/ ethyl acetate40:1-35:1-30:1-25:1-20:1-15:1-10:1-6:1) to give **SB-X** (15.6 g, 56.38 %) as off white solid. **¹H NMR (SB-X)** (400 MHz, CDCl₃), δ (ppm)=4.68-4.56 (m, 1H), 2.64-2.51 (m, 1H), 2.53-2.03 (m, 8H), 1.97-1.80 (m, 4H), 1.49-1.20 (m, 6H), 0.96-0.92 (m, 2H), 0.88-0.78 (m, 1H).

**Synthesis of compound SB-Y.** To a solution **of SB-X** (47 g, 160.75 mmol) in MeOH (600 mL) at 23 °C, then 2.35 g of TsOH was added in the mixture. The solution was stirred at 60 °C for 1.5 h .After the TLC showed the reaction was completed, and then the mixture was filtered and concentrated to give **SB-Y** (35 g, 64.33 %) as off white solid. **¹H NMR (SB-Y)** (400 MHz, CDCl₃), δ (ppm)=4.74-4.57 (m, 1H), 3.16 (s, 3H), 3.10 (s, 3H), 2.47-2.35 (m, 1H), 2.15-2.09 (m, 1H), 2.06-1.82 (m, 6H), 1.77-1.15 (m, 11H), 1.05-0.96 (m, 1H), 0.89 (s, 3H), 0.83-0.77 (m, 1H).

**Synthesis of compound SB-Z.** To a solution of ethyltriphenylphosphonium bromide (115.17 g, 310.23 mmol) in 150 mL THF, was added KOt-Bu (34.81 g, 310.23 mmol). The reaction mixture was heated to 60 °C for 1 h and **SB-Y** (35 g, 103.41 mmol) was added to the mixture which was stirred at 60 °C for an additional 15 h. The reaction mixture was cooled and extracted 1500 mL EtOAc, washed with brine and concentrated to afford **SB-Z** as the off white solid (120 g, crude). **¹H NMR (SB-Z)** (400 MHz, CDCl₃), δ (ppm)=5.13-5.07 (m, 1H), 4.67-4.54 (m, 1H), 3.14 (s, 3H), 3.09 (s, 3H), 2.42-2.15 (m, 3H) , 1.92-1.79 (m, 3H), 1.67-1.61 (m, 4H), 1.57-1.50 (m, 2H), 1.45-1.15 (m, 10H), 1.01-0.94 (m, 1H), 0.92 (s, 3H), 0.90-0.84 (m, 1H).

**Synthesis of compound SB-AA.** To a solution of **SB-Z** (120 g, crude) in 600 mL THF, was added 2M aqueous HCl 90 mL. the reaction mixture was stirred at 22 °C for 1h. After the TLC showed the reaction was completed, then the reaction was quenched with aq.NaHCO₃. The reaction was extracted with 500 mL EtOAc, washed with brine and evaporated in vacuo. The resulting residue was purified by chromatography (Petroleum ether/ethyl acetate =150:1-125:1-100:1-80:1-60:1- 50:1) to afford **SB-AA** as the off white solid (24 g, 76.23 % yield). **¹H NMR (SB-AA)** (400 MHz, CDCl₃), δ (ppm)=5.13 (m, 1H), 4.65-4.48 (m, 1H), 2.62-2.42 (m, 1H), 2.44-2.07 (m, 8H), 1.92-1.80 (m, 1H), 1.72-1.55 (m, 8H), 1.36-1.08 (m, 6H), 0.92 (s, 3H), 0.83-0.73 (m, 1H) .

**Synthesis of compound SB-BB.** To a solution of **Me₃SOI** (78.07 g, 354.75 mmol) in 50 mL THF, was added a solution of t-BuOK( 39.81 g, 354.75 mmol) in 50 mL THF. The reaction mixture was stirred at 60 °C for 1.5 h. Then a solution of **SB-AA** (24 g, 78.83 mmol) in THF (300 mL) was added in the reaction. The reaction was stirred for 2.5 h at 23 °C. After the TLC showed the reaction was completed, then the reaction was quenched with ice water. The reaction was extracted with 500 mL EtOAc, washed with brine and evaporated in vacuo to afford **SB-BB** as crude product (50 g). **¹H NMR (SB-BB)** (400 MHz, CDCl₃), δ (ppm)=5.20-5.11 (m, 1H), 4.65-4.52 (m, 1H), 2.74-2.68 (m, 2H), 2.48-1.81 (m, 9H), 1.72-1.64 (m, 4H), 1.55-1.06 (m, 10H), 0.97-0.89 (m, 3H), 0.85-0.77 (m, 1H).

**Synthesis of compound SB-CC.** To a solution of **SB-BB** (50 g, crude) in 300 mL THF, was added LiAlH₄ (8.99 g, 236.49 mmol) at 0 °C. the reaction mixture was stirred at 23 °C for 1.5 h . After the TLC showed the reaction was completed, then the reaction was quenched with water. The reaction was extracted with 1000 mL EtOAc, washed with brine and evaporated in vacuo. The resulting residue was purified by chromatography (Petroleum ether/ethyl acetate =100:1-80:1-60:1-50:1-40:1-30:1) to afford SB-CC as the off white solid (19 g, 75.19 % yield). **¹H NMR (SB-CC)** (400 MHz, CDCl₃), δ (ppm) = 5.17-5.07 (m, 1H), 4.66-4.48 (m, 1H), 2.41-2.32 (m, 1H), 2.28-2.15 (m, 2H), 2.09-2.05 (m, 1H), 1.88-1.75 (m, 2H), 1.68-1.64 (m, 3H), 1.40-1.31 (m, 1H), 1.25-1.13 (m, 9H), 0.89 (s, 3H), 0.81-0.72 (m, 1H).

**Synthesis of compound SB-DD.** To a solution of **SB-CC** (19 g, 59.29 mmol) in dry THF (500 mL) was added C₂H₉BS (59.29 mL; 10 M solution in THF) at 0 °C. After stirring at room temperature for 2 hour, the reaction mixture was cooled in an ice bath then quenched slowly with 3M aqueous NaOH (160 mL) followed by 30 % aqueous solution of H₂O₂ (100 mL). After stirring at 20 °C for 1.5 h, the mixture filtered and extracted with EtOAc (300 mL). The combined organic layers was treated with aq.Na₂S₂O₃, extracted, dried and concentrated to afford **SB-DD** as the crude (21 g, crude). The crude product was used in the next step without further purification.

**Synthesis of compound SB-EE.** To a solution of **SB-DD** (21 g, 59.29 mmol) in 200 mL CH₂Cl₂, was added PCC (25.56 g, 118.58 mmol) at 0 °C, stirred at 22°C for 2 h. The reaction mixture was filtered and extracted with 20 mL CH₂Cl₂, washed with aq.NaHCO₃, aq.Na₂S₂O₃, brine and evaporated *in vacuo.* The residue was purified by chromatography (Petroleum ether/ethyl acetate = 15:1-10:1-6:1) to afford **SB-EE** as the off white solid (12 g, 60.15% yield). **¹H NMR (SB-EE)** (400 MHz, CDCl₃), δ (ppm)=4.65-4.46 (m, 1H), 2.55-2.51 (m, 1H), 2.22-2.09 (m, 4H), 2.06-1.97 (m, 32H), 1.88-1.77 (m, 2H), 1.69-1.54 (m, 5H), 1.48-1.30 (m, 3H), 1.28-1.05 (m, 11H), 0.83-0.72 (m, 1H), 0.63 (s, 3H).

**Synthesis of compound SV.** To a solution of **SB-EE** (12 g, 35.66 mmol) in 1500 mL MeOH, was added HBr (5 drops) and Br₂ (2.01 mL, 39.23 mmol) at 0 °C. The reaction was stirred at 16 °C for 2 h.The reaction mixture was quenched with aq.NaHCO₃ and concentrated.Then the mixture was extracted with 1000 ml EtOAc, washed with brine and evaporated *in vacuo.* The product was purified by column chromatograph on silica gel eluted with (Petroleum ether/ethyl acetate = 12:1-10:1-8:1-6:1-3:1) to afford SV as the off white solid (12.3 g, 83.03% yield). **¹H NMR (SV)** (400 MHz, CDCl₃), δ (ppm)=4.64-4.47 (m, 1H), 3.95-3.86 (m, 2H), 2.89-2.80 (m, 1H), 2.23-2.16 (m, 1H), 2.07-1.64 (m, 8H) 1.46-1.06 (m, 14H), 0.83-0.74 (m, 1H), 0.67 (s, 3H).

### Reference Example 53. Synthesis of compounds SV-1 and SV-2.

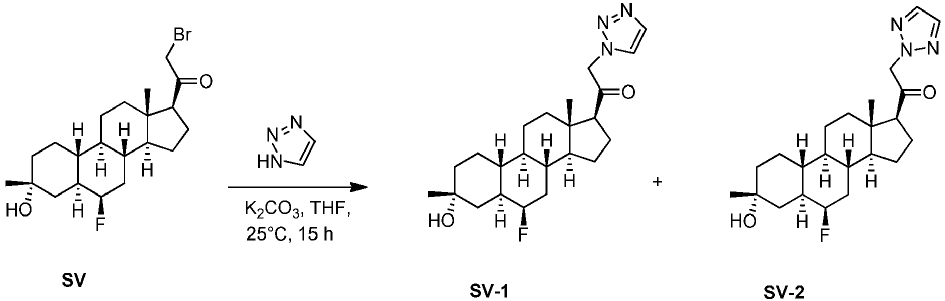

To a suspension of **SV** (40 mg, 0.09 mmol) in THF (5 mL) was added 1H-1,2,3-triazole (30 mg, 0.45 mmol) and K₂CO₃ (60 mg, 0.45mmol). The mixture was stirred at 25°C for 15h. The solution was then diluted with ethyl acetate (100 mL) and the resulting solution was washed with brine (100 mL), dried over sodium sulfate and concentrated *in vacuo.* The reaction mixture was purified with by reverse-phase prep-HPLC to afford **SV-1** as an off white solid ( 10 mg, 26% yield) and **SV-2** as an off white solid (10 mg, 26% yield).

**SV-1: ¹H NMR** (400 MHz, CDCl₃), δ (ppm), 7.75 (s, 1H), 7.65 (s, 1H), 5.29-5.25 (1H,AB), 5.25-5.17 (1H,AB), 4.61-4.52 (d, 1H), 2.6(1H,t),1.18 (s, 3H), 0.63 (s, 3H). **SV-2: ¹H NMR** (400 MHz, CDCl₃), δ (ppm), 7.68 (s, 2H), 5.24-5.23 (m, 2H), 4.60-4.50 (d, 1H), 2.6(1H,t,), 1.25 (s, 3H), 0.74 (s, 3H).

### Reference Example 54. Synthesis of compounds SV-3 and SV-4.

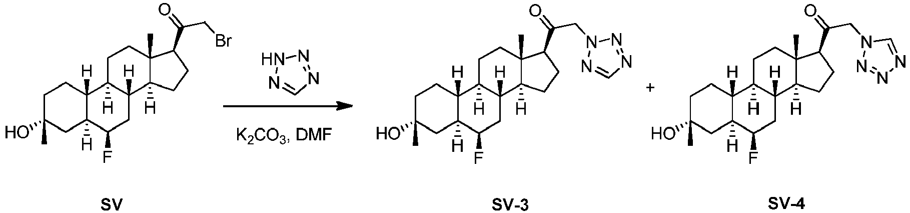

To a solution of **SV** (100 mg, 0.24 mmol) in 3 mL of DMF was added 2H-tetrazole (33.73 mg, 0.48mmol) and K₂CO₃(99-82 mg, 0.72 mmol). The reaction was stirred at 28°C for 2 h. The resulting solution was quenched with water and extracted with EtOAc (50 mL). The organic layer was washed with brine (20 mL), dried over Na₂SO₄ and concentrated in vacuum. The residue was purified by column chromatography on silica gel eluted with (PE/EA=12/1 to 2/1) to give **SV-3** (16.1 mg, yield: 16.67%) and **SV-4** (28.3 mg, yield:29.17 %) as off white solid. **¹H NMR (SV-3):** (400 MHz, CDC13) δ 8.60 (s, 1 H), 5.57-5.42 (m, 2H), 4.73-4.48 (m, 1H), 2.74-2.60 (m, 1H), 2.31-2.21 (m, 1H), 2.16-2.108(m, 1H), 1.97-1.89 (m, 1H), 1.86-1.60 (m, 7H), 1.55-1.11(m, 14H), 0.88-0.80 (m, 1H),0.77(s, 3H). **¹H NMR (SV-4):** (400 MHz, CDC13) δ 8.75 (s, 1 H), 5.36-5.16 (m, 2H), 4.66-4.47 (m, 1H), 2.73-2.62 (m, 1H), 2.30-2.18 (m, 1H), 2.09-1.74 (m, 6H), 1.67-1.60 (m, 3H), 1.38-1.16 (m, 11H), 0.88-0.75 (m, 1H), 0.70 (s, 3H).

### Reference Example 55. Synthesis of compounds SV-5 and SV-6.

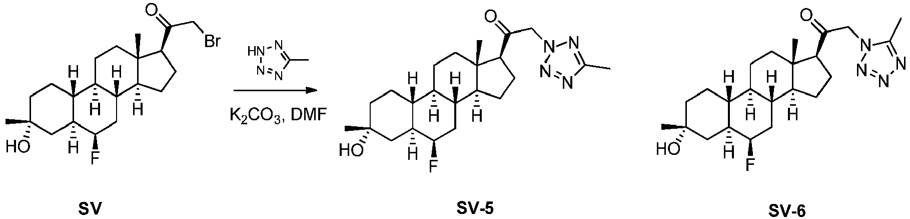

To a solution of **SV** (100 mg, 0.24 mmol) in 3 mL of DMF was added 5-methyl-2H-tetrazole (40.48 mg, 0.48 mmol) and K₂CO₃ (99.82 mg, 0.72 mmol) . The reaction was stirred for 1 h at 21°C. The resulting solution was quenched with water and extracted with EtOAc (50 mL). The organic layer was concentrated in vacuum. The residue was purified by column chromatography on silica gel (PE/EtOAc = 8/1 to 1/1) to give **SV-5** (21.3 mg, yield:21.14 %) and **SV-6** (27.1 mg, yield: 26.89 %) as off white solid. **¹H NMR (SV-5):** (400 MHz, CDCl₃) δ 5.43-5.31 (m, 2H), 4.68-4.49 (m, 1H), 2.69-2.62 (m, 1H), 2.59 (s, 3H), 2.31-2.20 (m, 1H), 2.14-2.09 (m, 1H), 1.95-1.88 (m, 1H), 1.85-1.60 (m, 8H), 1.46-1.20 (m, 12H), 1.02-0.93 (m, 1H), 0.89-0.80 (m, 1H), 0.77 (s, 3H). **¹H NMR (SV-6):** (400 MHz, CDCl₃) δ 5.21-5.05 (m, 2H), 4.69-4.50 (m, 1H), 2.73-2.63 (m, 1H), 2.50 (s, 3H), 2.30-2.19 (m, 1H), 2.13-2.01 (m, 2H), 1.98-1.57 (m, 9H), 1.45-1.14 (m, 12H),0.90-0.80 (m, 1H), 0.73 (s, 3H).

### Reference Example 56. Synthesis of compound SV-7.

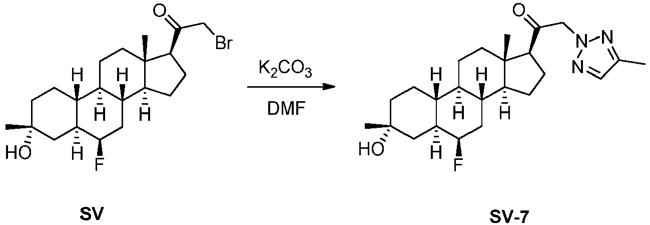

To a solution of **SV** (100 mg, 0.24 mmol) in 15 mL of DMF was added 4-methyl-2H-1,2, 3-triazole (40.01 mg, 0.48 mmol) and K₂CO₃ (99.82 mg, 0.72 mmol). The reaction was stirred at 28°C for 2 h. The resulting solution was quenched with water and extracted with EtOAc (50 mL). The organic layer was washed with brine (20 mL), dried over Na₂SO₄ and concentrated in vacuum. The residue was purified by prep-HPLC to give **SV-7** (20.6 mg, yield: 20.83%) as an off white solid. **¹H NMR (SV-7):** (400 MHz, CDC13) δ 7.45 (s, 1 H), 5.23-5.10 (m, 2H), 4.68-4.49 (m, 1H), 2.64-2.57 (m, 1H), 2.35 (s, 3H), 2.30-2.18 (m, 1H), 2.14-2.00 (m, 2H), 1.93-1.58 (m, 8H), 1.46-1.09 (m, 13H), 0.86-0.76 (m, 1H), 0.75 (s, 3H).

### Reference Example 57. Synthesis of compounds SV-8 and SV-9.

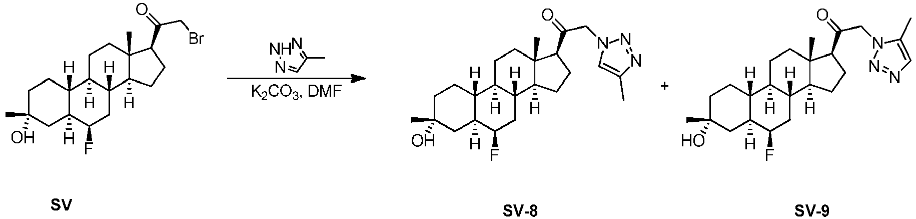

To a solution of **SV** (200 mg, 0.48 mmol) in 10 mL of DMF (5 mL) was added 4-methyl-2H-1,2,3-triazole (80.02 mg, 0.96 mmol) and K₂CO₃ (199.63 mg, 1.44 mmol). The reaction mixture was stirred at 17°C for 2 h. The resulting solution was quenched with water and extracted with EtOAc (50 mL). The organic layer was dried and concentrated. The residue was purified by silica gel to give a 90 mg mixture of **SV-8/SV-9** and a byproduct (60 mg). The mixture was split by SFC purification to give **SV-8** (38.8 mg, yield: 29.84%) and SV-9 (31.5 mg, yield: 23.3%) as off white solid. **¹H NMR (SV-8):** (400 MHz, CDC13) δ 7.347 (s, 1 H), 5.191-5.041 (q, *J₁*=17.6 HMz, *J₂*=42.4 HMz), 4.62-4.50 (m, 1H), 2.66-2.61 (m, 1H), 2.37 (s, 3H), 2.10-2.06 (m, 1H), 1.87-1.74 (m, 2H), 1.70-1.50 (m, 7H), 1.30-1.04 (m, 14H), 0.86-0.76 (m, 1H), 0.70 (s , 3H). **¹H NMR (SV-9):** (400 MHz, CDC13) δ 7.488 (s, 1 H), 5.08-5.07 (m, 2H), 4.63-4.50 (m, 1H), 2.68-2.63 (m, 1H), 2.22 (s, 3H), 2.04-1.89 (m, 2H), 1.80-1.73 (m, 7H), 1.64-1.60 (m, 1H), 1.56-1.20 (m, 14H), 0.80-0.70 (m, 1H), 0.64 (s, 3H).

### Reference Example 58. Synthesis of SW and SW intermediates.

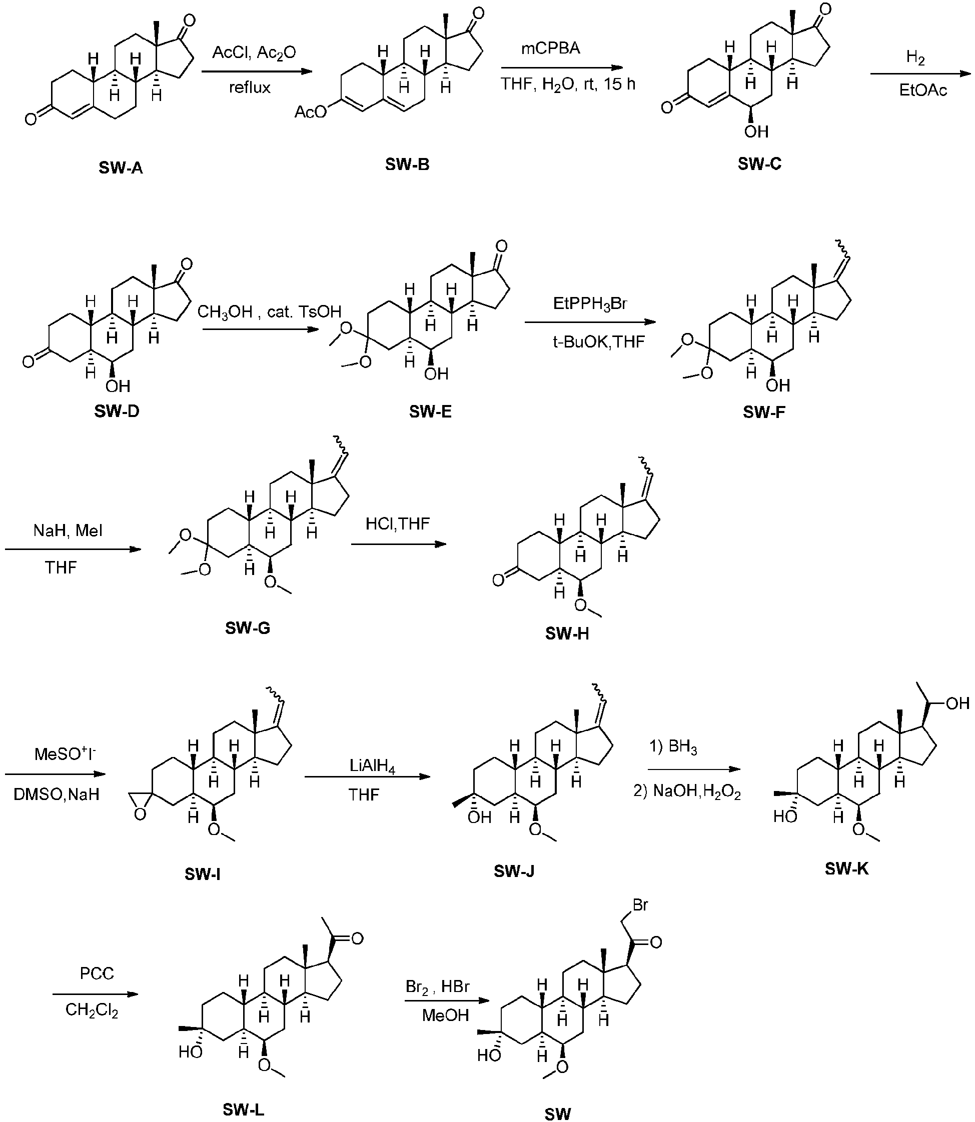

**Synthesis of compound SW-B. SW-A** (10 g, 36.7 mmol) was added to 50 mL acetyl chloride and 50 mL acetic anhydride. The reaction mixture was heated to 120°C for 5 h, evaporated *in vacuo* to afford SW-B as the off white solid (10 g, 87% yield). **¹H** NMR (400 MHz, CDCl₃), δ (ppm), 5.78 (s, 1H), 5.55 (s, 1H), 2.4(dd,2H),2.13 (s, 3H), 0.90 (s, 3H).

**Synthesis of compound SW-C.** To a solution of **SW-B** (10 g, 31.8 mmol) in 200 mL THF and 20 mL H₂O, was added mCPBA (11 g, 63.6 mmol) at 0°C, stirred at rt for 15 h, the reaction mixture was extracted 500 mL EtOAc, washed with 100 mL saturated Na₂SO₃, 100 ml saturated NaHCO₃ and 100 mL brine and evaporated *in vacuo* then purified by chromatography (PE:EtOAc = 5:1) to afford **SW-C** as an off white solid ( 2.2 g, 24% yield). **¹H NMR** (400 MHz, CDCl₃), δ (ppm), 5.92 (s, 1H), 4.44 (s, 1H), 0.95 (s, 3H).

**Synthesis of compound SW-D.** To a solution **of SW-C** (2 g, 6.94 mmol) in 50 mL EtOAc, was added Pd/C 200 mg. The reaction mixture was hydrogenated in 1 atm H₂ for 15 h. Then the reaction mixture was evaporated *in vacuo* and purified by chromatography (PE:EtOAc = 1:2) to afford **SW-D** as the off white solid (0.5 g, 25% yield). **¹H NMR** (400 MHz, CDCl₃), δ (ppm), 3.84 (s, 1H),2.62(1H,t) 0.95 (s, 3H).

**Synthesis of compound SW-E.** To a solution **of SW-D** (1 g, 3.4 mmol) in 100 mL MeOH, was added TsOH 50 mg, heated to 60 °C for 2 h. The reaction mixture was extracted 500 mL EtOAc, washed with 100 mL saturated NaHCO₃, 100 mL brine and evaporated *in vacuo* to afford **SW-E** as the off white solid (1 g, 91% yield).

**Synthesis of compound SW-F.** To a solution of ethyltriphenylphosphonium bromide (10.67 g, 28.84 mmol) in 30 mL THF, was added KOt-Bu (3.23 g, 28.80 mmol). The reaction was heated to 60 °C for 1 h. **SW-E** (3.23 g, 9.6 mmol) was added and the resulting mixture was stirred at 60 °C for 15 h. The reaction mixture was then extracted 500 mL EtOAc, washedwith brine and evaporated *in vacuo.* The resulting crude residue was purified by chromatography (PE:EtOAc = 3:1) to afford **SW-F** as the off white solid (2.17 g, 64% yield).

**Synthesis of compound SW-G.** To a solution **of SW-F** (1 g, 2.9 mmol) in 50 mL THF, was added NaH (2 g, 5.8 mmol) and the resulting mixture was stirred at rt for 1 h. Then 1 mL MeI was added to the mixture that was then stirred at rt overnight. The reaction mixture was quenched with 5 mL H₂O and extracted with 100 mL EtOAc, washed with brine and evaporated *in vacuo.* The resulting residue was purified by chromatography (PE:EtOAc = 10:1) to afford **SW-G** as the off white solid (587 mg, 59% yield).

**Synthesis of compound SW-H.** To a solution **of SW-G** (1 g, 2.8 mmol) in 20 mL THF, was added 2M aqueous HCl (2 mL), and the resulting reaction mixture was stirred at rt for 1 h. The reaction mixture was then quenched with 5 mL H₂O and extracted with 100 mL EtOAc, washed with brine and evaporated *in vacuo.* The residue was purified by chromatography (PE:EtOAc = 10:1) to afford **SW-H** as the off white solid (745 mg, 81% yield). **¹H NMR** (400 MHz, CDCl₃), δ (ppm), 5.05-5.03 (m, 1H), 3.24 (s, 3H), 3.11 (s, 1H), 2.6(1H,t),0.87 (s, 3H).

**Synthesis of compound SW-I.** To a stirred solution of trimethylsulfoxonium iodide (3.6 g, 16.5mmol) in 5 mL of THF was added potassium tert-butanolate (1.90 g, 16.5mmol). After stirring at 60°C for 1.5 h, a suspension of **SW-H** (1g, 3.3 mmol) in 10 mL of THF was added dropwise. After another 3 h, the reaction mixture was poured into ice-cold water and extracted with EtOAc (100 mL x 3), washed with brine (100 mLx3), dried (MgSO₄), filtered, and evaporated *in vacuo* to afford **SW-I** as the off white solid (800 mg, 73% yield). The crude product was used in the next step without further purification.

**Synthesis of compound SW-J.** To a solution of **SW-I** (150 mg, 0.45 mmol) in 10 mL THF, was added LiAH₄ (50 mg, 1.35 mmol), the resulting reaction mixture was stirred at rt for 1 h. The reaction mixture was then quenched with 5 mL H₂O and extracted with 100 mL EtOAc, washed with brine and evaporated *in vacuo.* The residue was purified by chromatography (PE:EA = 3:1) to afford **SW-J** as the off white solid (108mg, 72% yield). **¹H NMR** (400 MHz, CDCl₃), δ (ppm), 5.12-5.10 (m, 1H), 3.29 (s, 3H), 3.18 (s, 1H), 1.23 (s, 3H), 0.88 (s, 3H).

**Synthesis of compound SW-K.** To a solution **of SW-J** (100 mg, 0.3 mmol) in dry THF (5 mL) was added borane-tetrahydrofuran complex (1 mL; 1.0 M solution in THF). After stirring at room temperature for 1 hour, the reaction mixture was cooled in an ice bath then quenched slowly with 10% aqueous NaOH (1 mL) followed by 30% aqueous solution of H₂O₂ (1 mL). After stirring at room temperature for one hour, the mixture was extracted with EtOAc (3 × 100 mL). The combined organic layers were washed with 10% aqueous Na₂S₂O₃ (100 mL), brine (100 mL), dried over MgSO₄, filtered and concentrated to afford **SW-K** as the off white solid (90 mg, 81%). The crude product was used in the next step without further purification.

**Synthesis of compound SW-L.** To a solution **of SW-K** (100 mg, 0.29 mmol) in 20 mL DCM, was added PCC (190 mg, 0.87 mmol), stirred at rt for 2 h. The reaction mixture was quenched with 5 ml H₂O and extracted with 100 mL EtOAc, washed with brine and evaporated *in vacuo,* then purified by chromatography (PE:EtOAc = 3:1) to afford **SW-L** as the off white solid (52 mg, 51% yield). **¹H NMR** (400 MHz, CDCl₃), δ (ppm), 3.26 (s, 3H), 3.16 (s, 1H), 2.11 (s, 3H), 1.20 (s, 3H), 0.61 (s, 3H).

**Synthesis of compound SW.** To a solution **of SW-L** (40 mg, 0.11 mmol) in MeOH (5 mL) was added 2 drops of HBr (48%) followed by bromine (150 mg, 0.33 mmol). After stirring at room temperature for 1h, the reaction mixture was poured into ice-water then extracted with ethyl acetate (10 mL × 3). The combined organic layers were washed with brine (20 mL), dried over MgSO₄, filtered and concentrated to give crude compound **SW** as the off white solid (40 mg, 80% yield). The crude product was used in the next step without further purification.

### Reference Example 59. Synthesis of compounds SW-1 and SW-2.

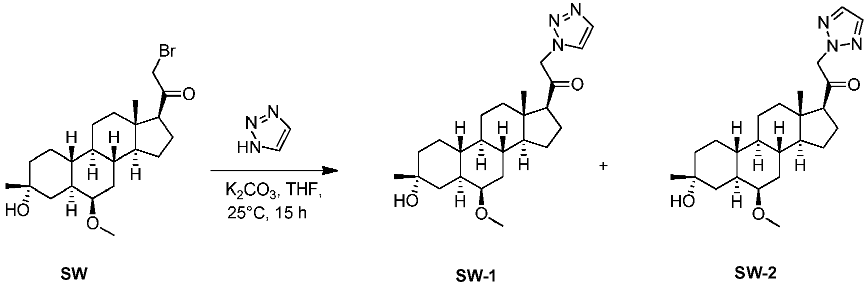

To a suspension of **SW** (40 mg, 0.09 mmol) in THF (5 mL) was added 1H-1,2,3-triazole (30 mg, 0.45 mmol) and K₂CO₃ (60 mg, 0.45mmol). The mixture was stirred at 25°C for 15h. The solution was then diluted with ethyl acetate (100 mL) and the resulting solution was washed with brine (100 mL), dried over sodium sulfate and concentrated *in vacuo.* The reaction mixture was purified with by reverse-phase prep-HPLC to afford **SW-1** as an off white solid ( 10 mg, 26% yield) and **SW-2** as an off white solid ( 8 mg, 20% yield). **SW-1: ¹H NMR** (400 MHz, CDCl₃), δ (ppm), 7.75 (s, 1H), 7.64 (s, 1H), 5.27-5.24 (1H,AB),5.17-5.13 (1H,AB), 3.28 (s, 3H), 3.17 (s, 1H),2.7(1H,t), 1.23 (s, 3H), 0.65 (s, 3H). **SW-2: ¹H NMR** (400 MHz, CDCl₃), δ (ppm), 7.68 (s, 2H), 5.28-5.25 (1H,AB), 5.23-5.20 (1H,AB), 3.28 (s, 3H), 3.17 (s, 1H), 2.6(1H,t), 1.24 (s, 3H), 0.75 (s, 3H).

### Reference Example 60. Synthesis of SZ and SZ intermediates.

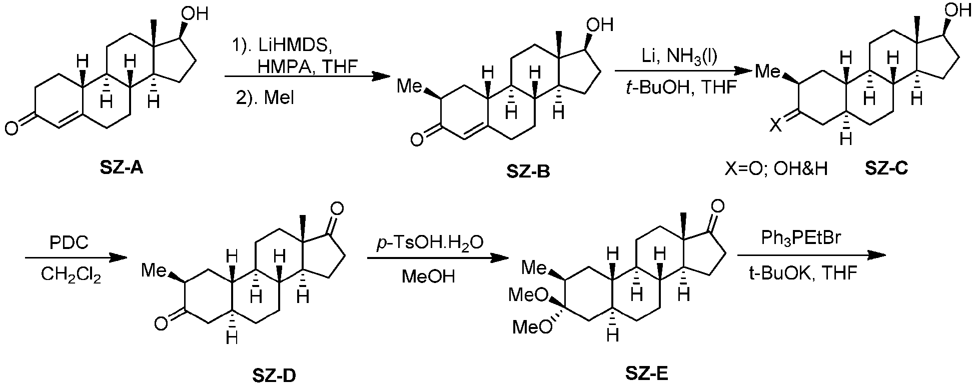

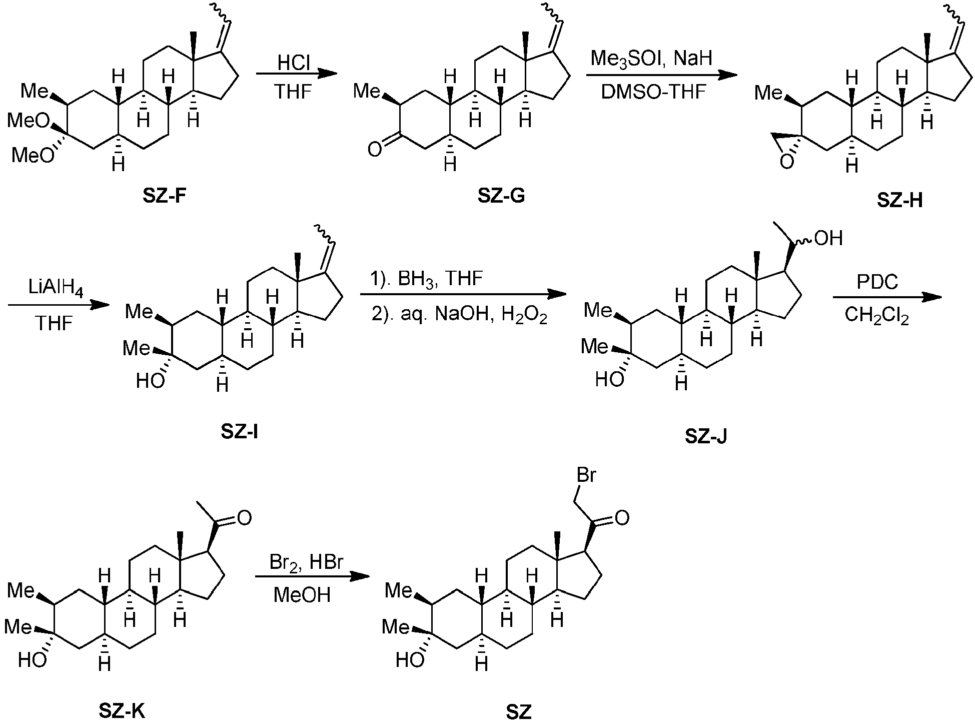

**Synthesis of compound SZ-B.** To a solution of compound **SZ-A** (500 mg, 1.82 mmol) in THF (18 mL) was added LiHMDS (1.0 M in THF solution, 4.00 mL, 4.00 mmol) at -78°C. The solution was stirred at - 78°C for 30 minutes. Then HMPA (0.69 mL, 4.00 mmol) was added. The solution was stirred at -78 °C for another 30 minutes. Then iodomethane (0.34 mL, 5.46 mmol) was added. The solution was further stirred at -78 °C for 2 hours and warmed to room temperature and stirred for 1 hour. The reaction was quenched by addition of water (2 mL). Most THF solvent was removed *in vacuo.* Then the residue was diluted with ethyl acetate (100 mL) and the resulting solution was washed with brine (2×100 mL), dried over magnesium sulfate. Removal of solvent *in vacuo* afforded crude product **SZ-B** (350mg, 67%) as thick oil. The crude product was used in the next step without further purification. **SZ-B: ¹HNMR** (500 MHz, CDCl₃) δ(ppm): 5.74 (1H, s), 3.67 (1H, t), 1.11 (3H, d), 0.81 (3H, s).

**Synthesis of compound SZ-C.** To liquid ammonia (100 mL) was added lithium (687 mg, 99.0 mmol) at - 78 °C. The liquid was turned to deep blue. Then a solution of reactant **SZ-B** (950 mg, 3.30 mmol) in t-BuOH (244 mg, 3.30 mmol) and THF (20 mL) was added to Li-ammonia solution. The mixture was stirred at -78 °C for 4 hours. Then NH₄Cl solid (7 g) was added to quench the reaction. The mixture was turned from deep blue to white. The mixture was allowed to warm to room temperature and ammonia was evaporated in a hood overnight. To the residue was added water (100 mL). The mixture was acidified by conc. HCl to pH 6-7. Then ethyl acetate (100 mL) was added. The separated aqueous layer was further extracted with ethyl acetate (2×100 mL). The combined organic extracts were washed with brine (200 mL), dried over magnesium sulfate and concentrated *in vacuo.* The crude product **SZ-C** was used directly without further purification in the next step.

**Synthesis of compound SZ-D.** To a solution of crude compound **SZ-C** (980 mg, 3.40 mmol) in dichloromethane (60 mL) was added pyridinium dichromate (PDC) (2.56 g, 6.80 mmol). The mixture was stirred at room temperature overnight. The solution was filtered through a short pad of celite. The celite was washed with CH₂Cl₂ (3×50 mL). The combined CH₂Cl₂ solution was concentrated *in vacuo.* The residue was purified by flash chromatography (eluant: petroleum ether/EtOAc=5:1) to afford product **SZ-D** (680 mg, 69%) as off white solid. **SZ-D:** ¹HNMR (500 MHz, CDCl₃) δ(ppm):1.02 (3H, d), 0.91 (3H, s).

**Synthesis of compound SZ-E.** To a solution of compound **SZ-D** (3.24 g, 11.24 mmol) in anhydrous methanol (100 mL) was added p-toluenesulfonic acid monohydrate (193 mg, 1.12 mmol). The solution was heated at 70 °C for 3 hours. The reaction was quenched by addition of sat. Na₂CO₃ solution (10 mL). Most methanol solvent was removed *in vacuo.* Then the residue was diluted with ethyl acetate (200 mL). The resulting solution was washed with saturated Na₂CO₃ solution (2×100 mL). The combined aqueous layers were extracted with ethyl acetate (50 mL). The combined organic extracts were washed with brine (100 mL), dried over magnesium sulfate and concentrated *in vacuo.* The residue was purified by flash chromatography (eluant: petroleum ether/ EtOAc= 15:1, added 0.1% NEt₃) to afford product **SZ-E** (1.76 g, 47%) as off white solid. Furthermore, starting compound **SZ-E** (1.34 g) was also recovered. The yield based on recovered starting material is 93%. **SZ-E:** ¹HNMR (500 MHz, d6-acetone) δ(ppm): 3.080 (3H, s), 3.076 (3H, s), 2.37 (1H, dd), 1.98 (1H, dd), 0.91 (3H, d), 0.85 (3H, s).

**Synthesis of compound SZ-F.** To a suspension of ethyltriphenylphosphonium bromide (6.67 g, 17.96 mmol) in anhydrous THF (25 mL) was added t-BuOK (2.01 g, 17.96 mmol). The solution was turned red in color and was then heated at 70 °C for 2 hours. Then compound **SZ-E** (2.00 g, 5.99 mmol) was added in one portion. The solution was heated at 70 °C overnight. The reaction was quenched by the addition of water (10 mL). The mixture was diluted with ethyl acetate (200 mL) and the resulting solution was washed with brine (2×100 mL), dried over magnesium sulfate and concentrated *in vacuo.* The crude product **SZ-F** was used directly in the next step without further purification.

**Synthesis of compound SZ-G:** To the crude product **SZ-F** (2.25 g, 6.50 mmol, theoretical amount) in THF (50 mL) was added 4 M HCl (2 mL). The solution was stirred at ambient temperature for 1 hour. The mixture was diluted with ethyl acetate (300 mL) and the resulting solution was washed with saturated Na₂CO₃ solution (2×100 mL). The combined aqueous layers were extracted with EtOAc (100 mL). The combined organic extracts were washed with brine (100 mL), dried over magnesium sulfate and concentrated *in vacuo.* The residue was purified by flash chromatography (eluant: petroleum ether/ EtOAc =20:1) to afford desired product **SZ-G,** 1.78 g (5.94 mmol, 91% yield). **SZ-G:** ¹HNMR (500 MHz, CDCl₃) δ(ppm): 5.13 (1H, qt), 1.66 (3H, dt), 1.02 (3H, d), 0.91 (3H, s).

**Synthesis of compound SZ-H:** To a solution of trimethylsulfoxonium iodide (6.53 g, 29.70 mmol) in anhydrous DMSO (30 mL) was added sodium hydride (60% wt, 1.19 mg, 29.70 mmol). The mixture was stirred at 25 °C for 1 hour. Then a solution of crude compound **SZ-G** (2.05 g, contaminated with some PPh₃, theoretical amount, 1.78 g, 5.94 mmol) in anhydrous THF (10 mL) was added. The mixture was stirred at 25 °C overnight. The reaction was quenched by addition of water (5 mL). The mixture was diluted with ethyl acetate (300 mL) and the resulting solution was washed with water (2×100 mL), followed by brine (100 mL) dried over magnesium sulfate and concentrated *in vacuo.* The crude product **SZ-H** was used directly in the next step without further purification.

**Synthesis of compound SZ-I:** To a solution of crude reactant **SZ-H** (theoretical amount, 1.21 g, 3.85 mmol) in anhydrous THF (30 mL) was added lithium alumium hydride (731 mg, 19.25 mmol) in portions. The suspension was stirred at 25 °C for 1 hour. Then the reaction was quenched by addition of EtOAc (5 mL) followed by water (5 mL). The off white solid was filtered and thoroughly washed with EtOAc (5×100 mL). The combined filtrate was washed with brine (200 mL), dried over magnesium sulfate and concentrated *in vacuo.* The residue was purified by flash chromatography (eluant: petroleum ether/ EtOAc=15:1) to afford product **SZ-I** (560 mg, 1.78 mmol, 2 steps total yield, 30%) as off white solid. **SZ-**I: ¹HNMR (500 MHz, CDC13) δ(ppm): 5.11 (1H, qt), 2.05 (1H, s), 1.56 (3H, s), 1.17 (3H, s), 0.91(3H, d), 0.88 (3H, s).

**Synthesis of compound SZ-J.** To a solution of reactant **SZ-I** (320 mg, 1.013 mmol) in anhydrous THF (20 mL) was added BH₃.THF (1.0 M, 5.07 mL, 5.065 mmol), This solution was stirred at 25 °C overnight then the reaction was quenched by addition of water (4 mL). 2 M aqueous NaOH solution (8 mL) was added followed by 30 % H₂O₂ (8 mL). The mixture was stirred at room temperature for 1 hour. The mixture was diluted with EtOAc (200 mL) and resulting solution was washed with brine (2×100 mL), dried over magnesium sulfate and concentrated *in vacuo.* The crude product **SZ-J** was used directly in the next step without further purification.

**Synthesis of compound SZ-K.** To a solution of crude compound **SZ-J** (320 mg, 1.013 mmol) in dichloromethane (30 mL) was added pyridinium dichromate (PDC) in portions (1.14 mg, 3.039 mmol). The solution was stirred at 25 °C overnight. Then the mixture was filtered through a short pad of silica gel and the silica gel was washed with dichloromethane (3×50 mL). All filtrate was combined and concentrated *in vacuo.* The residue was purified by flash chromatography (eluant: petroleum ether/ EtOAc =6:1) to afford product **SZ-K** (140 mg, 0.422 mmol, yield 42%, 2 steps) as off white solid. **SZ-K:** ¹HNMR (500 MHz, CDC13) δ(ppm): 2.54 (1H, t), 2.12 (3H, s), 1.99 (1H, td), 1.82-1.86 (1H, m), 1.18 (3H, s), 0.92 (3H, d), 0.61 (3H, s). **SZ-K:** ¹³CNMR (100 MHz, CDC13) δ(ppm): 209.79, 71.09, 63.94, 55.87, 47.94, 47.78, 46.97, 44.35, 41.16, 40.20, 39.04, 37.93, 34.48, 33.13, 31.55, 30.91, 28.45, 25.80, 24.20, 22.73, 15.15, 13.43.

**Synthesis of compound SZ.** To a solution of compound **SZ-K** (100 mg, 0.301 mmol) in methanol (10 mL) was added 48% hydrobromic acid (152 mg, 0.903 mmol) followed by bromine (241 mg, 0.077 mL, 1.505 mmol). The solution was heated at 25 °C for 2 hours. Then the mixture was poured into cooled water (50 mL). The resulting solid was extracted with ethyl acetate (2×50 mL). The combined organic extracts were washed with brine (50 mL), dried over magnesium sulfate and concentrated *in vacuo.* The crude product **SZ** was used directly without further purification in the next step.

### Reference Example 61. Synthesis of compounds SZ-1 and SZ-2.

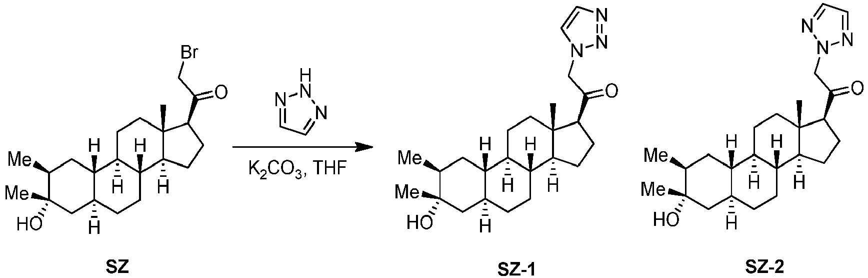

To a solution of crude compound **SZ** (80 mg, 0.195 mmol) in anhydrous THF (6 mL) was added 1,2,3-trizaole (40.4 mg, 0.585 mmol) followed by potassium carbonate (80.9 mg, 0.585 mmol). The solution was heated at 50 °C overnight. Then the solution was diluted with EtOAc (100 mL). The resulting solution was washed with brine (2×50 mL), dried over magnesium sulfate and concentrated *in vacuo.* The crude product was purified by reverse phase prep-HPLC to afford product **SZ-1** (15 mg, 19%) and product **SZ-2** (6 mg, 7.7%) as off white solid. **SZ-1:** ¹HNMR (500 MHz, CDC13) δ(ppm): 7.77 (1H, s), 7.65 (1H, s), 5.28 (1H, AB), 5.14 (1H, AB), 2.66 (1H, t), 1.18 (3H, s), 0.92 (3H, d), 0.68 (3H, s). **SZ-2: ¹HNMR** (500 MHz, CDC13) δ(ppm): 7.69 (2H, s), 5.25 (1H, AB), 5.23 (1H, AB), 2.60 (1H, t), 1.18 (3H, s), 0.92 (3H, d), 0.71 (3H, s).

### Reference Example 62. Synthesis of SS and SS intermediates.

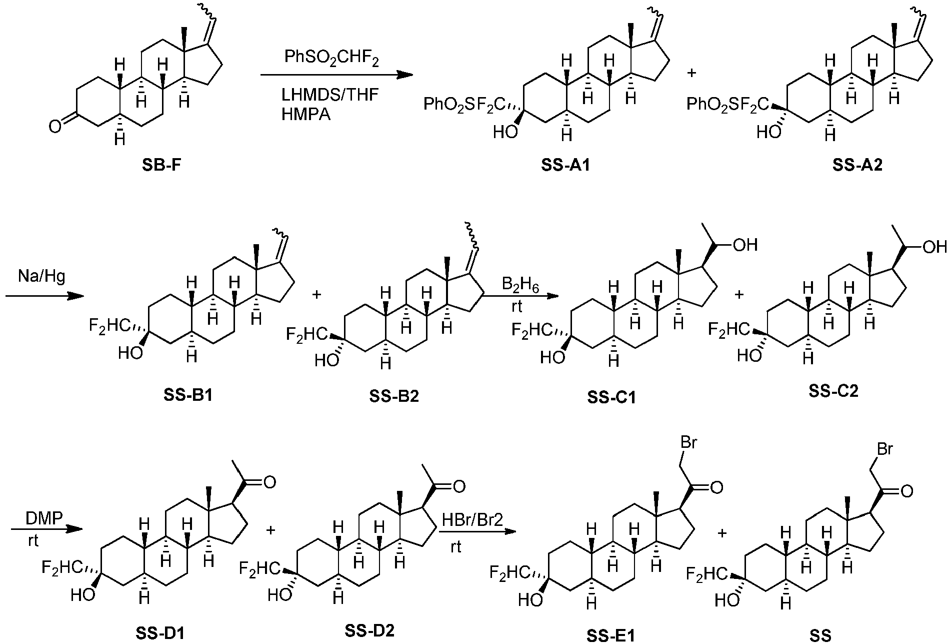

**Synthesis of compound SS-A1 and SS-A2.** To a solution of compound **SB-F** (800 mg, 2.79 mmol) and Ph₁SO₂CF₂H (540 mg, 2.79 mmol) in THF (25 mL) and HMPA (0.5 mL) at -78 °C under N₂ was added LHMDS (4 mL, 1M in THF) dropwise. After stirring at -78 °C for 2 h, the reaction mixture was quenched with saturated aqueous NH₄Cl solution (10 mL) and allowed to warm to room temperature then extracted with Et₂O (20 mL × 3). The combined organic layers were washed with brine, dried over sodium sulfate, filtered and concentrate. The residue was purified by silica gel column chromatography (pertroleum ether/ ethyl acetate =10/ 1) to give the mixture of compound **SS-A1** and **SS-A2** (650 mg). The mixture was further purified by chiral-HPLC to afford compound **SS-A1** (250 mg, t= 3.29 min) and **SS-A2** (230 mg, t= 3.89 min).

**Synthesis of compound SS-B2.** To a solution of compound **SS-A2** (230 mg, 0.489 mmol) and anhydrous Na₂HPO₄ (150 mg) in anhydrous methanol (5 mL) at -20 °C under N₂ was added Na/Hg amalgam (700 mg). After stirring at -20 °C to 0 °C for 1 h, the methanol solution was decanted out and the solid residue was washed with Et₂O (5 × 3 mL). The combined organic phase was removed under vacuum, and 20 ml brine was added, followed by extracting with Et₂O. The combined ether phase was dried with MgSO₄, filtered and concentrated. The crude product was purified by silica gel chromatography (PE/EA=10/1) to give compound **SS-B2** (120 mg, 73 %). **¹H NMR** (400 MHz, CD₃COCD₃), δ (ppm), 6.02-5.88 (t, 1H), 5.13-5.08 (m, 1H), 0.92(s, 3H).

**Synthesis of compound SS-C2.** To a solution of compound **SS-B2** (120 mg, 0.355 mmol) in dry THF (5 mL) was added borane-tetrahydrofuran complex (1.20 mL; 1.0 M solution in THF). After stirring at room temperature for 1 hour, the reaction mixture was cooled in an ice bath then quenched slowly with 10% aqueous NaOH (1 mL) followed by 30% aqueous solution of H₂O₂ (1.2 mL). The mixture was allowed to stir at room temperature for 1 hour then extracted with EtOAc (3 x 10 mL). The combined organic layers were washed with 10% aqueous Na₂S₂O₃ (10 mL), brine (10 mL), dried over MgSO₄, filtered and concentrated to afford compound **SS-C2** (180 mg, crude). The crude product was used in the next step without further purification.

**Synthesis of compound SS-D2.** To a solution of compound **SS-C2** (180 mg, crude) in 10 mL of wet dichloromethane (dichloromethane had been shaken with several milliliters of H₂O then separated from the water layer) was added Dess-Martin periodinate (380 mg, 0.896 mmol). After stirring at room temperature for 24 h, the reaction mixture was extracted with dichloromethane (3 x 10 mL). The combined organic layers were washed with 10 % aqueous Na₂S₂O₃ (10 mL), brine (10 mL), dried over MgSO₄, filtered and concentrated. The residue was purified by chromatography on silica gel (pertroleum ether/ ethyl acetate = 1: 5) to afford compound **SS-D2** (70 mg, 55.7% for two steps) as an off white solid. **¹H NMR** (400 MHz, CDC13), δ (ppm), 5.90-5.61 (t, 1H), 2.48-2.43 (m, 1H), 2.10 (s, 3H), 0.55 (s, 3H).

**Synthesis of compound SS.** To a solution of compound **SS-D2** (50 mg, 0.14 mmol) in MeOH (5 mL) was added 2 drops of HBr (48%) followed by bromine (100 mg, 0.62 mmol). After stirring at room temperature for 1h, the reaction mixture was poured into ice-water then extracted with ethyl acetate (15 mL x 3). The combined organic layers were washed with brine (20 mL), dried over MgSO₄, filtered and concentrated to give compound **SS** (72 mg, crude). The crude product was used in the next step without further purification.

### Reference Example 63. Synthesis of compound SS-1.

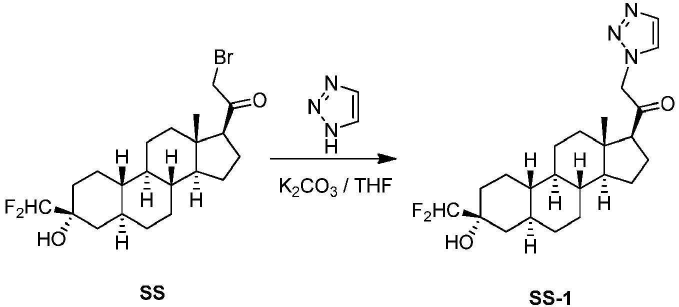

To a suspension of K₂CO₃ (126 mg, 0.92 mmol) in THF (10 mL) was added 1,2,3-1H-Triazole (22.4 mg, 0.92 mmol) and compound **SS** (200 mg, 0.46 mmol). After stirring at room temperature for 15h, the reaction mixture was poured into 5 mL H₂O and extracted with EtOAc (2 × 10 mL). The combined organic layers were washed with brine (2 × 10 mL), dried over sodium sulfate, filtered and concentrated in vacuum. The residue was purified by reverse-phase prep-HPLC to afford **SS-1** as an off white solid ( 53.8 mg, 0.13mmol, 27.7%). **SS-1: ¹HNMR** (400 MHz, CDCl₃) δ (ppm): 7.76 (d, 1H), 7.64 (d, 1H), 5.82 (t, 1H), 5.25 (AB, 1H), 5.13 (AB, 1H), 2.65 (t, 1H), 0.69 (s, 3H). LCMS: Rt = 2.01 min. m/z = 422.3 [M+H]⁺.

### Reference Example 64. Synthesis of SN and SN intermediates.

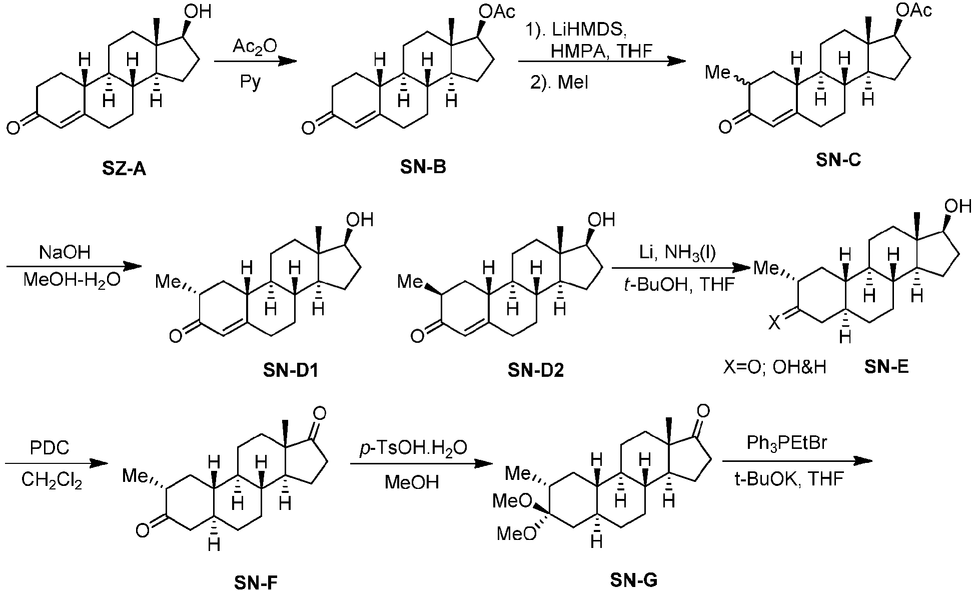

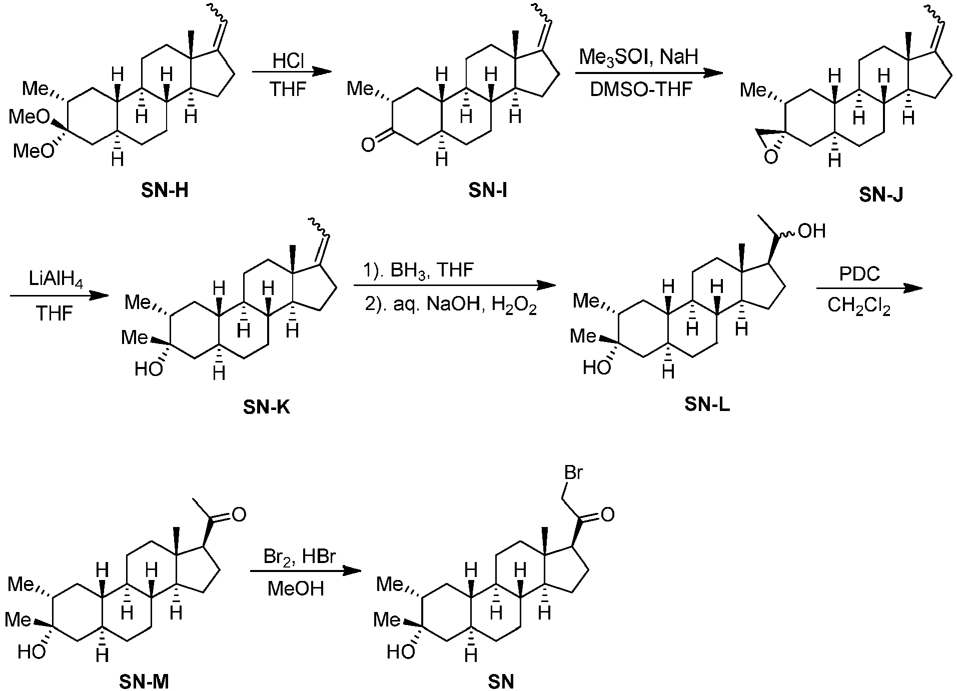

**Synthesis of compound SN-B.** To a solution of reactant **SN-A** (10.0 g, 36.44 mmol) in pyridine (30 mL) was added acetic anhydride (5.0 mL, 52.89 mmol). The mixture was stirred at 60 °C overnight. Then the solution was poured into ice-water (200 mL). The white precipitate was filtered and dissolved in ethyl acetate (300 mL). The resulting solution was washed with sat. CuSO₄.5H₂O solution (2 ×200 mL) in order to remove residual pyridine. The organic layer was further washed with brine (200 mL), dried over magnesium sulfate and concentrated *in vacuo.* The residue was purified by flash chromatography (eluant: petroleum ether/ ethyl acetate = 4:1) to afford product **SN-B** (11.125 g, 35.16 mmol, Yield=96%) as off white solid. **SN-B:** ¹HNMR (500 MHz, CDCl₃) δ(ppm):5.83 (1H, s), 4.62 (1H, dd), 2.05 (3H, s), 0.86 (3H, s).

**Synthesis of compound SN-C.** To a solution of reactant **SN-B** (4.68 g, 14.79 mmol) in THF (150 mL) was added LiHMDS (1.0 M in THF solution, 17.74 mL, 17.74 mmol) at -78°C. The solution was stirred at - 78°C for 30 minutes. Then HMPA (3.09 mL, 17.74 mmol) was added. The solution was stirred at -78 °C for another 30 minutes. Then iodomethane (2.76 mL, 44.37 mmol) was added. The solution was further stirred at -78 °C for 2 hours and warmed to room temperature and stirred for 1 hour. The reaction was quenched by addition of water (10 mL). Most THF solvent was removed *in vacuo.* Then the residue was diluted with ethyl acetate (300 mL) and the resulting solution was washed with brine (2×200 mL), dried over magnesium sulfate. Removal of solvent *in vacuo* afforded crude product SN-C (4.50 g, 13.62 mmol, Yield=92%) as thick oil. The crude product was used in the next step without further purification. **SN-C: ¹HNMR** (500 MHz, CDCl₃) δ(ppm): 5.75 (1H, s), 4.62 (1H, t), 2.05 (3H, s), 1.10 (3H, d), 0.86 (3H, s).

**Synthesis of compound SN-D1 & SN-D2.** To a solution of crude reactant **SN-C** (11.62 g, 35.16 mmol, theoretical amount) in methanol (100 mL) and water (20 mL) was added sodium hydroxide (2.81 g, 70.32 mmol). The solution was heated at 60 °C for 1 hour. Then most methanol solvent was removed *in vacuo.* The residual solution was acidified by 2 M HCl to pH 5-6. The aqueous layer was extracted with ethyl acetate (3 × 100 mL). The combined organic extracts were washed with brine (200 mL), dried over magnesium sulfate and concentrated in vacuo. The residue was purified by flash chromatography (eluant: petroleum ether/ ethyl acetate=5:1) to afford pure product **SN-D1** (2.354 g, 8.162 mmol, Yield=23%) and pure product **SN-D2** (5.306 g, 18.40 mmol, Yield=50%) as off white solid. **SN-D1:** ¹HNMR (500 MHz, CDCl₃) δ(ppm): 5.81 (1H, s), 3.67 (1H, t), 1.11 (3H, d), 0.81 (3H,s). **SN-D2: ¹HNMR** (500 MHz, CDCl₃) δ(ppm): 5.74 (1H, s), 3.67 (1H, t), 1.11 (3H, d), 0.81 (3H, s).

**Synthesis of compound SN-E.** To liquid ammonia (200 mL) was added lithium (1.80 g, 260 mmol) at -78 °C. The liquid then turned to deep blue. Then a solution of reactant **SN-D1** (3.0 g, 10.40 mmol) in t-BuOH (1.0 mL, 10.40 mmol ) and THF (100 mL) was added to Li-ammonia solution. The mixture was stirred at - 78 °C for 4 hours. Then NH₄Cl solid (20 g) was added to quench the reaction. The mixture was turned from deep blue to white. The mixture was allowed to warm to room temperature and ammonia was evaporated in a hood overnight. To the residue was added water (300 mL). The mixture was acidified by conc. HCl to pH 6-7. Then ethyl acetate (300 mL) was added. The separated aqueous layer was further extracted with ethyl acetate (2×100 mL). The combined organic extracts were washed with brine (300 mL), dried over magnesium sulfate and concentrated *in vacuo.* The crude product **SN-E** was used directly without further purification in the next step.

**Synthesis of compound SN-F.** To a solution of crude reactant **SN-E** (1.749 g, 6.022 mmol) in dichloromethane (60 mL) was added pyridinium dichromate (PDC) (3.398 g, 9.033 mmol). The mixture was stirred at room temperature overnight. The solution was filtered through a short pad of celite. The celite was washed with CH₂Cl₂ (3×50 mL). The combined CH₂Cl₂ solution was concentrated *in vacuo.* The residue was purified by flash chromatography (eluant: petroleum ether/ ethyl acetate=5:1) to afford product **SN-F** (1.298 g, 4.50 mmol, Yield=75%) as off white solid. **SN-F:** ¹HNMR (400 MHz, CDCl₃) δ(ppm): 1.02 (3H, d), 0.91 (3H, s).

**Synthesis of compound SN-G.** To a solution of reactant **SN-F** (1.948 g, 6.754 mmol) in anhydrous methanol (50 mL) was added p-toluenesulfonic acid monohydrate (128 mg, 0.6754 mmol). The solution was heated at 70 °C for 3 hours. The reaction was quenched by addition of sat. Na₂CO₃ solution (10 mL). Most methanol solvent was removed *in vacuo.* Then the residue was diluted with ethyl acetate (200 mL). The resulting solution was washed with sat. Na₂CO₃ solution (2×100 mL). The combined aqueous layers were extracted with ethyl acetate (50 mL). The combined organic extracts were washed with brine (100 mL), dried over magnesium sulfate and concentrated *in vacuo.* The residue was purified by flash chromatography (eluant: petroleum ether/ ethyl acetate= 10:1, added 0.1% NEt₃) to afford product **SN-G** (652 mg, 1.949 mmol, Yield=29%) as off white solid. Furthermore, starting material (1.338 g) was also recovered. So the yield based on recovered starting material is 92%. **SN-G:** ¹HNMR (500 MHz, d6-acetone) δ(ppm): 3.079 (3H, s), 3.075 (3H, s), 2.38 (1H, dd), 1.98 (1H, dd), 0.91 (3H, d, J=7.2 Hz), 0.85 (3H, s).

**Synthesis of compound SN-H.** To a solution of ethyltriphenylphosphonium bromide (8.795 g, 23.69 mmol) in anhydrous THF (20 mL) was added t-BuOK (2.658 g, 23.69 mmol). The solution then became reddish in color and was heated at 70 °C for 2 hours. Then the reactant **SN-G** (1.642 g, 4.909 mmol) was added in one portion. The solution was heated at 70 °C overnight. The reaction was quenched by the addition of water (10 mL). The mixture was diluted with ethyl acetate (200 mL) and the resulting solution was washed with brine (2×100 mL), dried over magnesium sulfate and concentrated *in vacuo.* The crude product **SN-H** was used directly in the next step without further purification.

**Synthesis of compound SN-I.** To the crude product **SN-H** (1.702 g, 4.909 mmol, theoretical amount) in THF (30 mL) was added 2 M HCl (3 mL). The solution was stirred at ambient temperature for 1 hour. The mixture was diluted with ethyl acetate (300 mL) and the resulting solution was washed with sat. Na₂CO₃ solution (2×100 mL). The combined aqueous layers were extracted with ethyl acetate (100 mL). The combined organic extracts were washed with brine (100 mL), dried over magnesium sulfate and concentrated *in vacuo.* The residue was purified by flash chromatography (eluant: petroleum ether/ ethyl acetate =100:3) to afford crude product **SN-I** (1.746 g) as off white solid which was contaminated with some inseparated PPh₃. Judged by the integration of ¹HNMR spectrum, the ratio of desired product to PPh₃ is 3:1, so the amount of desired product **SN-I** is 1.354 g (4.506 mmol), the yield is 92%. **SN-I:** ¹HNMR (500 MHz, CDC13) δ(ppm): 5.13 (1H, qt), 1.66 (3H, dt), 1.02 (3H, d), 0.91 (3H, s).

**Synthesis of compound SN-J.** To a solution of trimethylsulfoxonium iodide (5.213 g, 23.69 mmol) in anhydrous DMSO (30 mL) was added sodium hydride (60% wt, 948 mg, 23.69 mmol). The mixture was stirred at 25 °C for 1 hour. Then a solution of crude reactant (1.746 g, contaminated with some residual PPh3, theoretical amount, 1.354 g, 4.506 mmol) in anhydrous THF (10 mL) was added. The mixture was stirred at 25 °C overnight. The reaction was quenched by addition of water (5 mL). The mixture was diluted with ethyl acetate (300 mL) and the resulting solution was washed with water (2×100 mL), followed by brine (100 mL) dried over magnesium sulfate and concentrated *in vacuo.* The crude product **SN-J** was used directly in the next step without further purification.

**Synthesis of compound SN-K.** To a solution of crude reactant **SN-J** (theoretical amount, 1.417 g, 4.506 mmol) in anhydrous THF (30 mL) was added lithium aluminum hydride (342 mg, 9.012 mmol) in portions. The suspension was stirred at 25 °C for 1 hour. Then the reaction was quenched by addition of ethyl acetate (5 mL) followed by water (5 mL). The off white solid was filtered and thoroughly washed with ethyl acetate (5×100 mL). The combined filtrate was washed with brine (200 mL), dried over magnesium sulfate and concentrated *in vacuo.* The residue was purified by flash chromatography (eluant: petroleum ether/ ethyl acetate=20:1) to afford product **SN-K** (458 mg, 1.447 mmol, 2 steps total yield=32%) as off white solid.

**Synthesis of compound SN-L.** To a solution of reactant **SN-K** (458 mg, 1.447 mmol) in anhydrous THF (15 mL) was added BH₃.THF (1.0 M, 7.23 mL, 7.23 mmol), The solution was stirred at 25 °C overnight. Then the reaction was quenched by addition of water (5 mL). 2 M NaOH solution (10 mL) was added followed by 30 % H₂O₂ (10 mL). The mixture was stirred at room temperature for 1 hour. The mixture was diluted with ethyl acetate (200 mL) and resulting solution was washed with brine (2×100 mL), dried over magnesium sulfate and concentrated *in vacuo.* The crude product was used directly in the next step without further purification.

**Synthesis of compound SN-M.** To a solution of crude reactant **SN-L** (484 mg, 1.447 mmol, theoretical amount) in dichloromethane (40 mL) was added pyridinium dichromate (PDC) in portions (1633 mg, 4.341 mmol). The solution was stirred at 25 °C overnight. Then the mixture was filtered through a short pad of silica gel and the silica gel was washed with dichloromethane (3×50 mL). All filtrate was combined and concentrated *in vacuo.* The residue was purified by flash chromatography (eluant: petroleum ether/ ethyl acetate=8:1) to afford product **SN-M** (305 mg, 0.917 mmol, Yield=63% (2 steps)) as off white solid. **SN-**L: ¹HNMR (500 MHz, CDC13) δ(ppm): 2.54 (1H, t

0, 2.12-2.19 (1H, m), 2.12 (3H, s), 1.99 (1H, td), 1.80-1.86 (1H, m), 1.17 (3H, s), 0.92 (3H, d), 0.61 (3H, s). **SN-M:** ¹³CNMR (100 MHz, CDC13) δ(ppm): 209.75, 71.09, 63.96, 55.89, 47.96, 47.80, 47.00, 44.35, 41.19, 40.22, 39.05, 37.95, 34.49, 33.14, 31.54, 30.92, 28.46, 25.82, 24.22, 22.76, 15.14, 13.45.

**Synthesis of compound SN.** To a solution of reactant **SN-M** (100 mg, 0.301 mmol) in methanol (10 mL) was added 48% hydrobromic acid (152 mg, 0.903 mmol) followed by bromine (241 mg, 0.077 mL, 1.505 mmol). The solution was heated at 25 °C for 1.5 hours. Then the mixture was poured into cooled water (50 mL). The resulting solid was extracted with ethyl acetate (2×50 mL). The combined organic extracts were washed with brine (50 mL), dried over magnesium sulfate and concentrated *in vacuo.* The crude product **SN** was used directly without further purification in the next step.

### Reference Example 65. Synthesis of compounds SN-1 and SN-2.

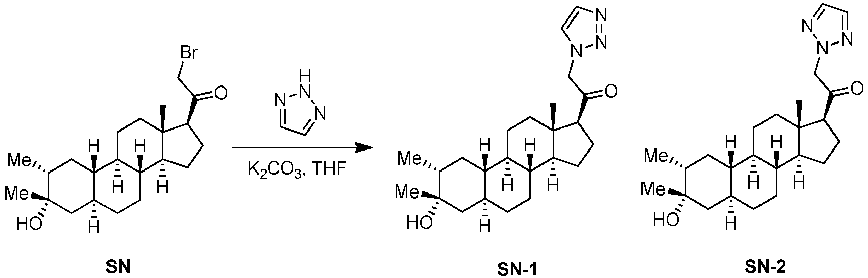

To a solution of crude reactant **SN** (124 mg, 0.301 mmol) in anhydrous THF (6 mL) was added 1,2,3-trizaole (31 mg, 0.45 mmol) followed by potassium carbonate (62 mg, 0.45 mmol). The solution was heated at 50 °C overnight. Then the solution was diluted with ethyl acetate (100 mL). The resulting solution was washed with brine (2×50 mL), dried over magnesium sulfate and concentrated *in vacuo.* The crude product was purified by reverse phase prep-HPLC to afford product **SN-1** (21 mg, 0.0526 mmol, Yield=17%) and product **SN-2** (16 mg, 0.0400 mmol, Yield=13%) as off white solid. **SN-1:** HNMR (400 MHz, CDC13) δ(ppm): 7.76 (1H, s), 7.65 (1H, s), 5.20 (1H, AB), 5.14 (1H, AB), 2.66 (1H, t), 2.21 (1H, dd), 1.18 (3H, s), 0.92 (3H, d), 0.68 (3H, s). **SN-2:** ¹HNMR (500 MHz, CDCl₃) δ(ppm): 7.69 (2H, s), 5.27 (1H, AB), 5.23 (1H, AB), 2.60 (1H, t), 2.20 (1H, dd), 1.17 (3H, s), 0.92 (3H, d), 0.71 (3H, s).

### Reference Example 66. Synthesis of SU and SU intermediates.

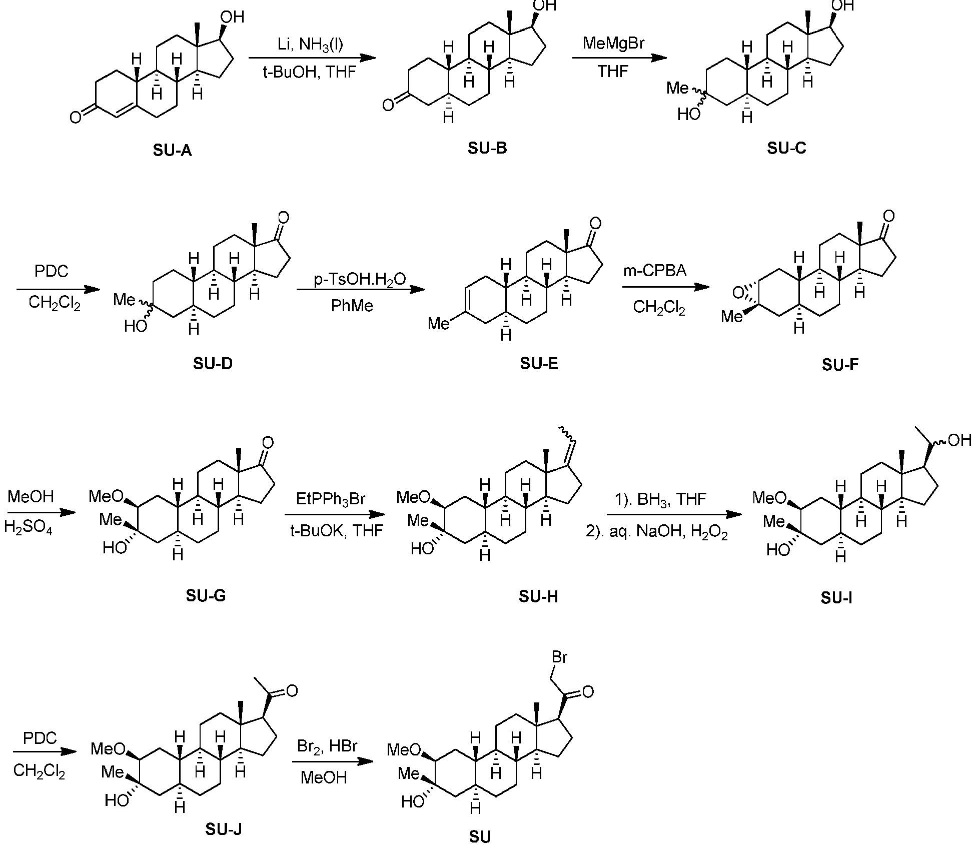

**Synthesis of compound SU-B.** To NH₃ (liquid, 2.0 L) was added lithium (7.0 g, 1 mol) at -78 °C. After the liquid was turned to deep blue, a solution of compound SU-A (27.0 g, 100 mmol) in t-BuOH (7.4 g, 100 mmol) and THF (20 mL) was added dropwise. The mixture was stirred at -78 °C for 4 hours. Then NH₄Cl solid (50 g) was added to quench the reaction. The mixture was turned from deep blue to white. The mixture was allowed to warm to room temperature and ammonia was evaporated overnight. The residue was dissolved in 0.5 N aqueous HCl (50 mL) and extracted with dichloromethane (200 mL×3). The combined organic layers were washed with saturated NaHCO₃ (200 mL) and brine (200 mL), dried over magnesium sulfate and concentrated *in vacuo.* The crude product was purified by flash chromatography (PE / EtOAc = 4:1) to get product **SU-B** (18.98 g, 68.7%) as off white solid. **SU-B:** ¹H NMR (500 MHz, CDCl₃) δ(ppm): 3.66 (1H, t, J=8.0Hz), 2.29-2.27 (2H, m), 2.12-2.07 (2H, m), 1.83-1.81 (2H, m), 1.50 (1H, s), 0.77 (3H, s).

**Synthesis of compound SU-C.** A sample of 19.0 g compound **SU-B** (68.84 mmol) was dissolved in 50 mL THF at 0 °C. Then 70 mL MeMgBr in THF(3M) was added dropwise in 30 min.The reaction was kept at 0 °C for 8 h. The reaction mixture was quenched with ice-cold water and extracted with EtOAc (200 mL×3).The combined organic layers were washed with brine, dried over sodium sulfate, filtered and concentrated. The white residue was purified by flash column chromatography (PE / EtOAc = 5:1) to give product SU-C (19.0 g, 94%) as off white solid. **SU-C:** ¹H NMR (500 MHz, CDCl₃) δ (ppm): 5.78 (1H, br), 5.36 (1H, t), 3.67 (1H, t), 1.73 (3H, s), 0.77 (3H, s).

**Synthesis of compound SU-D.** To a solution of compound **SU-C** (19.0 g, 65.07 mmol) in dichloromethane (100 mL) was added pyridinium dichromate (PDC) (48.9 g, 130.14 mmol). The mixture was stirred at room temperature overnight. The solution was filtered through a short pad of celite. The celite was washed with CH₂Cl₂ (3×100 mL). The combined CH₂Cl₂ solution was concentrated in vacuo. The residue was purified by flash chromatography (eluant: PE / EtOAc =5:1) to afford product **SU-D** (10.0 g, 53%) as off white solid. **SU-D:** ¹H NMR (500 MHz, CDCl₃) δ (ppm): 2.44 (1H, dd), 2.07 (1H, m), 1.21 (3H, s), 0.87 (3H, s).

**Synthesis of compound SU-E:** To a solution of compound **SU-D** (5.0 g, 17.2 mmol) in anhydrous toluene (100 mL) was added to the p-toluenesulfonic acid on sillica gel (80g), the mixture was stirred under 45 °C for 1 hour. The insouble bi-products were removed from sillica gel by elution with PE / EtOAc (10 / 1). The crude product **SU-E** (3.20 g, 11.75 mmol) was used in the next step without further purification.

**Synthesis of compound SU-F:** To a solution of compound **SU-E** (3.20 g, 11.75 mmol) in 10 mL anhydrous dichloromethane was added mCPBA (4.04 g, 23.50mmol), and the reaction mixture was stirred over night at room teperature. The reaction mixture then was extracted with CH₂Cl₂, the combined organic layer was washed twice with NaHCO₃ (100 mL) and brine, dried over Na₂SO₄ and concentrated. The crude product **SU-F** was used in the next step without further purification.

**Synthesis of compound SU-G.** To a solution of compound SU-F (11.75 mmol) in methanol was added H₂SO₄ (0.5mL), and the reaction mixture was stirred for 2h at room temperature. The reaction solution was then extracted with CH₂Cl₂ (200 mL ×3), the combined organic layer was washed with NaHCO₃ (100 mL) and brine, dried over Na₂SO₄ and concentrated. The residue was purified by chromatography (PE / EtOAc = 10:1) to afford compound **SU-G** (3.30 g, 10.30 mmol, Yield = 87% for two steps) as off white solid.

**Synthesis of compound SU-H.** To a solution of ethyltriphenylphosphonium bromide (11.52 g, 31.0 mmol) in anhydrous THF (20 mL) was added t-BuOK (3.48 g, 31.0 mmol). The solution was turned to reddish and heated at 70 °C for 3 hours. Then compound **SU-G** (3.30 g, 10.30 mmol) was added in one portion. The reaction solution was heated at 70 °C overnight, then was quenched by the addition of water (10 mL). The mixture was diluted with EtOAc (200 mL) and the resulting solution was washed with brine (2×100 mL), dried over magnesium sulfate and concentrated *in vacuo.* The crude product **SU-H** (1.90 g) was used directly in the next step without further purification.

**Synthesis of compound SU-I.** To a solution of compound **SU-H** (1.90 g, 5.72 mmol) in dry THF (20 mL) was added BH₃-THF (18 mL of 1.0M solution in THF). After stirring at room temperature for 1h, the reaction mixture was cooled in an ice bath then quenched slowly with 10% aqueous NaOH (12 mL) followed by 30% H₂O₂ (20 mL). The mixture was allowed to stir at room teperature for 1h then extracted with EA (100 mL×3). The combined organic layer was washed with 10% aqueous Na₂S₂O₃ (50 mL), brine, dried over Na₂SO₄, filtered and concentrated to afford crude compound **SU-I** (1.86 g, 5.31 mmol). The crude product was used in the next step without further purification.

**Synthesis of compound SU-J.** To a solution of crude compound **SU-I** (1.86 g, 5.31 mmol) in dichloromethane (50 mL) was added pyridinium dichromate (PDC) in portions (3.98 g, 10.62 mmol). The solution was stirred at 25 °C overnight. Then the mixture was filtered through a short pad of silica gel and the silica gel was washed with dichloromethane (3×50 mL). All filtrate was combined and concentrated *in vacuo.* The residue was purified by flash chromatography (eluant: PE / EtOAc =10:1) to afford product SU-**J** (1.20 g, 3.45 mmol, 65%) as off white solid.

**SU-J:** ¹HNMR (500 MHz, CDC13) δ(ppm): 3.33 (3H, s), 3.04 (1H, s), 2.53 (1H, t), 2.12 (3H, s), 1.26 (3H, s), 0.62 (3H, s)

**Synthesis of compound SU.** To a solution of reactant SU-J (100 mg, 0.287 mmol) in methanol (10 mL) was added 48% HBr (152 mg, 0.903 mmol) followed by bromine (0.08 mL, 1.505 mmol). The solution was heated at 25 °C for 1.5 hours. Then the mixture was poured into cooled water (50 mL). The resulting solid was extracted with ethyl acetate (2×50 mL). The combined organic extracts were washed with brine (50 mL), dried over magnesium sulfate and concentrated *in vacuo.* The crude product **SU** was used directly without further purification in the next step.

### Reference Example 67. Synthesis of SU-1 and SU-2.

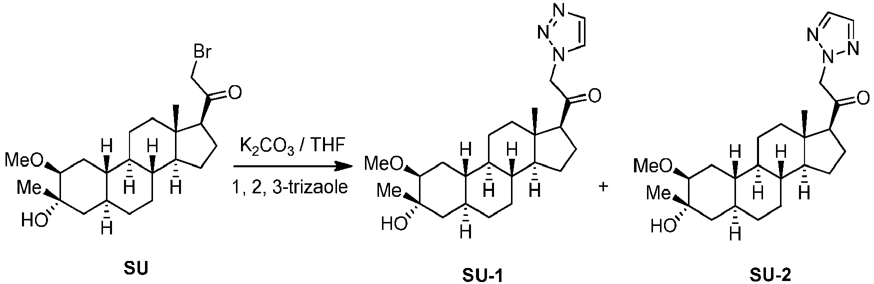

To a solution of crude compound **SU** (100 mg, 0.243 mmol) in anhydrous THF (6 mL) was added 1, 2, 3-trizaole (34 mg, 0.50 mmol) followed by potassium carbonate (70 mg, 0.50 mmol). The solution was heated at 50 °C overnight. Then the solution was diluted with EtOAc (100 mL). The resulting solution was washed with brine (2×50 mL), dried over magnesium sulfate and concentrated *in vacuo.* The crude product was purified by reverse phase prep-HPLC to afford product **SU-1** (35 mg, 0.084mmol, Yield=34%) and product **SU-2** (20 mg, 0.048mmol, 20%) as off white solid. **SU-1:** ¹H NMR (500 MHz, CDC13) δ (ppm): 7.76 (1H, s), 7.65 (1H, s), 5.27 (1H, AB), 5.14 (1H, AB), 3.34 (3H, s), 3.04 (1H, s), 2.65 (1H, t), 1.24 (3H, s), 0.68 (3H, s). SU-2: ¹H NMR (500 MHz, CDC13) δ(ppm): 7.68 (2H, s), 5.26 (1H, AB), 5.22 (1H, AB), 3.33 (3H, s), 3.04 (1H, s), 2.59 (1H, t), 1.24 (3H, s), 0.72 (3H, s).

### Reference Example 68. Synthesis of SY and SY intermediates.

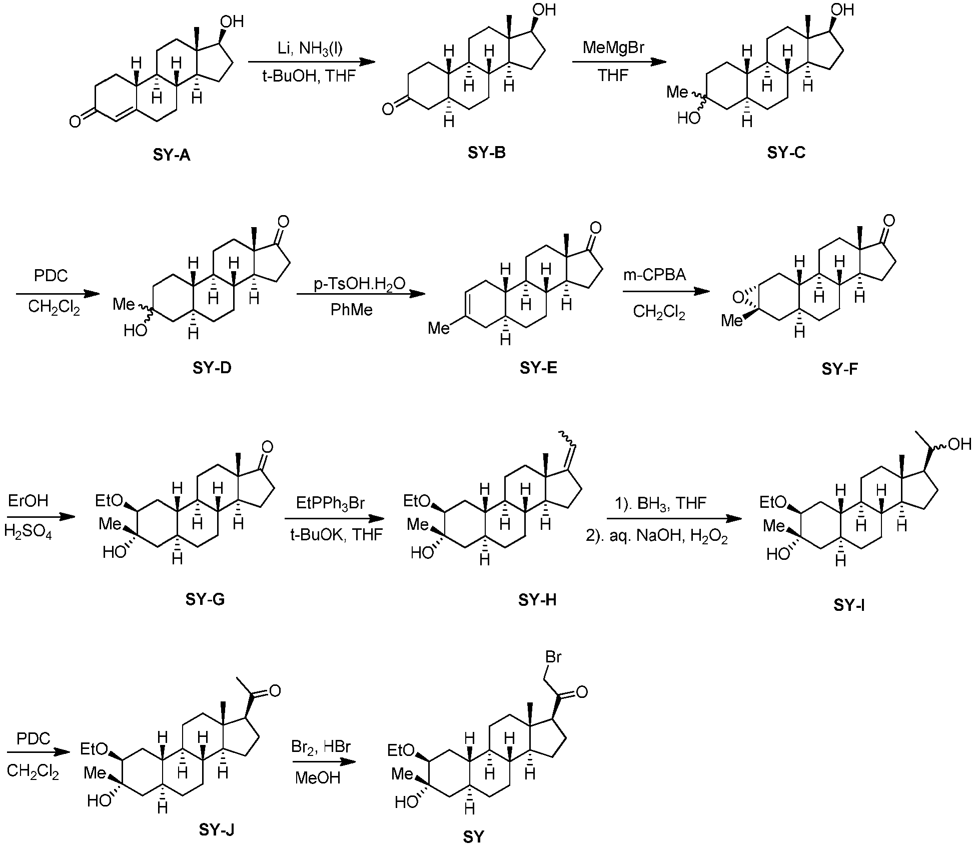

**Synthesis of compound SY-B.** To NH₃ (liquid, 2.0 L) was added lithium (7.0 g, 1 mol) at -78 °C. After the liquid turned to a deep blue, a solution of reactant SY-A (27.0 g, 100 mmol) in t-BuOH (7.4 g, 100 mmol) and THF (20 mL) was added dropwise. The mixture was stirred at -78 °C for 4 hours, then NH₄Cl solid (50 g) was added to quench the reaction. The mixture then turned from deep blue to white. The mixture was allowed to warm to room temperature and ammonia was evaporated in a fume hood overnight. The residue was dissolved in 0.5 N HCl (50 mL) and extracted with dichloromethane (200 mL×3). The combined organic layers were washed with saturated NaHCO₃ (200 mL) and brine (200 mL), dried over magnesium sulfate and concentrated *in vacuo.* The crude product was purified by flash chromatography (PE / EA = 4:1) to get product **SY-B** (18.98 g, 68.76 mmol, Yield=68.7%) as off white solid. **SY-B:** ¹H NMR (500 MHz, CDCl₃) δ(ppm): 3.66 (1H, t), 2.29-2.27 (2H, m), 2.12-2.07 (2H, m), 1.83-1.81 (2H, m), 1.50 (1H, s), 0.77 (3H, s).

**Synthesis of compound SY-C.** 19.0 g of compound **SY-B** (68.84 mmol) was dissloved in 50 mL THF at 0 °C. Then 70 mL MeMgBr in THF (3M) was added dropwise over 30 minutes then the reaction was kept at 0 °C for 8 h. The reaction mixture was quenched with ice-cold water and extracted with EA (200 mL×3).The combined organic layers were washed with brine, dried over sodium sulfate,filtered and concentrated. The white residue was purified by flash column chromatography (PE / EA = 5:1) to get product **SY-C** (19.0 g, 65.07 mmol Yield=94%) as off white solid. **SY-C:** ¹H NMR (500 MHz, CDCl₃) δ (ppm): 5.78 (1H, br), 5.36 (1H, t), 3.67 (1H, t), 1.73 (3H, s), 0.77 (3H, s).

**Synthesis of compound SY-D.** To a solution of reactant **SY-C** (19.0 g, 65.07 mmol) in dichloromethane (100 mL) was added pyridinium dichromate (PDC) (48.9 g, 130.14 mmol) at room temperature and the mixture was stirred at room temperature overnight. The solution was filtered through a short pad of celite. The celite was washed with CH₂Cl₂ (3×100 mL). The combined CH₂Cl₂ solution was concentrated *in vacuo.* The residue was purified by flash chromatography (eluant: PE / EA =5:1) to afford product **SY-D** (10.0 g, 34.48 mmol, Yield=53%) as off white solid. **SY-D:** ¹H NMR (500 MHz, CDCl₃) δ (ppm): 2.44 (1H, dd), 2.07 (1H, m), 1.21 (3H, s), 0.87 (3H, s).

**Synthesis of compound SY-E.** To a solution of reactant **SY-D** (5.0 g, 17.2 mmol) in anhydrous toluene (100 mL) was rapidly added to the p-toluenesulfonic acid on sillica gel (80g), the mixture is stirred at 45 °C for 1 hour. The product were removed from sillica gel by elution with (PE / EA =30:1). The crude product **SY-E** (3.20 g, 11.75 mmol) was used in the next step without further purification.

**Synthesis of compound SY-F.** To a solution **of SY-E** (3.20 g, 11.75 mmol) in 10 mL anhydrous dichloromethane was added mCPBA (4.04 g, 23.50mmol), and the reaction mixture was stirred overnight at room teperature. The solution was then extracted with CH₂Cl₂ (2x100 mL), and the combined organic layer was washed twice with NaHCO₃ (100 mL) and brine, dried over Na₂SO₄ and concentrated. The crude product **SY-E** was used in the next step without further purification.

**Synthesis of compound SY-G.** To a solution **of SY-F** (900 mg, 3.12 mmol) in ethanol (50mL) was added concentrated H₂SO₄ (0.5mL), and the reaction mixture was stirred for 2h at room teperature. As soon as TLC indicated complete conversion, the solution was extracted with CH₂Cl₂ (200 mL×3) , the combined organic layer was washed with NaHCO₃ (100 mL) and brine, dried over Na₂SO₄ and concentrated. The residue was purified by chromatography (PE / EA = 10:1) to afford compound **SY-G** (600mg, 1.80 mmol, Yield = 57.6% for two steps) as off white solid.

**Synthesis of compound SY-H.** To a solution of ethyltriphenylphosphonium bromide (1.99g, 5.96mmol) in anhydrous THF (10 mL) was added t-BuOK (500mg,4.48mmol). The solution was turned to reddish and heated at 70 °C for 3 hours. Then the reactant **SY-G** 600mg, 1.79mmol) was added in one portion. The solution was heated at 70 °C overnight. The reaction was quenched by the addition of water (5mL). The mixture was diluted with ethyl acetate (100 mL) and the resulting solution was washed with brine (2×50 mL), dried over magnesium sulfate and concentrated *in vacuo.* The residue was purified by flash chromatography (eluant: petroleum ether: elthyl acetate=20:1) to afford product **SY-H** (1.55g, 4.48mmol, 75.2%) as an off white solid.

**Synthesis of compound SY-I.** To a solution of compound **SY-H** (1.20 g, 3.47 mmol) in dry THF (20 mL) was added BH₃-THF (18 mL of 1.0M solution in THF). After stirring at room temperature for 1h, the reaction mixture was cooled in an ice bath then quenched slowly with 10% aqueous NaOH (10 mL) followed by 30% H₂O₂ (15 mL). The mixture was allowed to stir at room teperature for 1h then extracted with EA (100 mL×3). The combined organic layer was washed with 10% aqueous Na₂S₂O₃ (50 mL), brine, dried over Na₂SO₄, filtered and concentrated to afford crude compound **SY-I** (1.12 g). The crude product was used in the next step without further purification.

**Synthesis of compound SY-J.** To a solution of crude reactant **SY-I** (1.12 g, 3.08 mmol) in dichloromethane (50 mL) was added pyridinium dichromate (PDC) in portions (3.32 g, 6.16 mmol) at room temperature. The solution was stirred at 25 °C overnight, then the mixture was filtered through a short pad of silica gel and the silica gel was washed with dichloromethane (3×50 mL). All filtrate was combined and concentrated *in vacuo.* The residue was purified by flash chromatography (eluant: PE / EA =10:1) to afford product **SY-J** (0.95 g, 2.62mmol, Yield=85.1%) as off white solid. **SY-J:** ¹HNMR (500 MHz, CDC13) δ(ppm): 3.59 (1H, m), 3.36 (1H, m), 3.12 (1H, s), 2.53 (1H, t), 2.14 (3H, s), 1.23 (3H, s), 1.17 (3H, t), 0.62 (3H, s).

**Synthesis of compound SY.** To a solution of reactant **SY-J** (80 mg, 0.221 mmol) in methanol (5 mL) was added 48% hydrobromic acid (148 mg, 0.884mmol) followed by bromine (241 mg, 0.077 mL, 1.505 mmol). The solution was heated at 25 °C for 1.5 hours, then the mixture was poured into cooled water (50 mL). The resulting solid was extracted with ethyl acetate (2×50 mL). The combined organic extracts were washed with brine (20 mL), dried over magnesium sulfate and concentrated *in vacuo.* The crude product SY was used directly without further purification in the next step.

### Reference Example 69. Synthesis of compounds SY-1 and SY-2.

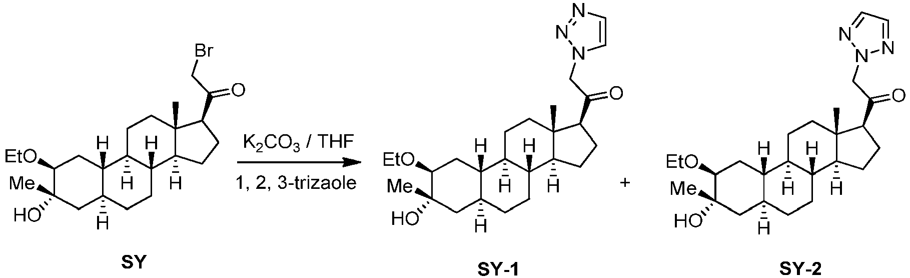

To a solution of compound **SY** (110 mg, crude) in dry THF (5 mL) was added potassium carbonate (100 mg) and 0.3 mL 1H-1,2,3-triazole. The reaction mixture was stirred at 50 °C for two days, and then extracted with EtOAc (3 × 10 mL). The combined EtOAc extracts were washed with brine (10 mL), dried over Na₂SO₄, filtered, and concentrated. The residue was purified by preparative HPLC to afford title compound **SY-1** (23 mg, yield =21%) and **SY-2** (9 mg, yield =8%) as off white solid. **SY-1:** ¹H NMR (500 MHz, CDC13) δ (ppm): 7.77 (1H, s), 7.66 (1H, s), 5.27 (1H, AB), 5.13 (1H, AB), 3.61-3.57 (1H, m), 3.38-3.34 (1H, m), 3.12 (1H, s), 2.64 (1H), 1.23 (3H, s), 1.16(3H, t), 0.68(3H, s). SY-2: ¹H NMR (500 MHz, CDC13) δ(ppm): 7.68 (2H, s), 5.26 (1H, AB), 5.21 (1H, AB), 3.61-3.57 (1H, m), 3.38-3.34 (1H, m), 3.12 (1H, s), 2.59 (1H, t), 2.08-1.97 (1H, m), 1.23 (3H, s), 1.16 (3H, t), 0.72 (3H, s).

### Reference Example 70. Synthesis of compounds SA-10, SA-11, SA-12, SA-13, and SA-14.

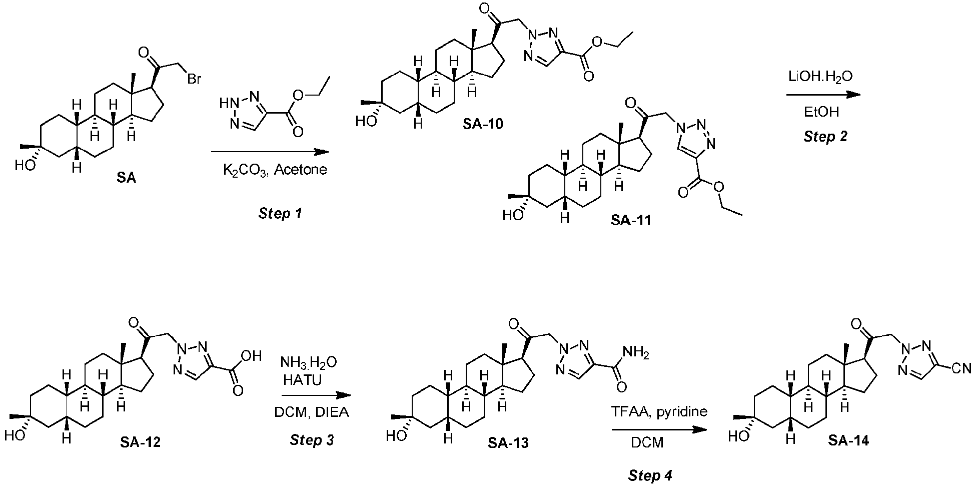

***Step 1**.* To a solution of **SA** (4.3 g, 10.8 mmol) in acetone (50 mL) was added K₂CO₃ (2.98 g, 21.6 mmol) and ethyl 2H-1,2,3-triazole-4-carboxylate (2.28 g, 16.2 mmol) at 25°C. The mixture was stirred at 25°C for 12 hours. TLC showed the starting material was disappeared. The reaction was quenched by water (30 mL) and then extracted with EA (30 mL^{∗}2). The combined organic phase was washed with saturated brine (50 mL), dried over anhydrous Na₂SO₄, filtered and concentrated in vacuum. The residue was purified by silica gel chromatography (100-200 mesh silica gel, Petroleum ether/Ethyl acetate=3/1 to EA) to afford **SA-10** (2.6 g, Purity: 95%, Yield: 50%, 54 mg for delivery) as a white solid and **SA-11** (1.5 g, Purity: 95%, Yield: 28.7%, 21 mg for delivery) as a light yellow solid. **SA-10:** 1H NMR CDCl₃ Bruker_P_400MHz δ 8.11 (s, 1H), 5.35-5.22 (m, 2H), 4.43 (q, *J* = 7.3 Hz, 2H), 2.64-2.55 (m, 1H), 2.24-2.03 (m, 2H), 1.91-1.60 (m, 7H), 1.50-1.23 (m, 18H), 1.18-1.04 (m, 3H), 0.71 (s, 3H). LCMS Rt = 1.081 min in 2 min chromatography, 30-90AB, purity 98.95%, MS ESI calcd. For C₂₆H₃₉N₃O₄ [M-H₂O+H]⁺ 440, found 440. **SA-11:** 1H NMR CDCl₃ Bruker_P_400MHz δ 8.17 (s, 1H), 5.33-5.12 (m, 2H), 4.43 (q, *J* = 7.1 Hz, 2H), 2.71-2.61 (m, 1H), 2.28-2.15 (m, 1H), 2.06 (d, *J* = 12.0 Hz, 1H), 1.90-1.70 (m, 6H), 1.69-1.60 (m, 1H), 1.55-1.38 (m, 10H), 1.37-1.21 (m, 8H), 1.18- 1.02 (m, 3H), 0.67 (s, 3H). LCMS Rₜ = 1.000 min in 2 min chromatography, 30-90AB, purity 96.6%, MS ESI calcd. For C₂₆H₃₉N₃O₄ [M-H₂O+H]⁺ 440, found 440.

***Step 2.*** To a solution of **SA-10** (300 mg, 655 umol) in EtOH (8 mL) was added LiOH.H₂O (137 mg, 3.27 mmol) at 25°C. The mixture was stirred at 25°C for 16 hrs. LCMS showed the reaction was complete. The reaction was poured into water (20 mL) and acidified with HCl (2 M) to pH 3-4, extracted with EA (50 mL^{∗}2). The combined organic layer was concentrated in vacuum. The residue was purified by prep-HPLC (0.05% HCl-ACN) to afford **SA-12** (90 mg, Purity: 100%, Yield: 32%, 28 mg for delivery) and a byproduct (13 mg, Purity: 100%, Yield: 4.62%) as a white solid. **SA-12:** 1H NMR DMSO Bruker_P_400MHz δ 8.24 (s, 1H), 5.76-5.39 (m, 2H), 4.27 (s, 1H), 2.78 (s, 1H), 2.05 (d, *J* = 11.5 Hz, 2H), 1.76-1.59 (m, 8H), 1.49-1.23 (m, 10H), 1.16-1.04 (m, 8H), 0.61 (s, 3H). LCMS Rₜ = 0.913 min in 2 min chromatography, 30-90AB, purity 96.6%, MS ESI calcd. For C₂₄H₃₅N₃O₄ [M+Na]⁺ 452, found 452.

***Step 3**.* To a solution of **SA-12** (300 mg, 698 umol) in DCM (15 mL) was added HATU (395 mg, 1.04 mmol), DIEA (224 mg, 1.74 mmol) at 25°C. The mixture was stirred at 25°C for 15 min. The ammonia hydrate (0.3 mL, 26% in water) was added to the solution. The mixture was stirred at 25°C for 30 min. LCMS showed the reaction was complete. The mixture was concentrated. The residue was purified by prep-HPLC (0.05%HCl-ACN) to afford **SA-13** (120 mg, Purity: 100%, Yield: 40.1%) as a white solid. **SA-13:** ¹H NMR DMSO Bruker A 400MHz δ 8.13 (s, 1H), 7.81 (s, 1H), 7.54 (s, 1H), 5.63-5.33 (m, 2H), 4.24 (s, 1H), 2.79-2.71 (m, 1H), 2.08-1.97 (m, 2H), 1.78-0.94 (m, 25H), 0.60 (s, 3H). LCMS Rt = 0.878 min in 2 min chromatography, 30-90AB, purity 100%, MS ESI calcd. For C₂₄H₃₆N₄O₃ [M-H₂O+H]⁺ 411, found 411.

***Step 4.*** To a solution of **SA-13** (82 mg, 191 umol) in DCM (4 mL) was added TFAA (120 mg, 573 umol), pyridine (60.3 mg, 764 umol) at 25°C. The mixture was stirred at 25°C for 16 hrs. LCMS showed the reaction was complete. The mixture was concentrated and the residue was purified by prep-HPLC (0.1%TFA-ACN) to afford **SA-14** (35 mg, Purity: 98.8%, Yield: 44%) as a white solid. **SA-14:** ¹H NMR CDCl₃ Bruker_P_400MHz δ 8.04-8.00 (m, 1H), 5.35-5.24 (m, 2H), 2.67-2.60 (m, 1H), 2.26-2.04 (m, 3H), 1.96-1.86 (m, 3H), 1.83-1.72 (m, 6H), 1.69-1.62 (m, 4H), 1.58-1.06 (m, 11H), 0.71 (s, 3H). LCMS Rt = 1.313 min in 2 min chromatography, 30-90AB, purity 98.8%, MS ESI calcd. For C₂₄H₃₄N₄O₂ [M-H₂O+H] 393, found 393.

### Reference Example 71. Synthesis of compounds SA-17, SA-18, and SA-20.

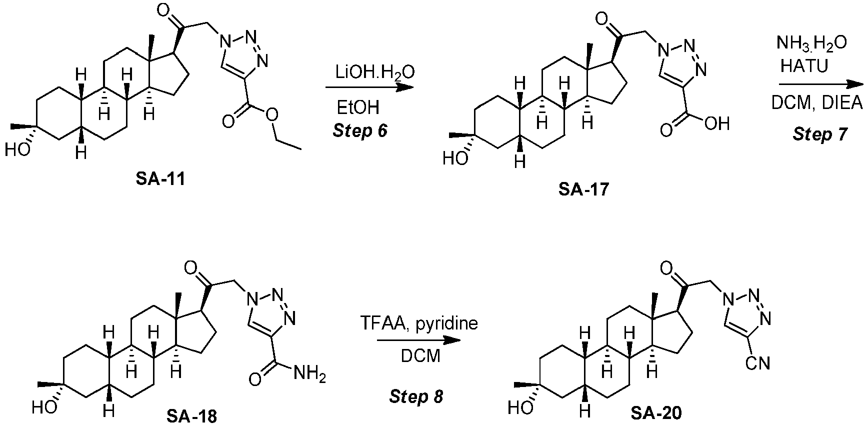

***Step 6*.** To a solution of **SA-11** (200 mg, 437 umol) in EtOH (5 mL) was added LiOH.H₂O (91.4 mg, 2.18 mmol) at 25°C. The mixture was stirred at 25°C for 16 hrs. LCMS showed the reaction was complete. The reaction was poured in to water (20 mL) and acidified with HCl (2 M) to pH 3-4, extracted with EA (50 mL^{∗}2). The combined organic layer was concentrated in vacuum. The residue was purified by prep-HPLC (0.05% HCl-ACN) to afford **SA-17** (86 mg, Purity: 100%, Yield: 45.9%, 26 mg for delivery) and a byproduct (12 mg, Purity: 100%, Yield:6.41%) as a white solid. **SA-17:** ¹H NMR DMSO Bruker_N_400MHz δ 8.58 (s, 1H), 5.62-5.33 (m, 2H), 4.28 (s, 1H), 2.81 (t, *J* = 8.7Hz, 1H), 2.14-2.00 (m, 2H), 1.81-1.61 (m, 7H), 1.54-1.20 (m, 10H), 1.19-0.97 (m, 8H), 0.61 (s, 3H). LCMS Rₜ = 0.867 min in 2 min chromatography, 30-90AB, purity 100%, MS ESI calcd. For C₂₄H₃₅N₃O₄ [M-H₂O+H] 412, found 412.

***Step 7*.** To a solution of **SA-17** (200 mg, 465 umol) in DCM (10 mL) was added HATU (264 mg, 697 umol), DIEA (149 mg, 1.16 mmol) at 25°C. The mixture was stirred at 25°C for 15 min. The methanamine hydrate (0.2 mL, 26% in water) was added to the solution. The mixture was stirred at 25°C for 30 min. LCMS showed the reaction was complete. The mixture was concentrated. The residue was purified by prep-HPLC (0.05%HCl-ACN) to afford **SA-18** (55 mg, Purity: 99.5%, Yield: 27.4%, 10 mg for delivery) as a white solid. **SA-18:** ¹H NMR DMSO Bruker_A_400MHz δ 8.40 (s, 1H), 7.86 (s, 1H), 7.47 (s, 1H), 5.59-5.31 (m, 2H), 4.24 (s, 1H), 2.83-2.74 (m, 1H), 2.12-2.00 (m, 2H), 1.78-0.96 (m, 23H), 0.59 (s, 3H). LCMS Rt = 0.877 min in 2 min chromatography, 30-90AB, purity 100%, MS ESI calcd. For C₂₄H₃₆N₄O₃ [M-H₂O+H]⁺ 411, found 411.

***Step 8.*** To a solution of **SA-18** (45 mg, 105 umol) in DCM (4 mL) was added TFAA (66.1 mg, 315 umol), pyridine (33.1 mg, 420 umol) at 25°C. The mixture was stirred at 25°C for 16 hrs. LCMS showed the reaction was complete. The mixture was concentrated and the residue was purified by prep-HPLC to afford **SA-20** (6.5 mg, Purity: 98.89%, Yield: 14.8%) as a white solid. **SA-20:** ¹H NMR CDCl₃ Bruker_P_400MHz δ 8.13 (s, 1H), 5.37-5.13 (m, 2H), 2.72-2.65 (m, 1H), 2.28-2.03 (m, 3H), 1.97-1.85 (m, 3H), 1.84-1.73 (m, 5H), 1.70-1.64 (m, 4H), 1.60-1.23 (m, 9H), 1.20-1.07 (m, 3H), 0.67 (s, 3H). LCMS Rt = 1.263 min in 2 min chromatography, 30-90AB, purity 98.89%, MS ESI calcd. For C₂₄H₃₄N₄O₂ [M-H₂O+H]⁺ 393, found 393.

### Assay Methods

Compounds provided herein can be evaluated using various assays; examples of which are described below.

### Steroid Inhibition of TBPS Binding

[35S]-t-Butylbicyclophosphorothionate (TBPS) binding assays using rat brain cortical membranes in the presence of 5 µM GABA has been described (Gee et al, J. Pharmacol. Exp. Ther. 1987, 241, 346-353; Hawkinson et al, Mol. Pharmacol. 1994, 46, 977-985; Lewin, A.H et al., Mol. Pharmacol. 1989, 35, 189-194).

Briefly, cortices are rapidly removed following decapitation of carbon dioxide-anesthetized Sprague-Dawley rats (200-250 g). The cortices are homogenized in 10 volumes of ice-cold 0.32 M sucrose using a glass/teflon homogenizer and centrifuged at 1500 × g for 10 min at 4 °C. The resultant supernatants are centrifuged at 10,000 × g for 20 min at 4 °C to obtain the P2 pellets. The P2 pellets are resuspended in 200 mM NaCl/50 mM Na-K phosphate pH 7.4 buffer and centrifuged at 10,000 × g for 10 min at 4 °C. This washing procedure is repeated twice and the pellets are resuspended in 10 volumes of buffer. Aliquots (100 µL) of the membrane suspensions are incubated with 3 nM [³⁵S]-TBPS and 5 µL aliquots of test drug dissolved in dimethyl sulfoxide (DMSO) (final 0.5%) in the presence of 5 µM GABA. The incubation is brought to a final volume of 1.0 mL with buffer. Nonspecific binding is determined in the presence of 2 µM unlabeled TBPS and ranged from 15 to 25 %. Following a 90 min incubation at room temp, the assays are terminated by filtration through glass fiber filters (Schleicher and Schuell No. 32) using a cell harvester (Brandel) and rinsed three times with ice-cold buffer. Filter bound radioactivity is measured by liquid scintillation spectrometry. Non-linear curve fitting of the overall data for each drug averaged for each concentration is done using Prism (GraphPad). The data are fit to a partial instead of a full inhibition model if the sum of squares is significantly lower by F-test. Similarly, the data are fit to a two component instead of a one component inhibition model if the sum of squares is significantly lower by F-test. The concentration of test compound producing 50% inhibition (IC₅₀) of specific binding and the maximal extent of inhibition (Iₘₐₓ) are determined for the individual experiments with the same model used for the overall data and then the means ± SEM.s of the individual experiments are calculated. Picrotoxin serves as the positive control for these studies as it has been demonstrated to robustly inhibit TBPS binding.

Various compounds are or can be screened to determine their potential as modulators of [³⁵S]-TBPS binding *in vitro.* These assays are or can be performed in accordance with the above discussed procedures.

### Patch clamp electrophysiology of recombinant α₁β₂γ₂ and α₄β3δ GABA_{A} receptors

Cellular electrophysiology is used to measure the pharmacological properties of our GABA_{A} receptor modulators in heterologous cell systems. Each compound is tested for its ability to affect GABA mediated currents at a submaximal agonist dose (GABA EC₂₀ = 2µM). LTK cells are stably transfected with the α*₁β₂γ₂* subunits of the GABA receptor and CHO cells are transiently transfected with the *α₄β3δ* subunits via the Lipofecatamine method. Cells were passaged at a confluence of about 50-80% and then seeded onto 35mm sterile culture dishes containing 2 ml culture complete medium without antibiotics or antimycotics. Confluent clusters of cells are electrically coupled (Pritchett et al., Science , 1988, 242, 1306-1308.). Because responses in distant cells are not adequately voltage clamped and because of uncertainties about the extent of coupling (Verdoorn et al., Neuron 1990, 4, 919-928.), cells were cultivated at a density that enables the recording of single cells (without visible connections to other cells).

Whole cell currents were measured with HEKA EPC-10 amplifiers using PatchMaster software or by using the high throughput QPatch platform (Sophion). Bath solution for all experiments contained (in mM): NaCl 137 mM, KCl 4 mM, CaCl₂ 1.8 mM, MgCl₂ 1 mM, HEPES 10 mM, D-Glucose 10 mM, pH (NaOH) 7.4. In some cases 0.005% cremophor was also added. Intracellular (pipette) solution contained: KCl 130 mM, MgCl₂ 1 mM, Mg-ATP 5mM, HEPES 10 mM, EGTA 5mM, pH 7.2. During experiments, cells and solutions were maintained at room temperature (19°C - 30°C). For manual patch clamp recordings, cell culture dishes were placed on the dish holder of the microscope and continuously perfused (1 ml/min) with bath solution. After formation of a Gigaohm seal between the patch electrodes and the cell (pipette resistance range: 2.5 MΩ - 6.0 MΩ; seal resistance range:>1 GΩ) the cell membrane across the pipette tip was ruptured to assure electrical access to the cell interior (whole-cell patch-configuration). For experiments using the QPatch system, cells were transferred as suspension to the QPatch system in the bath solution and automated whole cell recordings were performed.

Cells were voltage clamped at a holding potential of -80 mV. For the analysis of test articles, GABA receptors were stimulated by 2 µM GABA after sequential pre-incubation of increasing concentrations of the test article. Pre-incubation duration was 30 s and the duration of the GABA stimulus was 2s. Test articles were dissolved in DMSO to form stock solutions (10mM). Test articles were diluted to 0.01, 0.1, 1, and 10 µM in bath solution. All concentrations of test articles were tested on each cell. The relative percentage potentiation was defined as the peak amplitude in response to GABA EC₂₀ in the presence of the test article divided by the peak amplitude in response to GABA EC₂₀ alone, multiplied by 100.

### Loss of Righting Reflex in Rats

The plasma pharmacokinetics and a qualitative assessment of sedation were obtained in male Sprague Dawley rats according to the following procedure. Rats were dosed by intravenous bolus dose (60 seconds) via the foot dorsal vein at doses ranging from 5 to 15 mg/kg in an appropriate vehicle. In order to assess sedation, rats were gently restrained by hand to a lateral position for dose administration. If decreased muscle tone was observed during dose administration, restraint was gradually reduced. If the animal was unable to return to an upright position, the time was recorded as the onset of loss of righting reflex (LRR). In the event that LRR did not occur during dosing, the animals were evaluated at 5 minute intervals thereafter by being placed in dorsal recumbency. Sluggish or incomplete righting twice consecutively within a 30 second interval qualifies as a loss of righting reflex. After onset of LRR, animals were assessed every 5 minutes in the same manner. Recovery of righting reflex is defined as the ability of a rat to right itself completely within 20 seconds of being placed in dorsal recumbency. The duration of LRR is defined as the time interval between LRR and the return of righting reflex.

### Acute PTZ Method

The anticonvulsant effect of test compounds were assessed in the pentylenetetazol-induced seizure assay in mice similar to methods described in Giardina & Gasior (2009) Curr Protoc Pharmacol., Chapter 5. Male CD-1 mice were housed in groups of five under controlled conditions (temperature of 22±2°C and 12:12 light-dark cycle, lights on at 8:00 am) and water and food were available ad libitum. The mice were housed for 1 week prior to behavioral testing, at which time they weighed 25-35g. Pentylenetetrazol (PTZ, Sigma) was dissolved in sterile 0.9% saline at a concentration of 12 mg/mL concentration for subcutaneous administration. Test compounds were formulated and administered via oral gavage or intraperitoneal injection at a predetermined time-point (typically 30 or 60 minutes) prior to PTZ injection. All solutions were made fresh and were given in a volume of 10ml/kg body weight.

Mice were acclimated to the test room for at least 30 min before compound administration. Mice were randomized into at least four test groups (vehicle and at least three doses of the test compound) with 10 mice per group. After compound administration, mice were observed for qualitative assessment of sedation for a pre-determined time point (30 or 60 minutes). Following the drug pretreatment time the mice were injected s.c. with PTZ (120 mg/kg). Immediately following the PTZ injection, mice were individually placed into observation chambers (25×15×15cm) and a three-channel timer was started. Each mouse was continuously observed for 30 min and the following behaviors were recorded by observers blinded to the treatments: 1) latency to clonic convulsions that persist for 3 sec and followed by an absence of righting reflex 2) latency to tonic convulsions, characterized by the rigid extension of all four limbs that exceeded a 90 degree angle with the body 3) latency to death 4) number of clonic and tonic convulsions. Data are presented as mean ± S.E.M and one-way analysis of variance with Dunnett's or Bonferroni's post-hoc test was used to detect significant differences in latency and number between the vehicle and dose group. p values <0.05 were regarded as statistically significant.

**Table 1. TBPS binding of the exemplary compounds.**

| **Compound Structure** | **35S-TBPS Radioligand Displacement** |
|---|---|
| **SB-1** | C |
| **SA-1** | C |
| **SA-2** | B |
| **SB-2** | C |
| **SL-1** | D |
| **SL-2** | D |
| **SV-1** | C |
| **SV-2** | B |
| **SW-1** | D |
| **SW-2** | D |
| **SZ-1** | B |
| **SZ-2** | B |
| **SN-1** | B |
| **SN-2** | A |
| **SU-1** | c |
| **SU-2** | B |
| **SA-3** | A |
| **SH-1** | D |
| **SH-2** | D |
| **SY-1** | C |
| **SY-2** | C |
| **SE-3** | C |
| **SE-4** | B |
| **SI-1** | c |
| **SF-1** | c |
| **SD-4** | D |
| **SA-6** | A |
| **SA-7** | A |
| **SQ-1** | D |
| **SP-1** | D |
| **SP-2** | C |
| **SM-1** | C |
| **SP-4** | D |
| **SO-2** | D |
| **SE-5** | D |
| **SE-6** | D |
| **SQ-5** | D |
| **SA-9** | A |
| **SB-6** | B |
| **SI-2** | C |
| **SD-1** | D |
| **SS-1** | C |
| **SF-4** | C |
| **SG-5** | C |
| **SM-5** | C |

For **Table 1,** "A" indicates an IC₅₀ of 1 nM to 50 nM, "B" indicates an IC₅₀>50 nM to 100 nM, "C" indicates an IC₅₀> 100 nM to 500 nM, and "D" indicates IC₅₀> 500 nM.

**Table 2. Electrophysiological evaluation of the exemplary compounds at GABA_{A}-R.**

| **Name** | **GABA (α1β2γ2) Qpatch in Ltk, % efficacy at 10 µM** | **GABA (α4β3δ) Manual patch in CHO, % efficacy at 10 µM** |
|---|---|---|
| **SB-1** | D | C |
| **SA-1** | B | D |
| **SA-2** | C | B |
| **SB-2** | B | B |
| **SL-1** | A | D |
| **SL-2** | A | D |
| **SV-1** | B | B |
| **SV-2** | B | B |
| **SW-1** | B | D |
| **SZ-1** | B | D |
| **SZ-2** | B | B |
| **SN-1** | B | D |
| **SN-2** | B | B |
| **SU-1** | B | D |
| **SU-2** | B | C |
| **SA-3** | B | C |
| **SY-1** | B | D |
| **SY-2** | B | C |
| **SE-3** | B | D |
| **SE-4** | B | C |
| **SI-1** | B | C |
| **SF-1** | B | D |
| **SD-4** | B | D |
| **SA-6** | B | C |
| **SA-7** | C | C |
| **SE-1** | B | D |
| **SE-2** | C | D |
| **SQ-2** | B | D |
| **SP-2** | B | D |
| **SM-1** | B | D |
| **SQ-3** | C | D |
| **SP-3** | B | D |
| **SQ-4** | B | D |
| **SG-1** | B | B |
| **SA-4** | C | C |
| **SA-5** | C | D |
| **SB-4** | B | C |
| **SB-5** | B | C |
| **SD-2** | B | D |
| **SD-3** | B | D |
| **SI-3** | B | C |
| **SI-4** | B | B |
| **SG-3** | B | D |
| **SG-4** | B | D |
| **SV-3** | B | C |
| **SV-4** | B | B |
| **SM-3** | C | D |
| **SP-5** | B | D |
| **SQ-6** | B | D |
| **SF-2** | B | C |
| **SF-3** | B | B |
| **SV-5** | B | B |
| **SV-6** | B | B |
| **SA-8** | B | D |
| **SP-4** | A | D |
| **SO-1** | B | D |
| **SV-7** | B | D |
| **SE-6** | A | D |
| **SV-9** | B | D |
| **SV-8** | C | D |
| **SH-2** | B | D |
| **SM-4** | C | D |
| **SA-9** | B | C |
| **SB-6** | B | B |
| **SI-2** | B | C |
| **SG-5** | C | C |
| **SM-5** | C | D |
| **SF-4** | B | D |
| **SA-13*** | B | C |
| **SA-20*** | B | D |

For **Table 2.** GABA_{A} receptors α1β2γ2 and α4β3δ %efficacy: "A" 10-100, "B" >100-500, "C" >500; D indicates the data is not available or has not been determined. ^{∗}in cremophor

**Table 3. Loss of Righting Reflex (Rat IV, 5 mpk)**

| **Compound** | **Duration of Rat LRR** |
|---|---|
| **SV-2** | B |
| **SA-3** | B |
| **SE-4** | A |
| **SA-7** | B |
| **SB-5** | B |
| **SV-5** | B |
| **SF-3** | A |
| **SV-3** | A |
| **SI-4** | B |

| | |
|---|---|
| A ≤20 min; B > 20 min LRR: Loss of Righting Reflex | |

**Table 4. Minimal effective anticonvulsant doses are defined as the lowest dose which significantly reduces the latency to tonic seizures in PTZ-treated mice**

| **Compound** | **Anticonvulsive Effect Dose** |
|---|---|
| **SA-2** | B (PO) |
| **SB-2** | C (IP) |
| **SV-1** | B (PO) |
| **SV-2** | A (PO) |
| **SI-1** | B (PO) |
| **SA-6** | B (PO) |
| **SA-7** | A (PO) |
| **SB-5** | A (PO) |
| **SI-3** | A (PO) |
| **SI-4** | A (PO) |
| **SV-4** | B (PO) |
| **SF-3** | A (PO) |
| **SV-5** | A (PO) |
| **SA-9** | B (IP) |
| **SB-6** | B (IP) |
| **SI-2** | B (PO) |

| | |
|---|---|
| A ≤ 1 mpk; B >1- 3 mpk; C > 3 mpk; PO - oral administration; IP - intraperitoneal injection. | |

### Other Embodiments

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications. If there is a conflict between any of the references and the instant specification, the specification shall control.

## Claims

1. A compound of the formula: 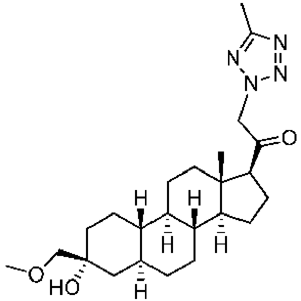 for use in a method for treating essential tremor in a subject in need thereof comprising administering to the subject the compound.

2. The compound for use as in claim 1, wherein the compound is administered orally, subcutaneously, intravenously, or intramuscularly.

3. The compound for use as in claim 1, wherein the compound is administered chronically.

4. The compound for use as in claim 2, wherein the compound is administered orally.

5. The compound for use as in claim 2, wherein the compound is administered intravenously.

6. A pharmaceutically acceptable salt of a compound of the formula: 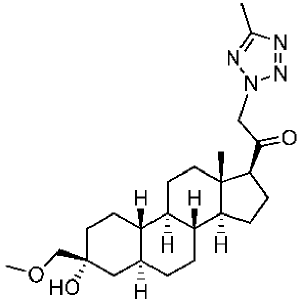 for use in a method for treating essential tremor in a subject in need thereof comprising administering to the subject the pharmaceutically acceptable salt of the compound.

7. The pharmaceutically acceptable salt for use as in claim 6, wherein the pharmaceutically acceptable salt is administered orally, subcutaneously, intravenously, or intramuscularly.

8. The pharmaceutically acceptable salt for use as in claim 6, wherein the pharmaceutically acceptable salt is administered chronically.

9. The pharmaceutically acceptable salt for use as in claim 7, wherein the pharmaceutically acceptable salt is administered orally.

10. The pharmaceutically acceptable salt for use as in claim 7, wherein the pharmaceutically acceptable salt is administered intravenously.

11. A pharmaceutical composition comprising a compound of the formula: 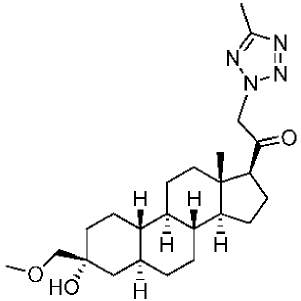 and a pharmaceutically acceptable excipient for use in a method for treating essential tremor in a subject in need thereof comprising administering to the subject the composition.

12. The pharmaceutical composition for use as in claim 11, wherein the pharmaceutical composition is administered orally, subcutaneously, intravenously, or intramuscularly.

13. The pharmaceutical composition for use as in claim 11, wherein the pharmaceutical composition is administered chronically.

14. The pharmaceutical composition for use as in claim 12, wherein the pharmaceutical composition is administered orally.

15. The pharmaceutical composition for use as in claim 12, wherein the pharmaceutical composition is administered intravenously.

## Patentansprüche

1. Verbindung der Formel: 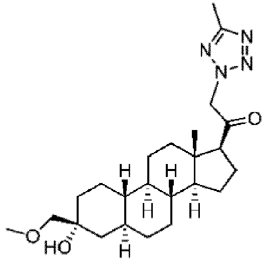 zur Verwendung bei dem Verfahren zur Behandlung von essentiellem Tremor bei einem Individuum, bei dem diesbezüglicher Bedarf besteht, bei dem man dem Individuum die Verbindung verabreicht.

2. Verbindung zur Verwendung nach Anspruch 1, wobei die Verbindung oral, subkutan, intravenös oder intramuskulär verabreicht wird.

3. Verbindung zur Verwendung nach Anspruch 1, wobei die Verbindung chronisch verabreicht wird.

4. Verbindung zur Verwendung nach Anspruch 2, wobei die Verbindung oral verabreicht wird.

5. Verbindung zur Verwendung nach Anspruch 2, wobei die Verbindung intravenös verabreicht wird.

6. Pharmazeutisch unbedenkliches Salz einer Verbindung der Formel: 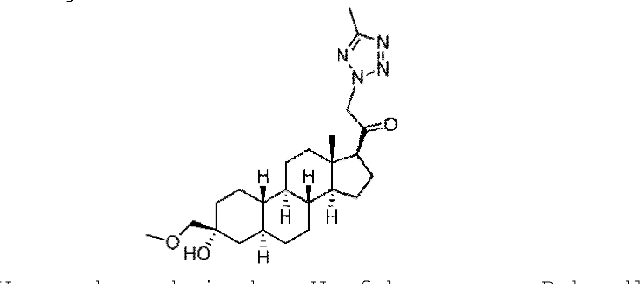 zur Verwendung bei dem Verfahren zur Behandlung von essentiellem Tremor bei einem Individuum, bei dem diesbezüglicher Bedarf besteht, bei dem man dem Individuum die Verbindung verabreicht.

7. Pharmazeutisch unbedenkliches Salz zur Verwendung nach Anspruch 6, wobei das pharmazeutisch unbedenkliche Salz oral, subkutan, intravenös oder intramuskulär verabreicht wird.

8. Pharmazeutisch unbedenkliches Salz zur Verwendung nach Anspruch 6, wobei das pharmazeutisch unbedenkliche Salz chronisch verabreicht wird.

9. Pharmazeutisch unbedenkliches Salz zur Verwendung nach Anspruch 7, wobei das pharmazeutisch unbedenkliche Salz oral verabreicht wird.

10. Pharmazeutisch unbedenkliches Salz zur Verwendung nach Anspruch 7, wobei das pharmazeutisch unbedenkliche Salz intravenös verabreicht wird.

11. Pharmazeutische Zusammensetzung, umfassend eine Verbindung der Formel: 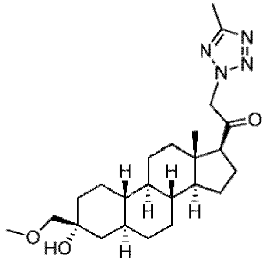 und einen pharmazeutisch unbedenklichen Träger zur Verwendung bei dem Verfahren zur Behandlung von essentiellem Tremor bei einem Individuum, bei dem diesbezüglicher Bedarf besteht, bei dem man dem Individuum die Verbindung verabreicht.

12. Pharmazeutische Zusammensetzung nach Anspruch 11, wobei die pharmazeutische Zusammensetzung oral, subkutan, intravenös oder intramuskulär verabreicht wird.

13. Pharmazeutische Zusammensetzung nach Anspruch 11, wobei die pharmazeutische Zusammensetzung chronisch verabreicht wird.

14. Pharmazeutische Zusammensetzung nach Anspruch 12, wobei die pharmazeutische Zusammensetzung oral verabreicht wird.

15. Pharmazeutische Zusammensetzung nach Anspruch 12, wobei die pharmazeutische Zusammensetzung intravenös verabreicht wird.

## Revendications

1. Composé de la formule : 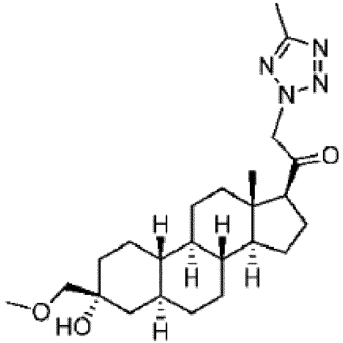 pour une utilisation dans un procédé pour le traitement d'un tremblement essentiel chez un sujet qui en a besoin comprenant l'administration du composé au sujet.

2. Composé pour une utilisation comme dans la revendication 1, le composé étant administré oralement, de manière sous-cutanée, de manière intraveineuse ou de manière intramusculaire.

3. Composé pour une utilisation comme dans la revendication 1, le composé étant administré de manière chronique.

4. Composé pour une utilisation comme dans la revendication 2, le composé étant administré oralement.

5. Composé pour une utilisation comme dans la revendication 2, le composé étant administré de manière intraveineuse.

6. Sel pharmaceutiquement acceptable d'un composé de la formule : 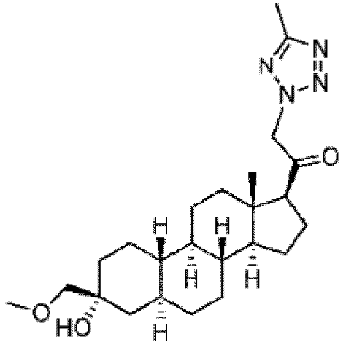 pour une utilisation dans un procédé pour le traitement d'un tremblement essentiel chez un sujet qui en a besoin comprenant l'administration du sel pharmaceutiquement acceptable du composé au sujet.

7. Sel pharmaceutiquement acceptable pour une utilisation selon la revendication 6, le sel pharmaceutiquement acceptable étant administré oralement, de manière sous-cutanée, de manière intraveineuse ou de manière intramusculaire.

8. Sel pharmaceutiquement acceptable pour une utilisation selon la revendication 6, le sel pharmaceutiquement acceptable étant administré de manière chronique.

9. Sel pharmaceutiquement acceptable pour une utilisation selon la revendication 7, le sel pharmaceutiquement acceptable étant administré oralement.

10. Sel pharmaceutiquement acceptable pour une utilisation selon la revendication 7, le sel pharmaceutiquement acceptable étant administré de manière intraveineuse.

11. Composition pharmaceutique comprenant un composé de la formule : 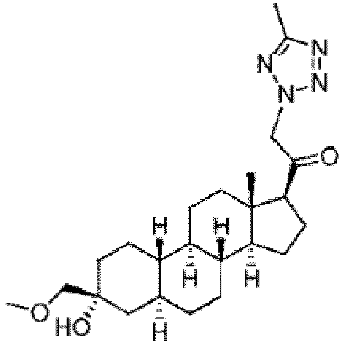 et un excipient pharmaceutiquement acceptable pour une utilisation dans un procédé pour le traitement d'un tremblement essentiel chez un sujet qui en a besoin comprenant l'administration de la composition au sujet.

12. Composition pharmaceutique pour une utilisation selon la revendication 11, la composition pharmaceutique étant administrée oralement, de manière sous-cutanée, de manière intraveineuse ou de manière intramusculaire.

13. Composition pharmaceutique pour une utilisation selon la revendication 11, la composition pharmaceutique étant administrée de manière chronique.

14. Composition pharmaceutique pour une utilisation selon la revendication 12, la composition pharmaceutique étant administrée oralement.

15. Composition pharmaceutique pour une utilisation selon la revendication 12, la composition pharmaceutique étant administrée de manière intraveineuse.
